# FASCICULE DE BREVET EUROPEEN

(11) **EP 1 885 695 B1**
(45) Date de publication et mention de la délivrance du brevet: **25.02.2009**
(21) Numéro de dépôt: 06794466.0
(22) Date de dépôt: 29.05.2006
(51) Int. Cl.: C07D 209/42, A61K 31/404, C07D 403/12, C07D 413/12, C07D 401/12

(54) **COMPOSES DE L'INDOLINE**
INDOLINVERBINDUNGEN
INDOLINE COMPOUNDS

(30) Priorité: 30.05.2005 FR 0505432
(43) Date de publication de la demande: 13.02.2008
(73) Titulaire: LABORATOIRES FOURNIER S.A., 21300 Chenove (FR)
(72) Inventeur: LEBRETON, Luc, F-Dijon 21000 (FR); DUMAS, Christine, F-21240 Talant (FR); MASSARDIER, Christine, F-21000 Dijon (FR); BONDOUX, Michel, F-21121 Fontaine Les Dijon (FR)
(74) Mandataire: Hubert, Philippe
(86) Numéro de dépôt international: PCT/FR2006/050487
(87) Numéro de publication internationale: WO 2007/000550

(56) Documents cités:
- WO-A-00/54759
- WO-A-03/043985
- WO-A-20/04005253
- US-A1- 2003 008 861

## Description

La présente invention a pour objet de nouveaux composés capables de moduler l'activité des récepteurs nucléaires LXR, leur procédé de fabrication ainsi que des compositions pharmaceutiques les contenant.

Les récepteurs « liver X receptor » (LXR) sont des facteurs de transcription qui appartiennent à la super famille des récepteurs nucléaires dont font également partie les récepteurs « retinoic acid receptor » (RXR), « farnesoid X receptor » (FXR) et « peroxisome proliferator-activated receptors » (PPARs). Les récepteurs LXR forment en se liant au récepteur RXR un hétérodimère qui se fixe de façon spécifique aux éléments de réponse de l'ADN (LXRE) pour conduire à la transactivation de gènes cibles *(*Genes Dev. 1995 ; 9 : 1033-45*).*

Ces récepteurs sont impliqués dans de multiples voies métaboliques et participent notamment à l'homéostasie du cholestérol, des acides biliaires, des triglycérides et du glucose.

La modulation de l'activité de ces récepteurs nucléaires influence la progression des désordres métaboliques tels que le diabète de type II, les dyslipidémies et le développement de l'athérosclérose.
L'hétérodimère LXR/RXR peut être activé par des ligands LXR et/ou RXR. La transactivation des gènes cibles nécessite le recrutement de co-activateurs tel que Grip-1. *(*Nature 1996; 383 : 728-31*).*

Les deux types de récepteurs LXR aujourd'hui identifiés, à savoir LXRα et LXRβ, présentent un haut degré de similarité au niveau de leur séquence en acides aminés mais diffèrent quant à leur distribution tissulaire. Le LXRα est fortement exprimé dans le foie et dans une moindre mesure dans les reins, l'intestin, le tissu adipeux et la rate. Le LXRβ est distribué de façon ubiquitaire *(*Gene 2000; 243: 93-103*,* N. Y. Acad. Sci. 1995; 761 : 38- 49*).*

Si le cholestérol n'active pas directement les récepteurs LXR, des dérivés mono-oxydés du cholestérol (oxystérols) le font et plus particulièrement les 22 (R)-hydroxycholestérol, 24(S)-hydroxycholestérol et 24(S),25-époxycholestérol. Ces oxystérols sont considérés comme les ligands physiologiques des récepteurs LXR *(*Nature 1996 ; 383 : 728-31*,* J.Biol.chem 1997; 272: 3137-40*).* En outre, il a été montré que l'oxystérol 5,6,24(S),25-diépoxycholestérol est un ligand spécifique du LXRα, ce qui suggère qu'il est possible de développer des ligands spécifiques des LXR α et/ou LXRβ *(*Proc. Natl. Acad. Sci USA 1999 ; 96 : 26-71*,* Endocrinology 2000 ; 141 : 4180 - 4*).*

Par ailleurs, on a pu démontrer que le plasma humain contient des antagonistes naturels des LXRα et β *(*Steroïds 2001 ; 66 : 473-479*).*

A partir d'hépatocytes de rat, il a pu être montré que des acides gras non saturés augmentent de façon importante l'expression du LXRα sans affecter LXRβ (Mol Endocrinol 2000 ; 14: 161-171). De plus, les activateurs des PPARs α et γ induisent également l'expression du LXRα dans des macrophages primaires humains.

Les fortes concentrations de LXRα dans le foie et l'identification des ligands endogènes du LXR ont suggéré que ces récepteurs jouent un rôle essentiel dans le métabolisme du cholestérol. Dans des conditions physiologiques, l'homéostasie du cholestérol est maintenue, via la régulation des voies de synthèse de novo et de catabolisme. L'accumulation de stérols dans le foie conduit par un mécanisme de "feed-back" impliquant des facteurs de transcription tels que SREBP-1 et

SREBP-2 à une inhibition de la biosynthèse de cholestérol. *(*Ce// 1997; 89: 331-40*).* L'excès de cholestérol active également une autre voie métabolique qui conduit à la conversion du cholestérol en acides biliaires. La conversion du cholestérol en 7α-hydroxy-cholestérol est réalisée par une enzyme localisée dans le foie (CYP7A : 7α-hydroxylase) *(*J.Biol. Chem. 1997 ; 272 : 313 -40*).*

L'implication du LXR dans la synthèse d'acides biliaires et donc dans la régulation de l'homéostasie du cholestérol a été démontrée au moyen de souris déficientes en LXRα qui, lorsqu'elles sont soumises à un régime gras, accumulent de grandes quantités d'esters de cholestérol au niveau hépatique *(*Cell 1998 ; 93 693-704*).* Les souris déficientes en LXRβ ont la même résistance physiologique que les souris normales vis-à-vis d'un régime enrichi en graisses. L'expression du LXRβ inchangé chez les souris déficientes en LXR tend à démontrer que le LXRβ est incapable à lui seul d'augmenter de façon importante le métabolisme du cholestérol (J. Clin. Invest. 2001 ; 107: 565-573*).*

Les récepteurs LXR exprimés au niveau du macrophage jouent également un rôle important dans la régulation de certaines fonctions de celui-ci. Ils sont plus particulièrement impliqués dans le contrôle du transport inverse du cholestérol qui permet d'exporter l'excès de cholestérol des tissus périphériques vers le foie. Le cholestérol est pris en charge par des pré-bHDL via l'apoA1 et ABCA1 pour être transporté vers le foie où il est catabolisé en acides biliaires puis éliminé.

L'ABCA1 est un membre de la super famille des protéines de transport (ATP - binding cassette) dont l'importance est illustrée par le fait qu'une mutation au niveau du gène de l'ABCA1 est responsable de la maladie de Tangier *(*Nat. Genet. 1999 ; 22 : 336-45*).*

L'expression de l'ABCA1 et l'efflux de cholestérol sont induits par le chargement des macrophages humains en cholestérol et l'activation des récepteurs LXR *(*Biochem. Biophys. Res. Comm. 1999 ; 257 : 29-33*).* Il a été également démontré ultérieurement que l'expression au niveau intestinal de l'ABCG1, l'ABCG5 et ABCG8, autres membres de la famille des transporteurs de type ABC, est régulée également par l'hétérodimère RXR/LXR *(*J. Biol. Chem. 2000 ; 275 : 14700-14707*,* Proc. Natl. Acad. Sci. USA 2000 ; 97 : 817-22*,* J. Biol. Chem. 2002 ; 277: 18793-18800*,* Proc. Natl. Aca d. Sci. USA 2002 ; 99 : 16237-16242*).*

Il a aussi été montré que des ligands agonistes LXR réduisaient les lésions athéromateuses dans deux différents modèles murins (souris ApoE-/- et souris LDLR-/-) *(*Proc. Natl. Acad. Sci. USA 2002 ; 99 : 7604-7609, FEBS Letters 2003 ; 536 :6-11). Ces résultats suggèrent que les ligands LXR peuvent constituer des agents thérapeutiques pour traiter l'athérosclérose.

Enfin, on sait que les macrophages jouent un rôle important dans l'inflammation en particulier dans la pathogénèse de l'athérosclérose. Il a été montré que l'activation des LXRs inhibe l'expression des gènes impliqués dans l'inflammation au niveau du macrophage. *(*Nature Medecine, 2003 ; 9 : 213-219*).* In vitro, l'expression des médiateurs, tels que la nitric oxide synthase, la cyclo oxygénase-2(COX-2) et l'interleukine-6 (IL-6) est inhibée. In vivo, les agonistes LXR réduisent l'inflammation dans un modèle de dermatite et inhibent l'expression des gènes impliqués dans l'inflammation des aortes de souris athéromateuses.

Parce que l'homéostasie du cholestérol semble jouer également un rôle essentiel dans le fonctionnement du système nerveux central et les mécanismes de la neurodégénérescence, l'expression de l'ABCA1 a également été étudiée dans des cultures de neurones primaires, d'astrocytes et de microglie isolés à partir de cerveaux d'embryons de rats. Les résultats de ces études montrent que l'activation des LXRs conduit à une diminution de la sécrétion d'amyloïde β et par voie de conséquence à une réduction des dépôts amyloïdes au niveau cérébral. Ces travaux suggèrent que l'activation des LXR peut constituer une nouvelle approche pour traiter la maladie d'Alzheimer (J. Biol. Chem. 2003, 275 (15) : 13244-13256, J. Biol. Chem. 2003, 278 (30) : 27688-27694).

Les récepteurs LXR sont également impliqués dans la régulation de l'expression de l'apolipoprotéine E (ApoE). Cette protéine est largement impliquée dans la clairance hépatique des lipoprotéines et favorise l'efflux de cholestérol à partir de macrophages riches en lipides. Il a été démontré que l'activation des récepteurs LXR conduit à une augmentation de l'expression de l'ApoE via un élément de réponse du LXR (LXRE) situé dans la séquence du promoteur de l'ApoE *(*Proc. Natl. Acad. Sci. USA 2001 ; 98 : 507-512).

L'activation de récepteurs LXR favoriserait également le transport inverse du cholestérol via la modulation de l'expression de la protéine CETP (cholesterol ester transfer protein) qui est impliquée dans le transfert du cholestérol estérifié des lipoprotéines HDL aux lipoprotéines riches en triglycérides éliminées par le foie (J. Clin. Invest. 2000 ; 105 : 513-520).

En résumé, l'activation des récepteurs LXR conduit à une augmentation de l'expression de nombreux gènes favorisant l'élimination du cholestérol en excès des tissus périphériques. Dans le macrophage chargé en cholestérol, l'activation des récepteurs LXRs augmente l'expression de l'ABCA1, l'ABCG1, l'ABCG5, l'ABCG8 et l'ApoE entraînant une augmentation de l'efflux de cholestérol des macrophages au foie où il est excrété sous forme d'acides biliaires. L'induction de l'expression de la CETP et de la CYP7A dans le foie conduit respectivement à une augmentation de la clairance hépatique des esters de cholestérol des lipoprotéines HDL et au catabolisme du cholestérol.

Par ailleurs, il a été également démontré que les récepteurs LXRs jouent un rôle important dans le métabolisme du glucose. Le traitement de rongeurs diabétiques avec un agoniste LXR conduit à une diminution drastique des taux de glucose plasmatique. En particulier chez le rat Zucker (fa/fa) insulino-résistant, l'activation du LXR inhibe l'expression des gènes impliqués dans la gluconéogénèse dont plus particulièrement la phosphoénolpyruvate carboxykinase (PEPCK) *(*J. Biol. Chem. 2003, 278 (2) : 1131-1136*).*

En outre, il a été montré qu'un agoniste LXR augmente la tolérance au glucose dans un modèle murin d'insulino-résistance et d'obésité *(*Proc. Natl. Acad. Sci. USA 2003 ; 100 : 5419-5424*).* L'analyse de l'expression des gènes démontre une régulation des gènes impliqués dans le métabolisme du glucose dans le foie :
- diminution de l'expression du « peroxisome proliferator-activated receptor coactivator-1α » (PGC-1), de la « phosphoenol pyruvate carboxykinase » (PEPCK) et de la « glucose-6-phosphatase »
- induction de l'expression de la glucokinase qui favorise l'utilisation du glucose hépatique.

Une induction transcriptionnelle du transporteur de glucose sensible à l'insuline (GLUT4) dans le tissu adipeux a également été démontrée.

Ces résultats soulignent l'importance des LXRs dans la coordination du métabolisme du glucose.

On sait aussi que les récepteurs LXR interviennent dans les processus de régulation de l'inflammation (Nature Medecine 2003 9, 213-219).

Des composés modulateurs de l'activité des récepteurs LXR sont connus dans l'état de la technique notamment des documents WO 03/090869, WO 03/90746, WO 03/082192 ou WO 03/082802 ; ou encore des documents WO 03/043985 et WO 04/005253 qui décrivent des composés de type benzènesulfonamide agonistes des récepteurs PPAR.

Le document WO 00/54759 décrit des composés modulateurs de l'activité des récepteurs LXR qui peuvent être dérivés de l'indole parmi de nombreuses possibilités.

Le document US 2003/008861 décrit des composés qui peuvent être dérivés de sulfonylindoline qui sont des antagonistes de VLA-4 utiles pour l'inhibition ou la prévention des maladies liées à l'adhésion cellulaire.

Dans ce contexte, il existe un intérêt significatif à trouver de nouveaux composés modulateurs de l'activité des récepteurs LXR qui seraient utiles dans le traitement de certaines pathologies telles que les maladies cardiovasculaires, les hypercholestérolémies, les dyslipidémies, l'infarctus du myocarde, l'athérosclérose, le diabète, l'obésité, l'inflammation et les maladies neurodégénératives.

La présente invention est précisément fondée sur la découverte de nouveaux composés modulateurs de l'activité des récepteurs LXR.

Ainsi, selon un premier aspect, la présente invention vise à protéger, en tant que produit industriel nouveau, un composé de sulfonylindoline, caractérisé en ce qu'il est choisi parmi :
i) les composés de formule : 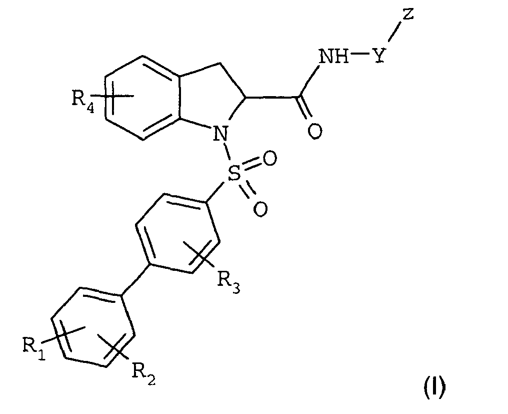 dans laquelle :
   - R₁ représente un atome d'hydrogène, un halogène, un groupe alkyle en C₁-C₄, un groupe alcoxy en C₁-C₄, un groupe trifluorométhyle ou un groupe méthoxy totalement ou partiellement halogéné,
   - R₂ représente un atome d'hydrogène, un halogène, un groupe alkyle en C₁-C₄ ou un groupe trifluorométhyle,
   - R₃ représente un atome d'hydrogène, un halogène, un groupe alkyle en C₁-C₄ ou un groupe trifluorométhyle, avec la condition que R₁, R₂ et R₃ ne représentent pas simultanément un atome d'hydrogène,
   - R₄ représente un atome d'hydrogène ou un groupe alcoxy en C₁-C₄,
   - Y représente un groupe alkylène en C₁-C₈ linéaire, ramifié, éventuellement substitué par un groupe trifluorométhyle ou par un noyau phényle, ou comportant une partie cyclisée ayant 3 à 6 atomes de carbone, ou représente un groupe -(CH₂)ₙ-W-,
   - W représente un atome d'oxygène, un groupe -NH- ou un atome de soufre,
   - n représente 2, 3 ou 4,
   - Z représente un groupe alkyle en C₁-C₄, éventuellement partiellement halogéné, trifluorométhyle, -CORₐ, -CH₂-N(R)₂, un noyau aromatique, hétéroaromatique ou hétérocyclique choisi parmi les groupes phényle, pyrrolidinyle, pyrrolidinyl-one, imidazolyle, pyridinyle, pyridinyle-oxyde, pipéridinyle, pipérazinyle, pyridazinyle, morpholinyle, indolinylone, éventuellement substitué par un, deux ou trois substituants, identiques ou différents, choisis parmi un halogène, un groupe alkyle en C₁-C₄, alcoxy en C₁-C₄, trifluorométhyle, nitro, N(R)₂, -CH₂-N(R)₂, -O-(CH₂)ₙ-N(R)₂, hydroxy, cyano, cyanoalkyle en C₂-C₃, 5-oxo-1,2,4-oxadiazolidinyle ou un groupe de formule -X-[C(R)₂]ₚ-CORₐ,
   - X représente une liaison simple, un atome d'oxygène, -O-CH₂-, un atome de soufre, un groupe -NR-, ou un groupe 1,1-cyclopropylène,
   - Rₐ représente OR ou N(R)₂,
   - R représente l'atome d'hydrogène ou un groupe alkyle en C₁-C₄**,**
   - p est égal à 0, 1, 2, 3 ou 4 ;
ii) les sels pharmaceutiquement acceptables desdits composés de formule (I).

Selon un deuxième aspect, l'invention concerne les composés précités pour leur utilisation en tant que substance pharmacologiquement active.

En particulier, l'invention concerne l'utilisation d'au moins un composé de formule (I) ou l'un de ses sels pharmaceutiquement acceptable en tant que principe actif pour la préparation d'un médicament destiné à une utilisation en thérapeutique, notamment pour lutter contre les hypercholestérolémies, les dyslipidémies, les hypertriglycéridémies, l'obésité ainsi que les maladies cardiovasculaires qui sont la conséquence d'un déséquilibre des lipoprotéines sériques. Les composés selon l'invention sont également utiles comme principes actifs de médicaments destinés à prévenir ou traiter l'athérosclérose, l'infarctus du myocarde, certaines maladies inflammatoires comme par exemple les dermatites, et les neurodégénérescences comme par exemple la maladie d'Alzheimer. Les composés selon l'invention trouvent également leur utilité en tant que principes actifs de médicaments destinés au traitement du diabète et des hyperglycémies.

### Description détaillée

Dans le cadre de la présente demande, on entend par :
- groupe alkyle en C₁-C₄ une chaîne hydrocarbonée saturée ayant de 1 à 4 atomes de carbone, linéaire ou ramifiée, ou bien encore comprenant un cycle ayant 3 ou 4 atomes de carbone comme par exemple les groupes méthyle, éthyle, propyle, butyle, 1-méthyléthyle, 1-méthylpropyle, 2-méthylpropyle, 1,1-diméthyléthyle, cyclopropyle, méthylcyclopropyle ou cyclopropylméthyle ;
- groupe alkyle en C₁-C₄ partiellement halogéné : un groupe alkyle en C₁-C₄ tel que défini précédemment dont un (ou plusieurs) atome(s) d'hydrogène a (ont) été remplacé(s) par un nombre correspondant d'atome d'halogène.
- groupe alcoxy en C₁-C₄ : un groupe O-alkyle en C₁-C₄ dans lequel le groupe alkyle en C₁-C₄ est tel que défini précédemment, comme par exemple les groupes méthoxy, éthoxy, propoxy, butoxy, 1-méthyléthoxy, 1-éthyléthoxy, 1- ou 2-méthylpropoxy ;
- groupe méthoxy totalement ou partiellement hydrogéné : un groupe méthoxy dont 1 à 3 atomes d'hydrogène a (ont) été remplacé(s) par un nombre correspondant d'atome d'halogène, comme par exemple les groupes -O-CH₂F, -O-CHF₂ ou -O-CF₃,
- halogène : un atome de fluor, chlore, brome ou iode, les atomes de fluor et de chlore étant préférés,
- alkylène : une chaîne hydrocarbonée saturée.

Les composés dans lesquels Rₐ représente OH sont des acides carboxyliques qui peuvent être utilisés sous la forme d'acides libres ou sous la forme de sels, lesdits sels étant obtenus par combinaison de l'acide avec une base minérale ou organique non toxique pharmaceutiquement acceptable. Parmi les bases minérales, on utilise par exemple les hydroxydes de sodium, de potassium, de magnésium ou de calcium. Parmi les bases organiques, on utilise par exemple les amines, les aminoalcools, des acides aminés basiques tels que la lysine ou l'arginine ou encore des composés porteurs d'une fonction ammonium quaternaire tels que par exemple la bétaïne ou la choline.

Dans le groupe de formule -X-[C(R)₂]ₚ-CORₐ, les substituants R portés par le carbone peuvent être identiques ou différents. Par exemple l'un peut représenter un atome d'hydrogène et le second représente un groupe alkyle : dans ce cas, le groupe comprend un carbone asymétrique et le composé peut être soit un mélange racémique, soit l'un des deux énantiomères R ou S.

Les composés selon l'invention comprennent un carbone asymétrique (porteur de la fonction carboxamide liée au groupe indoline) qui peut être soit racémique, soit de configuration R, soit encore de configuration S (fig. la). Parmi ces différentes configurations, on préfère les composés de formule (la) dans lesquels le carbone asymétrique du groupe indoline est de configuration S.

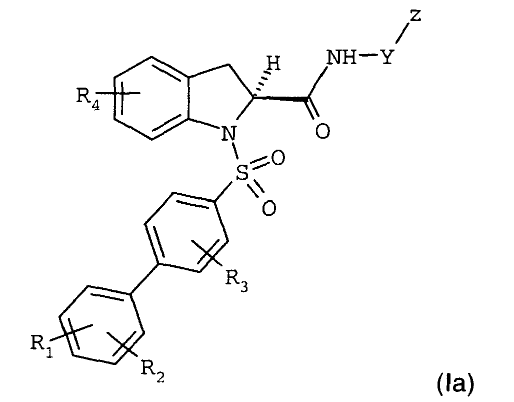

Parmi les composés de formule (I), on préfère encore les composés dans lesquels le carbone asymétrique est de configuration S et/ou R₁ représente un atome de fluor ou un groupe trifluorométhyle.

On préfère également les composés dans lesquels Y représente un groupe -CH₂- ou un groupe -(CH₂)₂-O- et Z représente un noyau aromatique substitué par un groupe contenant une fonction acide carboxylique, ledit noyau aromatique comportant éventuellement un ou deux autres substituants choisis parmi un halogène, un groupe alkyle en C₁-C₄, de préférence méthyle, un groupe alcoxy en C₁-C₄, de préférence méthoxy, un groupe trifluorométhyle.

Les composés selon l'invention peuvent être préparés selon un procédé faisant appel aux étapes consistant à
a) faire réagir un acide de formule : 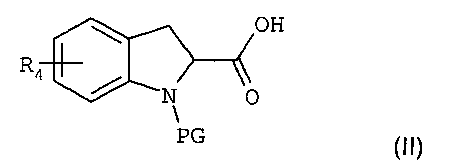 dans laquelle PG représente un groupe amino-protecteur, comme par exemple un groupe Boc (1,1-diméthyléthoxycarbonyle), et R₄ représente un atome d'hydrogène ou un groupe alcoxy en C₁-C₄ avec une amine de formule

   H₂N-Y-z (III)

   dans laquelle :
   - Y représente un groupe alkylène en C₁-C₈ linéaire, ramifié, éventuellement substitué par un groupe trifluorométhyle ou par un noyau phényle, ou comportant une partie cyclisée ayant 3 à 6 atomes de carbone, ou représente un groupe -(CH₂)ₙ-W-,
   - W représente un atome d'oxygène, un groupe -NH- ou un atome de soufre,
   - n représente 2, 3 ou 4,
   - Z représente un groupe alkyle en C₁-C₄, éventuellement partiellement halogéné, trifluorométhyle, CORₐ, CH₂-N(R)₂, un noyau aromatique, hétéroaromatique ou hétérocyclique choisi parmi les groupes phényle, pyridinyle, 1-imidazolyle, 1-pipéridinyle, 4-alkyl-1-pipérazinyle, 4-morpholinyle, 1-pyrrolidinyle, indolinylone, ou pyrrolidinylone, éventuellement substitué par un, deux ou trois substituants identiques ou différents choisis parmi un halogène, un groupe alkyle en C₁-C₄, alcoxy en C₁-C₄, trifluorométhyle, nitro, N(R)₂, -O-(CH₂)ₙ-N(R)₂, hydroxy, cyano cyanoalkyl en C₂-C₃, 5-oxo-1,2,4-oxadiazolidinyle ou un groupe de formule -X-[C(R)₂]ₚ-CORₐ,
   - X représente une liaison simple, -O-CH₂- un atome d'oxygène, un atome de soufre, ou un groupe 1,1-cyclopropylène,
   - Rₐ représente OR ou N(R)₂,
   - R représente un groupe alkyle en C₁-C₄,
   - p est égal à 0, 1, 2, 3 ou 4,
      dans un solvant anhydre tel que par exemple le dichlorométhane et en présence d'un agent de couplage tel que par exemple l'EDCI (chlorhydrate de 1-(3-diméthylaminopropyl)-3-éthylcarbodiimide), le DCC (dicyclohexylcarbodiimide) libre ou greffé sur une résine ou le HOAT (1-hydroxy-7-azabenzotriazole), à une température proche de la température ambiante et pendant 2 à 20 heures, pour obtenir l'amide de formule (IV) 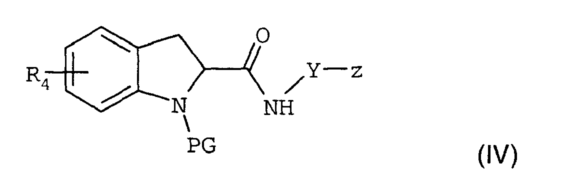
   dans laquelle Y, Z, R₄ et PG conservent la même signification que dans les composés de départ,
b) faire réagir le composé de formule (IV) obtenu précédemment avec l'acide trifluoroacétique, dans un solvant tel que le dichlorométhane, à température ambiante pendant 2 à 20 heures pour obtenir le composé de formule (V) 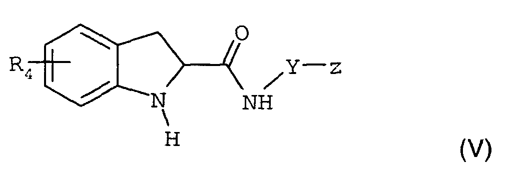 dans lequel Y, R₄ et Z conservent la même signification que dans le composé (IV),
c) faire réagir le composé de formule (V) avec un chlorure de benzènesulfonyle de formule (VI) 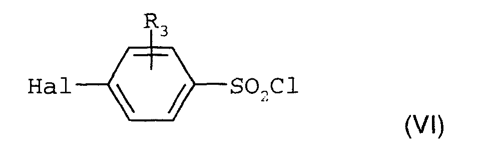 dans laquelle
   - R₃ représente un atome d'hydrogène, un atome de fluor, un atome de chlore, un groupe alkyle en C₁-C₄ ou un groupe trifluorométhyle,
   - Hal représente un atome d'iode ou de brome,
      dans un solvant tel que par exemple le dichlorométhane, à température ambiante pendant 2 à 20 heures pour obtenir le composé de formule (VII) 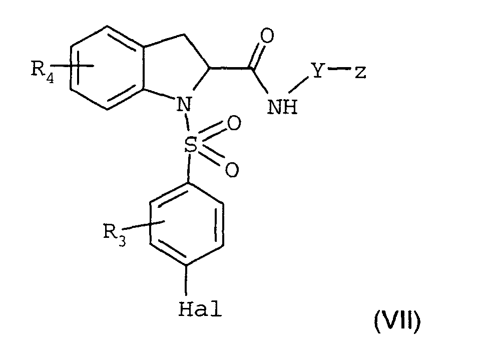
   dans laquelle Y, Z, R₃, R₄ et Hal conservent la même signification que dans les composés de départ,
d) faire réagir le composé de formule (VII) obtenu ci-dessus, avec un acide phénylboronique de formule : 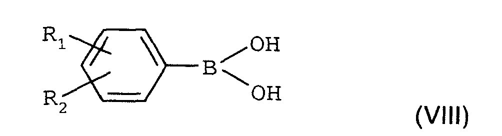 dans laquelle
   - R₁ représente un atome d'hydrogène, un atome de fluor ou de chlore, un groupe alkyle en C₁-C₄, un groupe alcoxy en C₁-C₄, un groupe trifluorométhyle ou un groupe méthoxy totalement ou partiellement halogéné,
   - R₂ représente un atome d'hydrogène, un atome de fluor, un atome de chlore, un groupe alkyle en C₁-C₄ ou un groupe trifluorométhyle, selon une réaction dite de SUZUKI (voir par exemple Chem. Rev., 1995, 95, 2457) en présence d'un catalyseur organométallique tel que le tétrakis-(triphénylphosphine)palladium et d'une base comme par exemple le carbonate de sodium, dans un solvant tel que par exemple un mélange d'éther de glycol et d'eau, à une température comprise entre 30°C et la température de reflux du solvant, pendant 5 à 24 heures pour obtenir le composé de formule I 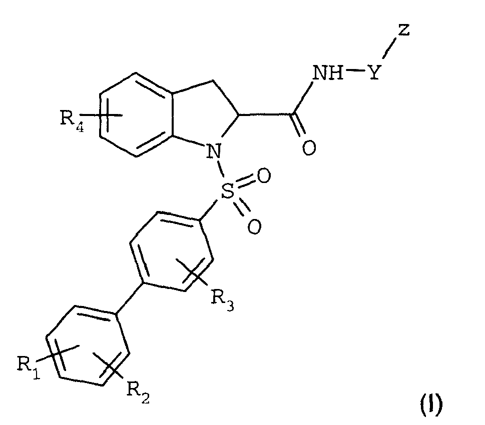
   dans laquelle R₁, R₂, R₃, R₄, Y et Z conservent la même signification que dans les composés de départ,
e) si nécessaire, lorsque le substituant Z comprend un groupe ester, effectuer une hydrolyse de la fonction ester par exemple par action de la lithine ou de la soude si l'hydrolyse peut être conduite en milieu alcalin, ou par action de l'acide trifluoroacétique s'il s'agit d'un ester *t*-butylique, pour obtenir le dérivé acide carboxylique correspondant,
f) si nécessaire, à partir de l'acide obtenu précédemment, préparer un sel avec une base minérale ou organique selon des méthodes bien connues de l'homme de métier.

En variante du procédé décrit ci-dessus, les composés de formule I selon l'invention peuvent être obtenus selon un procédé consistant :
à faire réagir un chlorure de benzènesulfonyle de formule 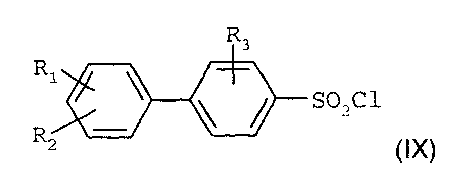 dans laquelle
   - R₁ représente un atome d'hydrogène, un halogène, un groupe alkyle en C₁-C₄, un groupe alcoxy en C₁-C₄, un groupe trifluorométhyle ou un groupe méthoxy totalement ou partiellement halogéné,
   - R₂ représente un atome d'hydrogène, un halogène, un groupe alkyle en C₁-C₄ ou un groupe trifluorométhyle,
   - R₃ représente un atome d'hydrogène, un halogène, un groupe alkyle en C₁-C₄ ou un groupe trifluorométhyle, avec la condition que R₁, R₂ et R₃ ne représentent pas simultanément un atome d'hydrogène,
      avec un dérivé d'indoline de formule 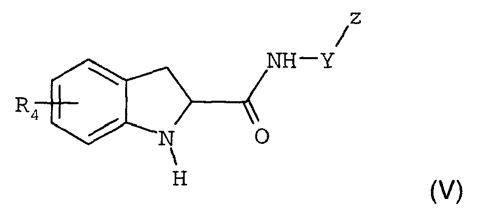
   dans laquelle
   - R₄ représente un atome d'hydrogène ou un groupe alcoxy en C₁-C₄,
   - Y représente un groupe alkylène en C₁-C₈ linéaire, ramifié, éventuellement substitué par un groupe trifluorométhyle ou par un noyau phényle, ou comportant une partie cyclisée ayant 3 à 6 atomes de carbone, ou représente un groupe -(CH₂)ₙ-W-,
   - W représente un atome d'oxygène, un groupe -NH- ou un atome de soufre,
   - n représente 2, 3 ou 4,
   - Z représente un groupe alkyle en C₁-C₄, éventuellement partiellement halogéné, trifluorométhyle, CORₐ, CH₂-N(R)₂, un noyau aromatique, hétéroaromatique ou hétérocyclique choisi parmi les groupes phényle, 1-pyrrolidinyle, pyrrolidinylone, 1-imidazolyle, pyridinyle, 1-pipéridinyle, 4-alkyl-1-pipérazinyle, pyridazinyle, 4-morpholinyle, indolinylone, éventuellement substitué par un, deux ou trois substituants identiques ou différents choisis parmi un halogène, un groupe alkyle en C₁-C₄, alcoxy en C₁-C₄, trifluorométhyle, nitro, N(R)₂, -CH₂-N(R)₂, -O-(CH₂)ₙ-N(R)₂, hydroxy, cyano, cyanoalkyle en C₂-C₃ ou un groupe de formule -X-[C(R)₂]ₚ-CORₐ,
   - X représente une liaison simple, -O-CH₂-, un atome d'oxygène, un atome de soufre, un groupe -NR-, ou un groupe 1,1-cyclopropylène,
   - Rₐ représente OR ou N(R)₂,
   - R représente un groupe alkyle en C₁-C₄, p est égal à 0, 1, 2, 3 ou 4 ;
      dans un solvant anhydre tel que par exemple le dichlorométhane, à température ambiante et pendant 2 à 10 heures, pour obtenir le composé de formule 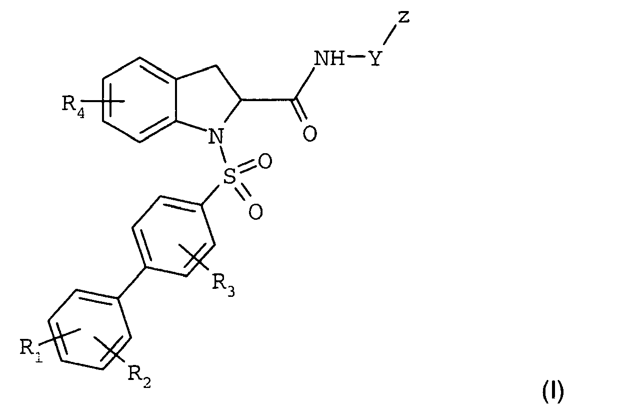
   dans laquelle R_{1,} R₂, R₃, R₄, Y et Z conservent la même signification que dans les composés de départ.

Selon une autre variante du procédé d'obtention des composés de formule (I), on réalise les étapes consistant à
a) faire réagir un chlorure de benzènesulfonyle de formule 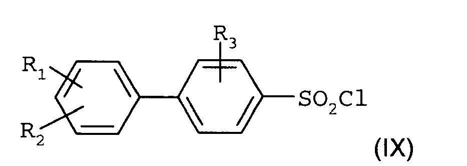 dans laquelle
   - R₁ représente un atome d'hydrogène, un halogène, un groupe alkyle en C₁-C₄, un groupe alcoxy en C₁-C₃, un groupe trifluorométhyle ou un groupe méthoxy totalement ou partiellement halogéné,
   - R₂ représente un atome d'hydrogène, un halogène, un groupe alkyle en C₁-C₄ ou un groupe trifluorométhyle,
   - R₃ représente un atome d'hydrogène, un halogène, un groupe alkyle en C₁-C₄ ou un groupe trifluorométhyle, avec la condition que R₁, R₂ et R₃ ne représentent pas simultanément un atome d'hydrogène,
      avec un ester d'indoline de formule 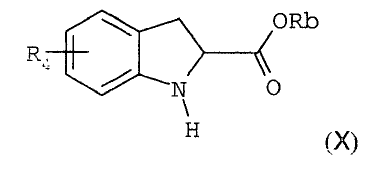
   dans laquelle Rb représente un groupe alkyle en C₁-C₄, préférentiellement un groupe méthyle, et R₄ représente un atome d'hydrogène ou un groupe alcoxy en C₁-C₄,
   dans des conditions analogues à celles du procédé ci-dessus,
   pour obtenir le composé de formule 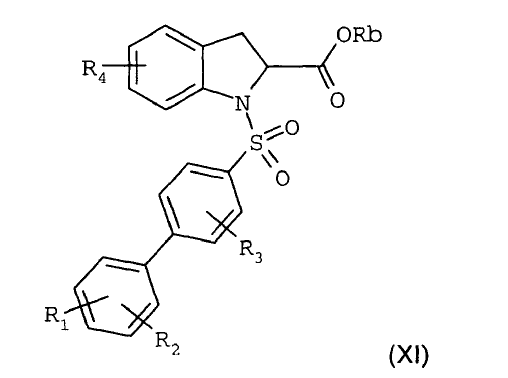 dans laquelle R₁, R₂, R₃, R₄ et Rb conservent la même signification que dans les composés de départ,
b) transformer l'ester (XI) en acide, par action d'une base en milieu hydroalcoolique selon des techniques bien connues de l'homme de métier pour obtenir l'acide de formule (XII) 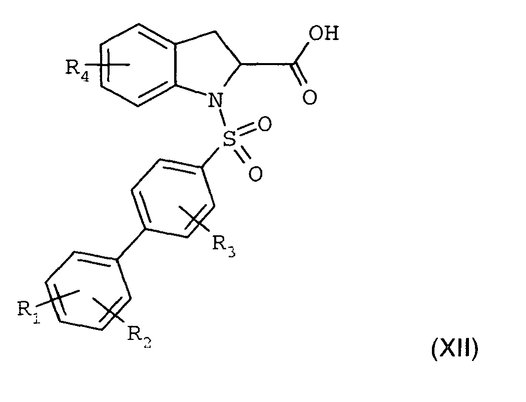 dans laquelle R₁, R₂, R₃ et R₄ restent inchangés,
c) faire réagir le composé acide (XII) avec une amine primaire de formule (III)

   NH₂-Y-z (III)

   dans laquelle
   - Y représente un groupe alkylène en C₁-C₈ linéaire, ramifié, éventuellement substitué par un groupe trifluorométhyle ou par un noyau phényle, ou comportant une partie cyclisée ayant 3 à 6 atomes de carbone, ou représente un groupe -(CH₂)ₙ-W-,
   - W représente un atome d'oxygène, -NH- ou un atome de soufre,
   - n représente 2, 3 ou 4,
   - Z représente un groupe alkyle en C₁-C₄, éventuellement partiellement halogéné, trifluorométhyle, CORₐ, CH₂-N(R)₂, un noyau aromatique, hétéroaromatique ou hétérocyclique choisi parmi les groupes phényle, pyridinyle, 1-imidazolyle, 1-pipéridinyle, 4-alkyl-1-pipérazinyle, pyridazinyle, 4-morpholinyle, 1-pyrrolidinyle ou pyrrolidinone, indolinylone, éventuellement substitué par un, deux ou trois substituants identiques ou différents choisis parmi un halogène, un groupe alkyle en C₁-C₄, alcoxy en C₁-C₄, trifluorométhyle, nitro, N(R)₂, -CH₂-N(R)₂, -O-(CH₂)ₙ-N(R)₂, hydroxy, cyano, cyanoalkyle en C₂-C₃ ou un groupe de formule -X-[C(R)₂]ₚ-CORₐ dans laquelle
   - X représente une liaison simple, un atome d'oxygène, un groupe -O-CH₂-, un atome de soufre ou un groupe -NR-,
   - Rₐ représente OR ou N(R)₂
   - R représente un groupe alkyle en C₁-C₃
   - p est égal à 0, 1, 2, 3 ou 4,
      selon un mode opératoire analogue à celui décrit à l'étape (a) du procédé décrit en premier lieu ci-dessus, pour obtenir le composé de formule (I) 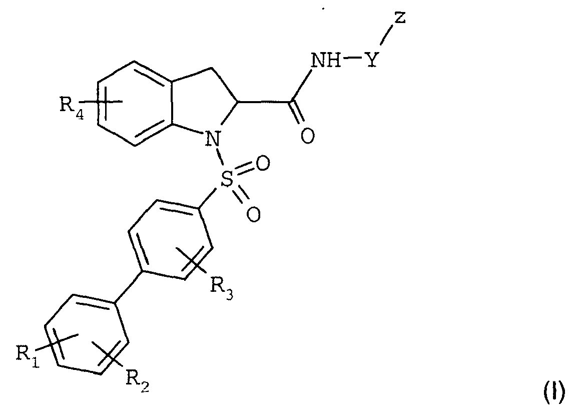
   dans laquelle R₁, R₂, R₃, R₄, Y et Z conservent la même signification que dans les composés de départ.

Les composés de formule (I) dans lesquels Z comprend un groupe amino, peuvent être obtenus à partir de leurs homologues nitrés, par une réaction de réduction chimique ou d'hydrogénation catalytique, selon des procédés classiques bien connus de l'homme de métier. Ces composés sont également accessibles à partir des composés de formule I comprenant un groupe cyano que l'on réduit suivant des procédés connus de l'homme de métier, notamment par hydrogénation catalytique.

Les composés dans lesquels Z comprend un groupe NH₂ ou NH libre peuvent également être obtenus au départ d'amines ou d'hétérocycles azotés dans lesquels la fonction NH₂ ou NH aura été préalablement protégée par un groupe aminoprotecteur tel que par exemple un groupe Boc (1,1-diméthyléthoxycarbonyle), ce groupe aminoprotecteur étant ensuite éliminé après avoir réalisé la liaison de couplage pour former la liaison amide -CONH- ou sulfonamide -SO₂N= du composé de formule I.

Les composés de formule I dans lesquels le carbone asymétrique est de configuration déterminée R ou S sont obtenus de préférence au départ d'acides indoline-2-carboxyliques de configuration R ou S. Il est possible également de les obtenir par séparation à partir du composé racémique d'une façon connue en soi, mais cette seconde méthode est généralement moins avantageuse.

Les exemples suivants de préparation de composés selon la formule (I) permettront de mieux comprendre l'invention.

Dans ces exemples, on désigne par « préparation » les exemples décrivant la synthèse de composés intermédiaires et par « exemples » ceux décrivant la synthèse de composés de formule (I) selon l'invention. Parmi les abréviations, « mmol » signifie millimole. Les points de fusion sont mesurés au banc Kofler ou en tube capillaire et les valeurs spectrales de Résonance Magnétique Nucléaire sont caractérisées par le déplacement chimique calculé par rapport au TMS, par le nombre de protons associés au signal et par la forme du signal (s pour singulet, d pour doublet, dd pour doublet dédoublé, t pour triplet, q pour quadruplet, m pour multiplet). La fréquence de travail et le solvant utilisé sont indiqués pour chaque composé. La température ambiante est de 20°C ± 2°C.

Les abréviations suivantes sont utilisées dans la description de ces exemples et préparations :
HOAT: 1 -hydroxy-7-azabenzotriazole
HOBT : 1-hydroxybenzotriazole
EDCI : chlorhydrate de 1-(3-diméthytaminopropyl)-3-éthylcarbodiimide),
DBAD : di-*t*-butylazodicarboxylate
DCC : dicyclohexylcarbodiimide
DCM : dichlorométhane
DIAD : diisopropylazodicarboxylate
DME : diméthoxyéthane
DMF : diméthylformamide
DMSO : diméthylsulfoxyde
MTBE : méthyl-*t*-butylétherTFA : acide trifluoroacétique
THF : tétrahydrofurane
PdCl₂dppf : dichloro-1,1'-bis(diphénylphosphino)ferrocène-palladium(II).

### PREPARATION I

### Acide 2-[[[2-(2-fluorophényl)éthyl]amino]carbonyl]-2,3-dihydro-(2S)-1H-indole-1-carboxylique 1,1-diméthyléthyl ester

On prépare une solution de 2 g (7,6 mmol) d'acide 2,3-dihydro-(2S)-1*H-*indole-1,2-dicarboxylique, 1-(1,1-diméthyléthyl) ester dans 15ml de dichlorométhane puis on ajoute 1,74 g (9,11 mmol) de EDCI (chlorhydrate de 1-(3-diméthylaminopropyl)-3-éthylcarbodiimide) et 206 mg (1,52 mmol) de HOAT (1-hydroxy-7-azabenzotriazole). Après 10 minutes sous agitation à température ambiante, on ajoute 1,20 ml (9,11 mmol) de 2-fluoro-benzèneéthanamine.Le milieu réactionnel est ensuite agité à température ambiante pendant 20 heures. Le milieu est alors traité par addition de dichlorométhane et la phase organique est lavée par de l'eau. La phase organique est séchée sur sulfate de magnésium, puis concentrée sous pression réduite. Le produit brut obtenu est ensuite purifié par chromatographie sur gel de silice en éluant à l'aide d'un mélange dichlorométhane/acétate d'éthyle (90/10 ; v/v). On obtient ainsi le produit attendu sous forme d'un solide blanc (rendement = 84 %).
F = 133°C.

### PREPARATION II

### N-[2-(2-fluorophényl)éthyl]-2,3-dihydro-(2S)-1H-indole-2-carboxamide

On prépare une solution de 2,38 g (6,19 mmol) du composé obtenu selon la préparation I dans 25 ml de dichlorométhane et on ajoute 9,3 ml d'acide trifluoroacétique. Le mélange réactionnel est agité à température ambiante pendant 4 heures puis le solvant est chassé sous pression réduite. Le résidu d'évaporation est ensuite repris à l'eau et le mélange est neutralisé par addition d'une solution saturée de bicarbonate de sodium. Un précipité blanc se forme, ce dernier est filtré et lavé à l'eau. Le solide obtenu est séché sur pentoxyde de phosphore, sous vide. On obtient ainsi le composé attendu sous forme d'un solide blanc (rendement = 98 %).
F = 135°C.

### PREPARATION III

### N-[2-(2-fluorophényl)éthyl]-2,3-dihydro-1-[(4-iodophényl)sulfonyl]-(2S)-1H-indole-2-carboxamide

On prépare une solution de 100 mg (0,351 mmol) du composé obtenu selon la préparation II dans 2 ml de dichlorométhane et on ajoute 74 µl de triéthylamine et 117 mg (0,386 mmol) de chlorure de 4-iodobenzènesulfonyle. Le mélange réactionnel est agité à température ambiante pendant 4 heures. Le milieu est alors traité par addition de dichlorométhane et la phase organique est lavée par de l'eau. La phase organique est séchée sur sulfate de magnésium, puis concentrée sous pression réduite. Le produit brut obtenu est ensuite purifié par chromatographie sur gel de silice en éluant à l'aide d'un mélange toluène/acétate d'éthyle (90/10 ; v/v). On obtient ainsi le produit attendu sous forme d'une huile incolore (rendement = 57 %).
RMN ¹H (DMSO, 300 MHz) δ : 8,26 (t, NH) ; 7,92 (d, 2H) ; 7,50 (d, 2H) ; 7,25 (d, 1 H) ; 7,3-7 (m, 7H) ; 4,71 (dd, 1 H) ; 3,5-3,2 (m, 2H) ; 3,1 (dd, 1H) ; 2,9-2,70 (dd et m, 3H).

### Exemple 1

### N-[2-(2-fluorophényl)éthyl]-2,3-dihydro-1-[[4'-(trifluorométhyl)[1,1'-biphényl]-4-yl]sulfonyl]-(2S)-1H-indole-2-carboxamide

Dans un tube pour réaction sous micro-ondes, on mélange 102 mg (0,185 mmol) du composé obtenu selon la préparation III dans 2 ml de DME, on ajoute 0,66 ml d'eau, puis 30 mg (0,278 mmol) de carbonate de sodium, 15 mg (0,0185 mmol) de PdCl₂dppf {dichloro-1,1'-bis(diphényl-phosphino)ferrocène-palladium(II)} et 71 mg (0,37 mmol) d'acide 4-(trifluorométhyl)phénylboronique. Le milieu réactionnel est chauffé 10 min à 110°C au four micro-ondes. 15 mg (0,0185 mmol) de dichloro-1,1'-bis(diphénylphosphino)ferrocène-palladium(II) sont additionnés à nouveau et le milieu réactionnel est de nouveau chauffé 30 min à 110°C au four micro-ondes. Après refroidissement, on ajoute du dichlorométhane au milieu réactionnel, et la phase organique est lavée par une solution de carbonate de sodium, puis à l'eau. La phase organique est séchée sur sulfate de magnésium et concentrée sous pression réduite. Le produit brut est purifié par chromatographie sur gel de silice en éluant à l'aide d'un mélange toluène/acétate d'éthyle (9/1 ; v/v). On obtient le produit sous forme d'une mousse incolore (rendement = 66 %).
RMN ¹H (DMSO, 300 MHz) δ : 8,30 (t, NH) ; 8,0-7,8 (m, 8H) ; 7,50 (d, 1H) ; 7,4-7,15 (m, 3H) ; 7,12-6,95 (m, 4H) ; 4,78 (dd, 1 H) ; 3,5-3,2 (m, 2H) ; 3,1 (dd, 1 H) ; 2,9-2,70 (dd et m, 3H).

### Exemple 2

### N-[2-(2-fluorophényl)éthyl]-2,3-dihydro-1-[[3'-(trifluorométhyl)[1,1'-biphényl]-4-yl]sulfonyl]-(2S)-1H-indole-2-carboxamide

Dans un tube pour réaction sous micro-ondes, on mélange 100 mg (0,181 mmol) du composé obtenu selon la préparation III dans 2 ml de DME, on ajoute 0,66 ml d'eau, puis 29 mg (0,272 mmol) de carbonate de sodium, 15 mg (0,0185 mmol) de PdCl₂dppf et 69 mg (0,363 mmol) d'acide 3-(trifluorométhyl)phénylboronique. Le milieu réactionnel est chauffé 10 min à 110°C au micro-ondes. Après refroidissement, on ajoute du dichlorométhane au milieu réactionnel, et la phase organique est lavée par une solution de carbonate de sodium, puis à l'eau. La phase organique est séchée sur sulfate de magnésium et concentrée sous pression réduite.
Le produit brut est purifié par chromatographie sur gel de silice en éluant à l'aide d'un mélange toluène/acétate d'éthyle (9/1 ; v/v). On obtient le produit sous forme d'une mousse blanche (rendement = 70 %).
RMN ¹H (DMSO, 300 MHz) δ : 8,28 (t, NH) ; 8,1-8,0 (m, 2H) ; 7,93 (d, 2H) ; 7,86 (d, 2H) ; 7,85-7,60 (m, 2H) ; 7,50 (d, 1H) ; 7,30-7 (m, 7H) ; 4,78 (dd, 1 H) ; 3,5-3,2 (m, 2H) ; 3,08 (dd, 1 H) ; 2,9-2,70 (dd et m, 3H).

### Exemple 3

### N-[2-(2-fluorophényl)éthyl]-2,3-dihydro-1-[[2'-(trifluorométhyl)[1,1'-biphényl]-4-yl]sulfonyl]-(2S)-1H-indole-2-carboxamide

En opérant de manière analogue à l'exemple 2, au départ d'acide 2-(trifluorométhyl)phénylboronique, on obtient le produit attendu sous forme d'une mousse rose (rendement = 67 %).
RMN ¹H (DMSO, 250 MHz) δ : 8,29 (t, NH) ; 7,9-7,6 (m, 5H) ; 7,55-7,35 (m, 4H) ; 7,3-7,0 (m, 7H) ; 4,77 (dd, 1H) ; 3,5-3,2 (m, 2H) ; 3,05-2,07 (m, 4H).

### PREPARATION IV

### Acide (2S)-2,3-dihydro-1-[[3'-(trifluorométhyl)[1,1'-biphényl]-4-yl]sulfonyl]-1H-indole-2-carboxylique.

### a) acide (2S)-2,3-dihydro-1-[(4-iodophényl)sulfonyl]indole-2-carboxylique

On prépare un mélange de 16,18 g (99,2 mmol) d'acide (2S)-2,3-dihydroindole-2-carboxylique, 1700 ml d'acétonitrile, 400 ml d'eau et 2,37 g (99,2 mmol) de lithine. Ce mélange est agité jusqu'à dissolution et on ajoute à température ambiante, 30 g (99,2 mmol) de chlorure de 4-iodobenzènesulfonyle. Le milieu réactionnel est agité 15 heures à température ambiante, puis concentré sous pression réduite. Le résidu d'évaporation est repris dans une solution de carbonate de sodium et la phase aqueuse obtenue est lavée par 200 ml d'acétate d'éthyle, puis acidifiée à l'aide d'acide chlorhydrique jusqu'à pH 2. Le précipité obtenu est filtré et recristallisé dans 500 ml d'acide acétique. On obtient ainsi 32 g d'acide attendu sous forme de cristaux beiges (rendement = 76 %).
F = 194-198°C.

### b) Acide (2S)-2,3-dihydro-1-[[3'-(trifluorométhyl)[1,1'-biphényl]-4-yl]-sulfonyl]-1H-indole-2-carboxylique.

On prépare un mélange de 20 g (46,6 mmol) d'acide obtenu ci-dessus, 1 700 ml d'acétonitrile, 9,73 g (51,2 mmol) d'acide 3-(trifluorométhyl)-phénylboronique, 190 ml d'eau, 14,81 g (139,8 mmol) de carbonate de sodium, et 1,04 g (4,6 mmol) d'acétate de palladium. Ce mélange est agité pendant 3 heures à température ambiante. On ajoute ensuite 400 ml d'acétate d'éthyle et acidifie le milieu jusqu'à pH 3 à l'aide d'une solution N d'acide chlorhydrique. La phase organique est séparée, lavée à l'eau puis séchée sur sulfate de magnésium et concentrée sous pression réduite. Le produit brut est repris à chaud dans 200 ml d'un mélange acide acétique/eau. Après refroidissement, les cristaux formés sont séparés par filtration et séchés. On obtient ainsi 19,2 g de l'acide attendu sous forme de cristaux beiges (rendement = 92 %).
F = 108-112°C.

### Exemple 4

### Acide 3-[2-[[[(2S)-2,3-dihydro-1-[[3'-(trifluorométhyl)[1,1'-biphényl]-4-yl]sulfonyl]-1H-indol-2-yl]carbonyl]amino]éthyl]benzoïque, méthyl ester

En opérant de manière analogue à la préparation I, au départ de l'acide obtenu selon la préparation IV et de l'ester méthylique de l'acide 3-(2-aminoéthyl)benzoïque, on obtient le produit attendu sous forme d'une mousse blanche (rendement = 48 %).
RMN ¹H (DMSO, 300 MHz) δ : 8,23 (t, NH) ; 8,1-7,6 (m, 10H) ; 7,55-7,35 (m, 3H) ; 7,3-7,0 (m, 3H) ; 4,78 (dd, 1H) : 3,5-3,2 (m, 2H) ; 3,07 (dd, 1 H) ; 2,95-2,75 (m, 3H).

### Exemple 5

### Acide 3-[2-[[[(2S)-2,3-dihydro-1-[[3'-(triftuorométhyl)[1,1'-biphényl]-4-yl]sulfonyl]-1H-indol-2-yl]carbonyl]amino]éthyl]benzoïque

On mélange 663 mg (1,08 mmol) de l'ester obtenu selon l'exemple 4 avec 5 ml de tétrahydrofurane et 5 ml d'eau et on ajoute, sous agitation et à température ambiante, 104 mg (4,35 mmol) de lithine. Ce mélange réactionnel est agité à température ambiante pendant 24 heures, puis acidifié jusqu'à pH 3 par une solution d'acide acétique N, dilué à l'eau puis extrait par de l'acétate d'éthyle. Les phases organiques rassemblées sont séchées sur sulfate de magnésium et concentrées sous pression réduite. On obtient ainsi le produit attendu sous forme d'une mousse blanche (rendement = 75 %).
RMN ¹H (DMSO, 250 MHz) δ : 13 (bs, CO2H) ; 8,25 (t, NH) ; 8,1-7,65 (m, 10H) ; 7,55-7,30 (m, 3H) ; 7,3-6,95 (m, 3H) ; 4,78 (dd, 1 H) ; 3,5-3,2 (m, 2H) ; 3,08 (dd, 1 H) ; 2,95-2,70 (m, 3H).

### Exemple 6

### 2,3-dihydro-N-[(1-phénylcyclohexyl)méthyl]-1-[[3'-(triftuorométhyl)-[1,1'-biphényl]-4-yl]sulfonyl]-(2S)-1H-indole-2-carboxamide

En opérant de manière analogue à l'exemple 4, au départ de 1-phénylcyclohexaneméthanamine, on obtient le produit attendu sous forme d'une mousse blanche (rendement = 33 %).
RMN ¹H (DMSO, 250 MHz) δ : 8,05-8,0 (m, 2H) ; 7,93 (d, 2H) ; 7,86 (d, 2H) ; 7,82-7,67 (m, 2H) ; 7,47-7,38 (m, 1 H et NH) ; 7,37-7,30 (m, 4H) ; 7,28-7,17 (m, 2H) ; 7,11 (d, 1 H) ; 7,02 (td, 1H) ; 4,87 (dd, 1 H) ; 3,5-3,1 (m, 2H) ; 3,02 (dd, 1H) ; 2,73 (dd, 1 H) ; 2,15-2,0 (m, 2H) ; 1,7-1,1 (m, 8H).

### PREPARATION V

### Acide 2,3-dihydro-1-[[2'-(trifluorométhyl)[1,1'-biphényl]-4-yl]sulfonyl]-(2S)-1H-indole-2-carboxylique

On prépare une solution de 2,15 g (4,66 mmol) d'acide 2,3-dihydro-1-[(4-iodophényl)sulfonyl]-(2S)-1*H*-indole-2-carboxylique obtenu selon la préparation IV(a) dans 40 ml de DME et on ajoute 13 ml d'eau, puis 1,24 g (11,6 mmol) de carbonate de sodium, 380 mg (0,46 mmol) de PdCl₂dppf et 1,77 g (9,3 mmol) d'acide 2-(trifluorométhyl)phénylboronique. Le milieu réactionnel est agité à température ambiante pendant 30 mn puis à 90°C pendant 30 mn. Après refroidissement, on ajoute de l'eau et on acidifie le milieu à l'aide d'acide chlorhydrique N. Le mélange est ensuite extrait par l'acétate d'éthyle. La phase organique obtenue est lavée à l'eau, séchée sur sulfate de magnésium et concentrée sous pression réduite. Le produit brut est purifié par chromatographie en phase inverse sur gel de silice greffée RP 18 en éluant à l'aide d'un mélange acétonitrile/eau/acide trifluoroacétique (8/2/0,02 ; v/v/v). On obtient le produit sous forme d'une huile orange (rendement = 80 %).
RMN ¹H (CDCl₃, 250 MHz) δ : 7,8-7,7 (m, 3H) ; 7,65 (d, 1 H) ; 7,65-7,45 (m, 2H) ; 7,38 (d, 2H) ; 7,3-7,2 (m, 2H) ; 7,15-7,0 (m, 2H) ; 4,84 (dd, 1H) ; 3,23 (dd, 1 H) ; 3,09 (dd, 1 H).

### PREPARATION VI

### Chlorure de l'acide 2,3-dihydro-1-[[2'-(trifluorométhyl)[1,1'-biphényl]-4-yl]sulfonyl]-(2S)-1H-indole-2-carboxylique.

On dissout 1,57 g (3,5 mmol) d'acide obtenu selon la préparation V dans 10 ml de toluène. On additionne à température ambiante 1 goutte de DMF, puis au goutte à goutte une solution de 0,603 ml (7,02 mmol) de chlorure d'oxalyle dans 5 ml de toluène. Le milieu réactionnel est agité 30 min à température ambiante. On ajoute du toluène au milieu réactionnel et on concentre sous pression réduite afin de chasser l'excès de chlorure d'oxalyle. On obtient ainsi le produit attendu sous forme d'une huile brune (rendement = 92 %).
RMN ¹H (CDCl₃, 250 MHz) δ : 7,85-7,65 (m, 3H) ; 7,60-7,45 (m, 3H) ; 7,45-7,35 (d, 2H) ; 7,3-7 (m, 4H) ; 5,11 (dd, 1 H) ; 3,35-3,2 (m, 2H).

### Exemple 7

### 2,3-dihydro-N-[2-(1-piperidinyl)éthyl]-1-[[2'-(trifluorométhyl)[1,1'-biphényl]-4-yl]sulfonyl]-(2S)-1H-indole-2-carboxamide

Dans une seringue, on mélange 168 mg (0,644 mmol) de résine DIEA (résine polystyrène greffée par un groupe 4-(diisopropylaminométhyl)-phényl dans 2 ml de dichlorométhane, et on laisse la résine gonfler. La résine est filtrée, puis rincée au dichlorométhane. On ajoute ensuite, à cette résine, 1 ml de dichlorométhane, puis 27,5 mg (0,214 mmol) de 1-pipéridineéthanamine et 100 mg (0,214 mmol) du chlorure d'acide obtenu selon la préparation VI dans 2 ml de dichlorométhane. Le milieu réactionnel est agité 1 h à température ambiante, puis on filtre le milieu et la résine est lavée par du dichlorométhane. Les filtrats sont réunis et concentrés sous pression réduite. Le produit brut est purifié par chromatographie sur gel de silice en éluant à l'aide d'un mélange dichlorométhane/éthanol (95/5 ; v/v). On obtient le produit sous forme d'une huile incolore (rendement = 49 %).
RMN ¹H (DMSO, 300 MHz) δ: 8,04 (t, NH) ; 7,95-7,60 (m, 5H) ; 7,55-7,45 (m, 3H) ; 7,39 (d, 1H) ; 7,24 (t, 1H) ; 7,14 (d, 1 H) ; 7,05 (t, 1H) ; 4,84 (dd, 1H) ; 3,4-3,05 (m, 2H) ; 2,98 (d, 2H) ; 2,45-2,2 (m, 6H) ; 1,6-1,25 (m, 6H).
En opérant de manière analogue à l'exemple 7, au départ d'amines connues, on obtient : **Exemple 8**

### 2,3-dihydro-N-[3-(1-pyrrolidinyl)propyl]-1-[[2'-(trifluorométhyl)[1,1'-biphényl]-4-yl]sulfonyl]-(2S)-1H-indole-2-carboxamide

Rendement = 68 % ; mousse incolore ;
RMN ¹H (DMSO, 300 MHz) δ: 8,45 (t, NH) ; 7,95-7,8 (m, 3H) ; 7,8-7,6 (m, 2H) ; 7,5-7,45 (m, 3H) ; 7,40 (d, 1H) ; 7,25 (t, 1H) ; 7,15(d,1H) ; 7,05 (t, 1H) ; 4,79 (dd, 1H) ; 3,5-2,8 (m, 10H) ; 2-1,7 (m, 6H).

### Exemple 9

### 2,3-dihydro-N-[3-(2-méthyl-1-piperidinyl)propyl]-1-[[2'-(trifluorométhyl)[1,1'-biphényl]-4-yl]sulfonyl]-(2S)-1H-indole-2-carboxamide

Rendement = 75 % ; mousse incolore ;
RMN ¹H (DMSO, 300 MHz) δ : 8,0 (sl, NH) ; 7,95-7,8 (m, 3H) ; 7,8-7,6 (m, 2H) ; 7,5-7,45 (m, 3H) ; 7,36 (d, 1 H) ; 7,24 (t, 1 H) ; 7,15 (d, 1 H) ; 7,05 (t, 1 H) ; 4,82 (dd, 1 H) ; 3,4-2,6 (m, 9H) ; 2-1,35 (m, 8H) ; 1,22 (d, CH3).

### Exemple 10

### 2,3-dihydro-N-[4-(1-pyrrolidinyl)butyl]-1-[[2'-(trifluorométhyl)[1,1'-biphényl]-4-yl]sulfonyl]-(2S)-1H-indole-2-carboxamide

Rendement = 72 % ; mousse incolore ;
RMN 1 H (DMSO, 300 MHz) δ : 8,31 (t, NH) ; 7,9-7,8 (m, 3H) ; 7,8-7,6 (m, 2H) ; 7,55-7,45 (m, 3H) ; 7,40 (d, 1H) ; 7,25 (t, 1H) ; 7,15 (d, 1 H) ; 7,06 (t, 1H); 4,81 (dd, 1 H) ; 3,4-2,8 (m, 10H) ; 2-1,8 (m, 4H) ; 1,75-1,4 (m, 4H).

### Exemple 11

### 2,3-dihydro-N-[2-(4-morpholinyl)éthyl]-1-[[2'-(trifluorométhyl)[1,1'-biphényl]-4-yl]sulfonyl]-(2S)-1H-indole-2-carboxamide

Rendement = 81 % ; mousse incolore ;
RMN ¹H (DMSO, 300 MHz) δ : 8,08 (t, NH) ; 7,9-7,8 (m, 3H) ; 7,8-7,6 (m, 2H) ; 7,55-7,45 (m, 3H) ; 7,39 (d, 1 H) ; 7,25 (t, 1 H) ; 7,15 (d, 1 H) ; 7,05 (t, 1H) ; 4,85 (t, 1 H) ; 3,54 (t, 4H) ; 3,4-3,1 (m, 2H) ; 2,98 (d, 2H) ; 2,45-2,25 (m, 6H).

### Exemple 12

### 2,3-dihydro-N-[3-(4-méthyl-1-piperazinyl)propyl]-1-[[2'-(trifluorométhyl)[1,1'-biphényl]-4-yl]sulfonyl]-(2S)-1H-indole-2-carboxamide

Rendement = 70 % ; mousse incolore ;
RMN ¹H (DMSO, 300 MHz) δ : 8,17 (t, NH) ; 7,9-7,8 (m, 3H) ; 7,8-7,6 (m, 2H) ; 7,55-7,45 (m, 3H) ; 7,39 (d, 1H) ; 7,24 (t, 1 H) ; 7,14 (d, 1 H) ; 7,04 (t, 1H) ; 4,79 (dd, 1 H) ; 3,2-2,85 (m, 4H) ; 2,5-2,1 (m, 10H) ; 2,20 (s, CH3) ; 1,65-1,50 (m, 2H).

### Exemple 13

### 2,3-dihydro-N-[3-(méthoxy)propyl]-1-[[2'-(trifluorométhyl)[1,1'-biphényl]-4-yl]suifonyl]-(2S)-1H-indole-2-carboxamide

Rendement = 30 % ; huile incolore ;
RMN ¹H (DMSO, 300 MHz) δ : 8,19 (t, NH) ; 7,9-7,8 (m, 3H) ; 7,8-7,6 (m, 2H) ; 7,55-7,45 (m, 3H) ; 7,40 (d, 1H) ; 7,24 (t, 1 H) ; 7,14 (d, 1 H) ; 7,04 (t, 1H) ; 4,79 (dd, 1 H) ; 3,4-3,25 (m, 2H) ; 3,22 (s, 3H) ; 3,25-3,10 (m, 2H) ; 3,10-2,90 (m, 2H) ; 1,75-1,60 (m, 2H).

### Exemple 14

### 2,3-dihydro-N-[3-(2-oxo-1-pyrrolidinyl)propyl]-1-[[2'-(trifluorométhyl)-[1,1'-biphényl]-4-yl]sulfonyl]-(2S)-1H-indole-2-carboxamide

Rendement = 36 % ; huile incolore ;
RMN ¹H (DMSO, 300 MHz) δ : 8,24 (t, NH) ; 7,9-7,8 (m, 3H) ; 7,8-7,6 (m, 2H) ; 7,55-7,45 (m, 3H) ; 7,40 (d, 1 H) ; 7,24 (t, 1 H) ; 7,14 (d, 1 H) ; 7,04 (t, 1H) ; 4,79 (dd, 1 H) ; 3,4-3,25 (m, 2H) ; 3,25-2,9 (m, 6H) ; 2,20 (t, 2H) ; 1,91 (quint, 2H) ; 1,61 (quint, 2H).

### Exemple 15

### 2,3-dihydro-N-[2,2,2-trifluoroéthyl]-1-[[2'-(trifluorométhyl)[1,1'-biphényl]-4-yl]sulfonyl]-(2S)-1H-indole-2-carboxamide

Rendement = 30 % ; huile incolore ;
RMN ¹H (DMSO, 300 MHz) δ : 8,88 (t, NH) ; 7,9-7,8 (m, 3H) ; 7,8-7,6 (m, 2H) ; 7,55-7,45 (m, 3H) ; 7,40 (d, 1 H) ; 7,25 (t, 1 H) ; 7,14 (d, 1 H) ; 7,06 (t, 1 H) ; 4,95 (dd, 1 H) ; 4,15-3,8 (m, 2H) ; 3,13 (dd, 1H) ; 2,91 (dd, 1 H).

### Exemple 16

### 2,3-dihydro-N-[3-(1H-imidazol-1-yl)propyl]-1-[[2'-(trifluorométhyl)[1,1'-biphényl]-4-yl]sulfonyl]-(2S)-1H-indole-2-carboxamide

Rendement = 36 % ; huile incolore ;
RMN ¹H (DMSO, 300 MHz) δ : 8,33 (t, NH) ; 7,9-7,8 (m, 3H) ; 7,8-7,6 (m, 2H) ; 7,58 (s, 1 H) ; 7,55-7,45 (m, 3H) ; 7,40 (d, 1 H) ; 7,25 (t, 1 H) ; 7,2-7,1 (m, 2H) ; 7,06 (t, 1H) ; 6,88 (s, 1 H) ; 4,79(dd,1H) ; 3,97 (t, 2H) ; 3,2-2,9 (m, 4H) ; 1,88 (quint, 2H).

### Exemple 17

### Acide 4-[[[[(2S)-2,3-dihydro-1-[[2'-(trifluorométhyl)[1,1'-biphényl]-4-yl]-sulfonyl]-1H-indol-2-yl]carbonyl]amino]méthyl]benzoïque

On mélange 304 mg (2,01 mmol) d'acide 4-(aminométhyl)-benzoïque avec 25 ml d'acétonitrile, 7,3 ml d'eau, et 4,8 ml de triéthyl-amine. Le milieu réactionnel est limpide. On additionne alors 624 mg (1,34 mmol) du chlorure d'acide obtenu selon la préparation VI dissous dans 4,8 ml de dichlorométhane. Le mélange réactionnel est agité à température ambiante pendant 1 heure, puis les solvants sont évaporés sous pression réduite. Le produit brut résiduel est alors repris à l'acétate d'éthyle et la phase organique est lavée à l'eau. On additionne ensuite du noir animal à la phase organique et on filtre sur un tampon de silice. Le filtrat est alors concentré sous pression réduite. On obtient ainsi le produit attendu sous forme d'une mousse blanche (rendement = 66 %).
RMN ¹H (DMSO, 250 MHz) δ : 8,85 (t, NH) ; 7,9-7,8 (m, 5H) ; 7,8-7,6 (m, 2H) ; 7,55-7,45 (m, 3H) ; 7,45-7,35 (m, 3H) ; 7,26 (t, 1H) ; 7,15 (d, 1H) ; 7,07 (t, 1H) ; 4,91 (dd, 1 H) ; 4,40 (t, 2H) ; 3,2-2,9 (m, 2H).

En opérant de manière analogue à l'exemple 17, au départ d'amines connues on obtient :

### Exemple 18

### Acide 4-[2-[[[(2S)-2,3-dihydro-1-[[2'-(trifluorométhyl)[1,1'-biphényl]-4-yl]sulfonyl]-1H-indol-2yl]carbonyl]amino]éthoxy]benzoïque

Rendement = 56 % ; mousse blanche ;
F = colle à 94°C.

### Exemple 19

### Acide 5-[[[(2S)-2,3-dihydro-1-[[2-(trifluorométhyl)[1,1'-biphényl]-4-yl]sulfonyl]-1H-indol-2-yl]carbonyl]amino]pentanoïque

Rendement = 23 % ; huile incolore ;
RMN ¹H (DMSO, 250 MHz) δ : 12,1-11,9 (sl, COOH) ; 8,18 (t, NH) ; 7,9-7,8 (m, 3H) ; 7,8-7,6 (m, 2H) ; 7,55-7,45 (m, 3H) ; 7,40 (d, 1H) ; 7,24 (t, 1 H) ; 7,13 (d, 1H) ; 7,05 (t, 1H) ; 4,79 (dd, 1H) ; 3,25-2,85 (m, 4H) ; 2,22 (t, 2H) ; 1,6-1,3 (m, 4H).

### Exemple 20

### 2,3-dihydro-N-[4,4,4-trifluorobutyl]-1-[[2'-(trifluorométhyl)[1,1'-biphényl]-4-yl]sulfonyl]-(2S)-1H-indole-2-carboxamide

Rendement = 29 % ; huile incolore ;
RMN ¹H (DMSO, 250 MHz) δ : 8,34 (t, NH) ; 7,9-7,8 (m, 3H) ; 7,8-7,6 (m, 2H) ; 7,55-7,45 (m, 3H) ; 7,40 (d, 1 H) ; 7,24 (t, 1 H) ; 7,14 (d, 1 H) ; 7,04 (td, 1H) ; 4,78 (dd, 1H) ; 3,20 (q, 2H) ; 3,07 (dd, 1H) ; 2,94 (dd, 1 H) ; 2,4-2,1 (m, 2H) ; 1,75-1,60 (m, 2H).

### Exemple 21

### Acide 4-[[[[(2S)-2,3-dihydro-1-[[21-(trifluorométhyl)[1, 11-biphényl]-4-yl]sulfonyl]-1H-indol-2-yl]carbonyl]amino]méthyl]benzèneacétique, méthyl ester

On prépare une solution de 500 mg (1,11 mmol) d'acide obtenu selon la préparation V dans 6 ml de dichlorométhane puis on ajoute 235 mg (1,23 mmol) de EDCI 167 mg (1,23 mmol) de HOAT et 0,343 ml (2,46 mmol) de triéthylamine. Après 10 minutes sous agitation à température ambiante, on ajoute 265 mg (1,23 mmol) d'ester méthylique de l'acide 4-(aminométhyl)benzèneacétique. Le milieu réactionnel est ensuite agité à température ambiante pendant 2 heures 30. Le milieu est alors traité par addition de dichlorométhane et la phase organique est lavée par de l'eau. La phase organique est ensuite séchée sur sulfate de magnésium, puis concentrée sous pression réduite. Le produit brut obtenu est ensuite purifié par chromatographie sur gel de silice en éluant à l'aide d'un mélange dichlorométhane/acétate d'éthyle (98/2 ; v/v). On obtient ainsi le produit attendu sous forme d'une huile incolore (rendement = 59 %).
RMN ¹H (DMSO, 300 MHz) δ : 8,74 (t, NH) ; 7,9-7,8 (m, 3H) ; 7,8-7,6 (m, 2H) ; 7,55-7,45 (m, 3H) ; 7,40 (d, 1 H) ; 7,3-7,1 (m, 6H) ; 7,05 (t, 1 H) ; 4,89 (dd, 1 H) ; 4,31 (t, 2H) ; 3,64 (s, CH₂) ; 3,60 (s, CH₃) ; 3,15-2,9 (m, 2H).

### Exemple 22

### Acide 4-[[[[(2S)-2,3-dihydro-1-[[2'-(trifluorométhy)[1,1'-biphényl]-4-yl]sulfonyl]-1H-indol-2-yl]carbonyl]amino]méthyl]benzèneacétique

On mélange 379 mg (0,622 mmol) d'ester obtenu selon l'exemple 21 avec 5 ml de tétrahydrofurane et 2 ml d'eau et on ajoute, sous agitation et à température ambiante, 60 mg (2,49 mmol) de lithine. Ce mélange réactionnel est agité à température ambiante pendant 4 heures, puis acidifié jusqu'à pH 3-4 par une solution d'acide chlorhydrique, dilué à l'eau puis extrait à l'acétate d'éthyle. Les phases organiques rassemblées sont séchées sur sulfate de magnésium et concentrées sous pression réduite. On obtient ainsi le produit attendu sous forme d'une huile incolore (rendement = 93 %).
RMN ¹H (DMSO, 250 MHz) δ : 8,72 (t, NH) ; 7,9-7,8 (m, 3H) ; 7,8-7,6 (m, 2H) ; 7,55-7,45 (m, 3H) ; 7,40 (d, 1H) ; 7,3-7,1 (m, 6H) ; 7,05 (t, 1 H) ; 4,89 (dd, 1H) ; 4,31 (t, 2H) ; 3,53 (s, CH₂) ; 3,15-2,9 (m, 2H).

### Exemple 23

### 2,3-dihydro-1-[[2'-(trifluorométhyl)[1,1'-biphényl]-4-yl]sulfonyl]-N-[3,3,3-trifluoropropyl]-(2S)-1H-indole-2-carboxamide

En opérant de manière analogue à l'exemple 21, au départ de la 3,3,3-trifluoropropanamine, on obtient le composé attendu sous forme d'une huile incolore (rendement = 84 %).
RMN ¹H (DMSO, 250 MHz) δ : 8,42 (t, NH) ; 7,9-7,6 (m, 5H) ; 7,55-7,45 (m, 3H) ; 7,40 (d, 1 H) ; 7,25 (t, 1 H) ; 7,14 (d, 1 H) ; 7,06 (t, 1 H) ; 4,81 (dd, 1H) ; 3,5-3,25 (m, 2H) ; 3,15-2,85 (m, 2H) ; 2,55-2,3 (m, 2H).

### Exemple 24

### Acide 4-[2-[[[(2S)-2,3-dihydro-1-[[2'-(trifluorométhyl)[1,1'-biphényl]-4-yl]sulfonyl]-1H-indol-2-yl]carbonyl]amino]éthyl]benzèneacétique, 1,1-diméthyléthyl ester

En opérant de manière analogue à l'exemple 21, au départ de l'ester t-butylique de l'acide 4-(2-aminoéthyl)benzèneacétique, on obtient le composé attendu sous forme d'une mousse blanche (rendement = 60 %).
RMN ¹H (DMSO, 250 MHz) δ : 8,22 (t, NH) ; 7,9-7,6 (m, 5H) ; 7,55-7,45 (m, 3H) ; 7,39 (d, 1 H) ; 7,25 (t, 1 H) ; 7,2-7,0 (m, 6H) ; 4,78 (dd, 1 H) ; 3,49 (s, 2H) ; 3,4-3,2 (m, 2H) ; 3,05-2,8 (m, 2H) ; 2,71 (t, 2H) ; 1,39 (s, 9H).

### Exemple 25

### Acide 4-[2-[[[(2S)-2,3-dihydro-1-[[2'-(trifluorométhyl)[1,1'-biphényl]-4-yl]sulfonyl]-1H-indol-2-yl]carbonyl]amino]éthyl]benzèneacétique

On prépare une solution de 541 mg (0,813 mmol) de l'ester obtenu selon l'exemple 24 dans 5 ml de dichlorométhane. On additionne à température ambiante 1,2 ml d'acide trifluoroacétique. Le milieu réactionnel est agité 4 heures à température ambiante. Le milieu est ensuite concentré sous pression réduite et repris à l'acétate d'éthyle. La phase organique est lavée par une solution à 10% de carbonate de sodium, puis à l'eau. Les phases aqueuses sont extraites 3 fois par du dichlorométhane. Les phases organiques rassemblées sont séchées sur sulfate de magnésium et concentrées sous pression réduite. On obtient ainsi le produit attendu sous forme d'un solide blanc (rendement = 89 %).
F = 152°C.

### Exemple 26

### Acide 3-[2-[[[(2S)-2,3-dihydro-1-[[2'-(trifluorométhyl)[1,1'-biphényl]-4-yl]sulfonyl]-1H-indol-2-yl]carbonyl]amino]éthyl]benzèneacétique, 1,1-diméthyléthyl ester

En opérant de manière identique à l'exemple 21, au départ de l'ester *t-*butylique de l'acide 3-(2-aminoéthyl)benzèneacétique, on obtient le composé attendu sous forme d'une mousse blanche (rendement = 80 %).
RMN ¹H (DMSO, 300 MHz) δ : 8,25 (t, NH) ; 7,9-7,8 (m, 3H) ; 7,8-7,6 (m, 2H) ; 7,55-7,45 (m, 3H) ; 7,40 (d, 1H) ; 7,3-7,15 (m, 2H) ; 7,15-7 (m, 5H) ; 4,80 (dd, 1H) ; 3,51 (s, 2H) ; 3,45-3,20 (m, 2H) ; 2,98 (dd, 1 H) ; 2,88 (dd, 1 H) ; 2,72 (t, 2H) ; 1,39 (s, 9H).

### Exemple 27

### Acide 3-[2-[[[(2S)-2,3-dihydro-1-[[2'-(trifluorométhyl)[1,1'-biphényl]-4-yl]sulfonyl]-1H-indol-2-yl]carbonyl]amino]éthyl]benzèneacétique,

En opérant de manière identique à l'exemple 25, au départ de l'ester obtenu selon l'exemple 26, on obtient le composé attendu sous forme d'un solide blanc (rendement = 95 %).
F = 78°C.

### Exemple 28

### Acide 6-[[[(2S)-2,3-dihydro-1-[[2'-(trifluorométhyl)[1,1'-biphényl]-4-yl]sulfonyl]-1H-indol-2-yl]carbonyl]amino]hexanoïque, méthyl ester

En opérant de manière analogue à la préparation I, au départ de l'acide obtenu selon la préparation V et d'ester méthylique de l'acide 6-aminohexanoïque, on obtient le composé attendu sous forme d'une huile incolore (rendement = 83 %).
RMN ¹H (DMSO, 250 MHz) δ : 8,15 (t, NH) ; 7,9-7,8 (m, 3H) ; 7,8-7,6 (m, 2H) ; 7,55-7,45 (m, 3H) ; 7,40 (d, 1H) ; 7,24 (t, 1H) ; 7,14(d,1H); 7,05 (t, 1H) ; 4,79 (dd, 1H) ; 3,57 (s, CH3) ; 3,2-2,85 (m, 4H) ; 2,27 (t, 2H) ; 1,6-1,35 (m, 4H) ; 1,35-1,15 (m, 2H).

### Exemple 29

### Acide 6-[[[(2S)-2,3-dihydro-1-[[2'-(trifluorométhyl)[1,1'-biphényl]-4-yl]sulfonyl]-1H-indol-2-yl]carbonyl]amino]hexanoïque

En opérant de manière analogue à l'exemple 22, au départ de l'ester obtenu selon l'exemple 28, on obtient le composé attendu sous forme d'une huile incolore (rendement = 83 %).
RMN ¹H (DMSO, 250 MHz) δ : 8,15 (t, NH) ; 7,9-7,8 (m, 3H) ; 7,8-7,6 (m, 2H) ; 7,55-7,45 (m, 3H) ; 7,40 (d, 1H) ; 7,24 (t, 1 H) ; 7,14 (d, 1 H) ; 7,05 (t, 1H) ; 4,79 (dd, 1 H) ; 3,2-2,85 (m, 4H) ; 2,17 (t, 2H) ; 1,6-1,1 (m, 6H).

### Exemple 30

### 2,3-dihydro-N-[4-(diméthylamino)butyl]-1-[[2'-(trifluorométhyl)[1,1'-biphényl]-4-yl]sulfonyl]-(2S)-1H-indole-2-carboxamide

En opérant de manière analogue à l'exemple 28, au départ de N,N-diméthylbutanediamine, on obtient le composé attendu sous forme d'une huile incolore (rendement = 27 %).
RMN ¹H (DMSO, 300 MHz) δ : 8,18 (t, NH) ; 7,9-7,8 (m, 3H) ; 7,8-7,6 (m, 2H) ; 7,55-7,45 (m, 3H) ; 7,40 (d, 1H) ; 7,24 (t, 1H) ; 7,14 (d, 1H) ; 7,05 (t, 1H) ; 4,79 (dd, 1 H) ; 3,2-2,85 (m, 4H) ; 2,16 (t, 2H) ; 2,08 (s, 6H) ; 1,5-1,3 (m, 4H).

### Exemple 31

### Acide 4-[[[(2S)-2,3-dihydro-1-[[2'-(trifluorométhyl)[1,1'-biphényl]-4-yl]sulfonyl]-1H-indol-2-yl]carbonyl]amino]butanoïque, méthyl ester

En opérant de manière analogue à l'exemple 28, au départ d'ester méthylique de l'acide 4-aminobutanoïque, on obtient le composé attendu sous forme d'une huile incolore (rendement = 33 %).
RMN ¹H (DMSO, 300 MHz) δ : 8,26 (t, NH) ; 7,9-7,8 (m, 3H) ; 7,8-7,6 (m, 2H) ; 7,55-7,45 (m, 3H) ; 7,40 (d, 1H) ; 7,24 (t, 1H) ; 7,14 (d, 1H) ; 7,05 (t, 1H) ; 4,77 (dd, 1H) ; 3,58 (s, CH3) ; 3,2-2,85 (m, 4H) ; 2,33 (t, 2H) ; 1,70 (quint, 2H).

### Exemple 32

### Acide 4-[[[(2S)-2,3-dihydro-1-[[2'-(trifluorométhyl)[1,1'-biphényl]-4-yl]-sulfonyl]-1H-indol-2-yl]carbonyl]amino]butanoïque

En opérant de manière analogue à l'exemple 22, au départ de l'ester obtenu selon l'exemple 31, on obtient le composé attendu sous forme d'une huile incolore (rendement = 57 %).
RMN ¹H (DMSO, 250 MHz) δ 8,24 (t, NH) ; 7,9-7,8 (m, 3H) ; 7,8-7,6 (m, 2H) ; 7,55-7,45 (m, 3H) ; 7,40 (d, 1 H) ; 7,24 (td, 1 H) ; 7,14 (d, 1 H) ; 7,04 (td, 1 H) ; 4,78 (dd, 1 H) ; 3,2-3 (m, 3H) ; 2,93 (dd, 1 H) ; 2,23 (t, 2H) ; 1,67 (quin, 2H).

### PREPARATION VIIa

### Acide 4-[2-[[[(2S)-2,3-dihydro-1-[(4-iodophényl)sulfonyl]-1H-indol-2-yl]carbonyl]amino]éthoxy]benzèneacétique, méthyl ester

En opérant de manière analogue à la préparation I, au départ de l'acide obtenu selon la préparation IV(a) et d'ester méthylique de l'acide 4-(2-aminoéthoxy)benzèneacétique, on obtient le composé attendu sous forme d'une huile blanche (rendement = 65 %).
RMN ¹H (DMSO, 250 MHz) δ : 8,38 (t, NH) ; 7,93 (dd, 2H) ; 7,54 (dd, 2H) ; 7,41 (d, 1 H) ; 7,25-7,10 (m, 4H) ; 7,02 (td, 1 H) ; 6,89 (dd, 2H) ; 4,82 (dd, 1H) ; 4,1-3,9 (m, 2H) ; 3,60 (s, 5H) ; 3,6-3,3 (m, 2H) ; 3,13 (dd, 1H) ; 2,91 (dd, 1 H).

### PREPARATION VIIb

### Acide 4-[2-[[[(2S)-2,3-dihydro-1-[(4-iodophényl)sulfonyl]-1H-indol-2-yl]carbonyl]amino]éthoxy]benzèneacétique

En opérant de manière analogue à l'exemple 22, au départ de l'ester obtenu selon la préparation VIIa, on obtient le composé attendu sous forme d'une mousse blanche (rendement = 96 %).
RMN ¹H (DMSO, 250 MHz) δ : 12,2 (sl, COOH) ; 8,41 (t, NH) ; 7,93 (dd, 2H) ; 7,54 (dd, 2H) ; 7,41 (d, 1 H) ; 7,25-7,10 (m, 4H) ; 7,02 (td, 1 H) ; 6,88 (dd, 2H) ; 4,82 (dd, 1 H) ; 4,1-3,9 (m, 2H) ; 3,48 (s, 2H) ; 3,6-3,2 (m, 2H) ; 3,13 (dd, 1H) ; 2,91 (dd, 1H).

### Exemple 33

### Acide 4-[2-[[[(2S)-2,3-dihydro-1-[[2'-(trifluorométhyl)[1,1'-biphényl]-4-yl]sulfonyl]-1H-indol-2-yl]carbonyl]amino]éthoxy]benzèneacétique

En opérant de manière analogue à l'exemple 1, au départ de l'acide obtenu selon la préparation Vllb et d'acide 2-(trifluorométhyl)phénylboronique, on obtient le composé attendu sous forme d'une mousse beige (rendement = 78 %).
RMN ¹H (DMSO 300 MHz) δ : 8,42 (t, NH) ; 7,9-7,8 (m, 3H) ; 7,8-7,6 (m, 2H) ; 7,55-7,45 (m, 3H) ; 7,39 (d, 1H) ; 7,24 (t, 1 H) ; 7,2-7,1 (m, 3H) ; 7,05 (t, 1H) ; 6,87 (d, 2H) ; 4,88 (dd, 1H) ; 4,1-3,9 (m, 2H) ; 3,6-3,15 (m, 2H) ; 3,45 (s, 2H) ; 3,2-2,85 (m, 2H).

### Exemple 34

### Acide 4-[2-[[[(2S)-1-[[4'-chloro-3'-(trifluorométhyl)[1,1'-biphényl]-4-yl]sulfonyl]-2,3-dihydro-1H-indol-2I]carbonyl]amino]éthoxy]benzène-acétique

En opérant de manière analogue à l'exemple 33, au départ d'acide 4-chloro-3-(trifluorométhyl)phénylboronique, on obtient le composé attendu sous forme d'une mousse beige (rendement = 54 %).
RMN ¹H (DMSO, 300 MHz) δ : 8,43 (t, NH) ; 8,10 (d, 1 H) ; 8,02 (dd, 1H) ; 8,00-7,8 (m, 5H) ; 7,48 (d, 1H) ; 7,24 (t, 1H) ; 7,2-7,1 (m, 3H) ; 7,03 (t, 1H) ; 6,89 (d, 2H) ; 4,89 (dd, 1H) ; 4,01 (t, 2H) ; 3,65-3,3 (m, 2H) ; 3,48 (s, 2H) ; 3,15 (dd, 1 H) ; 2,93 (dd, 1 H).

### Exemple 35

### Acide 4-[2-[[[(2S)-1-[[2'-chloro-5'-(trifluorométhyl)[1,1'-biphényl]-4-yl]sulfonyl]-2,3-dihydro-1H-indol-2l]carbonyl]amino]éthoxy]-benzèneacétique

En opérant de manière analogue à l'exemple 33, au départ d'acide 2-chloro-5-(trifluorométhyl)phénylboronique, on obtient le composé attendu sous forme d'une mousse beige (rendement = 37 %).
RMN ¹H (DMSO, 300 MHz) δ : 8,43 (t, NH) ; 7,91 (d, 1 H) ; 7,85-7,75 (m, 3H) ; 7,68 (d, 2H) ; 7,48 (d, 1H) ; 7,24 (t, 1H) ; 7,2-7,1 (m, 3H) ; 7,04 (t, 1H) ; 6,88 (d, 2H) ; 4,89 (dd, 1H) ; 4,01 (t, 2H) ; 3,65-3,3 (m, 2H) ; 3,48 (s, 2H) ; 3,16 (dd, 1H) ; 2,94 (dd, 1 H).

### Exemple 36

### Acide 4-[2-[[[(2S)-1-[[3'-chloro-4'-(trifluorométhyl)[1,1'-biphényl]-4-yl]sulfonyl]-2,3-dihydro-1H-indol-2-yl]carbonyl]amino]éthoxy]-benzèneacétique

En opérant de manière analogue à l'exemple 33, au départ d'acide 3-chloro-4-(trifluorométhyl)phénylboronique, on obtient le composé attendu sous forme d'une mousse beige (rendement = 60 %).
RMN ¹H (DMSO, 300 MHz) δ : 8,42 (t, NH) ; 8,10 (d, 1 H) ; 8,02 (dd,1 H) ; 7,95 (d, 2H) ; 7,89 (d, 2H) ; 7,83 (d, 1H) ; 7,48 (d, 1 H) ; 7,24 (t, 1 H) ; 7,2-7,1 (m, 3H) ; 7,02 (t, 1H) ; 6,89 (d, 2H) ; 4,89 (dd, 1 H) ; 4,01 (t, 2H) ; 3,65-3,3 (m, 2H) ; 3,48 (s, 2H) ; 3,15 (dd, 1 H) ; 2,94 (dd, 1H).

### Exemple 37

### Acide 4-[2-[[[(2S)-2,3-dihydro-1-[[2'-(trifluorométhoxy)[1,1'-biphényl]-4-yl]sulfonyl]-1H-indol-2-yl]carbonyl]amino]éthoxy]benzèneacétique

En opérant de manière analogue à l'exemple 33, au départ d'acide 2-(trifluorométhoxy)phénylboronique, on obtient le composé attendu sous forme d'une mousse beige (rendement = 74 %).
RMN ¹H (DMSO, 300 MHz) δ : 8,42 (t, NH) ; 7,86 (d, 2H) ; 7,65-7,45 (m, 7H) ; 7,24 (t, 1H) ; 7,2-7,1 (m, 3H) ; 7,03 (t, 1 H) ; 6,88 (d, 2H) ; 4,89 (dd, 1H) ; 4,01 (t, 2H) ; 3,65-3,3 (m, 4H) ; 3,1-2,9 (m, 2H).

### Exemple 38

### Acide 4-[2-[[[(2S)-2,3-dihydro-1-[[3'-(trifluorométhoxy)[1,1'-biphényl]-4-yl]sulfonyl]-1H-indol-2-yl]carbonyl]amino]éthoxy]benzèneacétique

En opérant de manière analogue à l'exemple 33, au départ d'acide 3-(trifluorométhoxy)phénylboronique, on obtient le composé attendu sous forme d'une mousse beige (rendement = 71 %).
RMN ¹H (DMSO, 300 MHz) δ : 12,2 (sl, COOH) ; 8,43 (t, NH) ; 7,90 (s, 4H); 7,76 (d, 1 H) ; 7,70 (s, 1 H) ; 7,63 (t, 1 H) ; 7,48 (d, 1 H) ; 7,44 (d, 1H) ; 7,24 (t, 1H) ; 7,2-7,1 (m, 3H) ; 7,02 (t, 1 H) ; 6,89 (d, 2H) ; 4,89 (dd, 1H) ; 4,02 (t, 2H) ; 3,65-3,35 (m, 2H) ; 3,48 (s, 2H) ; 3,16 (dd, 1H) ; 2,93 (dd, 1H).

### Exemple 39

### Acide 4-[2-[[[(2S)-2,3-dihydro-1-[[3'-(méthoxy)[1,1'-biphényl]-4-yl]-sulfonyl]-1H-indol-2-yl]carbonyl]amino]éthoxy]benzèneacétique

En opérant de manière analogue à l'exemple 33, au départ d'acide 3-(méthoxy)phénylboronique, on obtient le composé attendu sous forme d'une mousse marron (rendement = 69 %).
RMN ¹H (DMSO, 300 MHz) δ : 12,2 (sl, COOH) ; 8,42 (t, NH) ; 7,85 (s, 4H) ; 7,48 (d, 1H) ; 7,40 (t, 1H) ; 7,3-7,1 (m, 6H) ; 7,1-6,95 (m, 2H) ; 6,89 (d, 2H) ; 4,87 (dd, 1 H) ; 4,02 (t, 2H) ; 3,81 (s, OCH3) ; 3,65-3,35 (m, 2H) ; 3,48 (s, 2H) ; 3,16 (dd, 1H) ; 2,92 (dd, 1 H).

### Exemple 40

### Acide 4-[2-[[[(2S)-2,3-dihydro-1-[[2'-fluoro-[1,1'-biphényl]-4-yl]-sulfonyl]-1H-indol-2-yl]carbonyl]amino]éthoxy]benzèneacétique

En opérant de manière analogue à l'exemple 33, au départ d'acide 2-fluoro-phénylboronique, on obtient le composé attendu sous forme d'une mousse blanche (rendement = 71 %).
RMN ¹H (DMSO, 250 MHz) δ : 12,2 (sl, COOH) ; 8,42 (t, NH) ; 7,90 (d, 2H) ; 7,72 (d, 2H) ; 7,60-7,45 (m, 3H) ; 7,40-7,10 (m, 6H) ; 7,02 (t, 1H) ; 6,89 (d, 2H) ; 4,89 (dd, 1 H) ; 4,01 (t, 2H) ; 3,65-3,35 (m, 2H) ; 3,48 (s, 2H) ; 3,17 (dd, 1 H) ; 2,93 (dd, 1 H).

### Exemple 41

### Acide 4-[2-[[[(2S)-2,3-dihydro-1-[[2'-méthyl-[1,1'-biphényl]-4-yl]-sulfonyl]-1H-indol-2-yl]carbonyl]amino]éthoxy]benzèneacétique

En opérant de manière analogue à l'exemple 33, au départ d'acide 2-méthyl-phénylboronique, on obtient le composé attendu sous forme d'une mousse blanche (rendement = 78 %).
RMN ¹H (DMSO, 250 MHz) δ : 8,40 (t, NH) ; 7,83 (d, 2H) ; 7,55-7,4 (m, 3H) ; 7,35-7,10 (m, 8H) ; 7,03 (td, 1 H) ; 6,90 (dd, 2H) ; 4,88 (dd, 1 H) ; 4,0 (t, 2H) ; 3,48 (s, 2H) ; 3,65-3,25 (m, 2H) ; 3,13 (dd, 1 H) ; 2,93 (dd, 1H) ; 2,16 (s, CH3).

### Exemple 42

### Acide 4-[2-[[[(2S)-2,3-dihydro-1-[[3'-(1-méthyléthyl)[1,1'-biphényl]-4-yl]sulfonyl]-1H-indol-2-yl]carbonyl]amino]éthoxy] benzèneacétique

En opérant de manière analogue à l'exemple 33, au départ d'acide 3-(1-méthyléthyl)phénylboronique, on obtient le composé attendu sous forme d'une mousse beige (rendement = 79 %).
RMN ¹H (DMSO, 250 MHz) δ : 12,2 (sl, COOH) ; 8,40 (t, NH) ; 7,84 (s, 4H) ; 7,60-7,45 (m, 3H) ; 7,40 (t, 1H) ; 7,35-7,10 (m, 5H) ; 7,02 (t, 1 H) ; 6,89 (d, 2H) ; 4,87 (dd, 1 H) ; 4,01 (t, 2H) ; 3,65-3,35 (m, 2H) ; 3,48 (s, 2H) ; 3,14 (dd, 1 H) ; 3,05-2,85 (m, 2H) ; 1,23 (d, 6H).

### Exemple 43

### Acide 4-[2-[[[(2S)-2,3-dihydro-1-[[2'-(méthoxy)[1,1'-biphényl]-4-yl]-sulfonyl]-1H-indol-2-yl]carbonyl]amino]éthoxy]benzèneacétique

En opérant de manière analogue à l'exemple 33, au départ d'acide 2-(méthoxy)phénylboronique, on obtient le composé attendu sous forme d'une mousse blanche (rendement = 63 %).
RMN ¹H (DMSO, 250 MHz) δ : 12,2 (sl, COOH) ; 8,41 (t, NH) ; 7,83 (d, 2H) ; 7,64 (d, 2H) ; 7,48 (d, 1H) ; 7,45-7,1 (m, 7H) ; 7,1-7,0 (m , 2H) ; 6,88 (d, 2H) ; 4,88 (dd, 1 H) ; 4,01 (t, 2H) ; 3,75 (s, OCH3) ; 3,65-3,35 (m, 2H) ; 3,48 (s, 2H) ; 3,18 (dd, 1H) ; 2,93 (dd, 1 H).

### Exemple 44

### Acide 4-[2-[[[(2S)-2,3-dihydro-1-[[3'-chloro-[1,1'-biphényl]-4-yl]-sulfonyl]-1H-indol-2-yl]carbonyl]aminoléthoxy]benzèneacétique

En opérant de manière analogue à l'exemple 33, au départ d'acide 3-chloro-phénylboronique, on obtient le composé attendu sous forme d'une mousse beige (rendement = 79 %).
RMN ¹H (DMSO, 300 MHz) δ : 8,42 (t, NH) ; 7,88 (s, 4H) ; 7,78 (dd, 1H) ; 7,75-7,65 (m, 1 H) ; 7,55-7,45 (m, 3H) ; 7,24 (t, 1 H) ; 7,2-7,1 (m, 3H) ; 7,02 (t, 1 H) ; 6,88 (d, 2H) ; 4,88 (dd, 1H) ; 4,01 (t, 2H) ; 3,65-3,35 (m, 2H) ; 3,48 (s, 2H) ; 3,15 (dd, 1 H) ; 2,93 (dd, 1 H).

### Exemple 45

### Acide 4-[2-[[[(2S)-2,3-dihydro-1-[[4'-chloro-[1,1'-biphényl]-4-yl]-sulfonyl]-1H-indol-2-yl]carbonyl]amino]éthoxy]benzèneacétique

En opérant de manière analogue à l'exemple 33, au départ d'acide 4-chloro-phénylboronique, on obtient le composé attendu sous forme d'une mousse beige (rendement = 74 %).
RMN ¹H (DMSO, 250 MHz) δ : 8,41 (t, NH) ; 7,86 (s, 4H) ; 7,74 (dd, 2H) ; 7,52 (dd, 2H) ; 7,48 (d, 1 H) ; 7,3-7,1 (m, 4H) ; 7,01 (t, 1 H) ; 6,89 (dd, 2H) ; 4,88 (dd, 1 H) ; 4,01 (t, 2H) ; 3,65-3,35 (m, 2H) ; 3,48 (s, 2H) ; 3,15 (dd, 1 H) ; 2,93 (dd, 1 H).

### Exemple 46

### Acide 4-[2-[[[(2S)-2,3-dihydro-1-[[2'-chloro-[1,1'-biphényl]-4-yl]-sulfonyl]-1H-indol-2-yl]carbonyl]amino]éthoxy]benzèneacétique

En opérant de manière analogue à l'exemple 33, au départ d'acide 2-chloro-phénylboronique, on obtient le composé attendu sous forme d'une mousse beige (rendement = 75 %).
RMN ¹H (DMSO, 300 MHz) δ : 12,2 (sl, COOH) ; 8,43 (t, NH) ; 7,88 (d, 2H) ; 7,65-7,55 (m, 3H) ; 7,51-7,49 (m, 4H) ; 7,25 (t, 1 H) ; 7,2-7,1 (m, 3H) ; 7,03 (t, 1H) ; 6,89 (d, 2H) ; 4,89 (dd, 1 H) ; 4,01 (t, 2H) ; 3,65-3,25 (m, 2H) ; 3,48 (s, 2H) ; 3,15 (dd, 1 H) ; 2,95 (dd, 1 H).

### Exemple 47

### Acide 4-[2-[[[(2S)-2,3-dihydro-1-[[3'-fluoro-[1,1'-biphényl]-4-yl]-sulfonyl]-1H-indol-2-yl]carbonyl]amino]éthoxy]benzèneacétique

En opérant de manière analogue à l'exemple 33, au départ d'acide 3-fluoro-phénylboronique, on obtient le composé attendu sous forme d'une mousse beige (rendement = 39 %).
RMN ¹H (DMSO, 300 MHz) δ : 12,2 (sl, COOH) ; 8,42 (t, NH) ; 7,88 (s, 4H) ; 7,65-7,45 (m, 4H) ; 7,31-7,09 (m, 5H) ; 7,02 (t, 1 H) ; 6,89 (d, 2H) ; 4,89 (dd, 1 H) ; 4,01 (t, 2H) ; 3,65-3,25 (m, 2H) ; 3,48 (s, 2H) ; 3,15 (dd, 1H) ; 2,93 (dd, 1 H).

### Exemple 48

### Acide 4-[2-[[[(2S)-2,3-dihydro-1-[[2'-(trifluorométhyl)-[1,1'-biphényl]-4-yl]sulfonyl]-1H-indol-2-yl]carbonyl]amino]éthoxy]benzèneacétique

En opérant de manière analogue à l'exemple 33, au départ d'acide 2-(trifluorométhyl)-phénylboronique, on obtient le composé attendu sous forme d'une mousse beige (rendement = 78 %).
RMN ¹H (DMSO, 300 MHz) δ : 8,42 (t, NH) ; 7,9-7,8 (m, 3H) ; 7,8-7,6 (m, 2H) ; 7,55-7,45 (m, 3H) ; 7,39 (d, 1 H) ; 7,24 (t, 1H) ; 7,2-7,1 (m, 3H) ; 7,04 (td, 1 H) ; 6,87 (dd, 2H) ; 4,88 (dd, 1 H) ; 4,1-3,9 (m, 2H) ; 3,45 (s, 2H) ; 3,6-3,15 (m, 2H) ; 3,04 (dd, 1 H) ; 2,95 (dd, 1 H).

### Exemple 49

### Acide 4-[2-[[[(2S)-2,3-dihydro-1-[[3'-méthyl-[1,1'-biphényl]-4-yl]-sulfonyl]-1H-indol-2-yl]carbonyl]amino]éthoxy]benzèneacétique

En opérant de manière analogue à l'exemple 33, au départ d'acide 3-méthyl-phénylboronique, on obtient le composé attendu sous forme d'une mousse blanche (rendement = 85 %).
RMN¹H (DMSO, 300 MHz) δ : 12,2 (sl, COOH) ; 8,41 (t, NH) ; 7,84 (d, 2H) ; 7,82 (d, 2H) ; 7,55-7,4 (m, 3H) ; 7,36 (t, 1H) ; 7,3-7,1 (m, 5H) ; 7,02 (td, 1 H) ; 6,89 (dd, 2H) ; 4,88 (dd, 1H) ; 4,01 (t, 2H) ; 3,60 (s, 2H) ; 3,6-3,4 (m, 2H) ; 3,13 (dd, 1 H) ; 2,92 (dd, 1H) ; 2,36 (s, CH3).

### PREPARATION VIII

### Acide 1-[[2'-(trifluorométhyl)[1,1'-biphényl]-4-yl]sulfonyl]-(2S)-1H-indole-2-carboxylique, sel de tétrabutylammonium

Dans un tube micro-ondes, on mélange 2,4 g (5,59 mmol) d'acide obtenu selon la préparation V dans 48 ml d'eau, on ajoute 1,802g (5,59 mmol) de bromure de tétrabutylammonium, 1,062 g (5,59 mmol) d'acide 2-(trifluorométhyl)phénylboronique, 50 mg (0,224 mmol) d'acétate de palladium et 1,778 g (16,77 mmol) de carbonate de sodium. Le milieu réactionnel est chauffé pendant 5 min à 150°C au four micro-ondes. Après refroidissement, on ajoute du dichlorométhane au milieu réactionnel puis de l'eau, et la phase organique est séparée de la phase aqueuse. La phase organique est alors lavée 2 fois par de l'eau, séchée sur sulfate de magnésium et concentrée sous pression réduite. Le produit brut est purifié par cristallisation dans le toluène et on obtient le produit sous forme d'un solide blanc (rendement = 100 %).
F = 128 °C.

### Exemple 50

### 2,3-dihydro-N-[2-(4-pyridmyl)éthyl]-1-[[3'-(trifluorométhyl)[1,1'-biphényl]-4-yl]sulfonyl]-(2S)-1H-indole-2-carboxamide

En opérant de manière analogue à l'exemple 4, au départ de 4-(2-aminoéthyl)pyridine, on obtient le composé attendu sous forme d'une huile incolore (rendement = 76 %).
RMN ¹H (DMSO, 300 MHz) δ : 8,41 (dd, 2H) ; 8,29 (t, NH) ; 8,05-8,0 (m, 2H) ; 7,95 (d, 2H) ; 7,86 (d, 2H) ; 7,83-7,68 (m, 2H) ; 7,50 (d, 1 H) ; 7,26 (t, 1H) ; 7,19 (dd, 2H) ; 7,13 (d, 1H) ; 7,04 (td, 1H) ; 4,79 (dd, 1 H) ; 3,40 (dd, 2H) ; 3,06 (dd, 1H) ; 2,9-2,7 (m, 3H).

### Exemple 51

### 2,3-dihydro-N-[2-(3-pyridinyl)éthyl]-1-[[3'-(trifluorométhyl)[1,1'-biphényl]-4-yl]sulfonyl]-(2S)-1H-indole-2-carboxamide

En opérant de manière analogue à l'exemple 50, au départ de 3-(2-aminoéthyl)pyridine, on obtient le composé attendu sous forme d'une mousse jaune (rendement = 93 %).
RMN¹H (DMSO, 300 MHz) δ : 8,5-8,4 (m, 2H) ; 8,3 (t, NH) ; 8,05-8,0 (m, 2H) ; 7,94 (d, 2H) ; 7,87 (d, 2H) ; 7,85-7,68 (m, 2H) ; 7,65-7,55 (m, 1H) ; 7,50 (d, 1 H) ; 7,3-7,2 (m, 2H) ; 7,12 (d, 1 H) ; 7,06 (t, 1 H) ; 4,79 (dd, 1H) ; 3,5-3,3 (m, 2H) ; 3,2-3 (m, 1H) ; 2,9-2,7 (m, 3H).

### Exemple 52

### 2,3-dihydro-N-[(4-pyridinyl)méthyl]-1-[[3'-(trifluorométhyl)[1,1'-biphényl]-4-yl]sulfonyl]-(2S)-1H-indole-2-carboxamide

En opérant de manière analogue à l'exemple 50, au départ de 4-(aminométhyl)pyridine, on obtient le composé attendu sous forme d'une mousse blanche (rendement = 78 %).
RMN ¹H (DMSO, 250 MHz) δ :8,9 (t, NH) ; 8,49 (dd, 2H) ; 8,05-8,0 (m, 2H) ; 7,95 (d, 2H) ; 7,91 (d, 2H) ; 7,85-7,68 (m, 2H) ; 7,53 (d, 1 H) ; 7,28 (dd, 2H) ; 7,35-7,20 (m, 1 H) ; 7,15 (d, 1 H) ; 7,05 (t, 1 H) ; 4,92 (dd, 1H) ; 4,38 (d, 2H) ; 3,21 (dd, 1 H) ; 2,99 (dd, 1 H).

### Exemple 53

### 2,3-dihydro-N-[(3-pyridinyl)méthyl]-1-[[3'-(trifluorométhyl)[1,1'-biphényl]-4-yl]sulfonyl]-(2S)-1H-indole-2-carboxamide

En opérant de manière analogue à l'exemple 50, au départ de 3-(aminométhyl)pyridine, on obtient le composé attendu sous forme d'une mousse blanche (rendement = 74 %).
RMN ¹H (DMSO, 250 MHz) δ : 8,86 (t, NH) ; 8,51 (d, 1 H) ; 8,46 (dd, 1H) ; 8,05-8,0 (m, 2H) ; 7,94 (d, 2H) ; 7,90 (d, 2H) ; 7,85-7,65 (m, 3H) ; 7,52 (d, 1H) ; 7,36 (dd, 1 H) ; 7,25 (t, 1 H) ; 7,15 (d, 1 H) ; 7,07 (td, 1 H) ; 4,89 (dd, 1H) ; 4,38 (d, 2H) ; 3,19 (dd, 1 H) ; 2,97 (dd, 1 H).

### Exemple 54

### Acide 3-[2-[[[(2S)-2,3-dihydro-1-[[3'-(trifluorométhyl)[1,1'-biphényl]-4-yl]sulfonyl]-1H-indol-2-yl]carbonyl]amino]éthoxy]benzdique, méthyl ester

En opérant de manière analogue à l'exemple 4, au départ d'ester méthylique de l'acide 3-(2-aminoéthoxy)benzoïque, on obtient le composé attendu sous forme d'un solide rose (rendement = 28 %).
RMN ¹H (DMSO, 250 MHz) δ : 8,43 (t, NH) ; 8,1-8.0 (m, 2H) ; 7,94 (d, 2H) ; 7,89 (d, 2H) ; 7,85-7,65 (m, 2H) ; 7,.6-7,4 (m, 4H) ; 7,3-7,15 (m, 2H) ; 7,12 (d, 1 H) ; 7,02 (td, 1H) ; 4,89 (dd, 1 H) ; 4,10 (t, 2H) ; 3,85 (s, 3H) ; 3,7-3,4 (m, 2H) ; 3,15 (dd, 1H) ; 2.93 (dd, 1 H).

### Exemple 55

### Acide 3-[2-[[[(2S)-2,3-dihydro-1-[[3'-(trifluorométhyl)[1,1'-biphényl]-4-yl]sulfonyl]-1H-indol-2-yl]carbonyl]amino]éthoxy]benzoïque

En opérant de manière analogue à l'exemple 22, au départ de l'ester obtenu selon l'exemple 54, on obtient le composé attendu sous forme d'un solide blanc (rendement = 60 %).
PF = 85-92.

### PREPARATION IX

### Acide 2,3-dihydro-2-[[[2-[4-(2-méthoxy-2-oxoéthyl)phénoxy]-éthyl]-amino]carbonyl]-(2S)-1H-indole-1-carboxylique, 1,1-diméthyl-éthyl ester

En opérant de manière analogue à la préparation 1, au départ d'ester méthylique de l'acide 4-(2-aminoéthoxy)benzèneacétique, on obtient le composé attendu sous forme d'une mousse blanche (rendement = 48 %).

### PREPARATION X

### Acide 4-[2-[[[(2S)-2,3-dihydro-1H-indol-2-yl]carbonyl]amino]éthoxy]-benzèneacétique, méthyl ester

En opérant de manière analogue à la préparation II au départ de l'ester obtenu selon la préparation IX, on obtient le composé attendu sous forme d'un solide beige (rendement = 89 %).
RMN ¹H (DMSO, 250 MHz) δ : 8,03 (t, NH) ; 7,16 (dd, 2H) ; 7,05-6,80 (m, 4H) ; 6,65-6,5 (m, 2H) ; 5,93 (d, NH) ; 4,22 (ddd, 1H) ; 3,99 (t, 2H) ; 3,59 et 3,58 (2d, 5H) ; 3,55-3,4 (m, 2H) ; 3,31 (dd, 1 H) ; 2,90 (dd, 1 H).

### PREPARATION XI

### Acide 4-[2-[[[(2S)-1-[[4-bromo-3-(trifluorométhyl)phényl]sulfonyl]-2,3-dihydro-1H-indol-2-yl]carbonyl]amino]éthoxy]benzèneacétique, méthyl ester

On prépare une solution de 750 mg (2,12 mmol) du composé obtenu selon la préparation X dans 60 ml d'acétonitrile et on ajoute 512 µL de N-méthylmorpholine (4,65 mmol) et 0,486 ml (2,75 mmol) de chlorure de 4-bromo-3-(trifluorométhyl)benzènesulfonyle. Le mélange réactionnel est agité à température ambiante pendant 4 heures. Le milieu est alors traité par addition d'acétate d'éthyle et la phase organique est lavée par de l'eau. La phase organique est ensuite séchée sur sulfate de magnésium, puis concentrée sous pression réduite. Le produit brut obtenu est ensuite purifié par chromatographie sur gel de silice en éluant à l'aide d'un mélange dichlorométhane/acétate d'éthyle (99/1 puis 90/10 ; v/v). On obtient ainsi le produit attendu sous forme d'une mousse blanche (rendement = 93 %).
RMN ¹H (DMSO, 300 MHz) δ : 8,46 (t, NH) ; 8,11 (d, 1 H) ; 8,05-7,95 (m, 2H) ; 7,43 (d, 1 H) ; 7,25 (td, 1H) ; 7,2-7,1 (m, 3H) ; 7,06 (td, 1H) ; 6,89 (dd, 2H) ; 4,93 (dd, 1H) ; 4,0 (t, 2H) ; 3,60 (s, 5H) ; 3,6-3,35 (m, 2H) ; 3,17 (dd, 1H); 2,92(dd, 1 H).

### Exemple 56

### Acide 4-[2-[[[(2S)-2,3-dihydro-1-[[2-(trifluorométhyl)[1,1'-biphényl]-4-yl]sulfonyl]-1H-indol-2-yl]carbonyl]amino]éthoxy]benzèneacétique, méthyl ester

En opérant de manière analogue à l'exemple 2, au départ du composé obtenu selon la préparation XI et d'acide phénylboronique, on obtient le composé attendu sous forme d'une huile translucide (rendement = 79 %).

### Exemple 57

### Acide 4-[2-[[[(2S)-2,3-dihydro-1-[[2-(trifluorométhyl)[1,1'-biphényl]-4-yl]sulfonyl]-1H-indol-2-yl]carbonyl]amino]éthoxy]benzèneacétique

En opérant de manière analogue à l'exemple 5 au départ du composé obtenu selon l'exemple 56, on obtient le composé attendu sous forme d'une mousse blanche (rendement = 36 %).
RMN ¹H (DMSO, 250 MHz) δ : 12,2 (sl, COOH) ; 8,48 (t, NH) ; 8,13 (dd, 1H) ; 8,08 (s, 1H) ; 7,62 (d, 1 H) ; 7,55-7,4 (m, 4H) ; 7,48-7,1 (m, 6H) ; 7,06 (td, 1 H) ; 6,89 (d, 2H) ; 4,97 (dd, 1 H) ; 4,01 (t, 2H) ; 3,6-3,3 (m, 2H) ; 3,48 (s, 2H) ; 3,20 (dd, 1 H) ; 2,95 (dd, 1 H).

### Exemple 58

### Acide 3-[2-[[[(2S)-2,3-dihydro-1-[[3'-(trifluorométhyl)[1,1'-biphényl]-4-yl]sulfonyl]-1H-indol-2-yl]carbonyl]amino]éthoxy]benzèneacétique, méthyl ester

En opérant de manière analogue à l'exemple 4, au départ d'ester méthylique de l'acide 3-(2-aminoéthoxy)benzèneacétique, on obtient le composé attendu sous forme d'une huile incolore (rendement = 33 %).
RMN ¹H (DMSO, 300 MHz) δ : 8,43 (t, NH) ; 8,08-8 (m, 2H) ; 7,94 (d, 2H), 7,89 (d, 2H) ; 7,85-7,65 (m, 2H) ; 7,49 (d, 1H) ; 7,3-7,2 (m, 2H) ; 7,13 (d, 1H) ; 7,02 (td, 1 H) ; 6,9-6,8 (m, 3H) ; 4,90 (dd, 1 H) ; 4,02 (t, 2H) ; 3,64 (s, 2H) ; 3,61 (s, 3H) ; 3,6-3,4 (m, 2H) ; 3,16 (dd, 1 H) ; 2,94 (dd, 1 H).

### Exemple 59

### Acide 3-[2-[[[(2S)-2,3-dihydro-1-[[3'-(trifluorométhyl)[1,1'-biphényl]-4-yl]sulfonyl]-1H-indol-2-yl]carbonyl]amino]éthoxy]benzèneacétique

En opérant de manière analogue à l'exemple 5, au départ de l'ester obtenu selon l'exemple 58, on obtient le composé attendu sous forme d'une mousse blanche (rendement = 85 %).
RMN ¹H (DMSO, 250 MHz) δ : 8,42 (t, NH) ; 8,08-8 (m, 2H) ; 7,94 (d, 2H), 7,89 (d, 2H) ; 7,85-7,65 (m, 2H) ; 7,49 (d, 1 H) ; 7,3-7,2 (m, 2H) ; 7,13 (d, 1 H) ; 7,02 (td, 1 H) ; 6,9-6,8 (m, 3H) ; 4,90 (dd, 1 H) ; 4,02 (t, 2H) ; 3,52 (s, 2H) ; 3,65-3,3 (m, 2H) ; 3,15 (dd, 1 H) ; 2,94 (dd, 1 H).

### Exemple 60

### 2,3-dihydro-N-[(2-nitrophényl)méthyl]-1-[[3'-(trifluorométhyl)[1,1'-biphényl]-4-yl]sulfonyl]-(2S)-1H indole-2-carboxamide

En opérant de manière analogue à l'exemple 21, au départ de 2-(aminométhyl)nitrobenzène, on obtient le composé attendu sous forme d'une mousse blanche (rendement = 71 %).
RMN ¹H (DMSO, 250 MHz) δ : 8,89 (t, NH) ; 8,1-8,0 (m, 3H) ; 8,0-7,85 (m, 4H) ; 7,85-7,65 (m, 3H) ; 7,65-7,45 (m, 3H) ; 7,3-7,1 (m, 2H) ; 7,04 (dd, 1 H) ; 4,97 (dd, 1H) ; 4,68 (dd, 1 H) ; 4,60 (dd, 1H) ; 3,21 (dd, 1 H) ; 2,98 (dd, 1 H).

### Exemple 61

### M-[(2-aminophényl)méthyl]-2,3-dihydro-1-[[3'-(trifluorométhyl)[1,1'-biphényl]-4-yl]sulfonyl]-(2S)-1H-indole-2-carboxamide

683 mg (1,17 mmol) du composé obtenu selon l'exemple 60 sont dissous dans 10 ml de méthanol. On additionne 68 mg de charbon palladié à 10 %. Le milieu réactionnel est agité à température ambiante sous pression atmosphérique d'hydrogène pendant 2 h. Le milieu réactionnel est alors filtré, et le filtrat est concentré sous pression réduite. Le produit brut obtenu est ensuite purifié par chromatographie sur gel de silice en éluant à l'aide d'un mélange dichlorométhane/acétate d'éthyle (98/2 ; v/v). On obtient ainsi le produit attendu sous forme d'une mousse blanche (rendement = 80 %).
RMN ¹H (DMSO, 300 MHz) δ : 8,64 (t, NH) ; 8,08-8 (m, 2H) ; 7,94 (d, 2H), 7,89 (d, 2H) ; 7,85-7,65 (m, 2H) ; 7,50 (d, 1 H) ; 7,25 (t, 1 H) ; 7,13 (d, 1 H) ; 7,1-6,9 (m, 3H) ; 6,63 (dd, 1H) ; 6,51 (td, 1 H) ; 5,03 (sl, NH2) ; 4,91 (dd, 1H) ; 4,24 (dd, 1H) ; 4,14 (dd, 1 H) ; 3,17 (dd, 1 H) ; 2,96 (dd, 1 H).

### PREPARATION XII

### Acide 2-cyanobenzènebutanoïque, méthyl ester

On prépare, sous atmosphère d'argon, une solution de 369 mg (3,02 mmol) de 9-BBN (9-borabicyclo[3.3.1]nonane) dans 6 ml de THF anhydre. A cette solution, on additionne 0,32 ml (3,02 mmol) d'ester méthylique de l'acide 3-butènoïque. Le milieu est agité 3 h à température ambiante. Dans un second ballon, on prépare une solution de 500 mg (2,75 mmol) de 2-bromobenzonitrile dans 8 ml de THF anhydre. A cette solution sous argon, on ajoute successivement 445 mg (8,24 mmol) de méthylate de sodium, 68 mg (0,08 mmol) de PdCl₂dppf et la solution précédente au goutte à goutte. Le milieu réactionnel est alors porté au reflux pendant 2 h, puis agité à température ambiante pendant 16 h, et enfin au reflux pendant 3 h. On additionne de l'eau et de l'acétate d'éthyle au milieu réactionnel, les deux phases sont séparées. La phase organique est lavée par de l'eau, séchée sur sulfate de magnésium, filtrée et concentrée sous pression réduite. Le produit brut obtenu est ensuite purifié par chromatographie sur gel de silice en éluant à l'aide d'un mélange méthylcyclohexane/acétate d'éthyle (90/10 ; v/v). On obtient ainsi le produit attendu sous forme d'une huile incolore (rendement = 36 %).

RMN ¹H (DMSO, 300 MHz) δ : 7,78 (dd, 1 H) ; 7,62 (td, 1 H) ; 7,47 (dd, 1 H), 7,41 (td, 1 H) ; 3,58 (s, CH3) ; 2,81 (t, 2H) ; 2,36 (t, 2H) ; 1,88 (quint, 2H).

### PREPARATION XIII

### Acide 2-(aminométhyl)benzènebutanoïque, méthyl ester

On prépare une solution de 176 mg (0,866 mmol) d'ester obtenu selon la préparation XII dans 15 ml de méthanol et 0,1 ml d'acide chlorhydrique 10N. On additionne 20 mg de charbon palladié à 10 % et on agite 6 h à température ambiante sous pression atmosphérique d'hydrogène. Le milieu réactionnel est alors filtré, et le filtrat est concentré sous pression réduite. Le produit brut obtenu est ensuite purifié par chromatographie sur gel de silice en éluant à l'aide d'un mélange dichlorométhane/ méthanol (95/5 ; v/v). On obtient ainsi le produit attendu sous forme d'une huile incolore (rendement = 50 %).
RMN ¹H (DMSO, 250 MHz) δ : 7,5-7,35 (m, 1H) ; 7,35-7,1 (m, 3H) ; 3,97 (s, 2H) ; 3,60 (s, CH3) ; 2,64 (t, 2H) ; 2,39 (t, 2H) ; 1,76 (quint, 2H).

### Exemple 62

### Acide 2-[[[[(2S)-2,3-dihydro-1-[[3'-(triftuorométhyl)[1,1'-biphényl]-4-yl]sulfonyl]-1H-indol-2-yl]carbonyl]amino]méthyl]benzènebutanoïque, méthyl ester

En opérant de manière analogue à l'exemple 21, au départ de l'ester obtenu selon la préparation XIII, on obtient le composé attendu sous forme d'une huile incolore (rendement = 48 %).
RMN ¹H (DMSO, 500 MHz) δ : 8,65 (t, NH) ; 8,08-8 (m, 2H) ; 7,93 (d, 2H), 7,91 (d, 2H) ; 7,77 (d, 1H) ; 7,73 (t, 1 H) ; 7,51 (d, 1 H) ; 7,3-7,1 (m, 6H) ; 7,05 (t, 1H) ; 4,93 (dd, 1H) ; 4,41 (dd, 1 H) ; 4,33 (dd, 1 H) ; 3,60 (s, CH3) ; 3,17 (dd, 1 H) ; 2,97 (dd, 1 H) ; 2,64 (t, 2H) ; 2,38 (t, 2H) ; 1,80 (quint, 2H).

### Exemple 63

### Acide 2-[[[[(2S)-2,3-dihydro-1-[[3'-(trifluorométhyl)[1,1'-biphényl]-4-yl]sulfonyl]-1H-indol-2-yl]carbonyl]amino]méthyl]benzènebutanoïque

En opérant de manière analogue à l'exemple 5, au départ de l'ester méthylique obtenu selon l'exemple 62, on obtient le composé attendu sous forme d'un solide blanc (rendement = 81 %).
RMN ¹H (DMSO, 300 MHz) δ: 12,1 (sl ; COOH) ; 8,67 (t, NH) ; 8,08-8 (m, 2H) ; 7,94 (d, 2H), 7,90 (d, 2H) ; 7,85-7,65 (m, 2H) ; 7,50 (d, 1 H) ; 7,3-7,1 (m, 6H) ; 7,03 (td, 1H) ; 4,92 (dd, 1 H) ; 4,41 (dd, 1H) ; 4,32 (dd, 1 H) ; 3,17 (dd, 1H) ; 2,96 (dd, 1H) ; 2,63 (t, 2H) ; 2,28 (t, 2H) ; 1,76 (quint, 2H).

### Exemple 64

### 2,3-dihydro-N-[(2-hydroxyphényl)méthyl]-1-[[3'-(trifluorométhyl)[1,1'-biphényl]-4-yl]sulfonyl]-(2S)-1H-indole-2-carboxamide

On prépare une solution de 100 mg (0,224 mmol) d'acide obtenu selon la préparation IV dans 2 ml de dichlorométhane puis on ajoute 47 mg (0,246 mmol) de EDCI et 33 mg (0,246 mmol) de HOBT. Après 10 minutes sous agitation à température ambiante, on ajoute 28 mg (0,224 mmol) de 2-(aminométhyl)phénol. Le milieu réactionnel est ensuite agité à température ambiante pendant 1 heure 50 min. Le milieu est alors traité par addition de dichlorométhane et la phase organique est lavée par de l'eau. La phase organique est ensuite séchée sur sulfate de magnésium, puis concentrée sous pression réduite et on obtient ainsi le produit attendu sous forme d'une mousse blanche (rendement = 99 %).
RMN¹H (DMSO, 300 MHz) δ: 9,55 (s, OH) ; 8,54 (t, NH) ; 8,1-8 (m, 2H) ; 7,94 (d, 2H) ; 7,90 (d, 2H) ; 7,85-7,65 (m, 2H) ; 7,51 (d, 1 H) ; 7,25 (t, 1H) ; 7,2-7,00 (m, 4H) ; 6,81 (dd, 1 H) ; 6,73 (td, 1 H) ; 4,97 (dd, 1 H) ; 4,33 (dd, 1 H) ; 4,23 (dd, 1 H) ; 3,15 (dd, 1 H) ; 2,98 (dd, 1 H).

### Exemple 65

### 2,3-dihydro-N-[(2-cyanophényl)méthyl]-1-[[3'-(trifluorométhyl)[1,1'-biphényl]-4-yl]sulfonyl]-(2S)-1H-indole-2-carboxamide

En opérant de manière analogue à l'exemple 21, au départ de 2-(aminométhyl)benzonitrile, on obtient le composé attendu sous forme d'un solide rose (rendement = 23 %).
F = 70°C.

### PREPARATION XIV

### Acide (3-cyanophénoxy)acétique, méthyl ester

On prépare une solution de 5,95 g (50 mmol) de 3-hydroxy-benzonitrile dans 60 ml d'acétone et on ajoute 9,2 g (60 mmol) de bromoacétate de méthyle, 6,9 g de carbonate de potassium et 0,15 g d'iodure de sodium. Le mélange est agité à la température de reflux du solvant pendant 3 heures, puis concentré sous pression réduite. Le résidu est repris à l'eau et extrait à l'acétate d'éthyle. La phase organique est séchée sur sulfate de sodium puis concentrée sous pression réduite. Le produit brut est recristallisé une première fois dans l'éther isopropylique, puis dans 30 ml d'un mélange éthanol/eau (1/1). On obtient ainsi 6 g de l'ester attendu sous forme de cristaux blancs (rendement = 62 %).
F = 66°C.

### PREPARATION XV

### Acide 3-(aminométhyl)phénoxyacétique, méthyl ester, chlorhydrate

On prépare une solution de 1,9 g (10 mmol) d'ester méthylique de l'acide (3-cyanophénoxy)acétique dans 60 ml de méthanol et 1 ml d'acide chlorhydrique 10 N. On ajoute 0,2 g de charbon palladié à 10 % et on agite le mélange sous atmosphère d'hydrogène, à température ambiante et à pression atmosphérique, pendant 2 heures. Le catalyseur est éliminé par filtration et le filtrat est concentré sous pression réduite. Le produit brut est repris dans 10 ml de méthanol et la solution est diluée par 50 ml d'éther éthylique. Le produit ainsi cristallisé est séparé par filtration et séché sous pression réduite. On obtient ainsi 2 g de l'ester attendu sous forme de cristaux blancs (rendement = 86 %).
F = 118°C.

### Exemple 66

### Acide [3-[[[[(2S)-2,3-dihydro-1-[[3'-(trifluorométhyl)[1,1'-biphényl]-4-yl]sulfonyl]-1H-indol-2-yl]carbonyl]amino]méthyl]phénoxy]acétique, méthyl ester

En opérant de façon analogue à la préparation 1, au départ de l'acide obtenu à la préparation IV et de l'amine obtenue selon la préparation XV, on obtient le produit attendu sous forme d'un solide blanc (rendement = 47 %).
F = 50°C.

### Exemple 67

### Acide [4-[[[[(2S)-2,3-dihydro-1-[[3'-(trifluorométhyl)[1,1'-biphényi]-4-yl]sulfonyl]-1H-indol-2-yl]carbonyl]amino]méthyl]phénoxy]acétique, méthyl ester

En opérant de façon analogue à l'exemple 66, au départ de l'ester méthylique de l'acide (4-aminométhyl)phénoxyacétique, on obtient le produit attendu sous forme d'une poudre blanche (rendement = 27 %).
RMN 1H (DMSO, 300 MHz) δ : 8,70 (t, NH) ; 8,03 (m, 2H) ; 7,96-7,90 (m, 4H) ; 7,88-7,80 (m, 2H) ; 7,49 (d, 1 H) ; 7,25-7,10 (m,4H) ; 7,24 (t, 1 H) ; 6,90 (d, 2H) ; 4,91 (dd, 1 H) ; 4,77 (s, 2H) ; 4,27 (m, 2H) ; 3,69 (s, 3H) ; 3,3-3,1 (m, 1 H) ; 3,00-2,93 (m, 1 H).

### PREPARATION XVI

### N-(4-cyanophényl)-β-alanine

On prépare une solution de 3,54 g (30 mmol) de 4-amino-benzonitrile dans 10 ml d'acétonitrile et on ajoute, à température de reflux du solvant, 2,16 g (30 mmol) de propiolactone. Le mélange est agité à la température de reflux du solvant pendant 18 heures, puis refroidi. Le résidu est repris à l'eau et extrait à l'acétate d'éthyle. Le précipité blanc formé est séparé par filtration et lavé par 5 ml d'éther éthylique, puis séché. On obtient ainsi 3,4 g de l'acide attendu sous forme d'un solide blanc (rendement = 59 %).
F = 150°C.

### PREPARATION XVII

### N-(4-cyanophényl)-β-alanine, méthyl ester, chlorhydrate

On prépare une solution de 2 g (10,5 mmol) d'acide obtenu selon la préparation XVI dans 50 ml de méthanol et on ajoute, à 0°C, 2,5 g (21 mmol) de chlorure de thionyle. Le mélange est ensuite agité pendant 2 heures à température de reflux du solvant, puis concentré sous pression réduite. Le résidu est repris par du toluène et à nouveau concentré sous pression réduite. On obtient ainsi 2,72 g du sel d'ester attendu sous forme d'un solide blanc (rendement = 76 %).
F = 121°C.

### PREPARATION XVIII

### N-[4-(aminométhyl)phényl]-β-alanine, méthyl ester, dichlorhydrate

En opérant de façon analogue à la préparation XV, au départ du composé obtenu selon la préparation XVII, on obtient le produit attendu sous forme d'un solide blanc (rendement = 99 %).
F=201°C.

### Exemple 68

### N-[4-[[[[(2S)-2,3-dihydro-1-[[3'-(trifluorométhyl)[1,1'-biphényl]-4-yl]-sulfonyl]-1H-indol-2-yl]carbonyl]amino]méthyl]phényl]-β-alanine, méthyl ester

En opérant de façon analogue à l'exemple 66, au départ du composé obtenu selon la préparation XVIII, on obtient le produit attendu sous forme d'un solide blanc (rendement = 57 %).
F= 61°C.

### Exemple 69

### N-[4-[[[[(2S)-2,3-dihydro-1-[[3'-(trifluorométhyl)[1,1'-biphényl]-4-yl]sulfonyl]-1H-indol-2-yl]carbonyl]amino]méthyl]phényl]-β-alanine

En opérant de façon analogue à l'exemple 5, au départ du composé obtenu selon l'exemple 68, on obtient le produit attendu sous forme d'un solide blanc (rendement = 62 %).
F = 82°C.

### PREPARATION XIX

### N-(3-cyanophényl)-β-alanine

En opérant de façon analogue à la préparation XVI, au départ de 3-aminobenzonitrile, on obtient le produit attendu sous forme d'un solide écru (rendement = 14 %).
F= 119°C.

### PREPARATION XX

### N-(3-cyanophényl)-β-alanine, méthyl ester, chlorhydrate

En opérant de façon analogue à la préparation XVII, au départ du composé obtenu selon la préparation XIX, on obtient le produit attendu sous forme d'un solide blanc écru (rendement = 99 %).
F = 102°C.

### PREPARATION XXI

### N-[3-(aminométhyl)phényl]-β-alanine, méthyl ester, dichlorhydrate

En opérant de façon analogue à la préparation XVIII, au départ du composé obtenu selon la préparation XX, on obtient le produit attendu sous forme d'une huile jaune (rendement = 84 %).
RMN ¹H (DMSO, 300 MHz) δ : 8,40 (s large, 3 H) ; 7,14 (t, 1 H) ; 6,77-6,67 (m, 3H) ; 3,89 (m, 2H) ; 3,64 (s, 3H) ; 3,39 (t, 2H) ; 2,71 (t, 2 H).

### Exemple 70

### N-[3-[[[[(2S)-2,3-dihydro-1-[[3'-(trifluorométhyl)[1,1'-biphényl]-4yl]-sulfonyl]-1H-indol-2-yl]carbonyl]amino]méthyl]phényl]-β-alanine, méthyl ester

En opérant de façon analogue à l'exemple 66, au départ du composé obtenu selon la préparation XXI, on obtient le produit attendu sous forme d'un solide blanc (rendement = 25 %).
F = 57°C.

### Exemple 71

### N-[3-[[[[(2S)-2,3-dihydro-1-[[3'-(trifluorométhyl)[1,1'-biphényl]-4-yl]sulfonyl]-1H-indol-2-yl]carbonyl]amino]méthyl]phényl]-β-alanine

En opérant de façon analogue à l'exemple 5, au départ du composé obtenu selon l'exemple 70, on obtient le produit attendu sous forme d'un solide blanc (rendement = 94 %).
F= 85°C.

### PREPARATION XXII

### N-(2-cyanophényl)-β-alanine, méthyl ester

En opérant de façon analogue à la préparation XVII, au départ de *N-*(2-cyanophényl)-β-alanine, on obtient le produit attendu sous forme d'une huile jaune (rendement = 70 %).
RMN ¹H (DMSO, 250 MHz) δ : 7,47-7,39 (m, 2H) ; 6,80 (d, 1 H) ; 6,66 (t, 1 H) ; 6,05 (t, 1H) ; 3,66 (s, 3H) ; 3,44 (q, 2H) ; 2,63 (t, 2 H).

### PREPARATION XXIII

### N-[2-(aminométhyl)phényl]-β-alanine, méthyl ester, chlorhydrate

En opérant de façon analogue à la préparation XV, au départ du composé obtenu selon la préparation XXII, on obtient le produit attendu sous forme d'un solide jaune pâle (rendement = 44 %).
RMN ¹H (DMSO, 250 MHz) δ : 8,16 (s large, 3 H) ; 7,23-7,17 (m, 2H) ; 6,67 (d, 1 H) ; 6,62 (m, 1 H) ; 5,59 (s large, 1 H) ; 3,90 (s, 2H) ; 3,62 (s, 3H) ; 3,31 (t, 2H) ; 2,67 (t, 2H).

### Exemple 72

### N-[2-[[[[(2S)-2,3-dihydro-1-[[3'-(trifluorométhyl)[1,1'-biphényl]-4-yl]-sulfonyl]-1H-indol-2-yl]carbonyl]amino]méthyl]phényl]-β-alanine, méthyl ester

En opérant de façon analogue à l'exemple 66, au départ du composé obtenu selon la préparation XXIII, on obtient le produit attendu sous forme d'un solide blanc (rendement = 73 %).
F= 60°C.

### Exemple 73

### N-[2-[[[[(2S)-2,3-dihydro-1-[[3'-(trifluorométhyl)[1,1'-biphényl]-4-yl]-sulfonyl]-1H-indol-2-yl]carbonyl]amino]méthyl]phényl]-β-alanine

En opérant de façon analogue à l'exemple 5, au départ du composé obtenu selon l'exemple 72, on obtient le produit attendu sous forme d'un solide blanc (rendement = 79 %).
F = 94°C.

### PREPARATION XXIV

### Acide 3-(2-cyanophénoxy)propanoïque

En opérant de façon analogue à la préparation XVI, au départ de 2-hydroxybenzonitrile, on obtient le produit attendu sous forme d'un solide brun (rendement = 90 %).
RMN ¹H (DMSO, 300 MHz) δ : 12,42 (s large, 1 H) ; 7,73-7,66 (m, 2H) ; 7,29 (d, 1 H) ; 7,10 (t, 1 H) ; 4,36 (t, 2H) ; 2,77 (t, 2H).

### PREPARATION XXV

### Acide 3-(2-cyanophénoxy)propanoïque, méthyl ester

En opérant de façon analogue à la préparation XVII, au départ du composé obtenu selon la préparation XXIV, on obtient le produit attendu sous forme d'une huile jaune (rendement = 50 %).
RMN ¹H (DMSO, 250 MHz) δ: 7,73-7,63 (m, 2H) ; 7,29 (d, 1 H) ; 7,10 (t, 1H) ; 4,36 (t, 2H) ; 3,64 (s, 3H) ; 2,86 (t, 2H).

### PREPARATION XXVI

### Acide 3-[2-(aminométhyl)phénoxy]propanoïque, méthyl ester, chlorhydrate

En opérant de façon analogue à la préparation XV, au départ du composé obtenu selon la préparation XXV, on obtient le produit attendu sous forme d'un solide blanc écru (rendement = 54 %).
RMN ¹H (DMSO, 300 MHz) δ : 8,2 (s large, 3H) ; 7,41-7,34 (m, 2H) ; 7,3 (d, 1 H) ; 7,02 (t, 1 H) ; 4,26 (t, 2H) ; 3,91 (s, 2H) ; 3,65 (s, 3H) ; 2.88 (t, 2H).

### Exemple 74

### Acide 3-[2-[[[[(2S)-2,3-dihydro-1-[[3'-(trifluorométhyl)[1,1'-biphényl]-4-yl]sulfonyl]-1H-indol-2-yl]carbonyl]amino]méthyl]phénoxy] propanoïque, méthyl ester

En opérant de façon analogue à l'exemple 66, au départ du composé obtenu selon la préparation XXVI, on obtient le produit attendu sous forme d'un solide blanc (rendement = 25 %).
F=131°C.

### Exemple 75

### Acide 3-[2-[[[[(2S)-2,3-dihydro-1-[[3'-(trifluorométhyl)[1,1'-biphényl]-4-yl]sulfonyl]-1H-indol-2-yl]carbonyl]amino]méthyl]phénoxy]-propanoïque

On prépare un mélange de 22 mg (0,52 mmol) de lithine et 53 mg (1,57 mmol) d'eau oxygénée à 30 % dans 2 ml de THF (tétrahydrofurane) et on ajoute une solution de 278 mg (0,43 mmol) du composé obtenu selon l'exemple 74 dans 2 ml de THF. Le mélange réactionnel est agité à température ambiante pendant 20 heures, puis acidifié à l'aide d'une solution diluée d'acide chlorhydrique et extrait par 25 ml de dichlorométhane. La phase organique est lavée à l'eau, séchée sur sulfate de sodium et concentrée sous pression réduite. Le produit brut obtenu est purifié par chromatographie sur gel de silice en éluant à l'aide d'un mélange dichlorométhane/méthanol (9/1 ; v/v). On obtient ainsi 141 mg de l'acide attendu sous forme d'une poudre blanche (rendement = 52 %).
F = 100°C.

### PREPARATION XXVII

### Acide [[3-[[bis[(1,1-diméthyléthoxy)carbonyl]amino]méthyl]phényl]thio]acétique, méthyl ester

On prépare une suspension de 44 mg (1,1 mmol) d'hydrure de sodium à 60 % dans l'huile dans 8 ml de diméthylformamide (DMF) et on ajoute 238 mg (1,1 mmol) d'iminodicarboxylate de di-t-butyle. Le mélange réactionnel est agité à 60°C pendant 30 mn puis refroidi à température ambiante. On ajoute alors une solution de 230 mg (1 mmol) d'ester méthylique de l'acide [3-(chlorométhyl)phénylthio]acétique dans 3 ml de DMF. Le milieu réactionnel est agité à 50°C pendant 90 mn, puis refroidi et versé sur 30 ml d'eau. La phase aqueuse obtenue est extraite par l'acétate d'éthyle (2 fois) et les phases organiques rassemblées sont séchées sur sulfate de sodium et concentrées sous pression réduite. Le produit brut est purifié par chromatographie sur gel de silice en éluant à l'aide d'un mélange toluène/acétate d'éthyle (95/5 ; v/v). On obtient ainsi 300 mg du composé attendu sous forme d'une huile incolore (rendement = 73 %).
RMN ¹H (DMSO, 250 MHz) δ: 7,34-7,25 (m, 1 H) ; 7,18 (s, 1 H) ; 7,07 (d, 1 H) ; 4,64 (s, 2H) ; 3,86 (s, 2H) ; 3,61 (s, 3H) ; 1,39 (s, 18).

### PREPARATION XXVIII

### Acide [[3-(aminométhyl)phényl]thio]acétique, méthyl ester

On prépare une solution de 290 mg (0,7 mmol) du composé obtenu selon la préparation XXVII dans 4 ml de dioxane saturé de chlorure d'hydrogène. Le mélange réactionnel est agité pendant 2 heures à température ambiante puis concentré sous pression réduite. Le produit brut est cristallisé par trituration dans l'éther éthylique. On obtient ainsi 150 mg du composé attendu sous forme d'un solide blanc (rendement = 88 %).
F = 72°C.

### Exemple 76

### Acide [[3-[[[[(2S)-2,3-dihydro-1-[[3'-(trifluorométhyl)[1,1'-biphényl]-4-yl]sulfonyl]-1H-indol-2-yl]carbonyl]amino]méthyl]phényl]thio]acétique, méthyl ester

En opérant de façon analogue à l'exemple 66, au départ du composé obtenu selon la préparation XXVIII, on obtient le produit attendu sous forme d'un solide blanc (rendement = 63 %).
F=51°C.

### Exemple 77

### Acide [[3-[[[[(2S)-2,3-dihydro-1-[[3'-(trifluorométhyl)[1,1'-biphényl]-4-yl]sulfonyl]-1H-indol-2-yl]carbonyl]amino]méthyl]phényl]thio]acétique

En opérant de façon analogue à l'exemple 5, au départ du composé obtenu selon l'exemple 76, on obtient le produit attendu sous forme d'un solide blanc (rendement = 98 %).
F = 75°C.

### Exemple 78

### Acide 4-[[[[(2S)-2,3-dihydro-1-[[3'-(trifluorométhyl)[1,1'-biphényl]-4-yl]sulfonyl]-1H-indol-2-yl]carbonyl]amino]méthyl)benzoïque, méthyl ester

En opérant de façon analogue à l'exemple 66, au départ du chlorhydrate de l'ester méthylique de l'acide 4-(aminométhyl)benzoïque, on obtient le produit attendu sous forme d'un solide blanc (rendement = 51 %).
F = 70°C.

### Exemple 79

### Acide 4-[[[[(2S)-2,3-dihydro-1-[[3'-(trifluorométhyl)[1,1'-biphényl]-4-yl]sulfonyl]-1H-indol-2-yl]carbonyl]amino]méthyl]benzoïque

En opérant de façon analogue à l'exemple 5, au départ du composé obtenu selon l'exemple 78, on obtient le produit attendu sous forme d'un solide blanc (rendement = 50 %).
F = 120°C.

### Exemple 80

### Acide 3-[[[[(2S)-2,3-dihydro-1-[[3'-(trifluorométhyl)[1,1'-biphényl]-4-yl]sulfonyl]-1H-indol-2-yl]carbonyl]amino]méthyl]benzoïque, méthyl ester

En opérant de façon analogue à l'exemple 66, au départ du chlorhydrate de l'ester méthylique de l'acide 3-(aminométhyl)benzoïque, on obtient le produit attendu sous forme d'un solide blanc (rendement = 56 %).
F = 50°C.
RMN ¹H (DMSO, 300 MHz) δ : 8,84 (t, NH) ; 8,03 (m, 2H) ; 7,96-7,75 (m, 8H) ; 7,56-748 (m, 3H) ; 7,25 (t, 1 H) ; 7,14 (d, 1H) ; 7,05 (t, 1 H) ; 4,921 (dd, 1 H) ; 4,41 (m, 2H) ; 3,84 (s, 3H) ; 3,2-3,1 (m, 1H) ; 3,0-2,93 (m, 1 H).

### Exemple 81

### Acide 3-[[[[(2S)-2,3-dihydro-1-[[3'-(trifluorométhyl)[1,1'-biphényl]-4-yl]-sulfonyl]-1H-indol-2-yl]carbonyl]amino]méthyl]benzoïque

En opérant de façon analogue à l'exemple 5, au départ du composé obtenu selon l'exemple 80, on obtient le produit attendu sous forme d'un solide blanc (rendement = 82 %).
F = 96°C.

### PREPARATION XXIX

### Acide 2-(aminométhyl)benzoïque, 1,1-diméthyléthyl ester, chlorhydrate

En opérant de façon analogue à la préparation XV au départ de l'ester *t-*butylique de l'acide 2-cyanobenzoïque, on obtient le produit attendu sous forme d'un solide blanc (rendement = 87 %).
F = 177°C.

### Exemple 82

### Acide 2-[[[[(2S)-2,3-dihydro-1 -[[3'-(trif luorométhyl)[1,1'-biphényl]-4-yl]sulfonyl]-1H-indol-2-yl]carbonyl]amino]méthyl]benzoïque, 1,1-diméthyléthyl ester

En opérant de façon analogue à l'exemple 66, au départ du composé obtenu selon la préparation XXIX, on obtient le produit attendu sous forme d'un solide blanc (rendement = 69 %).
F = 68°C.

### Exemple 83

### Acide 2-[[[[(2S)-2,3-dihydro-1-[[3'-(trifluorométhyl)[1,1'-biphényl]-4-yl]-sulfonyl]-1H-indol-2-yl]carbonyl]amino]méthyl]benzoïque

En opérant de façon analogue à l'exemple 25, au départ du composé obtenu selon l'exemple 82, on obtient le produit attendu sous forme d'un solide blanc (rendement = 90 %).
F=118°C.

### Exemple 84

### Acide 4-[[[[(2S)-2,3-dihydro-1-[[3'-(trifluorométhyl)[1,1'-biphényl]-4-yl]-sulfonyl]-1H-indol-2-yl]carbonyl]amino]méthyl]benzèneacétique, méthyl ester

En opérant de façon analogue à l'exemple 66, au départ du chlorhydrate de l'ester méthylique de l'acide 4-(aminométhyl)-benzèneacétique, on obtient le produit attendu sous forme d'un solide blanc (rendement = 83 %).
F = 120°C.

### Exemple 85

### Acide 4-[[[[(2S)-2,3-dihydro-1-[[3'-(trifluorométhyl)[1,1'-biphényl]-4-yl]-sulfonyl]-1H-indol-2-yl]carbonyl]amino]méthyl]benzèneacétique

En opérant de façon analogue à l'exemple 5, au départ du composé obtenu selon l'exemple 84, on obtient le produit attendu sous forme d'un solide blanc (rendement = 56 %).
F = 90°C.

### Exemple 85a

### Acide 4-[[[[(2S)-2,3-dihydro-1-[[3'-(trifluorométhyl)[1,1'-biphényl]-4-yl]-sulfonyl]-1H-indol-2-yl]carbonyl]amino]méthyl]benzèneacétique, sel de potassium

On prépare une solution de 250 mg (0,42 mmol) de l'acide obtenu selon l'exemple 85 dans 1 ml d'acétonitrile et on ajoute 0,84 ml d'une solution aqueuse de potasse 0,5N. Le mélange est agité 1 heure à température ambiante puis dilué par 10 ml d'eau pure et lyophilisé. On obtient ainsi le sel attendu sous forme d'une poudre blanche.F = 150-160°C.

### Exemple 85b

### Acide 4-[[[[(2S)-2,3-dihydro-1-[[3'-(trifluorométhyl)[1,1'-biphényl]-4-yl]-sulfonyl]-1H-indol-2-yl]carbonyl]amino]méthyl]benzèneacétique, sel de diéthanolamine

En opérant de façon analogue à l'exemple 85a, avec de la diéthanolamine, on obtient le sel attendu sous forme d'une poudre blanche.
F = 55-60°C.

### Exemple 85c

### Acide 4-[[[[(2S)-2,3-dihydro-1-[[3'-(trifluorométhyl)[1,1'-biphényl]-4-yl]-sulfonyl]-1H-indol-2-yl]carbonyl]amino]méthyl]benzèneacétique, sel de choline

En opérant de façon analogue à l'exemple 85a, avec une solution d'hydroxyde de choline dans le méthanol, on obtient le sel attendu sous forme d'une poudre blanche.
F = 75-80°C.

### Exemple 85d

### Acide 4-[[[[(2S)-2,3-dihydro-1-[[3'-(trifluorométhyl)[1,1'-biphényl]-4-yl]-sulfonyl]-1H-indol-2-yl]carbonyl]amino]méthyl]benzèneacétique, sel de TRIS

En opérant de façon analogue à l'exemple 85a, avec du 2-amino-2-(hydroxyméthyl)-1,3-propanediol, on obtient le sel attendu sous forme d'une poudre blanche.
F = 66-70°C.

### Exemple 86

### Acide 3-[[[[(2S)-2,3-dihydro-1-[[3'-(trifluorométhyl)[1,1'-biphényl]-4-yl]-sulfonyl]-1H-indol-2-yl]carbonyl]amino]méthyl]benzèneacétique, méthyl ester

En opérant de façon analogue à l'exemple 66, au départ du chlorhydrate de l'ester méthylique de l'acide 3-(aminométhyl)benzèneacétique, on obtient le produit attendu sous forme d'un solide blanc (rendement = 66 %).
F = 60°C.

### Exemple 87

### Acide 3-[[[[(2S)-2,3-dihydro-1-[[3'-(trifluorométhyl)[1,1'-biphényl]-4-yl]-sulfonyl]-1H-indol-2-yl]carbonyl]amino]méthyl]benzèneacétique

En opérant de façon analogue à l'exemple 5, au départ du composé obtenu selon l'exemple 86, on obtient le produit attendu sous forme d'un solide blanc (rendement = 62 %).
F = 80°C.

### PREPARATION XXX

### Acide 4-(aminométhyl)benzènepropanoïque, méthyl ester, chlorhydrate

En opérant de façon analogue à la préparation XV au départ de l'ester méthylique de l'acide (2*E*)-3-(4-cyanophényl)-2-propènoïque, on obtient le produit attendu sous forme d'un solide blanc (rendement = 72 %).
F = 215°C.

### Exemple 88

### Acide 4-[[[[(2S)-2,3-dihydro-1-[[3'-(trifluorométhyl)(1,1'-biphényl]-4-yl]-sulfonyl]-1H-indol-2-yl]carbonyl]amino]méthyl]benzène propanoïque, méthyl ester

En opérant de façon analogue à l'exemple 66, au départ du composé obtenu selon la préparation XXX, on obtient le produit attendu sous forme d'un solide blanc (rendement = 91 %).
F = 61°C.

### Exemple 89

### Acide 4-[[[[(2S)-2,3-dihydro-1-[[3'-(trifluorométhyl)[1,1'-biphény]-4-yl]-sulfonyl]-1H-indol-2-yl]carbonyl]amino]méthyl]benzène propanoïque

En opérant de façon analogue à l'exemple 5, au départ du composé obtenu selon l'exemple 88, on obtient le produit attendu sous forme d'un solide blanc (rendement = 53 %).
F = 85°C

### Exemple 90

### Acide 3-[[[[(2S)-2,3-dihydro-1-[[3'-(trifluorométhyl)[1,1'-biphényl]-4-yl]-sulfonyl]-1H-indol-2-yl]carbonyl]amino]méthyl]benzènepropanoïque, éthyl ester

En opérant de façon analogue à l'exemple 66, au départ du chlorhydrate de l'ester éthylique de l'acide 3-[3-(aminométhyl)benzène]propanoïque, on obtient le produit attendu sous forme d'une huile incolore (rendement = 51 %).
RMN¹H (DMSO, 250 MHz) δ : 8,73 (t, NH) ; 8,02 (m, 2H) ; 7,93 (m, 4H) ; 7,77-7,72 (m, 2H) ; 7,50 (d, 1 H) ; 7,25-7,03 (m, 7H) ; 4,91 (dd, 1 H) ; 4,31 (d, 2H) ; 4,02 (q, 2H) ; 3,2-2,9 (m, 2H) ; 2,81 (t, 2H) ; 2,58 (t, 2H) ; 1,14 (t, 3H).

### Exemple 91

### Acide 3-[[[[(2S)-2,3-dihydro-1-[[3'-(trifluorométhyl)[1,1'-biphényl]-4-yl]sulfonyl]-1H-indol-2-yl]carbonyl]amino]méthyl]benzène propanoïque

En opérant de façon analogue à l'exemple 5, au départ du composé obtenu selon l'exemple 90, on obtient le produit attendu sous forme d'un solide blanc (rendement = 81 %).
F = 135°C.

### PREPARATION XXXI

### Acide 2-(aminométhyl)benzènepropanoïque, éthyl ester, chlorhydrate

En opérant de façon analogue à la préparation XXX au départ de l'ester éthylique de l'acide (2*E*)-3-(2-cyanophényl)-2-propènoïque, on obtient le produit attendu sous forme d'un solide blanc (rendement = 40 %).
F = 107-110°C.

### Exemple 92

### Acide 2-[[[[(2S)-2,3-dihydro-1-[[3'-(trifluorométhyl)[1,1'-biphényl]-4-yl]-sulfonyl]-1H-indol-2-yl]carbonyl]amino]méthyl]benzène propanoïque, éthyl ester

En opérant de façon analogue à l'exemple 66, au départ du composé obtenu selon la préparation XXXI, on obtient le produit attendu sous forme d'un solide blanc (rendement = 60 %).
F = 48°C.

### Exemple 93

### Acide 2-[[[[(2S)-2,3-dihydro-1-[[3'-(trifluorométhyl)[1,1'-biphényl]-4-yl]-sulfonyl]-1H-indol-2-yl]carbonyl]amino]méthyl]benzène propanoïque

En opérant de façon analogue à l'exemple 5, au départ du composé obtenu selon l'exemple 92, on obtient le produit attendu sous forme d'un solide blanc (rendement = 77 %).
F = 94°C.

### PREPARATION XXXII

### Acide 4-[4-[[bis[(1,1-diméthyléthoxy)carbonyl]amino]méthyl]phényl]-butanoïque, méthyl ester

En opérant de façon analogue à la préparation XXVII au départ de l'ester méthylique de l'acide 4-(chlorométhyl)benzènebutanoïque, on obtient le produit attendu sous forme d'une huile incolore (rendement = 36 %).

RMN ¹H (DMSO, 250 MHz) δ: 7,14 (s, 4H) ; 4,66 (s, 2H) ; 3,56 (s, 3H) ; 2,56 (t, 2H) ; 2,27 (t, 2H) ; 1,79 (q, 2H) ; 1,39 (s, 18H).

### PREPARATION XXXIII

### Acide 4-(aminométhyl)benzènebutanoïque, méthyl ester, chlorhydrate

En opérant de façon analogue à la préparation XXVIII au départ du composé obtenu selon la préparation XXXII, on obtient le produit attendu sous forme d'un solide blanc (rendement = 80 %).
F = 205°C.

### Exemple 94

### Acide 4-[[[[(2S)-2,3-dihydro-1-[[3'-(trifluorométhyl)[1,1'-biphényl]-4-yl]-sulfonyl]-1H-indol-2-yl]carbonyl]amino]méthyl]benzènebutanoïque, méthyl ester

En opérant de façon analogue à l'exemple 66, au départ du composé obtenu selon la préparation XXXIII, on obtient le produit attendu sous forme d'un solide blanc (rendement = 71 %).
F = 55°C.

### Exemple 95

### Acide 4-[[[[(2S)-2,3-dihydro-1-[[3'-(trifluorométhyl)[1,1'-biphény]-4-yl]-sulfonyl]-1H-indol-2-yl]carbonyl]amino]méthyl]benzènebutanoïque

En opérant de façon analogue à l'exemple 5, au départ du composé obtenu selon l'exemple 94, on obtient le produit attendu sous forme d'un solide blanc (rendement = 91 %).
F = 75°C.

### PREPARATION XXXIV

### Acide 3-(aminométhyl)benzènebutanoïque, méthyl ester, chlorhydrate

En opérant de façon analogue à la préparation XV au départ de l'ester méthylique de l'acide 3-cyanobenzènebutanoïque, on obtient le produit attendu sous forme d'un solide blanc (rendement = 84 %).
F = *90*°C.

### Exemple 96

### Acide 3-[[[[(2S)-2,3-dihydro-1-[[3'-(trifluorométhyl)[1,1'-biphényl]-4-yl]sulfonyl]-1H-indol-2-yl]carbonyl]amino]méthyl]benzènebutanoïque, méthyl ester

En opérant de façon analogue à l'exemple 66, au départ du composé obtenu selon la préparation XXXIV, on obtient le produit attendu sous forme d'une huile incolore (rendement = 81 %).
RMN¹H (DMSO, 300 MHz) δ : 8,72 (t, NH) ; 8,02 (m, 2H) ; 7,93 (m, 4H) ; 7,78-7,72 (m, 2H) ; 7,50 (d, 1H) ; 7,25-7,03 (m, 7H) ; 4,91 (dd, 1 H) ; 4,31 (d, 2H) ; 3,57 (s, 3H) ; 3,2-3,1 (m, 1 H) ; 3,0-294 (m, 1 H) ; 2,55 (t, 2H) ; 2,32 (t, 2H) ; 1,80 (m, 2H).

### Exemple 97

### Acide 3-[[[[(2S)-2,3-dihydro-1-[[3'-(trifluorométhyl)[1,1'-biphényl]-4-yl]-sulfonyl]-1H-indol-2-yl]carbonyl]amino]méthyl]benzènebutanoïque

En opérant de façon analogue à l'exemple 5, au départ du composé obtenu selon l'exemple 96, on obtient le produit attendu sous forme d'un solide blanc (rendement = 92 %).
F = 76°C.

### PREPARATION XXXV

### Chlorure de l'acide (2S)-2,3-dihydro-1-[[3'-(trifluorométhyl)[1,1'-biphényl]-4-yl]sulfonyl]-1H-indole-2-carboxylique.

En opérant de façon analogue à la préparation VI, au départ de l'acide obtenu selon la préparation IV, on obtient le produit attendu sous forme d'une huile incolore (rendement = 98 %).

### PREPARATION XXXVI

### Acide 4-[2-[[(phénylméthoxy)carbonyl]amino]éthoxy]benzène acétique, 1,1-diméthyléthyl ester

On refroidit à -30°C une solution de 3 g (9,1 mmol) d'acide 4-[2-[[(phénylméthoxy)carbonyl]amino]éthoxy]benzèneacétique dans 60 ml de chloroforme et on ajoute 1,18 g (10,9 mmol) de chloroformiate d'éthyle et 1,52 ml (10,9 mmol) de triéthylamine. Le mélange est agité pendant 1 heure à -30°C, et on ajoute ensuite 1,63 g (22 mmol) de t-butanol et 1,52 ml (10,9 mmol) de triéthylamine. Le mélange est agité pendant 16 heures à température ambiante puis concentré sous pression réduite. Le résidu d'évaporation est repris dans 100 ml d'acétate d'éthyle et la phase organique obtenue est lavée à l'eau puis séchée sur sulfate de magnésium et concentrée sous pression réduite. Le produit brut est purifié par chromatographie sur gel de silice en éluant à l'aide d'un mélange dichlorométhane/méthylcyclohexane (1/1 ; v/v). On obtient ainsi 930 mg du composé attendu (rendement = 26 %).
RMN ¹H (DMSO, 300 MHz) δ : 7,46 (t, 1H); 7,38-7,28 (m, 5H); 7,13 (d, 2H); 6,86 (d, 2H); 5,03 (s, 2H); 3,96 (t, 2H); 3,45 (s, 2H); 3,36 (q, 2H); 1,38 (s, 9H).

### PREPARATION XXXVII

### Acide 4-[2-(amino)éthoxy]benzèneacétique, 1,1-diméthyléthyl ester

On ajoute 91 mg de charbon palladié à 10 % à une solution de 910 mg (2,36 mmol) du composé obtenu selon la préparation XXXVI dans 15 ml de THF et 1 ml d'eau et on agite ce mélange sous atmosphère d'hydrogène, à température ambiante et pression atmosphérique pendant 1 heure. Le catalyseur est ensuite éliminé par filtration et le filtrat est concentré sous pression réduite. On obtient ainsi 575 mg du produit attendu sous forme d'une huile incolore (rendement = 97 %).
RMN ¹H (DMSO, 300 MHz) δ : 7,15 (d, 2H); 6,93 (d, 2H); 3,91 (t, 2H); 6,89 (d, 2H); 2,85 (t, 2H); 3,45 (s, 2H); 1,38 (s, 9H); (protons NH2 non visibles).

### Exemple 98

### Acide 4-[2-[[[(2S)-2,3-dihydro-1-[[3'-(trifluorométhyl)[1,1'-biphényl]-4-yl]sulfonyl]-1H-indol-2-yl]carbonyl]amino]éthoxy]benzèneacétique, 1,1-diméthyléthyl ester

On prépare une solution de 1,1 g (2,35 mmol) du chlorure d'acide obtenu selon la préparation XXXV dans 15 ml de dichlorométhane et on ajoute 575 mg (4,72 mmol) d'amine obtenue selon la préparation XXXVII et 0,658 ml de triéthylamine. Le mélange réactionnel est agité pendant 3 heures à température ambiante puis concentré sous pression réduite. Le résidu d'évaporation est purifié par chromatographie sur gel de silice en éluant à l'aide d'un mélange toluène/acétate d'éthyle (9/1 v/v). On obtient ainsi 1,2 g du composé attendu sous forme d'un solide amorphe beige (rendement =77%).
RMN ¹H (DMSO, 250 MHz) δ : 8,42 (t, 1 H); 8,02 (m, 2H); 7,95-7,72 (m, 6H); 7,48 (d, 1H); 7,16-7,11 (m, 5H); 7,03 (t, 1H); 6,89 (d,2H); 4,89 (dd, 1H); 4,01 (t, 2H); 3,57-3,42 (m, 4H); 3,20-2,89 (m, 2H); 1,38 (s, 9H).

### Exemple 99

### Acide 4-[2-[[[(2S)-2,3-dihydro-1-[[3'-(trifluorométhyl)[1,1'-biphényl]-4-yl]sulfonyl]-1H-indol-2-yl]carbonyl]amino]éthoxy]benzèneacétique

En opérant de façon analogue à l'exemple 25, au départ du composé obtenu selon l'exemple 98, on obtient le produit attendu sous forme d'un solide blanc (rendement = 97 %).
F = 112-113°C.

### Exemple 100

### Acide 4-[2-[[[(2S)-2,3-dihydro-1-[[3'-(trifluorométhyl)[1,1'-biphényl]-4-yl]sulfonyl]-1H-indol-2-yl]carbonyl]amino]éthoxy]benzoïque

On prépare une solution de 233 mg (1,288 mmol) d'acide 4-(2-aminoéthoxy)benzoïque dans 3,73 ml d'eau et 2,13 ml de triéthylamine. On ajoute ensuite, à température ambiante, 12,8 ml d'acétonitrile puis 300 mg (0,644 mmol) du chlorure d'acide obtenu selon la préparation XXXV en solution dans 2,13 ml de dichlorométhane. Le mélange réactionnel est agité pendant 3 heures à température ambiante puis concentré sous pression réduite. Le résidu d'évaporation est repris dans du dichlorométhane et lavé par une solution à 3 % d'acide acétique. La phase organique est concentrée sous pression réduite et le produit brut obtenu est purifié par chromatographie en phase inverse sur silice greffée RP18, en éluant à l'aide d'un mélange acétonitrile/eau (55/45; v/v). On obtient ainsi 350 mg du produit attendu sous forme d'un solide blanc (rendement = 89 %).
F = 95-100°C.

### Exemple 101

### Acide 4-[2-[[[(2S)-2,3-dihydro-1-[[3'-(trifluorométhyl)[1,1'-biphényl]-4-yl]sulfonyl]-1H-indol-2-yl]carbonyl]amino]éthyl]benzèneacétique, 1,1-diméthyléthyl ester

On prépare une solution de 177 mg (0,75 mmol) d'ester *t*-butylique de l'acide 4-(2-aminoéthyl)benzèneacétique dans 3 ml de dichlorométhane. On ajoute ensuite, à température ambiante, 385 mg (équivalent à 1 mmol) de morpholine supportée sur copolymère styrène-divinylbenzène, puis 350 mg (0,75 mmol) du chlorure d'acide obtenu selon la préparation XXXV en solution dans 2,13 ml de dichlorométhane. Le mélange réactionnel est agité pendant 3 heures à température ambiante puis filtré et concentré sous pression réduite. Le produit brut obtenu est purifié par chromatographie sur gel de silice, en éluant à l'aide d'un mélange toluène/acétate d'éthyle (8/2 v/v). On obtient ainsi 430 mg du produit attendu sous forme d'une huile (rendement = 95 %).
RMN ¹H (DMSO, 300 MHz) δ : 8,23 (t, 1 H); 8,02-7,72 (m, 8H); 7,49 (d, 1H); 7,25 (t, 1 H); 7,13-7,11 (m, 5H); 7,03 (t, 1 H); 4,80 (dd, 1 H); 3,50 (s, 2H); 3,38-3,29 (m, 2H); 3,07 (dd, 1 H); 2,84 (dd, 1 H); 2,72 (t, 2H); 1,39 (s, 9H).

### Exemple 102

### Acide 4-[2-[[[(2S)-2,3-dihydro-1-[[3'-(trifluorométhyl)[1,1'-biphényl]-4-yl]sulfonyl]-1H-indol-2-yl]carbonyl]amino]éthyl]benzèneacétique

En opérant de façon analogue à l'exemple 25, au départ du composé obtenu selon l'exemple 101, on obtient le produit attendu sous forme d'un solide blanc (rendement = 69 %).
F=110°C.

### Exemple 103

### Acide 3-[2-[[[(2S)-2,3-dihydro-1-[[3'-(trifluorométhyl)[1,1'-biphényl]-4-yl]sulfonyl]-11-Nindol-2-yl]carbonyl]amino]éthyl]benzèneacétique, 1,1-diméthyléthyl ester

En opérant de façon analogue à l'exemple 98, au départ d'ester t-butylique de l'acide 3-(2-aminoéthyl)benzèneacétique, on obtient le produit attendu sous forme d'une pâte blanche (rendement = 95 %).
RMN ¹H (DMSO, 300 MHz) δ : 8,26 (t, 1 H); 8,03-7,72 (m, 8H); 7,49 (d, 1 H); 7,25-7,03 (m, 7H); 4,81 (dd, 1 H); 3,51 (s, 2H); 3,36-3,30 (m, 2H); 3,21 (dd, 1 H); 2,90 (dd, 1 H); 2,73 (t, 2H); 1,38 (s, 9H).

### Exemple 104

### Acide 3-[2-[[[(2S)-2,3-dihydro-1-[[3'-(trifluorométhyl)[1,1'-biphényl]-4-yl]sulfonyl]-1H-indol-2-yl]carbonyl]amino]éthyl]benzèneacétique

En opérant de façon analogue à l'exemple 25, au départ du composé obtenu selon l'exemple 103, on obtient le produit attendu sous forme d'un solide blanc (rendement = 84 %).
F = 90°C.

### Exemple 105

### (2S)-2,3-dihydro-N-[2-(4-pyridinyloxy)éthyl]-1-[[3'-(trifluorométhyl) [1,1'-biphényl]-4-yl]sulfonyl]-1H-indole-2-carboxamide

En opérant de façon analogue à l'exemple 98, au départ de 2-(4-pyridinyloxy)éthanamine, on obtient le produit attendu sous forme d'une pâte blanche (rendement = 37 %).
RMN ¹H (DMSO, 300 MHz) δ : 8,45 (t, 1 H); 8,39-8,36 (m, 2H); 8,03-7,72 (m, 8H); 7,48 (d, 1 H); 7,27 (t, 1 H); 7,13 (d, 1 H); 7,03 (d, 1 H); 6,98-6,95 (m, 2H); 4,87 (dd, 1 H); 4,13 (t, 2H); 3,61-3,44 (m, 2H); 3,15 (dd, 1 H); 2,93 (dd, 1 H).

### Exemple 106

### (2S)-2,3-dihydro-N-[2-(3-pyridinyloxy)éthyl]-1-[[3'-(trifluorométhyl) [1,1'-biphényl]-4-yl]sulfonyl]-1H-indole-2-carboxamide

En opérant de façon analogue à l'exemple 98, au départ de 2-(3-pyridinyloxy)éthanamine, on obtient le produit attendu sous forme d'une mousse blanche (rendement = 63 %).
RMN ¹H (DMSO, 300 MHz) δ: 8,48 (t, 1 H); 8,30 (dd, 1 H); 8,17 (m, 1 H); 8,03-7,65 (m, 8H); 7,50-7,02 (m, 6H); 4,89 (dd, 1 H); 4,12 (t, 2H); 3,58-3,50 (m, 2H); 3,15 (dd, 1 H); 2,93 (dd, 1 H).

### Exemple 107

### Acide γ-[[[(2S)-2,3-dihydro-1-[[3'-(trifluorométhyl)[1,1'-biphényl]-4-yl] sulfonyl]-1H-indol-2-yl]carbonyl]amino]-(γR)-benzènepentanoïque

En opérant de façon analogue à l'exemple 100, au départ d'acide γ(R)-aminobenzènepentanoïque, on obtient le produit attendu sous forme d'un solide blanc (rendement = 50 %).
F = 82-84°C.

### Exemple 108

### Acide α-[[[[(2S)-2,3-dihydro-1-[[3'-(trifluorométhyl)[1,1'-biphényl]-4-yl] sulfonyl]-1H-indol-2-yl]carbonyl]amino]méthyl]benzènepropanoïque

En opérant de façon analogue à l'exemple 100, au départ d'acide α-(aminométhyl)benzènepropanoïque, on obtient le produit attendu sous forme d'un solide blanc (rendement = 48 %).
F = 85-90°C.

### Exemple 109

### Acide 7-[[[(2S)-2,3-dihydro-1-[[3'-(trifluorométhyl)[1,1'-biphényl]-4-yl] sulfonyl]-1H-indol-2-yl]carbonyl]amino]heptanoïque

En opérant de façon analogue à l'exemple 100, au départ d'acide 7-aminoheptanoïque, on obtient le produit attendu sous forme d'un solide blanc (rendement = 14 %).
F = 65-70°C.

### Exemple 110

### Acide 9-[[[(2S)-2,3-dihydro-1-[[3'-(trifluorométhyl)[1,1'-biphényl]-4-yl] sulfonyl]-1H-indol-2-yl]carbonyl]amino]nonanoïque

En opérant de façon analogue à l'exemple 100, au départ d'acide 9-aminononanoïque, on obtient le produit attendu sous forme d'un solide blanc (rendement = 23 %).
F = 60°C.

### Exemple 111

### Acide β-[[[[(2S)-2,3-dihydro-1-[[3'-(trifluorométhyl)[1,1'-biphényl]-4-yl] sulfonyl]-1H-indol-2-yl]carbonyl]amino]méthyl]benzènepropanoïque

On prépare un mélange de 38,5 mg (0,215 mmol) d'acide β-(aminométhyl)benzènepropanoïque, 2 ml de THF, 23 mg (0,217 mmol) de carbonate de sodium et 0,7 mg (0,02mmol) de bromure de dodécyltriméthylammonium. Le mélange est agité pendant 5 mn à température ambiante et on ajoute 100 mg (0,215 mmol) du chlorure d'acide obtenu selon la préparation XXXV. Le milieu réactionnel est agité à température ambiante pendant 2 heures, puis à température de reflux du solvant pendant 16 heures, puis refroidi et filtré. Le solide résiduel est lavé avec 5 ml de THF et les filtrats rassemblés sont concentrés sous pression réduite. Le produit brut est purifié par chromatographie en phase inverse sur silice greffée RP18 en éluant à l'aide d'un mélange eau/acétonitrile (45/55 v/v). On obtient ainsi 26 mg du composé attendu sous forme d'un solide blanc (rendement = 20 %).
F = 90°C.

### Exemple 112

### Acide β-[[[(2S)-2,3-dihydro-1-[[3'-(trifluorométhyl)[1,1'-biphényl]-4-yl]-sulfonyl]-1H-indol-2-yl]carbonyl]amino]-(βS)-benzènebutanoïque, 1,1-diméthyléthyl ester

En opérant de façon analogue à l'exemple 98, au départ d'ester t-butylique de l'acide (βs)-β-aminobenzènebutanoïque, on obtient le produit attendu sous forme d'un solide blanc (rendement = 67 %).
F = 50°C.

### Exemple 113

### Acide β-[[[(2S)-2,3-dihydro-1-[[3'-(trifluorométhyl)[1,1'-biphényl]-4-yl]-sulfonyl]-1H-indol-2-yl]carbonyl]amino]-(βS)-benzènebutanoïque

En opérant de façon analogue à l'exemple 25, au départ du composé obtenu selon l'exemple 112, on obtient le produit attendu sous forme d'un solide blanc (rendement = 98 %).
F = 78-82°C.

### PREPARATION XXXVIII

### Acide 5-(4-cyanophénoxy)pentanoïque, méthyl ester

On prépare une solution de 408,6 mg (3,43 mmol) de 4-hydroxybenzonitrile dans 15 ml d'acétonitrile et on ajoute 1,12 g (3,43 mmol) de carbonate de césium, puis 803 mg (4,12 mmol) d'ester méthylique de l'acide 5-bromopentanoïque. Le mélange réactionnel est agité pendant 16 heures à température ambiante puis concentré sous pression réduite. Le résidu d'évaporation est repris dans l'acétate d'éthyle et la phase organique obtenue est lavée à l'eau, puis séchée et concentrée. Le produit brut est purifié par chromatographie sur gel de silice en éluant à l'aide d'un mélange dichlorométhane/méthylcyclohexane (6/4 v/v). On obtient ainsi le produit attendu sous forme d'un solide pâteux (rendement = 71 %).

RMN ¹H (DMSO, 250 MHz) δ : 7,78-7,72 (m, 2H); 7,12-7,06 (m, 2H); 4,06 (t, 2H); 3,58 (s, 3H); 2,38 (t, 2H); 1,78-1,63 (m, 4H).

### PREPARATION XXXIX

### Acide 5-[4-(aminométhyl)phénoxy]pentanoïque , méthyl ester (chlorhydrate)

En opérant de façon analogue à la préparation XV, en présence d'acide chlorhydrique, au départ du composé obtenu selon la préparation XXXVIII, on obtient le produit attendu sous forme d'un solide blanc (rendement = 83%).
F = 198-200°C.

### Exemple 114

### Acide 5-[4-[[[[(2S)-2,3-dihydro-1-[[3'-(trifluorométhyl)[1,1'-biphényl]-4-yl]sulfonyl]-1H-indol-2-yl]carbonyl]amino]méthyl]phénoxy] pentanoïque, méthyl ester

En opérant de façon analogue à l'exemple 98, au départ du composé obtenu selon la préparation XXXIX, on obtient le produit attendu sous forme d'un solide blanc (rendement = 98 %).
F = 50°C.

### Exemple 115

### Acide 5-[4-[[[[(2S)-2,3-dihydro-1-[[3'-(trifluorométhyl)[1,1'-biphényl]-4-yl]sulfonyl]-1H-indol-2-yl]carbonyl]amino]méthyl]phénoxy] pentanoïque

En opérant de façon analogue à l'exemple 5, au départ du composé obtenu selon l'exemple 114, on obtient l'acide attendu sous forme d'un solide blanc (rendement = 98 %).
F = 70°C.

### PREPARATION XL

### Acide 4-(3-cyanophénoxy)butanoïque, méthyl ester

En opérant de façon analogue à la préparation XXXVIII, au départ de 3-hydroxybenzonitrile et d'ester méthylique de l'acide 4-bromobutanoïque, on obtient l'ester attendu sous forme d'un liquide orange (rendement = 98%).
RMN ¹H (DMSO, 250 MHz) δ : 7,49 (t, 1 H); 7,40-7,37 (m, 2H); 7,29-7,22 (m, 1 H); 4,05 (t, 2H); 3,60 (s, 3H); 2,51-2,45 (m, 2H); 2,05-1,93 (m, 2H).

### PREPARATION XLI

### Acide 4-[3-(aminométhyl)phénoxy]butanoïque, méthyl ester (chlorhydrate)

En opérant de façon analogue à la préparation XXXIX, au départ du composé obtenu selon la préparation XL, on obtient l'ester attendu sous forme d'un solide blanc (rendement = 87 %).
F = 104°C.

### Exemple 116

### Acide 4-[3-[[[[(2S)-2,3-dihydro-1-[[3'-(trifluorométhyl)[1,1'-biphényl]-4-yl]sulfonyl]-1H-indol-2-yl]carbonyl]amino]méthyl]phénoxy] butanoïque, méthyl ester

En opérant de façon analogue à l'exemple 98, au départ du composé obtenu selon la préparation XLI, on obtient le produit attendu sous forme d'un solide blanc (rendement = 96 %).
F = 50°C.

### Exemple 117

### Acide 4-[3-[[[[(2S)-2,3-dihydro-1-[[3'-(trifluorométhyl)[1,1'-biphényl]-4-yl]sulfonyl]-1H-indol-2-yl]carbonyl]amino]méthyl]phénoxy] butanoïque

En opérant de façon analogue à l'exemple 5, au départ du composé obtenu selon l'exemple 116, on obtient l'acide attendu sous forme d'un solide blanc (rendement = 97 %).
F = 70°C.

### PREPARATION XLII

### Acide 2-(2-aminoéthoxy)benzèneacétique, 1-méthyléthyl ester (chlorhydrate)

On prépare une solution de 865 mg (4,45 mmol) d'ester isopropylique de l'acide 2-hydroxybenzèneacétique dans 5 ml d'acétonitrile et on ajoute 1,45 g (4,45 mmol) de carbonate de césium. Le mélange est agité pendant 10 mn à température ambiante, puis on ajoute 500 mg (2,23 mmol) de (2-bromoéthyl)carbamate de t-butyle. Le milieu réactionnel est agité à température ambiante pendant 16 heures puis concentré sous pression réduite. Le résidu d'évaporation est ensuite traité par 4,7 ml d'acide trifluoroacétique pendant 2 heures à température ambiante, puis dilué avec de l'eau et de l'acétate d'éthyle. Le mélange est neutralisé par une solution de soude et extrait par l'acétate d'éthyle. La phase organique obtenue est séchée sur sulfate de magnésium et concentrée partiellement sous pression réduite. Le produit brut est fixé sur une silice greffée sulfonique (Varian Megabond SCX) et relargué par une solution d'ammoniaque dans le méthanol. Le produit attendu est obtenu sous forme de base par concentration du liquide d'élution, puis salifié par une solution de chlorure d'hydrogène dans l'éther éthylique. On obtient ainsi le sel attendu sous forme d'une huile jaune (rendement = 61 %).
RMN¹H (DMSO, 300 MHz) δ: 8,21 (s, 3H); 7,28-7,17 (m, 2H); 7,00-6,91 (m, 2H); 4,90 (hep, 1 H); 4,17 (t, 2H; 3,60 (s, 2H); 3,15 (hex, 2H); 1,18 (d, 6H).

### Exemple 118

### Acide 2-[2-[[[(2S)-2,3-dihydro-1-[[3'-(trifluorométhyl)[1,1'-biphényl]-4-yl]sulfonyl]-1H-indol-2-yl]carbonyl]amino]éthoxy]benzèneacétique, 1-méthyléthyl ester

En opérant de façon analogue à l'exemple 98, au départ du composé obtenu selon la préparation XLII, on obtient le produit attendu sous forme d'un solide blanc (rendement = 92 %).
RMN ¹H (DMSO, 250 MHz) δ: 8,32 (t, 1 H); 8,02-7,72 (m, 8H); 7,51 (d, 1 H); 7,25-6,89 (m, 7H); 4,93-4,83 (m, 2H); 4,01 (t, 2H); 3,57-3,39 (m, 4H); 3,20-2,92 (m, 2H); 1,16 (d, 6H).

### Exemple 119

### Acide 2-[2-[[[(2S)-2,3-dihydro-1-[[3'-(trifluorométhyl)[1,1'-biphényl]-4-yl]sulfonyl]-1H-indol-2-yl]carbonyl]amino]éthoxy]benzèneacétique

En opérant de façon analogue à l'exemple 5, au départ du composé obtenu selon l'exemple 118, on obtient le produit attendu sous forme d'un solide blanc (rendement = 52 %).
F = 131-134°C.

### Exemple 120

### N-[[4-[(diméthylamino)carbonyl]phényl]méthyl]-2,3-dihydro-1-[[3'-(trifluorométhyl)[1,1'-biphényl]-4-yl]sulfonyl]-(2S)-1H-indole-2-carboxamide

En opérant de façon analogue à l'exemple 98, au départ de 4-(aminométhyl)-*N*,*N-*diméthyl-benzènecarboxamide, on obtient le produit attendu sous forme d'un solide blanc (rendement = 54 %).
F = 70°C.

### PREPARATION XLIII

### Acide 4-(3-bromophénoxy)butanoïque, 1,1-diméthyléthyl ester

On place, dans un réacteur résistant à la pression, 1,3 g d'acide 4-(3-bromophénoxy)butanoïque, 8 ml d'isobutène liquide et 2 gouttes d'acide sulfurique. Le réacteur est fermé et agité à température ambiante pendant 16 heures. La pression résiduelle est libérée doucement et le produit est repris par de l'acétate d'éthyle et une solution de bicarbonate de sodium. La phase organique est lavée à l'eau puis séchée et concentrée sous pression réduite. On obtient ainsi l'ester attendu sous forme d'un liquide incolore (rendement = 94 %).
RMN ¹H (DMSO, 250 MHz) δ : 7,26-7,21 (m, 1H); 7,13-7,09 (m, 2H); 6,96-6,92 (m, 1 H); 3,98 (t, 2H); 2,34 (t, 2H); 1,95-1,86 (m, 2H); 1,41 (s, 9H).

### PREPARATION XLIV

### Acide 4-[3-[(E)-2-(1,3-dihydro-1,3-dioxo-2H-isoindol-2-yl)éthenyl] phénoxy]butanoïque 1,1-diméthyléthyl ester

On prépare un mélange de 342 mg (1,98 mmol) de N-(2-éthényl)phtalimide, 620 mg (1,98 mmol) d'ester obtenu selon la préparation XLIII, 13,3 mg (0,06 mmol) d'acétate de palladium, 450 µl (2,5 mmol) de diisopropyléthylamine, 53 µl (022 mmol) de tri-*t-*butylphosphine dans 8 ml d'acétonitrile et on chauffe ce mélange par micro-ondes pendant 20 mn à 150°C. Après élimination du solvant, le produit brut est purifié par chromatographie sur gel de silice en éluant à l'aide d'un mélange toluène/acétate d'éthyle (95/5 v/v). On obtient ainsi 568 mg du composé attendu sous forme d'un solide jaune (rendement = 70 %).
RMN ¹H (DMSO, 300 MHz) δ : 7,96-7,87 (m, 4H); 7,47-7,06 (m, 5H); 6,85-6,82 (m, 1 H); 4,02 (t, 2H); 2,38 (t, 2H); 1,98-1,92 (m, 2H); 1,42 (s, 9H).

### PREPARATION XLV

### Acide 4-[3-[2-(1,3-dihydro-1,3-dioxo-2H-isoindol-2-yl)éthyl] phénoxy]butanoïque 1,1-diméthyléthyl ester

On prépare une solution de 565 mg (1,38 mmol) du composé obtenu selon la préparation XLIV dans 8 ml de THF et 2 ml d'éthanol et on ajoute 209 mg de charbon palladié à 10 %. Ce mélange est hydrogéné sous 3500 hPa à température ambiante puis filtré et concentré sous pression réduite. On obtient ainsi 536 mg du composé attendu sous forme d'un solide beige (rendement = 94 %).
RMN ¹H (DMSO, 300 MHz) δ: 7,87-7,80 (m, 4H); 7,17-7,11 (m, 1 H); 6,74-6,71 (m, 3H); 3,92-3,76 (m, 4H); 2,88 (t, 2H); 2,30 (t, 2H); 1,91-1,82 (m, 2H); 1,39 (s, 9H).

### PREPARATION XLVI

### Acide 4-[3-[2-(amino)éthyl]phénoxy]butanoïque 1,1-diméthyléthyl ester

On prépare une solution de 250 mg (0,63 mmol) du composé obtenu selon la préparation XLV dans 2,5 ml d'éthanol et on ajoute 230 mg (0,46 mmol) d'hydrate d'hydrazine. Ce mélange est chauffé pendant 30 mn à reflux du solvant puis filtré et concentré sous pression. Le produit brut est purifié par chromatographie sur gel de silice en éluant à l'aide d'un mélange dichlorométhane/méthanol (95/5 v/v) avec 1 % d'ammoniaque. On obtient ainsi 244 mg du composé attendu sous forme d'une huile. (rendement = 67 %).
RMN ¹H (DMSO, 300 MHz) δ : 7,20-7,13 (m, 1 H); 6,76-6,70 (m, 3H); 3,94 (t, 2H); 2,74 (m, 2H); 2,58 (t, 2H); 2,35 (t, 2H); 1,96-1,88 (m, 2H); 1,39 (s, 9H).

### Exemple 121

### Acide 4-[3-[2-[[[(2S)-2,3-dihydro-1-[[3'-(trifluorométhyl)[1,1'-biphényl]4-yl]sulfonyl]-1H-indol-2-yl]carbonyl]amino]éthyl]phénoxy] butanoïque, 1,1-diméthyléthyl ester

En opérant de façon analogue à l'exemple 98, au départ du composé obtenu selon la préparation XLVI, on obtient le produit attendu sous forme d'un solide beige (rendement = 88 %).
RMN ¹H (DMSO, 300 MHz) δ : 8,26 (t, 1H); 8,03-7,72 (m, 8H); 7,49 (d, 1 H); 7,30-7,00 (m, 4H); 6,78-6,73 (m, 3H); 4,81 (dd, 1 H); 3,94 (t, 2H); 3,42-3,30 (m, 2H); 3,06 (dd, 1H); 2,85 (dd, 1 H); 2,71 (t, 2H); 2,35 (t, 2H); 1,93-1,88 (m, 2H); 1,37 (s, 9H).

### Exemple 122

### Acide 4-[3-[2-[[[(2S)-2,3-dihydro-1-[[3'-(trifluorométhyl)[1,1'-biphényl]4-yl]sulfonyl]-1H-indol-2-yl]carbonyl]amino]éthyl]phénoxy] butanoïque

En opérant de façon analogue à l'exemple 25, au départ du composé obtenu selon l'exemple 121, on obtient le produit attendu sous forme d'un solide blanc (rendement = 89 %).
F = 70°C.

En opérant de façon analogue aux préparations XLIII à XLVI, au départ de l'acide 3-(3-bromophénoxy)propanoïque, on obtient successivement les composés suivants :

### PREPARATION XLVII

### Acide 3-(3-bromophénoxy)propanoïque, 1,1-diméthyléthyl ester

Liquide incolore (rendement = 98 %).

RMN ¹H (DMSO, 300 MHz) δ : 7,24 (t, 1 H); 7,14-7,11 (m, 2H); 6,96-6,92 (m, 1 H); 4,17 (t, 2H); 2,65 (t, 2H); 1,40 (s, 9H).

### PREPARATION XLVIII

### Acide 3-[3-[(E)-2-(1,3-dihydro-1,3-dioxo-2H-isoindol-2-yl)éthenyl] phénoxy]propanoïque 1,1-diméthyléthyl ester

Solide jaune (rendement = 21 %).
RMN ¹H (DMSO, 300 MHz) δ: 7.96-7,87 (m, 4H); 7,47-7,07 (m, 5H) ; 6,86-6,81 (m, 1 H); 4,21 (t, 2H); 2,67 (t, 2H); 1,42 (s, 9H).

### PREPARATION IL

### Acide 3-[3-[2-(1,3-dihydro-1,3-dioxo-2H-isoindol-2-yl)éthyl]phénoxy]propanoïque 1,1-diméthyléthyl ester

Huile incolore (rendement = 98 %).
RMN ¹H (DMSO, 250 MHz) δ : 7,84-7,81 (m, 4H); 7,15 (t, 1 H); 6,76-6,71 (m, 3H); 4,07 (t, 2H); 3,81 (t, 2H); 2,89 (t, 2H); 2,61 (t, 2H); 1,39 (s, 9H).

### PREPARATION L

### Acide 3-[3-[2-(amino)éthyl]phénoxy]propanoïque 1,1-diméthyléthyl ester

Huile incolore (rendement = 91 %).
RMN ¹H (DMSO, 300 MHz) δ : 7,20-7,14 (m, 1H); 6,78-6,71 (m, 3H); 4,13 (t, 2H); 2,74 (m, 2H); 2,66-2,56 (m, 4H); 1,39 (s, 9H).

### Exemple 123

### Acide 3-[3-[2-[[[(2S)-2,3-dihydro-1-[[3'-(trifluorométhyl)[1,1'-biphényl]4-yl]sulfonyl]-1H-indol-2-yl]carbonyl]amino]éthyl]phénoxy] propanoïque, 1,1-diméthyléthyl ester

En opérant de façon analogue à l'exemple 98, au départ du composé obtenu selon la préparation L, on obtient le produit attendu sous forme d'un solide beige (rendement = 81 %).
RMN ¹H (DMSO, 300 MHz) δ : 8,23 (t, 1 H); 8,02-7,72 (m, 8H); 7,48 (d, 1 H); 7,30-7,11 (m, 3H); 7,02 (t, 1 H); 6,77-6,74 (m, 3H); 4,80 (dd, 1 H); 4,13 (t, 2H); 3,39-3,31 (m, 2H); 3,08 (dd, 1 H); 2,85 (dd, 1 H); 2,73-2,62 (m, 4H); 1,39(s,9H).

### Exemple 124

### Acide 3-[3-[2-[[[(2S)-2,3-dihydro-1-[[3'-(trifluorométhyl)[1,1'-biphényl]4-yl]sulfonyl]-1H-indol-2-yl]carbonyl]amino]éthyl]phénoxy] propanoïque

En opérant de façon analogue à l'exemple 25, au départ du composé obtenu selon l'exemple 123, on obtient le produit attendu sous forme d'un solide blanc (rendement = 45 %).
F = 78°C.

### PREPARATION LI

### Acide 4-[4-[2-(amino)éthyl]phénoxy]butanoique, méthyl ester (chlorhydrate)

En opérant de façon analogue à la préparation XV, au départ de l'ester méthylique de l'acide 4-[4-(cyanométhyl)phénoxy]butanoïque, on obtient le produit attendu sous forme d'un solide blanc (rendement = 68 %).
F = 168-172°C.

### Exemple 125

### Acide 4-[4-[2-[[[(2S)-2,3-dihydro-1-[[3'-(trifluorométhyl)[1,1'-biphényl]4-yl]sulfonyl]-1H-indol-2-yl]carbonyl]amino]éthyl]phénoxy] butanoïque, méthyl ester

En opérant de façon analogue à l'exemple 98, au départ du composé obtenu selon la préparation LI, on obtient le produit attendu sous forme d'un solide blanc (rendement = 98 %).
RMN ¹H (DMSO, 250 MHz) δ : 8,18 (t, 1H); 8,03-7,72 (m, 8H); 7,49 (d, 1 H); 7,26 (t, 1 H); 7,13-7,01 (m, 4H); 6,80 (d, 2H); 4,80 (dd, 1 H); 3,94 (t, 2H); 3,60 (s, 3H); 3,40-3,30 (m, 2H); 3,04 (dd, 1H); 2,85 (dd, 1 H); 2,66 (t, 2H); 2,46 (t, 2H); 2,00-1,89 (m, 2H).

### Exemple 126

### Acide 4-[4-[2-[[[(2S)-2,3-dihydro-1-[[3'-(trifluométhyl)[1,1'-biphényl]4-yl]sulfonyl]-1H-indol-2-yl]carbonyl]amino]éthyl]phénoxy] butanoïque

En opérant de façon analogue à l'exemple 5, au départ du composé obtenu selon l'exemple 125, on obtient le produit attendu sous forme d'un solide blanc (rendement = 75 %).
F = 70°C.

### PREPARATION LII

### Acide 3-[4-[2-(amino)éthyl]phénoxy]propanoïque (chlorhydrate)

En opérant de façon analogue à la préparation XV, au départ de l'acide 3-[4-(cyanométhyl)phénoxy]propanoïque, on obtient le produit attendu sous forme d'un solide blanc (rendement = 23 %).
F = 216°C.

### Exemple 127

### Acide 3-[4-[2-[[[(2S)-2,3-dihydro-1-[[3'-(trifluorométhyl)[1,1'-biphényl]4-yl]sulfonyl]-1H-indol-2-yl]carbonyl]amino]éthyl]phénoxy] propanoïque

On prépare une solution de 115 mg (0,47 mmol) du composé obtenu selon la préparation LII dans 1,3 ml d'eau et on ajoute 777 µl de triéthylamine et 4,7 ml d'acétonitrile. On ajoute enfin, sous agitation et à température ambiante, 240, 9 mg (0,51 mmol) du chlorure d'acide obtenu selon la préparation XXXV en solution dans 2 ml d'acétonitrile. Le milieu réactionnel est agité 30 mn puis concentré sous pression réduite. Le résidu d'évaporation est repris par de l'eau légèrement acidifiée et de l'acétate d'éthyle. La phase organique est séparée, lavée à l'eau puis séchée et concentrée sous pression réduite. Le produit brut est purifié par chromatographie sur gel de silice en éluant à l'aide d'un mélange dichlorométhane/acétate d'éthyle (95/5 v/v) avec 1 % d'acide acétique. On obtient ainsi 144 mg du composé attendu sous forme d'un solide blanc (rendement = 48 %).
F = 70°C.

### PREPARATION LIII

### Acide 5-[3-(aminométhyl)phénoxy]pentanoïque, méthyl ester (chlorhydrate)

En opérant de façon analogue à la préparation XV, au départ de l'ester méthylique de l'acide 5-(3-cyanophénoxy)pentanoïque, on obtient le produit attendu sous forme d'un solide blanc (rendement = 94 %).
F = 94-98°C.

### Exemple 128

### Acide 5-[3-[[[[(2S)-2,3-dihydro-1-[[3'-(trifluorométhyl)[1,1'-biphényl]-4-yl]sulfonyl]-1H-indol-2-yl]carbonyl]amino]méthyl]phénoxy] pentanoïque, méthyl ester

En opérant de façon analogue à l'exemple 98, au départ du composé obtenu selon la préparation LIII, on obtient le produit attendu sous forme d'un solide blanc (rendement = 98 %).
RMN ¹H (DMSO, 250 MHz) δ : 8,74 (t, 1H); 8,03-7,72 (m, 8H); 7,51 (d, 1H); 7,24-7,03 (m, 4H); 6,86-6,80 (m, 3H); 4,92 (dd, 1 H); 4,32 (d, 2H); 3,90 (t, 2H); 3,56 (s, 3H); 3,18 (dd, 1 H); 2,97 (dd, 1 H); 2,36 (t, 2H); 1,73-1,63 (m, 4H).

### Exemple 129

### Acide 5-[3-[[[[(2S)-2,3-dihydro-1-[[3'-(trifluorométhyl)[1,1'-biphényl]-4-yl]sulfonyl]-1H-indol-2-yl]carbonyl]amino]méthyl]phénoxy] pentanoïque

En opérant de façon analogue à l'exemple 5, au départ du composé obtenu selon l'exemple 128, on obtient le produit attendu sous forme d'un solide blanc (rendement = 75 %).
F = 70°C.

### PREPARATION LIV

### Acide 4-[4-(aminométhyl)phénoxy]butanoïque, méthyl ester (chlorhydrate)

En opérant de façon analogue à la préparation XV, au départ de l'ester méthylique de l'acide 4-(4-cyanophénoxy)butanoïque, on obtient le produit attendu sous forme d'un solide blanc (rendement = 63 %).
F = 218°C.

### Exemple 130

### Acide 4-[4-[[[[(2S)-2,3-dihydro-1-[[3'-(trifluorométhyl)[1,1'-biphényl]-4-yl]sulfonyl]-1H-indol-2-yl]carbonyl]amino]méthyl]phénoxy] butanoïque, méthyl ester

En opérant de façon analogue à l'exemple 127, au départ du composé obtenu selon la préparation LIV, on obtient le produit attendu sous forme d'un solide blanc (rendement = 98 %).
F = 50°C.

### Exemple 131

### Acide 4-[4-[[[[(2S)-2,3-dihydro-1-[[3'-(trifluorométhyl)[1,1'-biphényl]-4-yl]sulfonyl]-1H-indol-2-yl]carbonyl]amino]méthyl]phénoxy] butanoïque

En opérant de façon analogue à l'exemple 5, au départ du composé obtenu selon l'exemple 130, on obtient le produit attendu sous forme d'un solide blanc (rendement = 84 %).
F = 78-80°C.

### PREPARATION LV

### Acide 3-[3-(aminométhyl)phénoxy]propanoïque (chlorhydrate)

En opérant de façon analogue à la préparation XV, au départ de l'acide 3-(3-cyanophénoxy)propanoïque, on obtient le produit attendu sous forme d'un solide blanc (rendement = 73 %).
F = 134-138°C.

### Exemple 132

### Acide 3-[3-[[[[(2S)-2,3-dihydro-1-[[3'-(trifluorométhyl)[1,1'-biphényl]-4-yl]sulfonyl]-1H-indol-2-yl]carbonyl]amino]méthyl]phénoxy] propanoïque

En opérant de façon analogue à l'exemple 127, au départ du composé obtenu selon la préparation LV, on obtient le produit attendu sous forme d'un solide blanc (rendement = 98 %).
F = 70°C.

### PREPARATION LVI

### Acide 3-[4-(aminométhyl)phénoxy]propanoïque (chlorhydrate)

En opérant de façon analogue à la préparation XV, au départ de l'acide 3-(4-cyanophénoxy)propanoïque, on obtient le produit attendu sous forme d'un solide blanc (rendement = 78 %).
F = 163-165°C.

### Exemple 133

### Acide 3-[4-[[[[(2S)-2,3-dihydro-1-[[3'-(trifluorométhyl)[1,1'-biphényl]-4-yl]sulfonyl]-1H-indol-2-yl]carbonyl]amino]méthyl]phénoxy] propanoïque

En opérant de façon analogue à l'exemple 127, au départ du composé obtenu selon la préparation LVI, on obtient le produit attendu sous forme d'un solide blanc (rendement = 76 %).
F = 72°C.

### Exemple 134

### Acide 4-[2-[[[(2S)-2,3-dihydro-1-[[3'-(trifluorométhyl)[1,1'-biphényl]-4-yl]sulfonyl]-1H-indol-2-yl]carbonyl]amino]éthyl]benzdique

En opérant de façon analogue à l'exemple 127, au départ d'acide 4-(2-aminoéthyl)benzoïque, on obtient le produit attendu sous forme d'un solide blanc (rendement = 89 %).
F = 106°C.

### PREPARATION LVII

### Acide 3-(4-cyanophénoxy)-2,2-diméthylpropanoïque, méthyl ester

On prépare un mélange de 2,65 g (10,1 mmol) de triphénylphosphine dans 20 ml de toluène et on ajoute, à 0°C, 2 ml (10,2 mmol) de DIAD (diisopropylazodicarboxylate). La solution obtenue est ajoutée à une solution de 1 g (8,4 mmol) de 4-hydroxybenzonitrile et 1,30 ml (10,2 mmol) de 2,2-diméthyl-3-hydroxypropanoate de méthyle dans 10 ml de toluène, à 0°C. Le mélange réactionnel est ensuite agité pendant 30 mn à 80°C, puis concentré sous pression réduite. Le produit brut est purifié par chromatographie sur gel de silice en éluant à l'aide de toluène. On obtient ainsi le composé attendu sous forme d'une huile incolore (rendement = 89 %).
RMN ¹H (DMSO, 250 MHz) δ : 7,75 (d, 2H); 7,10 (d, 2H); 4,08 (s, 2H); 3,61 (s, 3H); 1,24 (s, 6H).

### PREPARATION LVIII

### Acide 3-[4-(aminométhyl)phénoxy]-2,2-diméthylpropanoïque, méthyl ester (chlorhydrate)

En opérant de façon analogue à la préparation XV, au départ du composé obtenu selon la préparation LVII, on obtient le produit attendu sous forme d'un solide blanc (rendement = 96 %).
F = 140-145°C.

### Exemple 135

### Acide 3-[4-[[[[(2S)-2,3-dihydro-1-[[2'-(trifluorométhyl)[1,1'-biphényl]-4-yl]sulfonyl]-1H-indol-2-yl]carbonyl]amino]méthyl]phénoxy]-2,2-diméthylpropanoïque, méthyl ester

En opérant de façon analogue à l'exemple 21, au départ de l'amine obtenue selon la préparation LVIII, on obtient le produit attendu sous forme d'un solide blanc (rendement = 75 %).
F = 68-75°C.

### Exemple 136

### Acide 3-[4-[[[[(2S)-2,3-dihydro-1-[[2-(trifluorométhyl)[1,1'-biphényl]-4-yl]sulfonyl]-1H-indol-2-yl]carbonyl]amino]méthyl]phénoxy]-2,2-diméthylpropanoïque

En opérant de façon analogue à l'exemple 5, au départ de l'ester obtenu selon l'exemple 135, on obtient le produit attendu sous forme d'un solide blanc (rendement = 74 %).
F = 98-105°C.

### Exemple 137

### Acide 3-[4-[[[[(2S)-2,3-dihydro-1-[[3'-(trifluorométhyl)[1,1'-biphényl]-4-yl]sulfonyl]-1H-indol-2-yl]carbonyl]amino]méthyl]phénoxy]-2,2-diméthylpropanoïque, méthyl ester

En opérant de façon analogue à l'exemple 4, au départ de l'amine obtenue selon la préparation LVIII, on obtient le produit attendu sous forme d'une huile incolore (rendement = 51 %).
RMN ¹H (DMSO, 300 MHz) δ : 8,66 (t, 1 H); 8,03-7,70 (m, 8H); 7,50 (d, 1 H); 7,24-7,12 (m, 4H); 7,03 (t, 1 H); 6,86 (d, 2H); 4,88 (dd, 1 H); 4,33-4,20 (m, 2H); 3,95 (s, 2H); 3,60 (s, 3H); 3,16 (dd, 1 H); 2,94 (dd, 1 H); 1,23 (s, 6H).

### Exemple 138

### Acide 3-[4-[[[((2S)-2,3-dihydro-1-[[3'-(trifluorométhyl)(1,1'-biphényl]-4-yl]sulfonyl]-1H-indol-2-yl]carbonyl]amino]méthyl]phénoxy]-2,2-diméthylpropanoïque

En opérant de façon analogue à l'exemple 5, au départ de l'ester obtenu selon l'exemple 137, on obtient le produit attendu sous forme d'un solide blanc (rendement = 88 %).
F = 95-105°C.

### Exemple 139

### 2,3-dihydro-N-[[2-(méthylamino)phényl]méthyl]-1-[[3'-(trifluorométhyl)[1,1'-biphényl]-4-yl]sulfonyl]-(2S)-1H-indole-2-carboxamide

On ajoute 0,028 ml (0,19 mmol) de triéthylamine et 0,018 ml (0,19 mmol) de sulfate de méthyle à une solution de 100 mg (0,18 mmol) du composé obtenu selon l'exemple 61 dans 3 ml d'éther éthylique. Le mélange est chauffé à reflux pendant 8 heures puis concentré sous pression réduite. Le produit brut est purifié par chromatographie en phase inverse sur silice greffée RP 18 en éluant à l'aide d'un mélange acétonitrile/eau (6/4 ; v/v). On obtient le produit attendu sous forme d'une poudre blanche (rendement = 62 %).
F = 86°C.

### Exemple 140

### 2,3-dihydro-N-[[2-(diméthylamino)phényl]méthyl]-1-[[3'-(trifluorométhyl)[1,1'-biphényl]-4-yl]sulfonyl]-(2S)-1H-indole-2-carboxamide

On prépare une solution de 100 mg (0,18 mmol) du composé obtenu selon l'exemple 61 dans 2 ml d'acétonitrile et on ajoute, à température ambiante, 0,07 ml (0,9 mmol) d'une solution de formaldéhyde à 36 % dans l'eau. Le mélange réactionnel est agité pendant 15 mn à température ambiante puis on ajoute 23 mg (0,36 mmol) de cyanoborohydrure de sodium. Le mélange est à nouveau agité pendant 15 mn et on ajoute 0,05 ml d'acide acétique et on poursuit l'agitation pendant 2 heures, à température ambiante. Le solvant est ensuite éliminé sous pression réduite et le résidu repris par du dichlorométhane. La phase organique est lavée avec une solution de soude N, puis à l'eau, séchée sur sulfate de magnésium et concentrée sous pression réduite. Le produit brut est purifié par chromatographie sur gel de silice en éluant à l'aide d'un mélange toluène/isopropanol (95/5;v/v). On obtient ainsi le produit attendu sous forme d'une mousse blanche (rendement = 95%).
F = 66°C.

### Exemple 141

### Acide 3-[4-[2-[[[(2S)-2,3-dihydro-1-[[3'-(trifluorométhyl)[1,1'-biphényl]4-yl]sulfonyl]-1H-indol-2-yl]carbonyl]amino]éthyl]phényl] propanoïque, 1,1-diméthyléthyl ester

En opérant de façon analogue à l'exemple 4, au départ de l'ester t-butylique de l'acide 4-(2-aminoéthyl)benzènepropanoïque, on obtient le produit attendu sous forme d'un solide amorphe (rendement = 89 %).
RMN ¹H (DMSO, 250 MHz) δ : 8,21 (t, 1 H); 8,03-7,72 (m, 8H); 7,49 (d, 1 H); 7,30-7,03 (m, 7H); 4,81 (dd, 1 H); 3,39-3,31 (m, 2H); 3,21 (dd, 1H); 2,88-2,66 (m, 5H); 2,50 (t, 2H); 1,35 (s, 9H).

### Exemple 142

### Acide 3-[4-[2-[[[(2S)-2,3-dihydro-1-[[3'-(trifluorométhyl)[1,1'-biphényl]-4-yl]sulfonyl]-1H-indol-2-yl]carbonyl]amino]éthyl]phényl] propanoïque

En opérant de façon analogue à l'exemple 25, au départ de l'ester obtenu selon l'exemple 141, on obtient le produit attendu sous forme d'un solide beige (rendement = 94 %).
F = 86°C.

### PREPARATION LIX

### Acide 4-[(2-aminoéthyl)thio]-3-chlorobenzèneacétique, méthyl ester (trifluoroacétate)

On prépare une solution de 957 mg (2,76 mmol) de d'ester méthylique de l'acide 3-chloro-4-[[2-[[(1,1-diméthyléthoxy)carbonyl]-amino]éthyl]thio]-benzèneacétique dans 7 ml d'acide trifluoroacétique. Le mélange réactionnel est agité pendant 16 heures à température ambiante puis concentré sous pression réduite. On obtient ainsi le composé attendu sous forme d'une huile beige (rendement = 98 %).
RMN ¹H (DMSO, 250 MHz) δ : 7,91 (s large, 3H); 7,44 (dd, 1 H); 7,41 (s, 1 H); 7,28 (dd, 1 H); 3,71 (s, 2H); 3,62 (s, 3H); 3,22 (t, 2H); 3,04 (m, 2H).

### Exemple 143

### Acide 3-chloro-4-[[2-[[[(2S)-2,3-dihydro-1-[[2'-(trifluorométhyl)[1,1'-biphényl]-4-yl]sulfonyl]-1H-indol-2-yl]carbonyl]amino]éthyl]thio]-, benzèneacétique, méthyl ester

En opérant de façon analogue à l'exemple 21, au départ de l'amine obtenue selon la préparation LIX, on obtient le produit attendu sous forme d'une huile (rendement = 88 %).
RMN ¹H (DMSO, 250 MHz) δ : 8,51 (t, 1 H); 7,83-7,60 (m, 5H); 7,51-7,04 (m, 10H); 4,82 (dd, 1 H); 3,68 (s, 2H); 3,61 (s, 3H); 3,41-2,99 (m, 6H).

### Exemple 144

### Acide 3-chloro-4-[[2-[[[(2S)-2,3-dihydro-1-[[2'-(trifluorométhyl)[1,1'-biphényl]-4-yl]sulfonyl]-1H-indol-2-yl]carbonyl]amino]éthyl]thio]-, benzèneacétique

En opérant de façon analogue à l'exemple 5, au départ de l'ester obtenu selon l'exemple 143, on obtient le produit attendu sous forme d'un solide blanc (rendement = 66 %).
F = 86°C.

### Exemple 145

### Acide 3-chloro-4-[[2-[[[(2S)-2,3-dihydro-1-[[3'-(trifluorométhyl)[1,1'-biphényl]-4-yl]sulfonyl]-1H-indol-2-yl]carbonyl]amino]éthyl]thio]-, benzèneacétique, méthyl ester

En opérant de façon analogue à l'exemple 4, au départ de l'amine obtenue selon la préparation LIX, on obtient le produit attendu sous forme d'une huile (rendement = 88 %).
RMN ¹H (DMSO, 300 MHz) : δ = 8,51 (t, 1 H); 8,03-7,72 (m, 8H); 7,53-7,47 (m, 2H); 7,39 (d, 1 H); 7,25-7,03 (m, 3H); 6,90 (td, 1 H); 4,83 (dd, 1 H); 3,66 (s, 2H); 3,61 (s, 3H); 3,42-2,95 (m, 6H).

### Exemple 146

### Acide 3-chloro-4-[[2-[[[(2S)-2,3-dihydro-1-[[3'-(trifluorométhyl)[1,1'-biphényl]-4-yl]sulfonyl]-1H-indol-2-yl]carbonyl]amino]éthyl]thio]-, benzèneacétique

En opérant de façon analogue à l'exemple 5, au départ de l'ester obtenu selon l'exemple 145, on obtient le produit attendu sous forme d'un solide blanc (rendement = 83 %).
F = 70°C.

### PREPARATION LX

### Acide 2-(2-aminoéthoxy)benzoïque, 1-méthyléthyl ester (chlorhydrate)

En opérant de façon analogue à la préparation XLII, au départ de l'ester isopropylique de l'acide 2-hydroxybenzoïque, on obtient le produit attendu sous forme d'un solide amorphe (rendement = 37 %).
RMN ¹H (DMSO, 300 MHz) δ : 8,20 (s large, 3H); 7,67 (dd, 1 H); 7,55 (td, 1 H); 7,20 (dd, 1 H); 7,07 (td, 1 H); 5,11 (hep, 1 H); 4,27 (t, 2H); 3,19 (t, 2H); 1,30 (d, 6H).

### Exemple 147

### Acide 2-[2-[[[(2S)-2,3-dihydro-1-[[3'-(trifluorométhyl)[1,1'-biphényl]-4-yl]sulfonyl]-1H-indol-2-yl]carbonyl]amino]éthoxy]benzoïque, 1-méthyléthyl ester

En opérant de façon analogue à l'exemple 4, au départ du composé obtenu selon la préparation LX, on obtient le produit attendu sous forme d'un solide blanc (rendement = 73 %).
RMN ¹H (DMSO, 250 MHz) δ : 8,31 (t, 1 H); 8,03-7,78 (m, 8H); 7,60 (dd, 1 H); 7,52-7,46 (m, 2H); 7,13 (t, 1 H); 7,12-6,99 (m, 4H); 5,11 (hep, 1 H); 4,86 (dd, 1 H); 4,09 (t, 2H); 3,53 (m, 2H); 3,20-2,92 (m, 2H); 1,29 (d, 6H).

### Exemple 148

### Acide 2-[2-[[[(2S)-2,3-dihydro-1-[[3'-(trifluorométhyl)[1,1'-biphényl]-4-yl]sulfonyl]-1H-indol-2-yl]carbonyl]amino]éthoxy]benzoïque

En opérant de façon analogue à l'exemple 5, au départ du composé obtenu selon l'exemple 147, on obtient le produit attendu sous forme d'un solide blanc (rendement = 11 %).
F = 70°C.

### Exemple 149

### N-[[4-[(diméthylamino)carbonyl]phényl]méthyl]-2,3-dihydro-1-[[2'-(trifluorométhyl)[1,1'-biphényl]-4-yl]sulfonyl]-(2S)-1H-indole-2-carboxamide

En opérant de façon analogue à l'exemple 21, au départ du 4-(aminométhyl)-N,N-diméthylbenzènecarboxamide, on obtient le produit attendu sous forme d'un solide blanc (rendement = 80 %).
F = 105-106°C.

### Exemple 150

### N-[[4-[(diméthylamino)méthyl]phényl]méthyl]-2,3-dihydro-1-[[2'-(trifluorométhyl)[1,1'-biphényl]-4-yl]sulfonyl]-(2S)-1H-indole-2-carboxamide

En opérant de façon analogue à l'exemple 21, au départ de *N*,*N-*diméthyl-1,4-benzènediméthanamine, on obtient le produit attendu sous forme d'un solide blanc (rendement = 32 %).
F = 53°C.

### Exemple 151

### N-[[4-[(diméthylamino)méthyl]phényl]méthyl]-2,3-dihydro-1-[[3'-(trifluorométhyl)[1,1'-biphényl]-4-yl]sulfonyl]-(2S)-1H-indole-2-carboxamide

En opérant de façon analogue à l'exemple 4, au départ de *N*,*N-*diméthyl-1,4-benzènediméthanamine, on obtient le produit attendu sous forme d'un solide blanc (rendement = 25 %).
F = 70°C.

### PREPARATION LXI

### Acide 4-(2-aminoéthoxy)-3-chlorobenzèneacétique, méthyl ester (trifluoroacétate)

En opérant de façon analogue à la préparation XLII, au départ de l'ester méthylique de l'acide 3-chloro-4-hydroxybenzèneacétique, on obtient le produit attendu sous forme d'un solide amorphe (rendement = 88 %).
RMN ¹H (DMSO, 300 MHz) δ : 7,99 (s large, 3H); 7,37 (d, 1H); 7,23-7,13 (m, 2H); 4,23 (t, 2H); 3,66 (s, 2H); 3,61 (s, 3H); 3,25 (m, 2H).

### Exemple 152

### Acide 3-chloro-4-[2-[[[(2S)-2,3-dihydro-1-[[3'-(trifluorométhyl)[1,1'-biphényl]-4-yl]sulfonyl]-1H-indol-2-yl]carbonyl]amino]éthoxy] benzèneacétique, méthyl ester

En opérant de façon analogue à l'exemple 4, au départ du composé obtenu selon la préparation LXI, on obtient le produit attendu sous forme d'un solide mal cristallisé (rendement = 49 %).
RMN ¹H (DMSO, 250 MHz) δ : 8,36 (t, 1 H); 8,03-7,72 (m, 8H); 7,49 (d, 1 H); 7,34 (s, 1 H); 7,15-7,02 (m, 5H); 4,89 (dd, 1 H); 4,12 (m, 2H); 3,63-3,44 (m, 7H); 3,20-2,92 (m, 2H)

### Exemple 153

### Acide 3-chloro-4-[2-[[[(2S)-2,3-dihydro-1-[[3'-(trifluorométhyl)[1,1'-biphényl]-4-yl]sulfonyl]-1H-indol-2-yl]carbonyl]amino]éthoxy]benzèneacétique

En opérant de façon analogue à l'exemple 5, au départ du composé obtenu selon l'exemple 152, on obtient le produit attendu sous forme d'un solide blanc (rendement = 80 %).
F = 96°C.

### Exemple 154

### 2,3-dihydro-N-[2-(2-pyridinyloxy)éthyl]-1-[[3'-(trifluorométhyl)[1,1'-biphényl]-4-yl]sulfonyl]-(2S)-1H-indole-2-carboxamide

En opérant de façon analogue à l'exemple 4, au départ du chlorhydrate de 2-(2-aminoéthoxy)pyridine, on obtient le produit attendu sous forme d'une mousse blanche (rendement = 84 %).
F = 85°C.

### Exemple 155

### 2,3-dihydro-N-[(3-nitrophényl)méthyl]-1-[[3'-(trifluorométhyl)[1,1'-biphényl]-4-yl]sulfonyl]-(2S)-1H-indole-2-carboxamide

En opérant de façon analogue à l'exemple 4, au départ du chlorhydrate de 3-nitro-benzèneméthanamine, on obtient le produit attendu sous forme d'une mousse blanche (rendement = 97 %).
RMN ¹H (DMSO, 250 MHz) δ: 8,97 (t, 1 H); 8,17-7,72 (m, 11 H); 7,64 (t, 1 H); 7,53 (d, 1 H); 7,26 (t, 1 H); 7,14 (d, 1 H); 7,04 (td, 1 H); 4,91 (dd, 1 H); 4,48 (d, 2H); 3,20 (dd, 1 H); 2,96 (dd, 1 H)

### Exemple 156

### N-[(3-aminophényl)méthyl]-2,3-dihydro-1-[[3'-(trifluorométhyl)[1,1'-biphényl]-4-yl]sulfonyl]-(2S)-1H-indole-2-carboxamide

En opérant de façon analogue à l'exemple 61, au départ du composé obtenu selon l'exemple 155, on obtient le produit attendu sous forme d'une mousse blanche (rendement = 96 %).
F = 82°C.

### Exemple 157

### 2,3-dihydro-N-[(4-nitrophényl)méthyl]-1-[[3'-(trifluorométhyl)[1,1'-biphényl]-4-yl]sulfonyl]-(2S)-1H-indole-2-carboxamide

En opérant de façon analogue à l'exemple 4, au départ du chlorhydrate de 4-nitro-benzèneméthanamine, on obtient le produit attendu sous forme d'une mousse blanche (rendement = 79 %).
RMN ¹H (DMSO, 250 MHz) δ : 8,96 (t, 1H); 8,19 (dd, 2H); 8,02-7,70 (m, 8H); 7,57-7,51 (m, 3H); 7,26-7,14 (m, 2H); 7,04 (td, 1 H); 4,91 (dd, 1 H); 4,55-4,40 (m, 2H); 3,21 (dd, 1 H); 2,99 (dd, 1 H)

### Exemple 158

### N-[(4-aminophényl)méthyl]-2,3-dihydro-1-[[3'-(trifluorométhyl)[1,1'-biphényl]-4-yl]sulfonyl]-(2S)-1H-indole-2-carboxamide

En opérant de façon analogue à l'exemple 61, au départ du composé obtenu selon l'exemple 157, on obtient le produit attendu sous forme d'une mousse blanche (rendement = 98 %).
F = 84°C.

### PREPARATION LXII

### Acide 2,3-dihydro-1-[(4'-fluoro-[1,1'-biphényl]-4-yl)sulfonyl]-(2S)-1H-indole-2-carboxylique, méthyl ester

On prépare un mélange de 0,545 g (2,55 mmol) du chlorhydrate de l'ester méthylique de l'acide 2,3-dihydro-(2*S*)-1*H*-indµole-2-carboxylique dans 10 ml d'acétonitrile et on ajoute 0,9 g (3,32 mmol) de chlorure de 4'-fluoro-[1,1'-biphényl]-4-sulfonyle et 0,62 ml de N-méthylmorpholine. Le mélange réactionnel est agité pendant 16 heures à température ambiante, puis concentré sous pression réduite. Le résidu est repris dans 100 ml d'acétate d'éthyle et lavé avec 3 fois 75 ml d'eau. La phase organique est séchée sur sulfate de magnésium, puis concentrée sous pression réduite. Le produit brut est purifié par chromatographie sur gel de silice en éluant à l'aide d'un mélange toluène/acétate d'éthyle (98/2 v/v). On obtient ainsi 0,83 g du composé attendu sous forme d'un solide blanc (rendement = 79 %).
RMN ¹H (DMSO, 300 MHz) δ : 7,93-7,74 (m, 6H); 7,42 (d, 1H); 7,34-7,14 (m, 4H); 7,01 (t, 1 H); 5,10 (dd, 1 H); 3,73 (s, 3H); 3,38 (dd, 1 H); 3,09 (dd, 1H)

### PREPARATION LXIII

### Acide 2,3-dihydro-1-[(4'-fluoro-[1,1'-biphényl]-4-yl)sulfonyl]-(2S)-1H-indole-2-carboxylique

En opérant de façon analogue à l'exemple 5, au départ du composé obtenu selon la préparation LXII, on obtient le produit attendu sous forme d'un solide blanc (rendement = 98 %).
F = 145-150°C.

### Exemple 159

### 2,3-dihydro-N-(phénylméthyl)-1-[(4'-fluoro-[1,1'-biphényl]-4-yl)-sulfonyl]-(2S)-1H-indole-2-carboxamide

On mélange une solution de 50 mg (0,126 mmol) d'acide obtenu selon la préparation LXIII dans 3 ml de dichlorométhane avec 140 mg de résine greffée DCC (préalablement activée par lavage au dichlorométhane), et on ajoute 5 mg (0,036 mmol) de HOAT et 18 mg (0,17 mmol) de benzylamine. Le mélange réactionnel est agité à température ambiante pendant 16 heures. On ajoute environ 50 mg de résine IR 120 et on agite à nouveau le mélange pendant 3 heures. Les résines sont ensuite éliminées par filtration et le filtrat est concentré sous pression réduite. On obtient ainsi le composé attendu sous forme d'un solide blanc (rendement = 85 %).
F = 65°C.

### Exemple 160

### N-[2-(4-chlorophényl)éthyl]-2,3-dihydro-1-[(4'-fluoro-[1,1'-biphényl]-4-yl)sulfonyl]-(2S)-1H-indole-2-carboxamide

En opérant de façon analogue à l'exemple 159, au départ de la 2-(4-chlorophényl)éthanamine, on obtient le produit attendu sous forme d'un solide blanc (rendement = 91 %).
F = 126°C.

### Exemple 161

### Acide 4-[2-[[[(2S)-1-[(4'-fluoro[1,1'-biphényl]-4-yl)sulfonyl]-2,3-dihydro -1 H-indol-2-yl]carbonyl]amino]éthoxy]-benzoïque, méthyl ester

En opérant de façon analogue à l'exemple 159, au départ de l'ester méthylique de l'acide 4-(2-aminoéthoxy)benzoïque, on obtient le produit attendu sous forme d'un solide blanc (rendement = 94 %).
F = 70-72°C.

### Exemple 162

### Acide 4-[2-[[[(2S)-1-[(4'-fluoro[1,1'-biphényl]-4-yl)sulfonyl]-2,3-dihydro -1H-indol-2-yl]carbonyl]amino]éthoxy]-benzoïque

En opérant de façon analogue à l'exemple 5, au départ de l'ester obtenu selon l'exemple 161, on obtient le produit attendu sous forme d'un solide blanc (rendement = 94 %).
F = 108°C.

### Exemple 162a

### Acide 4-[2-[[[(2S)-1-[(4'-fluoro[1,1'-biphényl]-4-yl)sulfonyl]-2,3-dihydro -1H-indol-2-yl]carbonyl]amino]éthoxy]-benzoïque, sel de sodium

Le sel est obtenu par lyophilisation de la solution obtenue en mélangeant l'acide et une quantité équimoléculaire de soude dans l'eau.
F = 214-224°C.

### Exemple 163

### 2,3-dihydro-N-(2-oxo-2-phényléthyl)-1-[(4'-fluoro-[1,1'-biphényl]-4-yl])sulfonyll-(2S)-1H-indole-2-carboxamide

En opérant de façon analogue à l'exemple 159, au départ de la 2-oxo-2-phényléthanamine, on obtient le produit attendu sous forme d'un solide jaune (rendement = 51 %).
F= 80-82°C.

### Exemple 164

### 2,3-dihydro-N-(2,2-diphényl-éthyl)-1-[(4'-fluoro-[1,1'-biphényl]-4-yl) sulfonyl]-(2S)-1H-indole-2-carboxamide

En opérant de façon analogue à l'exemple 159, au départ de la 2,2-diphényléthanamine, on obtient le produit attendu sous forme d'un solide blanc (rendement = 86 %).
F= 80-82°C.

### Exemple 165

### 1-[(4'-fluoro[1,1'-biphényl]-4-yl)sulfonyl]-2,3-dihydro-N-[(1S,2R)-2-phénylcyclopropyl]-(2S)-1H-indole-2-carboxamide

En opérant de façon analogue à l'exemple 159, au départ de la (1 S, 2R)-2-phénylcyclopropylamine, on obtient le produit attendu sous forme d'un solide jaune (rendement = 75 %).
F = 80°C.

### Exemple 166

### Acide 4-[2-([[(2S)-1-[(4'-fluoro[1,1'-biphényl]-4-yl)sulfonyl]-2,3-dihydro -1H-indol-2-yl]carbonyl]amino]éthoxy]benzèneacétique

On prépare une solution de 100 mg (0,252 mmol) d'acide obtenu selon la préparation LXIII dans 2 ml de dichlorométhane et on ajoute à 0°C, 40,5 µl de chlorure de thionyle et 77 µl de triéthylamine. Le mélange est agité pendant 15 mn à 0°C, puis concentré sous pression réduite. On ajoute ensuite au chlorure d'acide ainsi obtenu une suspension de 54 mg d'acide 4-(2-aminoéthoxy)benzèneacétique dans 2 ml de dichlorométhane et 77 µl de triéthylamine. Le milieu réactionnel est agité à température ambiante pendant 3 heures, puis dilué avec 10 ml de dichlorométhane et lavé à l'eau. La phase organique est séchée sur sulfate de magnésium et concentrée sous pression réduite. Le produit brut est purifié par chromatographie sur gel de silice en éluant à l'aide d'un mélange dichlorométhane/méthanol (95/5; v/v). On obtient ainsi 29 mg du composé attendu sous forme d'un solide jaune (rendement = 20 %).
F=81°C.

### Exemple 167

### Acide β-[[[(2S)-1-[(4'-fluoro[1,1'-biphényl]-4-yl)sulfonyl]-2,3-dihydro-1H-indol-2-yl]carbonyl]amino]-(βS)-benzènebutanoique,1,1-diméthyléthyl ester

En opérant de façon analogue à la préparation I, au départ de l'ester *t-*butylique de l'acide (β*S*)-β-amino-benzènebutanoique, on obtient le produit attendu sous forme d'un solide beige (rendement = 62 %).
F = 67-73°C.

### Exemple 168

### Acide β-[[[(2S)-1-[(4'-fluoro[1,1'-biphényl]-4-yl)sulfonyl]-2,3-dihydro-1H-indol-2-yl]carbonyl]amino]-(βS)-benzènebutanoique

En opérant de façon analogue à l'exemple 25, au départ de l'ester obtenu selon l'exemple 167 on obtient le produit attendu sous forme d'un solide blanc (rendement = 44 %).
F = 87-90°C.

### Exemple 169

### Acide 4-[[[[(2S)-2,3-dihydro-1-[[3'-(trifluorométhyl)[1,1'-biphényl]-4-yl]sulfonyl]-1H-indol-2-yl]carbonyl]amino]méthyl]benzèneacétique, sel de sodium

On prépare une solution de 540mg (0,91 mmol) d'acide obtenu selon l'exemple 85 dans 5 ml de THF et on ajoute à 0°C, 0,905 ml d'une solution d'hydroxyde de sodium 1 N dans l'eau. Le mélange réactionnel est agité pendant 1,5 heure à 0°C, puis concentré sous pression réduite jusqu'à environ 1,5 ml résiduel. Le résidu est repris en solution dans l'eau à température ambiante, la solution est filtrée puis lyophylisée. On obtient ainsi le sel attendu sous forme d'une poudre blanche.
F = 138-140°C.

### PREPARATION LXIV

### Acide 4-méthoxy-2,3-dihydro-1H-indole-2-carboxylique, méthyl ester

A une solution de 500 mg (2,44 mmol) d'acide 4-méthoxy-1 H-indole-2-carboxylique dans 10 ml de méthanol, on ajoute 119 mg (4,90 mmol) de tournures de magnésium. Le mélange est agité 3 heures dans un bain à 10°C puis 1 heure à température ambiante. On additionne 20 ml d'acide chlorhydrique à 0°C et on agite une heure. On additionne ensuite une solution d'ammoniaque 3N jusqu'à pH 10 et on extrait par 3 fois 50 ml d'acétate d'éthyle. Les phases organiques sont séchées sur sulfate de magnésium, filtrées et concentrées sous pression réduite. Le résidu d'évaporation est purifié par chromatographie sur colonne de silice en éluant à l'aide d'un mélange dichlorométhane/acétate d'éthyle (98/2 puis 95-5 ; v/v). On obtient le produit attendu sous forme d'une huile marron avec un rendement de 64 %.
RMN ¹H (DMSO, 250 MHz) δ : 6,91 (t, 1H); 6,24-6,20 (m, 2H); 5,97 (d, 1 H); 4,41-4,34 (m; 1 H); 3,71 (s, 3H); 3,65 (s, 3H); 3,17 (dd, 1 H); 2,97 (dd, 1 H).

### PREPARATION LXV

### Acide 1-[(4-iodophényl)sulfonyl]-4-méthoxy-2,3-dihydro-1H-indole-2-carboxylique, méthyl ester

En opérant de façon analogue à la préparation XI, au départ de l'ester obtenu selon la préparation LXIV et de chlorure de 4-iodobenzènesulfonyle, on obtient le composé attendu sous forme d'un solide blanc avec un rendement de 87 %.
RMN ¹H (DMSO, 300 MHz) δ : 7,95 (d, 2H); 7,59 (d, 2H); 7,20 (t, 1 H); 6,97 (d, 1 H); 6,68 (d, 1 H); 5,07 (dd, 1 H); 3,73 (s, 3H); 3,71 (s, 3H); 3,24 (dd, 1H); 2,93 (dd, 1 H).

### PREPARATION LXVI

### Acide 4-méthoxy-2,3-dihydro-1-[[3'-(trifluorométhyl)[1,1'-biphényl]-4-yl]sulfonyl]-1H-indole-2-carboxylique, méthyl ester

En opérant de façon analogue à l'exemple 2, au départ de l'ester obtenu selon la préparation LXV, on obtient le composé attendu sous forme d'une poudre blanche avec un rendement de 73 %.
RMN ¹H (DMSO, 300 MHz) δ : 8,04-8,02 (m, 2H); 7,95 (s; 4H); 7,81-7,69 (m, 2H); 7,21 (t, 1 H); 7,05 (d, 1 H); 6,68 (d, 1 H); 5,13 (dd, 1 H); 3,74 (s, 3H); 3,72 (s, 3H); 3,24 (dd, 1 H); 2,95 (dd, 1 H).

### PREPARATION LXVII

### Acide 4-méthoxy-2,3-dihydro-1-[[3'-(trifluorométhyl)[1,1'-biphényl]-4-yl]sulfonyl]-1H-indole-2-carboxylique

En opérant de façon analogue à l'exemple 5, au départ de l'ester obtenu selon la préparation LXVI, on obtient l'acide attendu sous forme d'une poudre blanche avec un rendement de 97 %.
RMN ¹H (DMSO, 300 MHz) δ : 13,27 (s large, 1 H); 8,04-8,02 (m, 2H); 7,94 (s; 4H); 7,81-7,69 (m, 2H); 7,21 (t, 1 H); 7,04 (d, 1 H); 6,67 (d, 1 H); 4,98 (dd, 1 H); 3,72 (s, 3H); 3,24 (dd, 1 H); 2,89 (dd, 1 H).

### Exemple 170

### Acide 4-[[[[2,3-dihydro-4-méthoxy-1-[[3'-(trifluorométhyl)[1,1'-biphényl]-4-yl]sulfonyl]-1H-indol-2-yl]carbonyl]amino]méthyl] benzèneacétique, méthyl ester.

En opérant de façon analogue à l'exemple 21, au départ de l'acide obtenu selon la préparation LXVII, on obtient le composé attendu sous forme d'une poudre blanche avec un rendement de 95 %.
F = 80°C.

### Exemple 171

### Acide 4-[[[[2,3-dihydro-4-méthoxy-1-[[3'-(trifluorométhyl)[1,1'-biphényl]-4-yl]sulfonyl]-1H-indol-2-yl]carbonyl]amino]méthyl] benzèneacétique

En opérant de façon analogue à l'exemple 5, au départ de l'ester obtenu selon l'exemple 170, on obtient l'acide attendu sous forme d'une poudre blanche avec un rendement de 97 %.
F = 100°C.

### PREPARATION LXVIII

### Acide 2-fluoro-4-[2-[[(phénylméthoxy)carbonyl]amino]éthoxy]benzoïque, méthyl ester

En opérant de façon analogue à la préparation XXXVIII, au départ de l'ester benzylique de l'acide (2-bromoéthyl)carbamique et de l'ester méthylique de l'acide 2-fluoro-4-hydroxybenzoïque, on obtient le composé attendu sous forme d'une huile avec un rendement de 42 %.
RMN ¹H (DMSO, 300 MHz) δ : 7,82 (t, NH) ; 7,40-7,20 (m, 6H) ; 6,95-6,80 (m, 2H) ; 5,01 (s, 2H) ; 4,08 (t, 2H) ; 3,79 (s, 3H) ; 3,38 (t, 2H).

### PREPARATION LXIX

### Acide 2-fluoro-4-(2-aminoéthoxy)benzoïque, méthyl ester, chlorhydrate

On prépare une solution de 1,93 g (5,55 mmol) du composé obtenu selon la préparation LXVIII dans 10 ml de méthanol et on ajoute 10 ml d'une solution saturée de chlorure d'hydrogène dans le méthanol, puis 0,2 g de charbon palladié à 10%. Le mélange est agité sous atmosphère d'hydrogène à pression atmosphérique et température ambiante pendant 14 heures, puis filtré et concentré sous pression réduite. On obtient ainsi 1,38 g du composé attendu sous forme d'un solide blanc (rendement = 99 %).
F = 231-234°C.

### PREPARATION LXX

### Acide 3,5-diméthyl-4-[2-[[(phénylméthoxy)carbonyl]amino]éthoxy]benzoïque, méthyl ester

En opérant de façon analogue à la préparation LXVIII, au départ de l'ester méthylique de l'acide 3,5-diméthyl-4-hydroxybenzoïque, on obtient le composé attendu sous forme d'une huile avec un rendement de 49 %.
RMN ¹H (DMSO, 300MHz) δ : 7,70-7,60 (m, 2H) ; 7,57 (t, NH) ; 7,45-7,20 (m, 5H) ; 5,05 (s, 2H) ; 3,90-3,70 (m, 5H) ; 3,50-3,30 (m, 2H) ; 2,24 (s, 6H).

### PREPARATION LXXI

### Acide 3,5-diméthyl-4-(2-aminoéthoxy)benzoïque, méthyl ester, chlorhydrate

On prépare une solution de 0,802 g (2,24 mmol) du composé obtenu selon la préparation LXX dans 14 ml de méthanol et on ajoute 2,7 ml d'acide chlorhydrique N, puis 0,1 g de charbon palladié à 10%. Le mélange est agité sous atmosphère d'hydrogène à pression atmosphérique et température ambiante pendant 17 heures, puis filtré et concentré sous pression réduite. Le solide résiduel est trituré dans l'éther éthylique et on obtient ainsi 0,516 g du composé attendu sous forme d'un solide blanc (rendement =88 %)
RMN ¹H (DMSO, 300MHz) δ : 8,29 (slarge, 3H) ; 7,67 (s, 2H) ; 3,99 (t, 2H) ; 3,81 (s, 3H) ; 3,23 (t, 2H) ; 2,30 (s, 6H).

### PREPARATION LXXII

### Acide 3-chloro-4-[2-[[(phénylméthoxy)carbonyl]amino]éthoxy]benzoïque, méthyl ester

En opérant de façon analogue à la préparation LXVIII, au départ de l'ester méthylique de l'acide 3-chloro-4-hydroxybenzoïque, on obtient le composé attendu sous forme d'un solide blanc avec un rendement de 68 %.
F=81°C.

### PREPARATION LXXIII

### Acide 3-chloro-4-(2-aminoéthoxy)benzoïque, méthyl ester, chlorhydrate

En opérant de façon analogue à la préparation LXXI, au départ du composé obtenu selon la préparation LXXII, on obtient le composé attendu sous forme d'un solide blanc avec un rendement de 99 %.
RMN ¹H (DMSO, 300MHz) δ : 8,26 (slarge, NH2) ; 8,00-7,90 (m, 2H) ; 7,33 (d, 1 H) ; 4,39 (t, 2H) ; 3,84 (s, 3H) ; 3,40-3,20 (m, 2H).

### PREPARATION LXXIV

### Acide 3-fluoro-4-[2-[[(phénylméthoxy)carbonyl]amino]éthoxy]benzoïque, méthyl ester

En opérant de façon analogue à la préparation LXVIII, au départ de l'ester méthylique de l'acide 3-fluoro-4-hydroxybenzoïque, on obtient le composé attendu sous forme d'un solide blanc avec un rendement de 80 %.
RMN ¹H (CDCl3, 300MHz) δ : 7,85-7,70 (m, 2H) ; 7,40-7,30 (m, 5H ; 6,95 (t, 1 H) ; 5,29 (t, 1 H) ; 5,12 (s, 2H) ; 4,16 (t, 2H) ; 3,89 (s, 3H) ; 3,66 (q, 2H).

### PREPARATION LXXV

### Acide 3-fluoro-4-(2-aminoéthoxy)benzoïque, méthyl ester, chlorhydrate

En opérant de façon analogue à la préparation LXIX, au départ du composé obtenu selon la préparation LXXIV, on obtient le composé attendu sous forme d'un solide blanc avec un rendement de 98 %.
F = 240-247°C.

### PREPARATION LXXVI

### Acide α-méthyl-4-[2-[[(phénylméthoxy)carbonyl]amino]éthoxy]benzèneacétique, méthyl ester

En opérant de façon analogue à la préparation LXVIII, au départ de l'ester méthylique de l'acide α-méthyl-4-hydroxybenzèneacétique, on obtient le composé attendu sous forme d'une huile avec un rendement de 53 %.
RMN ¹H (DMSO, 300MHz) δ : 7,5 (t, NH) ; 7,40-7,05 (m, 7H); 6,87 (d, 2H) ; 5,03 (s, 2H) ; 3,96 (t, 2H) ; 3,72 (q, 1H) ; 3,56 (s, 3H) ; 3,40-3,30 (m, 2H) ; 1,34 (d, 3H).

### PREPARATION LXXVII

### Acide 4-(2-aminoéthoxy)- α-méthyl-benzèneacétique, méthyl ester , chlorhydrate

En opérant de façon analogue à la préparation LXXI au départ du composé obtenu selon la préparation LXXVI, on obtient le composé attendu sous forme d'un solide beige avec un rendement de 76 %.
RMN ¹H (DMSO, 300MHz) δ 8,19 (s large, NH3) ; 7,21 (d, 2H) ; 6,95 (d, 2H) ; 4,16 (t, 2H) ; 3,75 (q, 1 H) ; 3,57 (s, 3H) ; 3,25-3,10 (m, 2H) ; 1,35 (d, 3H).

### PREPARATION LXXVIII

### Acide 3-méthoxy-4-[2-[[(phénylméthoxy)carbonyl]amino]éthoxy]benzèneacétique, méthyl ester

En opérant de façon analogue à la préparation LXVIII, au départ de l'ester méthylique de l'acide 4-hydroxy-3-méthoxybenzèneacétique, on obtient le composé attendu sous forme d'une huile jaune avec un rendement de 51 %.
RMN ¹H (CDCl₃, 300MHz) δ : 7,50-7,30 (m, 5H) ; 6,95-6,65 (m, 3H) ; 5,11 (s, 2H) ; 4,20-4,0 (m, 2H) ; 3,84 (s, 3H) ; 3,69 (s, 3H) ; 3,65-3,50 (m, 4H).

### PREPARATION LXXIX

### Acide 4-(2-aminoéthoxy)- 3-méthoxybenzèneacétique, méthyl ester (chlorhydrate)

En opérant de façon analogue à la préparation LXXI au départ du composé obtenu selon la préparation LXXVIII, on obtient le composé attendu sous forme d'un solide blanc cassé avec un rendement de 86 %.
F = 77-80°C.

### PREPARATION LXXX

### Acide 2,6-difluoro-4-[2-[[(phénylméthoxy)carbonyl]amino]éthoxy]benzoïque, méthyl ester

En opérant de façon analogue à la préparation LXVIII, au départ de l'ester méthylique de l'acide 2,6-difluoro-4-hydroxybenzoïque, on obtient le composé attendu sous forme d'un solide blanc avec un rendement de 75 %.
F = 95-97°C.

### PREPARATION LXXXI

### Acide 2,6-difluoro-4-(2-aminoéthoxy)benzoïque, méthyl ester, chlorhydrate

En opérant de façon analogue à la préparation LXIX, au départ du composé obtenu selon la préparation LXXX, on obtient le composé attendu sous forme d'un solide blanc avec un rendement de 70 %.
F = 185°C.

### PREPARATION LXXXII

### Acide 4-[2-[[(phénylméthoxy)carbonyl]amino]éthoxy]-3-(trifluorométhyl)benzoïque, méthyl ester

En opérant de façon analogue à la préparation LXVIII, au départ de l'ester méthylique de l'acide 4-hydroxy-3-(trifluorométhyl)benzoïque, on obtient le composé attendu sous forme d'un solide pâteux incolore avec un rendement de 51 %.
RMN ¹H (DMSO, 300MHz) δ : 8,25-8,05 (m, 2H) ; 7,50-7,25 (m, 6H) ; 5,01 (s, 2H) ; 4,25 (t, 2H) ; 3,86 (s, 3H) ; 3,43 (t, 2H).

### PREPARATION LXXXIII

### Acide 4-(2-aminoéthoxy)-3-(trifluorométhyl)benzoïque, méthyl ester, chlorhydrate

En opérant de façon analogue à la préparation LXIX, au départ du composé obtenu selon la préparation LXXXII, on obtient le composé attendu sous forme d'un solide blanc avec un rendement de 99 %.
F = 220-222°C.

### PREPARATION LXXXIV

### Acide 4-[2-[[(phénylméthoxy)carbonyl]amino]éthoxy]-2-(trifluorométhyl)benzoïque, méthyl ester

En opérant de façon analogue à la préparation LXVIII, au départ de l'ester méthylique de l'acide 4-hydroxy-2-(trifluorométhyl)benzoïque, on obtient le composé attendu sous forme d'une huile incolore avec un rendement de 64%.
RMN ¹H (CDCl3, 300MHz) δ : 8,95-8,85 (m, 1 H) ; 7,54 (t, NH) ; 7,45-7,25 (m, 7H) ; 5,03 (s, 2H) ; 4,16 (t, 2H); 3,83 (s, 3H) ; 3,42 (t, 2H).

### PREPARATION LXXXV

### Acide 4-(2-aminoéthoxy)-2-(trifluorométhyl)benzoïque, méthyl ester, chlorhydrate

En opérant de façon analogue à la préparation LXIX, au départ du composé obtenu selon la préparation LXXXIV, on obtient le composé attendu sous forme d'un solide blanc avec un rendement de 93 %.
F = 172-175°C.

### PREPARATION LXXXVI

### Acide 3-méthyl-4-[2-[[(phénylméthoxy)carbonyl]amino]éthoxy]benzoïque, méthyl ester

En opérant de façon analogue à la préparation LXVIII, au départ de l'ester méthylique de l'acide 3-méthyl-4-hydroxybenzoïque, on obtient le composé attendu sous forme d'une huile jaune avec un rendement de 79 %.
RMN ¹H (DMSO, 300MHz) δ : 7,85-7,70 (m, 2H) ; 7,50 (t, NH) ; 7,40-7,35 (m, 5H) ; 7,03 (d, 1 H) ; 5,04 (s, 2H) ; 4,07 (t, 2H) ; 3,80 (s, 3H) ; 3,50-3,40 (m, 2H) ; 2.17 (s, 3H).

### PREPARATION LXXXVII

### Acide 4-(2-aminoéthoxy)-3-méthyl-benzoïque, méthyl ester, chlorhydrate

En opérant de façon analogue à la préparation LXIX, au départ du composé obtenu selon la préparation LXXXVI, on obtient le composé attendu sous forme d'un solide rose avec un rendement de 60 %.
F = 200-201°C

### PREPARATION LXXXVIII

### Acide 3-[(4-cyanophényl)thio]propanoïque, éthyl ester

On mélange, dans un réacteur, 1 g (3,46 mmol) d'ester éthylique de l'acide 3-[(4-bromophényl)thio]-propanoïque et 0,62 g (7 mmol) de cyanure cuivreux dans 10 ml de DMF. Le mélange réactionnel est agité à doux reflux pendant 7 heures, puis refroidi et versé sur 50 ml d'eau et extrait par l'acétate d'éthyle. La phase organique séparée est lavée à l'eau puis séchée sur sulfate de magnésium et concentrée sous pression réduite. Le produit brut obtenu est purifié par chromatographie sur gel de silice en éluant à l'aide d'un mélange toluène/acétate d'éthyle (98/2 ;v/v). On obtient ainsi le produit attendu avec un rendement de 98 %.
RMN ¹H (CDCl₃, 300MHz) δ : 7,55 (d, 2H) ; 7,32 (d, 2H) ; 4,16 (q, 2H) ; 3,25 (t, 2H) ; 2,68 (t, 2H) ; 1,26 (t, 3H).

### PREPARATION LXXXIX

### Acide 3-[[4-(aminométhyl)phényl]thio]propanoïque, éthyl ester

On charge, dans un réacteur, 0,83 g (3,5 mmol) du composé obtenu selon la préparation LXXXVIII, 15 ml d'éthanol, 8 ml d'éthanol saturé par l'ammoniac et 0,4 g de Nickel préparé selon Raney. Le mélange est agité sous atmosphère d'hydrogène à pression atmosphérique pendant 2h à température ambiante, puis filtré et concentré sous pression réduite.Le résidu d'évaporation est repris par de l'ether éthylique et extrait par une solution d'acide chlorhydrique N. La phase aqueuse acide obtenue est lavée par 20 ml d'éther éthylique, puis amenée à pH basique par addition d'une solution concentrée de bicarbonate de sodium et extraite par l'acétate d'éthyle. Cette phase organique est lavée à l'eau, séchée sur sulfate de magnésium et concentrée sous pression réduite. On obtient ainsi 360 mg du produit attendu sous forme d'une huile beige (rendement = 42 %).
RMN ¹H (DMSO, 300MHz) δ : 7,30 (m, 4H) ; 4,05 (q, 2H) ; 3,69 (s, 2H) ; 3,12 (t, 2H) ; 2,57 (t, 2H) ; 1,17 (t, 3H).

### PREPARATION XC

### Acide 3-[[2-(hydroxyméthyl)phényl]thio]propanoïque éthyl ester

On prépare une solution de 3 g (21,4 mmol) de 2-(hydroxyméthyl)thiophénol dans 30 ml d'éthanol absolu et on ajoute par portions 1,46 g (21,4 mmol) d'éthylate de sodium. Le mélange est agité pendant 10 mn à température ambiante, puis on ajoute 2,74 ml (21,4 mmol) d'ester éthylique de l'acide 3-bromopropanoïque. Le mélange est agité pendant 30 mn à température ambiante, puis concentré sous pression réduite. Le résidu d'évaporation est repris par 25 ml d'acide chlorhydrique 2N et extrait par l'acétate d'éthyle. La phase organique est lavée à l'eau, séchée sur sulfate de magnésium et concentrée sous pression réduite. Le produit brut obtenu est purifié par chromatographie sur gel de silice en éluant à l'aide d'un mélange cyclohexane/acétate d'éthyle (80/20 v/v). On obtient ainsi le produit attendu sous forme d'une huile jaune avec un rendement de 68 %.
RMN ¹H (CDCl3, 300MHz) δ : 7,50-7,40 (m, 2H) ; 7,40-7,20 (m, 2 H) ; 4,77 (s, 2H) ; 4,11 (q, 2H) ; 3,16 (t, 2H) ; 2,60 (t, 2H) ; 1,24 (t, 3H).

### PREPARATION XCI

### Acide 3-[[2-(chlorométhyl)phényl]thio]propanoïque éthyl ester

On prépare une solution de 0,480 g (2 mmol) du composé obtenu selon la préparation XC dans 10 ml de DCM et on ajoute progressivement, à 0°C, 0,174 ml (2,4 mmol) de chlorure de thionyle. Le mélange est agité pendant 30 mn à cette température, puis versé sur une solution saturée de bicarbonate de sodium et extrait par 50 ml de DCM. La phase organique est lavée à l'eau, séchée sur sulfate de magnésium et concentrée sous pression réduite. On obtient ainsi le produit attendu sous forme d'une huile jaune avec un rendement de 98 %.
RMN ¹H (CDCl3, 300MHz) δ : 7,50-7,40 (m, 2H) ; 7,40-7,20 (m, 2 H) ; 4,80 (s, 2H) ; 4,14 (q, 2H) ; 3,20 (t, 2H) ; 2,62 (t, 2H) ; 1,26 (t, 3H).

### PREPARATION XCII

### Acide 3-[[2-(azidométhyl)phényl]thio]propanoïque éthyl ester

On prépare une solution de 0,260 g (1 mmol) du composé obtenu selon la préparation XCI dans 1 ml d'acétone et on ajoute 68 mg (1,05 mmol) d'azoture de sodium dans 0,5 ml d'eau. Le mélange est agité pendant 1 heure à température ambiante, puis extrait par l'acétate d'éthyle. La phase organique est lavée à l'eau salée, séchée sur sulfate de magnésium et concentrée sous pression réduite. On obtient ainsi le produit attendu sous forme d'une huile incolore avec un rendement de 90%.
RMN ¹H (CDCl3, 300MHz) δ : 7,46 (dd, 1 H) ; 7,40-7,20 (m,3 H) ; 4,54 (s, 2H) ; 4,14 (q, 2H) ; 3,18 (t, 2H) ; 2,62 (t, 2H) ; 1,26 (t, 3H).

### PREPARATION XCIII

### Acide 3-[[2-(aminométhyl)phényl]thio]propanoïque éthyl ester

On prépare dans un réacteur adapté à l'hydrogénation sous pression, une solution de 0,1 g (0,38 mmol) du composé obtenu selon la préparation XCII dans 2 ml de THF et on ajoute 1 ml de méthanol saturé par le chlorure d'hydrogène et 10 mg de charbon palladié à 10 %. Le mélange est agité pendant 48 heures à température ambiante, sous atmosphère d'hydrogène, sous une pression de 5000 hPa, puis filtré et concentré sous pression réduite. On obtient ainsi le produit attendu sous forme d'une huile incolore avec un rendement de 98 %, engagé sans autre purification dans les étapes suivantes.

### PREPARATION XCIV

### Acide 3-[(2-cyanophényl)amino]propanoïque méthyl ester

On prépare une solution de 0,66 g (3,47 mmol) d'acide 3-[(2-cyanophényl)amino]propanoïque dans 10 ml de méthanol et on ajoute 0,3 ml de chlorure de thionyle. Le mélange est agité pendant 48 heures à doux reflux du méthanol, puis concentré sous pression réduite. Le solide résiduel est repris dans l'acétate d'éthyle et la phase organique obtenue est lavée à l'eau jusqu'à pH 7, puis séchée sur sulfate de magnésium et concentrée sous pression réduite. On obtient ainsi le produit attendu sous forme d'une huile beige avec un rendement de 90 %.
RMN ¹H (DMSO, 300MHz) δ : 7,50-7,35 (m, 2H) ; 6,80 (d, 1 H) ; 6,66, (t, 1 H); 6,07 (t, NH) ; 3,61 (s, 3H) ; 3,50-3,35 (m, 2H) ; 2,63 (t, 2H).

### PREPARATION XCV

### Acide 3-[(2-(aminométhyl)phényl]amino]propanoïque méthyl ester

On prépare dans un réacteur adapté à l'hydrogénation sous pression, une solution de 0,64 g (3,13 mmol) du composé obtenu selon la préparation XCIV dans 10 ml de méthanol et on ajoute 0,26 ml d'acide chlorhydrique concentré et 128 mg de charbon palladié à 10 %. Le mélange est agité pendant 18 heures à température ambiante, sous atmosphère d'hydrogène, sous une pression de 10000 hPa, puis filtré et concentré sous pression réduite. Le produit brut est repris par de l'acétate d'éthyle et extrait par de l'acide chlorhydrique N. La phase aqueuse acide est lavée par l'acétate d'éthyle puis amenée à pH basique à l'aide d'une solution de bicarbonate de sodium et extraite par l'acétate d'éthyle. Cette phase organique est lavée à l'eau puis séchée et concentrée sous pression réduite. On obtient ainsi le produit attendu sous forme d'une huile marron clair avec un rendement de 55 %.
RMN ¹H (CDCl3, 300MHz) δ : 7,18 (td, 1H) ; 7,03 (d, 1 H) ; 6,75-6,60 (m, 2H) ; 3,87 (s, 2H) ; 3,70 (s, 3H) ; 3,49 (t, 2H) ; 2,68 (t, 2H).

### PREPARATION XCVI

### Acide [2-(5-méthyl-2-nitrophénoxy)éthyl]carbamique, 1,1-diméthyléthyl ester

On prépare une solution de 1 g (6,53 mmol) de 5-méthyl-2-nitrophénol dans 40 ml de THF et on ajoute 1,61g (10 mmol) d'ester t-butylique de l'acide 2-hydroxyéthylcarbamique et 2,62 g (10 mmol) de triphénylphosphine. Le mélange est agité pendant 10 mn à température ambiante puis on ajoute, à 0°C, 1,98 g (9,8 mmol) de DIAD. Le milieu réactionnel est agité pendant 4 heures à température ambiante, puis concentré sous pression réduite. L'huile jaune obtenue est purifiée par chromatographie sur gel de silice en éluant à l'aide d'un mélange toluène/acétate d'éthyle (98/2 ; v/v). On obtient ainsi le produit attendu sous forme d'un solide jaune avec un rendement de 91 %.
F = 68°C.

### PREPARATION XCVII

### 2-(5-méthyl-2-nitrophénoxy)éthanamine, chlorhydrate

En opérant de façon analogue à la préparation XXVIII, au départ du composé obtenu selon la préparation XCVI, on obtient le composé attendu sous forme d'un solide beige avec un rendement de 93 %.
F = 184°C

### PREPARATION XCVIII

### Acide [2-(5-fluoro-2-nitrophénoxy)éthyl]carbamique, 1,1-diméthyléthyl ester

En opérant de façon analogue à la préparation XCVI, au départ du 5-fluoro-2-nitrophénol, on obtient le composé attendu sous forme d'un solide fin blanc avec un rendement de 96 %.
RMN ¹H (DMSO, 250MHz) δ : 7,99 (dd, 1 H) ; 7,33 (dd, 1 H) ; 6,95 (ddd, 1H) ; 4,18 (t, 2H) ; 3,40-3,20 (m, 2H) ; 1,37 (s, 9H).

### PREPARATION IC

### 2-(5-fluoro-2-nitrophénoxy)éthanamine, chlorhydrate

En opérant de façon analogue à la préparation XXVIII, au départ du composé obtenu selon la préparation XCVIII, on obtient le composé attendu sous forme d'un solide blanc avec un rendement de 84 %.
RMN ¹H (DMSO, 250 MHz) δ : 8,40-8,10 (slarge, NH3); 8,05 (dd, 1H) ; 7,42 (dd, 1 H) ; 7,03 (ddd, 1H) ; 4,42 (t, 2H) ; 3,30-3,10 (m, 2H).

### PREPARATION C

### Acide 3-[[3-(hydroxyméthyl)phényl]thio]propanoïque éthyl ester

On prépare, sous atmosphère d'argon, une solution de 1,27 g (9,07 mmol) de 3-mercapto-benzèneméthanol dans 20 ml d'éthanol et on ajoute 0,93 g (13,7 mmol) d'éthylate de sodium en solution dans 6 ml d'éthanol. Le mélange est agité 5 mn à température ambiante puis on ajoute 1,81 g (10 mmol) d'ester éthylique de l'acide 3-bromopropanoïque en solution dans 6 ml d'éthanol. Le mélange est agité pendant 72 heures à température de reflux du solvant, puis refroidi et versé sur de l'eau et extrait trois fois par l'éther éthylique. Les phases organiques rassemblées sont lavées à l'eau puis séchées sur sulfate de magnésium et concentrées sous pression réduite. L'huile orange obtenue est purifiée par chromatographie sur gel de silice en éluant à l'aide d'un mélange DCM/méthylcyclohexane (98/2 ;v/v). On obtient ainsi le produit attendu sous forme d'une huile jaune avec un rendement de 65 %.
RMN ¹H (DMSO, 300MHz) δ : 7,35-7,10 (m, 4H) ; 5,21 (t, OH) ; 4,47 (d, 2H) ; 4,06 (q, 2H) ; 3,15 (t, 2H) ; 2,60 (t, 2H), 1,17 (t, 3H).

### PREPARATION CI

### Acide 3-[[3-(chlorométhyl)phényl]thio]propanoïque éthyl ester

On prépare une solution de 1,3 g (5,4 mmol) de l'ester obtenu selon la préparation C dans 15 ml de DCM et on ajoute, à 0°C, 0,9 ml de chlorure de thionyle. Le mélange est agité pendant 3 heures à température ambiante, puis concentré sous pression réduite. Le produit résiduel est repris par du toluène et à nouveau concentré sous pression réduite. L'huile jaune obtenue est purifiée par chromatographie sur gel de silice en éluant à l'aide d'un mélange acétate d'éthyle/méthylcyclohexane (1/9 ;v/v). On obtient ainsi le produit attendu sous forme d'une huile jaune avec un rendement de 93 %.
RMN ¹H (DMSO, 300MHz) δ : 7,50-7,20 (m, 4H) ; 4,74 (, 2H) ; 4,06 (q, 2H) ; 3,18 (t, 2H) ; 2,62 (t, 2H), 1,17 (t, 3H).

### PREPARATION CII

### Acide 3-[[3-[[bis[(1,1-diméthyléthoxy)carbonyl]amino]méthyl]phényl] thio]propanoïque, éthyl ester

En opérant de façon analogue à la préparation XXVII, au départ du composé obtenu selon la préparation CI, on obtient le composé attendu sous forme d'une huile translucide avec un rendement de 68 %.
RMN ¹H (DMSO, 250MHz) δ : 7,40-7,00 (m, 4H) ; 4,66 (s, 2H) ; 4,05 (q, 2H) ; 3,14 (t, 2H) ; 2,59 (t, 2H), 1,38 (s, 18H) ; 1,17 (t, 3H).

### PREPARATION CIII

### Acide 3-[[3-(aminométhyl)phényl]thio]propanoïque éthyl ester, chlorhydrate

En opérant de façon analogue à la préparation XXVIII, au départ du composé obtenu selon la préparation CII, on obtient le composé attendu sous forme d'une huile incolore avec un rendement de 22 %.
RMN ¹H (DMSO, 300MHz) δ : 7,40-7,10 (m, 4H) ; 4,05 (q, 2H) ; 3,68 (s, 2H); 3,15 (t, 2H) ; 2,60 (t, 2H), 1,88 (slarge, NH2) ; 1,17 (t, 3H).

### PREPARATION CIV

### Acide 2,3-dihydro-1-[[4'-fluoro-2'-méthyl-[1,1-biphényl]-4-yl]sulfonyl](2S)-1H-indole-2-carboxylique.

En opérant de façon analogue à la préparation IV, au départ d'acide 4-fluoro-2-méthylphénylboronique, on obtient le composé attendu sous forme d'un solide jaune avec un rendement de 95 %.
F =62-67°C.

### PREPARATION CV

### Acide 2,3-dihydro-1-[[2',4'-difluoro-[1,1'-biphényl]-4-yl]sulfonyl]-(2S)-1H-indole-2-carboxylique.

En opérant de façon analogue à la préparation IV, au départ d'acide 2,4-difluorophénylboronique, on obtient le composé attendu sous forme d'un solide jaune avec un rendement de 74 %.
F =88-92°C.

### PREPARATION CVI

### Acide 2,3-dihydro-1-[(3'-méthyl-[1,1'-biphényl]-4-yl]sulfonyl]-(2S)-1H-indole-2-carboxylique.

En opérant de façon analogue à la préparation IV, au départ d'acide 3-méthylphénylboronique, on obtient le composé attendu sous forme d'un solide blanc avec un rendement de 98 %.
F = 123-128°C.

### PREPARATION CVII

### Acide 2,3-dihydro-1-[[2'-chloro-4'-fluoro-[1,1'-biphényl]-4-yl]sulfonyl](2S)-1H-indole-2-carboxylique.

En opérant de façon analogue à la préparation IV, au départ d'acide 2-chloro-4-fluorophénylboronique, on obtient le composé attendu sous forme d'un solide mal cristallisé avec un rendement de 97 %.
RMN ¹H (DMSO, 300MHz) δ : 8,15 (d, 2H) ; 7,90-7,50 (m, 6H) ; 7,50-7,30 (m, 2H) ; 7,21 (t, 1 H) ; 5,20 (dd, 1 H) ; 3,58 (dd, 1 H) ; 3,27 (dd, 1 H).

### PREPARATION CVIII

### Acide 2,3-dihydro-1-[[3'-chloro-[1,1'-biphényl]-4-yl]sulfonyl]-(2S)-1H-indole-2-carboxylique.

En opérant de façon analogue à la préparation IV, au départ d'acide 3-chlorophénylboronique, on obtient le composé attendu sous forme d'un solide jaune pâle avec un rendement de 73 %.
F = 78°C.

### PREPARATION CIX

### Acide 2,3-dihydro-1-[[3'-éthyl-[1,1'-biphényl]-4-yl]sulfonyl]-(2S)-1H-indole-2-carboxylique.

En opérant de façon analogue à la préparation IV, au départ d'acide 3-éthylphénylboronique, on obtient le composé attendu sous forme d'un solide blanc avec un rendement de 78 %.
F = 70-72°C

### PREPARATION CX

### Acide 4-[2-[[[(2S)-2,3-dihydro-1-[[4-(4,4,5,5-tétraméthyl-1,3,2-dioxaborolan-2-yl)phényl]sulfonyl]-1H-indol-2-yl]carbonyl]amino]éthoxy]benzèneacétique, méthyl ester

On charge dans un ballon 792 mg (8,08 mmol) d'acétate de potassium, 66 mg (0,081 mmol) de PdCl₂dppf , 753 mg (2,96 mmol) de bis(pinacolato)dibore (ou : 4,4,4',4',5,5,5',5'-octaméthyl-2,2'-bi-1,3,2-dioxaborolane) et 18 ml de DMSO préalablement dégazé. On ajoute ensuite sous agitation, à température ambiante, 1,67 g (2,70 mmol) du composé iodé obtenu selon la préparation VIIa. Le mélange réactionnel est ensuite agité à 80°C pendant 3 heures, puis refroidi et dilué dans du toluène. La phase organique est lavée à l'eau, séchée sur sulfate de magnésium et concentrée sous pression réduite. On obtient ainsi le produit attendu sous forme d'une huile marron clair avec un rendement de 98%.
RMN ¹H (DMSO, 300MHz) δ : 8,37 (t, NH) ; 7,78 (s, 4H) ; 7,45 (d, 1H) ; 7,30-6,80 (m, 7H) ; 4,83 (dd, 1 H) ; 4,00 (t, 2H) ; 3,60 (s, 3H) ; 3,60-3,35 (m, 2H) ; 3,07 (dd, 1 H) ; 2,89 (dd, 1 H) ; 2,30 (s, 2H) ; 1,28 (s, 12H).

### PREPARATION CXI

### Acide 2,3-dihydro-1-[(4'-fluoro-2'-méthoxy-[1,1'-biphényl]-4-yl)-sulfonyl]-(2S)-1H-indole-2-carboxylique

En opérant de façon analogue à la préparation IV, au départ d'acide 4-fluoro-2-méthoxyphénylboronique, on obtient le composé attendu sous forme d'un solide beige avec un rendement de 97 %.
F = 93-100°C.

### PREPARATION CXII

### Acide 2,3-dihydro-1-[[2'-chloro-5'-(trifluorométhyl)[1,1'-biphényl]-4-yl]sulfonyl]-(2S)-1H-indole-2-carboxylique

En opérant de façon analogue à la préparation IV, au départ d'acide 2-chloro-5-(trifluorométhyl)phénylboronique, on obtient le composé attendu sous forme d'un solide beige avec un rendement de 93 %.
F = 79-83°C.

### Exemple 172

### Acide 4-[[[[(2S)-1-[(4'-fluoro[1,1'-biphényl]-4-yl)sulfonyl]-2,3-dihydro-1H-indol-2-yl]carbonyl]amino]méthyl]benzoïque, méthyl ester

On prépare une solution de 150 mg (0,38 mmol) d'acide obtenu selon la préparation LXIII dans 5 ml de DCM et on ajoute 160 mg (0,84 mmol) de EDCI, 114 mg (0,84 mmol) de HOAT, 3,32 ml (2,26 mmol) de triéthylamine et enfin 76 mg (0,38 mmol) d'ester méthylique de l'acide 4-(aminométhyl) benzoïque (sous forme de son chlorhydrate). Le mélange réactionnel est ensuite agité à température ambiante pendant 20 heures, puis dilué dans 10 ml de DCM. La phase organique est lavée par une solution d'acide chlorhydrique N, puis par une solution de bicarbonate de sodium, séchée sur sulfate de magnésium et concentrée sous pression réduite. Le produit brut obtenu est purifié par chromatographie sur gel de silice en éluant à l'aide d'un mélange toluène/acétate d'éthyle/acide acétique (8/2/0,1; v/v/v). On obtient ainsi le produit attendu sous forme d'une poudre beige avec un rendement de 57 %.
F = 66-73°C.

### Exemple 173

### Acide β-[[[(2S)-1-[(4'-fluoro[1,1'-biphényl]-4-yl)sulfonyl]-2,3-dihydro-1H-indol-2-yl]carbonyl]amino]-(βS)-benzènebutanoique, 1,1-diméthyléthyl ester

En opérant de façon analogue à l'exemple 172, au départ d'ester t-butylique de l'acide (βS)-β-aminobenzènebutanoïque, on obtient le produit attendu sous forme d'une poudre beige (rendement = 62 %).
F = 67-73°C.

### Exemple 174

### N-[(2-chlorophényl)méthyl]-2,3-dihydro-1-[[3'-(trifluorométhyl)[1,1'-biphényl]-4-yl]sulfonyl]-(2S)-1H-indole-2-carboxamide

### a) chlorure de l'acide 2,3-dihydro-1-[[3'-(trifluorométhyl)[1,1'-biphényl]-4-yl]sulfonyl]-(2S)-1H-indole-2-carboxylique

Le chlorure d'acide est obtenu à partir de l'acide correspondant en opérant de façon analogue à la préparation VI.

### b) N-[(2-chlorophényl)méthyl]-2,3-dihydro-1-[[3'-(trifluorométhyl)[1,1'-biphényl]-4-yl]sulfonyl]-(2S)-1H-indole-2-carboxamide

En opérant de façon analogue à l'exemple 17, au départ du chlorure d'acide ci-dessus et de la 2-chlorobenzylamine, on obtient le composé attendu sous forme d'une poudre fine blanche (rendement = 70 %).
F = 142-144°C.

En opérant de façon analogue à l'exemple 174, on obtient les composés suivants :

### Exemple 175

### N-[(2-fluorophényl)méthyl]-2,3-dihydro-1-[[3'-(trifluorométhyl)[1,1'-biphényl]-4-yl]sulfonyl]-(2S)-1H-indole-2-carboxamide

Solide blanc (rendement = 83 %).
F = 68-74°C.

### Exemple 176

### N-[[2-(trifluorométhyl)phényl]méthyl]-2,3-dihydro-1-[[3'-(trifluorométhyl)[1,1'-biphényl]-4-yl]sulfonyl]-(2S)-1H-indole-2-carboxamide

Solide blanc (rendement = 70 %).
F = 127-129°C.

### Exemple 177

### N-[(2-méthylphényl)méthyl]-2,3-dihydro-1-[[3'-(trifluorométhyl)[1,1'-biphényl]-4-yl]sulfonyl]-(2S)-1H-indole-2-carboxamide

Solide blanc (rendement = 85 %).
F = 146-148°C.

### Exemple 178

### N-[(2-méthoxyphényl)méthyl]-2,3-dihydro-1-[[3'-(trifluorométhyl)[1,1'-biphényl]-4-yl]sulfonyl]-(2S)-1H-indole-2-carboxamide

Solide blanc (rendement = 76 %)
F = 153-155°C.

### Exemple 179

### N-[(2-pyridinyl)méthyl]-2,3-dihydro-1-[[3'-(trifluorométhyl)[1,1'-biphényl]-4-yl]sulfonyl]-(2S)-1H-indole-2-carboxamide

Solide blanc (rendement = 65 %).
F = 77-79°C.

### Exemple 18

### N-[(1-phénylcyclopropyl)méthyl]-2,3-dihydro-1-[[3'-(trifluorométhyl)-[1,1'-biphényl]-4-yl]sulfonyl]-(2S)-1H-indole-2-carboxamide

En opérant de façon analogue à la préparation I, au départ de l'acide obtenu selon la préparation IV et de la 1-phénylcyclopropane-méthanamine, on obtient le composé attendu sous forme d'une huile beige (rendement = 56 %).
RMN ¹H (DMSO, 250MHz) δ : 8,08 (t, NH) ; 8,05-7,95 (m, 2H) ; 7,94 (d, 2H) ; 7,88 (d, 2H) ; 7,85-7,65 (m, 2H) ; 7,45 (d, 1 H) ; 7,30-7,05 (m, 7H) ; 7,02 (td, 1H) ; 4,87 (dd, 1 H) ;3,53 (dd, 1H) ; 3,27 (dd, 1 H) ; 3,08 (dd, 1H) ; 2,75 (dd, 1H) ; 1,00-0,60 (m, 4H).

### Exemple 181

### N-[(2-hydroxyphényl)méthyl]-2,3-dihydro-1-[[3'-(trifluorométhyl)[1,1'-biphényl]-4-yl]sulfonyl]-(2S)-1H-indole-2-carboxamide

En opérant de façon analogue à l'exemple 64, au départ de l'acide obtenu selon la préparation IV et de la 2-hydroxybenzylamine, on obtient le composé attendu sous forme d'un solide fin blanc (rendement = 99 %).
RMN ¹H (DMSO, 300MHz) δ : 9,55 (s, OH) ; 8,54 (t, NH) ; 8,10-7,60 (m, 8H) ; 7,51 (d, 1 H) ; 7,25 (t, 1H) ; 7,20-7,00 (m, 4H) ; 6,85-6,70 (m, 2H) ; 4,96 (dd, 1 H) ; 4,40-4,15 (m, 2H) ; 3,15 (dd, 1 H) ; 2,98 (dd, 1 H).

### Exemple 182

### Acide 4-[2-([[[(2S)-2,3-dihydro-1-[[3'-(trifluorométhyl)[1,1'-biphényl]-4-yl]sulfonyl]-1H-indol-2-yl]carbonyl]amino]méthyl]phénoxy] butanoïque, méthyl ester

Dans un tube pour réaction sous micro-ondes, on mélange 302 mg (0,55 mmol) du composé obtenu selon l'exemple 181 dans 5 ml d'acétonitrile et on ajoute 105 mg (0,55 mmol) de carbonate de césium et 198 mg (1,1 mmol) d'ester méthylique de l'acide 4-bromobutanoïque. Le milieu réactionnel est chauffé 1 heure à 110°C au four micro-ondes, puis l'acétonitrile est chassé par évaporation sous pression réduite. Le résidu est repris dans de l'eau et de l'acétate d'éthyle. La phase organique est séparée, séchée sur sulfate de magnésium et concentrée sous pression réduite. Le produit brut est purifié par chromatographie sur gel de silice en éluant à l'aide d'un mélange DCM/acétate d'éthyle (99/1 ; v/v). On obtient le produit sous forme d'une mousse blanche (rendement = 49 %).
RMN ¹H (DMSO, 300MHz) δ : 8,50 (t, NH) ; 8,10-7,60 (m, 8H) ; 7,51 (d, 1H) ; 7,30-7,10 (m, 4H) ; 7,10-6,85 (m, 3H) ; 4,95 (dd, 1H) ; 4,40-4,15 (m, 2H) ; 4,02 (t, 2H) ; 3,60 (s, 3H) ; 3,18 (dd, 1 H) ; 2,98 (dd, 1 H) ; 2,52 (t, 2H) ; 2,10-1,90 (m, 2H).

### Exemple 183

### Acide 4-[2-[[[[(2S)-2,3-dihydro-1-[[3'-(trifluorométhyl)[1,1'-biphényl]-4-yl]sulfonyl]-1H-indol-2-yl]carbonyl]amino]méthyl]phénoxy] butanoïque

En opérant de façon analogue à l'exemple 22, au départ de l'ester obtenu selon l'exemple 182, on obtient le composé attendu sous forme d'une huile translucide (rendement = 93 %).
RMN ¹H (DMSO, 300MHz) δ : 12,12 (slarge, COOH) ; 8,51 (t, NH) ; 8,10-7,60 (m, 8H) ; 7,52 (d, 1 H) ; 7,30-7,10 (m, 4H) ; 7,10-6,85 (m, 3H) ; 4,95 (dd, 1 H) ; 4,40-4,15 (m, 2H) ; 4,02 (t, 2H) ; 3,17 (dd, 1 H) ; 2,99 (dd, 1H) ; 2,43 (t, 2H) ; 2,10-1,90 (m, 2H).

### Exemple 184

### Acide 4-[2-[[[(2S)-1-[(4'-fluoro-2'-méthyl[1,1'-biphényl]-4-yl)sulfonyl]-2,3-dihydro-1H-indol-2-yl]carbonyl]amino]éthoxy]benzèneacétique, méthyl ester

### a) chlorure de l'acide 2,3-dihydro-1-[(4'-fluoro-2'-méthyl[1,1'-biphényl]-4-yl)sulfonyl]-(2S)-1H-indole-2-carboxylique

Le chlorure d'acide est obtenu à partir de l'acide correspondant (Préparation CIV) en opérant de façon analogue à la préparationVI.

### b) acide 4-[2-[[[(2S)-1-[(4'-fluoro-2'-méthyl[1,1'-biphényl]-4-yl)sulfonyl]-2,3-dihydro-1H-indol-2-yl]carbonyl]amino]éthoxy]benzèneacétique, méthyl ester

En opérant de façon analogue à l'exemple 98, au départ du chlorure d'acide ci-dessus et du chlorhydrate de l'ester méthylique de l'acide 4-(2-aminoéthoxy)benzèneacétique, on obtient le composé attendu sous forme d'un solide jaune (rendement = 25 %).
F = 58-62°C.

### Exemple 185

### Acide 4-[2-[[[(2S)-1-[(4'-fluoro-2'-méthyl[1,1'-biphényl]-4-yl)sulfonyl]-2,3-dihydro-1H-indol-2-yl]carbonyl]amino]éthoxy]benzèneacétique

En opérant de façon analogue à l'exemple 22, au départ de l'ester obtenu selon l'exemple 184, on obtient le composé attendu sous forme d'un solide beige (rendement = 89 %).
F = 99-105°C.

### Exemple 186

### Acide 4-[[[[(2S)-1-[(4'-fluoro-2'-méthyl[1,1'-biphényl]-4-yl)sulfonyl]-2,3-dihydro-1H-indol-2-yl]carbonyl]amino]méthyl]benzèneacétique, méthyl ester

En opérant de façon analogue à l'exemple 184, au départ d'un équivalent du chlorhydrate de l'ester méthylique de l'acide 4-(aminométhyl)benzèneacétique, on obtient le composé attendu sous forme d'un solide jaune (rendement = 35 %).
F = 71-75°C.

### Exemple 187

### Acide 4-[[[[(2S)-1-[(4'-fluoro-2'-méthyl[1,1'-biphényl]-4-yl)sulfonyl]-2,3-dihydro-1H-indol-2-yl]carbonyl]amino]méthyl]benzèneacétique

En opérant de façon analogue à l'exemple 22, au départ de l'ester obtenu selon l'exemple 186, on obtient le composé attendu sous forme d'un solide beige (rendement = 86 %).
F = 95-100°C.

### Exemple 188

### N-[2-[[[[(2S)-1-[(4'-fluoro-2'-méthyl[1,1'-biphényl]-4-yl)sulfonyl]-2,3-dihydro-1H-indol-2-yl]carbonyl]amino]méthyl]phényl]-β-alanine, méthyl ester

En opérant de façon analogue à l'exemple 186, au départ du composé obtenu selon la préparation XCV, on obtient le composé attendu sous forme d'un solide blanc (rendement = 30 %).
F = 78-80°C.

### Exemple 189

### N-[2-[[[[(2S)-1-[(4'-fluoro-2-méthyl[1,1'-biphényl]-4-yl)sulfonyl]-2,3-dihydro-1H-indol-2-yl]carbonyl]amino]méthyl]phényl]-β-alanine

En opérant de façon analogue à l'exemple 22, au départ de l'ester obtenu selon l'exemple 188, on obtient le composé attendu sous forme d'un solide blanc (rendement = 94 %).
F = 118-126°C.

### Exemple 190

### N-[2-[[[[(2S)-1-[(4'-fluoro-2'-chloro[1,1'-biphényl]-4-yl)sulfonyl]-2,3-dihydro-1H-indol-2-yl]carbonyl]amino]méthyl]phényl]-β-alanine, méthyl ester

En opérant de façon analogue à l'exemple 21, au départ du composé obtenu selon la préparation XCV et d'acide obtenu selon la préparation CVII, on obtient le composé attendu sous forme d'un solide blanc (rendement = 46 %).
F = 70-73°C.

### Exemple 191

### N-[2-[[[[(2S)-1-[(4'-fluoro-2'-chloro[1,1'-biphényl]-4-yl)sulfonyl]-2,3-dihydro-1H-indol-2-yl]carbonyl]amino]méthyl]phényl]-β-alanine

En opérant de façon analogue à l'exemple 22, au départ de l'ester obtenu selon l'exemple 190, on obtient le composé attendu sous forme d'un solide blanc (rendement = 87 %).
F = 105-111°C.

### Exemple 192

### N-[2-[[[[(2S)-1-[(4'-fluoro-2'-méthoxy[1,1'-biphényl]-4-yl)sulfonyl]-2,3-dihydro-1H-indol-2-yl]carbonyl]amino]méthyl]phényl]-β-alanine, méthyl ester

En opérant de façon analogue à l'exemple 184, au départ de l'acide obtenu selon la préparation CXI et du composé obtenu selon la préparation XCV, on obtient le composé attendu sous forme d'un solide blanc (rendement = 28 %).
F = 78-80°C.

### Exemple 193

### N-[2-[[[[(2S)-1-[(4'-fluoro-2'-méthoxy[1,1'-biphényl]-4-yl)sulfonyl]-2,3-dihydro-1H-indol-2-yl]carbonyl]amino]méthyl]phényl]-β-alanine

En opérant de façon analogue à l'exemple 22, au départ de l'ester obtenu selon l'exemple 192, on obtient le composé attendu sous forme d'un solide blanc (rendement = 49 %).
F = 121-125°C.

### Exemple 194

### N-[2-[[[[(2S)-1-[(2',4'-difluoro[1,1'-biphényl]-4-yl)sulfonyl]-2,3-dihydro-1H-indol-2-yl]carbonyl]amino]méthyl]phényl]-β-alanine, méthyl ester

En opérant de façon analogue à l'exemple 192, au départ de l'acide obtenu selon la préparation CV, on obtient le composé attendu sous forme d'un solide blanc (rendement = 51 %).
F = 63-66°C.

### Exemple 195

### N-[2-[[[[(2S)-1-[(2',4'-difluoro[1,1'-biphényl]-4-yl)sulfonyl]-2,3-dihydro-1H-indol-2-yl]carbonyl]amino]méthyl]phényl]-β-alanine

En opérant de façon analogue à l'exemple 22, au départ de l'ester obtenu selon l'exemple 194, on obtient le composé attendu sous forme d'un solide blanc (rendement = 87 %).
F = 106-110°C.

### Exemple 196

### Acide 3-[[4-[[[[(2S)-2,3-dihydro-1-[[3'-(trifluorométhyl)[1,1'-biphényl]-4-yl]sulfonyl]-1H-indol-2-yl]carbonyl]amino]méthyl]phényl]thio] propanoïque, éthyl ester

En opérant de façon analogue à l'exemple 98, au départ de l'amine obtenue selon la préparation LXXXIX, on obtient le composé attendu sous forme d'une huile (rendement = 30 %).
RMN ¹H (DMSO, 300MHz) δ : 8,78 (t, NH) ; 8,10-8,00 (m, 2H) ; 8,00-7,85 (m, 4H) ; 7,85-7,65 (m, 2H) ; 7,51 (d, 1 H) ; 7,35-7,20 (m, 5H) ; 7,15 (d,1H) ; 7,04 (t, 1 H) ; 4,90 (dd, 1 H) ; 4,32 (t, 2H) ; 4,05 (q, 2H) ; 3,30-3,05 (m, 3H) ; 2,95 (dd, 1 H) ; 2,58 (t, 2H) ; 1,17 (t, 3H).

### Exemple 197

### Acide 3-[[4-[[[[(2S)-2,3-dihydro-1-[[3'-(trifluorométhyl)[1,1'-biphényl]-4-yl]sulfonyl]-1H-indol-2-yl]carbonyl]aminolméthyl]phényl]thio] propanoïque

En opérant de façon analogue à l'exemple 22, au départ de l'ester obtenu selon l'exemple 196, on obtient le composé attendu sous forme d'un solide blanc (rendement = 53 %).
F=77-81°C.

### Exemple 198

### Acide 3-[[2-[[[[(2S)-2,3-dihydro-1-[[3'-(trifluorométhyl)[1,1'-biphényl]4-yl]sulfonyl]-1 H-indol-2-yl]carbonyl]amino]méthyl]phényl]thio] propanoïque, éthyl ester

En opérant de façon analogue à l'exemple 21, au départ de l'acide obtenu selon la préparation IV et du composé obtenu selon la préparation XCIII, on obtient le composé attendu sous forme d'une huile (rendement = 53 %).
RMN ¹H (CDCl₃, 300MHz) δ : 7,80-7,05 (m,16 H) ; 4,73 (dd, 1H) ; 4,75-4,50 (m, 2H) ; 4,14 (q, 2H) ; 3,32 (dd, 1 H) ; 3,14 (t, 2H) ; 2,84 (dd, 1 H); 2,60 (t, 2H) ; 1,25 (t, 3H).

### Exemple 199

### Acide 3-[[2-[[[[(2S)-2,3-dihydro-1-[[3'-(trifluorométhyl)[1,1'-biphényl]-4-yl]sulfonyl]-1 H-indol-2-yl]carbonyl]amino]méthyl]phényl]thio] propanoïque

En opérant de façon analogue à l'exemple 22, au départ de l'ester obtenu selon l'exemple 198, on obtient le composé attendu sous forme d'un solide blanc (rendement = 51 %).
F = 85-89°C.

### Exemple 200

### Acide 3,5-diméthyl-4-[2-[[[(2S)-1-[[3'-(trifluorométhyl)[1,1'-biphényl]-4-yl]sulfonyl]-2,3-dihydro-1 H-indol-2-yl]carbonyl]amino]éthoxy] -benzoïque, méthyl ester

En opérant de façon analogue à l'exemple 198, au départ du composé obtenu selon la préparation LXXI, on obtient le produit attendu sous forme d'un solide blanc (rendement = 51 %).
F = 81-83°C.

### Exemple 201

### Acide 3,5-diméthyl-4-[2-[[[(2S)-1-[[3-(trifluorométhyl)[1,1'-biphényl]-4-yl]sulfonyl]-2,3-dihydro-1H-indol-2-yl]carbonyl]amino]éthoxy] -benzoïque

En opérant de façon analogue à l'exemple 22, au départ de l'ester obtenu selon l'exemple 200, on obtient le composé attendu sous forme d'un solide blanc (rendement = 59 %).
F=120-126°C.

### Exemple 202

### Acide 3,5-diméthyl-4-[2-[[[(2S)-1-[[4'-fluoro[1,1'-biphényl]-4-yl]sulfonyl]-2,3-dihydro-1H-indol-2-yl]carbonyl]amino]éthoxy]benzoïque, méthyl ester

En opérant de façon analogue à l'exemple 200, au départ de l'acide obtenu selon la préparation LXIII, on obtient le produit attendu sous forme d'un solide blanc (rendement = 68 %).
F = 91°C.

### Exemple 203

### Acide 3,5-diméthyl-4-[2-[[[(2S)-1-[[4'-fluoro[1,1'-biphényl]-4-yl]sulfonyl]-2,3-dihydro-1H-indol-2-yl]carbonyl]amino]éthoxy]benzoïque

En opérant de façon analogue à l'exemple 22, au départ de l'ester obtenu selon l'exemple 202, on obtient le composé attendu sous forme d'un solide blanc (rendement = 59 %).
F = 124-128°C.

### Exemple 204

### Acide 3-chloro-4-[2-[[[(2S)-1-[[3'-(trifluorométhyl)[1,1'-biphényl]-4-yl]sulfonyl]-2,3-dihydro-1H-indol-2-yl]carbonyl]amino]éthoxy]benzoïque, méthyl ester

En opérant de façon analogue à l'exemple 172, au départ de l'acide obtenu selon la préparation IV et du composé obtenu selon la préparation LXXIII, on obtient le composé attendu sous forme d'une poudre blanche (rendement = 42 %).
F = 84-88°C.

### Exemple 205

### Acide 3-chloro-4-[2-[[[(2S)-1-[[3'-(trifluorométhyl)[1,1'-biphényl]-4-yl]sulfonyl]-2,3-dihydro-1H-indol-2-yl]carbonyl]amino]éthoxy]benzoïque

En opérant de façon analogue à l'exemple 22, au départ de l'ester obtenu selon l'exemple 204, on obtient le composé attendu sous forme d'un solide blanc (rendement = 46 %).
F = 135-140°C.

### Exemple 206

### Acide 3-chloro-4-[2-[[[(2S)-1-[(4'-fluoro[1,1'-biphényl]-4-yl)sulfonyl]-2,3-dihydro-1H-indol-2-yl]carbonyl]amino]éthoxy]benzoïque, méthyl ester

En opérant de façon analogue à l'exemple 204, au départ de l'acide obtenu selon la préparation LXIII, on obtient le composé attendu sous forme d'un solide blanc (rendement = 55 %).
F = 84-90°C.

### Exemple 207

### Acide 3-chloro-4-[2-[[[(2S)-1-[[3'-(trifluorométhyl)[1,1'-biphényl]-4-yl]sulfonyl]-2,3-dihydro-1H-indol-2-yl]carbonyl]amino]éthoxy]benzoïque

En opérant de façon analogue à l'exemple 22, au départ de l'ester obtenu selon l'exemple 206, on obtient le composé attendu sous forme d'un solide jaune (rendement = 56 %).
F = 175-182°C.

### Exemple 208

### Acide 3-fluoro-4-[2-[[[(2S)-1-[[3'-(trifluorométhyl)[1,1'-biphényl]-4-yl]sulfonyl]-2,3-dihydro-1H-indol-2-yl]carbonyl]amino]éthoxy]-benzoïque, méthyl ester

En opérant de façon analogue à l'exemple 204, au départ du composé obtenu selon la préparation LXXV, on obtient le composé attendu sous forme d'un solide blanc (rendement = 50 %).
F = 65-70°C.

### Exemple 209

### Acide 3-fluoro-4-[2-[[[(2S)-1-[[3-(trifluorométhyl)[1,1'-biphényl]-4-yl]sulfonyl]-2,3-dihydro-1H-indol-2-yl]carbonyl]amino]éthoxy]-benzoïque

En opérant de façon analogue à l'exemple 22, au départ de l'ester obtenu selon l'exemple 208, on obtient le composé attendu sous forme d'un solide blanc (rendement = 64 %).
F = 96-104°C.

### Exemple 210

### Acide 3-fluoro-4-[2-[[[(2S)-1-[(4'-fluoro[1,1'-biphényl]-4-yl)sulfonyl]2,3-dihydro-1H-indol-2-yl]carbonyl]amino]éthoxy]-benzoïque, méthyl ester

En opérant de façon analogue à l'exemple 206, au départ du composé obtenu selon la préparation LXXV, on obtient le composé attendu sous forme d'un solide blanc. (Rendement = 56 %)
F = 82-87°C

### Exemple 211

### Acide 3-fluoro-4-[2-[[[(2S)-1-[(4'-fluoro[1,1'-biphényl]-4-yl)sulfonyl]-2,3-dihydro-1H-indol-2-yl]carbonyl]amino]éthoxy]-benzoïque

En opérant de façon analogue à l'exemple 22, au départ de l'ester obtenu selon l'exemple 210, on obtient le composé attendu sous forme d'un solide blanc (rendement = 56 %).
F = 118°C.

### Exemple 212

### Acide 4-[2-[[[(2S)-2,3-dihydro-1-[[3'-(trifluorométhyl)[1,1'-biphényl]-4-yl]sulfonyl]-1H-indol-2-yl]carbonyl]amino]éthoxy]-α-méthyl-benzène-acétique, méthyl ester

En opérant de façon analogue à l'exemple 204, au départ du composé obtenu selon la préparation LXXVII, on obtient le composé attendu sous forme d'un solide blanc (rendement = 49 %).
F=71-74°C.

### Exemple 213

### Acide 4-[2-[[[(2S)-2,3-dihydro-1-[[3'-(trifluorométhyl)[1,1'-biphényl]-4-yl]sulfonyl]-1H-indol-2-yl]carbonyl]amino]éthoxy]-α-méthyl-benzèneacétique

En opérant de façon analogue à l'exemple 22, au départ de l'ester obtenu selon l'exemple 212, on obtient le composé attendu sous forme d'un solide blanc (rendement = 93 %).
F = 92-96°C.

### Exemple 214

### Acide 4-[2-[[[(2S)-2,3-dihydro-1-[[3'-(trifluorométhyl)[1,1'-biphényl]-4-yl]sulfonyl]-1H-indol-2-yl]carbonyl]amino]éthoxy]-3-méthoxybenzène-acétique, méthyl ester

En opérant de façon analogue à l'exemple 98, au départ d'un équivalent du composé obtenu selon la préparation LXXIX, on obtient le composé attendu sous forme d'une poudre blanche (rendement = 40 %).
F = 52-60°C.

### Exemple 215

### Acide 4-[2-[[[(2S)-2,3-dihydro-1-[[3'-(trifluorométhyl)[1,1'-biphényl]-4-yl]sulfonyl]-1 H-indol-2-yl]carbonyl]amino]éthoxy]-3-méthoxybenzène-acétique

En opérant de façon analogue à l'exemple 22, au départ de l'ester obtenu selon l'exemple 214, on obtient le composé attendu sous forme d'un solide blanc (rendement = 86 %).
F = 89-98°C.

### Exemple 216

### N-[4-[[[[(2S)-2,3-dihydro-1-[[3-(trifluorométhyl)[1,1'-biphényl]-4-yl]sulfonyl]-1H-indol-2-yl]carbonyl]amino]méthyl]phényl]glycine méthyl ester

En opérant de façon analogue à l'exemple 198, au départ du chlorhydrate de l'ester méthylique de la N-[4-(aminométhyl)phényl]glycine, on obtient le composé attendu sous forme d'une poudre blanche (rendement = 32 %).
F = 85°C.

### Exemple 217

### N-[4-[[[[(2S)-2,3-dihydro-1-[[3'-(trifluorométhyl)[1,1'-biphényl]-4-yl]sulfonyl]-1H-indol-2-yl]carbonyl]amino]méthyl]phényl]glycine

En opérant de façon analogue à l'exemple 22, au départ de l'ester obtenu selon l'exemple 216, on obtient le composé attendu sous forme d'un solide jaune (rendement = 40 %).
F = 139°C.

### Exemple 218

### Acide 2,6-difluoro-4-[2-[[[(2S)-1-[[3'-(trifluorométhyl)[1,1'-biphényl]-4-yl]sulfonyl]-2,3-dihydro-1H-indol-2-yl]carbonyl]amino]éthoxy]benzoïque, méthyl ester

En opérant de façon analogue à l'exemple 198, au départ du composé obtenu selon la préparation LXXXI, on obtient le composé attendu sous forme d'un solide blanc (rendement = 65 %).
F=68-71°C.

### Exemple 219

### Acide 2,6-difluoro-4-[2-[[[(2S)-1-[(4'-fluoro[1,1'-biphényl]-4-yl)-sulfonyl]-2,3-dihydro-1H-indol-2-yl]carbonyl]amino]éthoxy]benzoïque, méthyl ester

En opérant de façon analogue à l'exemple 218, au départ de l'acide obtenu selon la préparation LXIII, on obtient le composé attendu sous forme d'un solide blanc (rendement = 55 %).
F = 83°C.

### Exemple 220

### Acide 4-[2-[[[(2S)-1-[[3'-(trifluorométhyl)[1,1'-biphényl]-4-yl]sulfonyl]-2,3-dihydro-1H-indol-2-yl]carbonyl]amino]éthoxy]-3-(trifluorométhyl)-benzoïque, méthyl ester

En opérant de façon analogue à l'exemple 198, au départ du composé obtenu selon la préparation LXXXIII, on obtient le composé attendu sous forme d'un solide blanc (rendement = 74 %).
F = 92-98°C.

### Exemple 221

### Acide 4-[2-[[[(2S)-1-[[3'-(trifluorométhyl)[1,1'-biphényl]-4-yl]sulfonyl]2,3-dihydro-1H-indol-2-yl]carbonyl]amino]éthoxy]-3-(trifluorométhyl)-benzoïque

En opérant de façon analogue à l'exemple 22, au départ de l'ester obtenu selon l'exemple 220, on obtient le composé attendu sous forme d'un solide blanc (rendement = 51 %).
F=111-116°C.

### Exemple 222

### Acide 4-[2-[[[(2S)-1-[(4'-fluoro[1,1'-biphényl]-4-yl)sulfonyl]-2,3-dihydro-1H-indol-2-yl]carbonyl]amino]éthoxy]-3-(trifluorométhyl)-benzoïque, méthyl ester

En opérant de façon analogue à l'exemple 220, au départ de l'acide obtenu selon la préparation LXIII, on obtient le composé attendu sous forme d'un solide blanc (rendement = 69 %).
F = 90-95°C.

### Exemple 223

### Acide 4-[2-[[[(2S)-1-[(4'-fluoro[1,1'-biphényl]-4-yl)sulfonyl]-2,3-dihydro-1H-indol-2-yl]carbonyl]amino]éthoxy]-3-(trifluorométhyl)-benzoïque

En opérant de façon analogue à l'exemple 22, au départ de l'ester obtenu selon l'exemple 222, on obtient le composé attendu sous forme d'un solide blanc (rendement = 85 %).
F = 139-142°C.

### Exemple 224

### Acide 4-[2-[[[(2S)-1-[[3-(trifluorométhyl)[1,1'-biphényl]-4-yl]sulfonyl]-2,3-dihydro-1H-indol-2-yl]carbonyl]amino]éthoxy]-2-(trifluorométhyl)-benzoïque, méthyl ester

En opérant de façon analogue à l'exemple 198, au départ du composé obtenu selon la préparation LXXXV, on obtient le composé attendu sous forme d'un solide blanc (rendement = 48 %).
F = 75-79°C.

### Exemple 225

### Acide 4-[2-[[[(2S)-1-[(4'-fluoro[1,1'-biphényl]-4-yl)sulfonyl]-2,3-dihydro-1H-indol-2-yl]carbonyl]amino]éthoxy]-2-(trifluorométhyl)-benzoïque, méthyl ester

En opérant de façon analogue à l'exemple 224, au départ de l'acide obtenu selon la préparation LXIII, on obtient le composé attendu sous forme d'un solide blanc (rendement = 83 %).
F = 62-70°C.

### Exemple 226

### Acide 3-méthyl-4-[2-[[[(2S)-1-[[3'-(trifluorométhyl)[1,1'-biphényl]-4-yl]sulfonyl]-2,3-dihydro-1H-indol-2-yl]carbonyl]amino]éthoxy]benzoïque, méthyl ester

En opérant de façon analogue à l'exemple 198, au départ du composé obtenu selon la préparation LXXXVII, on obtient le composé attendu sous forme d'un solide blanc (rendement = 21 %).
F = 68-74°C.

### Exemple 227

### Acide 3-méthyl-4-[2-[[[(2S)-1-[[3'-(trifluorométhyl)[1,1'-biphényl]-4-yl]sulfonyl]-2,3-dihydro-1H-indol-2-yl]carbonyl]amino]éthoxy]benzoïque

En opérant de façon analogue à l'exemple 22, au départ de l'ester obtenu selon l'exemple 226, on obtient le composé attendu sous forme d'un solide blanc (rendement = 83 %).
F = 102-108°C.

### Exemple 228

### Acide 4-[2-[[[(2S)-1-[(4'-fluoro[1,1'-biphényl]-4-yl)sulfonyl]-2,3-dihydro-1H-indol-2-yl]carbonyl]amino]éthoxy]-3-méthylbenzoïque, méthyl ester

En opérant de façon analogue à l'exemple 226, au départ de l'acide obtenu selon la préparation LXIII, on obtient le composé attendu sous forme d'un solide rose (rendement = 61 %).
F = 81-85°C.

### Exemple 229

### Acide 4-[2-[[[(2S)-1-[(4-fluoro[1,1'-biphényl]-4-yl)sulfonyl]-2,3-dihydro-1 H-indol-2-yl]carbonyl]amino]éthoxy]-3-méthylbenzoïque

En opérant de façon analogue à l'exemple 22, au départ de l'ester obtenu selon l'exemple 226, on obtient le composé attendu sous forme d'un solide blanc (rendement = 48 %).
F=112-118°C.

### Exemple 230

### Acide 2-fluoro-4-[2-[[[(2S)-1-[(4'-fluoro[1,1'-biphényl]-4-yl)sulfonyl]-2,3-dihydro-1H-indol-2-yl]carbonyl]amino]éthoxy]benzoïque, méthyl ester

En opérant de façon analogue à l'exemple 228, au départ du composé obtenu selon la préparation LXIX, on obtient le composé attendu sous forme d'un solide blanc (rendement = 58 %).
F=77-81°C.

### Exemple 231

### Acide 2-fluoro-4-[2-[[[(2S)-1-[(4'-fluoro[1,1'-biphényl]-4-yl)sulfonyl]-2,3-dihydro-1H-indol-2-yl]carbonyl]amino]éthoxy]benzoïque

En opérant de façon analogue à l'exemple 22, au départ de l'ester obtenu selon l'exemple 230, on obtient le composé attendu sous forme d'un solide beige (rendement = 52 %).
F = 109-113°C.

### Exemple 232

### Acide 2-fluoro-4-[2-[[[(2S)-1-[[3'-(trifluorométhyl)[1,1'-biphényl]-4-yl]sulfonyl]-2,3-dihydro-1H-indol-2-yl]carbonyl]amino]éthoxy]-benzoïque, méthyl ester

En opérant de façon analogue à l'exemple 198, au départ du composé obtenu selon la préparation LXIX, on obtient le composé attendu sous forme d'un solide blanc (rendement = 22 %).
F = 63-70°C.

### Exemple 233

### Acide 2-fluoro-4-[2-[[[(2S)-1-[[3'-(trifluorométhyl)[1,1'-biphényl]-4-yl]sulfonyl]-2,3-dihydro-1H-indol-2-yl]carbonyl]amino]éthoxy] benzoïque

En opérant de façon analogue à l'exemple 22, au départ de l'ester obtenu selon l'exemple 232, on obtient le composé attendu sous forme d'un solide blanc (rendement = 90 %).
F=97-105°C.

### Exemple 234

### 2,3-dihydro-N-[2-(5-méthyl-2-nitrophénoxy)éthyl]-1-[[3'-(trifluorométhyl)[1,1'-biphényl]-4-yl]sulfonyl]-(2S)-1H-indole-2-carboxamide

On prépare une solution de 480 mg (1,07 mmol) d'acide obtenu selon la préparation IV dans 10 ml de dichlorométhane puis on ajoute 226 mg (1,18 mmol) de EDCI et 30 mg (0,22 mmol) de HOAT. Après 10 minutes sous agitation à température ambiante, on ajoute 250 mg (1,07 mmol) du composé obtenu selon la préparation XCVII et 0,315 ml (2,25 mmol) de triéthylamine. Le milieu réactionnel est ensuite agité à température ambiante pendant 20 heures. Le mélange est alors traité par addition de dichlorométhane et la phase organique est lavée par de l'eau. La phase organique est ensuite séchée sur sulfate de magnésium, puis concentrée sous pression réduite. Le produit brut obtenu est ensuite purifié par chromatographie sur gel de silice en éluant à l'aide d'un mélange dichlorométhane/acétate d'éthyle (99/1 ; v/v). On obtient ainsi le produit attendu sous forme d'une mousse beige (rendement = 42 %).
RMN ¹H (DMSO, 250MHz) δ : 8,37 (t, NH) ; 8,10-7,60 (m, 9H) ; 7,49 (d, 1H) ; 7,30-6,85 (m, 5H) ; 4,86 (dd, 1 H) ; 4,30-4,10 (m, 2H) ; 3,70-3,40 (m, 2H) ; 3,12 (dd, 1 H) ; 2,94 (dd, 1 H) ; 2,37 (s, 3H).

### Exemple 235

### 2,3-dihydro-N-[2-(2-amino-5-méthylphénoxy)éthyl]-1-[[3'-(trifluorométhyl)[1,1'-biphényl]-4-yl]sulfonyl]-(2S)-1H-indole-2-carboxamide

En opérant de façon analogue à l'exemple 61, au départ du dérivé nitré obtenu selon l'exemple 234, on obtient le composé attendu sous forme d'un solide blanc (rendement = 86 %).
RMN ¹H (DMSO, 250MHz) δ : 8,46 (t, NH) ; 8,10-7,60 (m, 8H) ; 7,49 (d, 1H) ; 7,24 (td, 1 H) ; 7,13 (d, 1 H) ; 7,02 (td, 1 H) ; 6,70-6,40 (m, 3H) ; 4,86 (dd, 1 H) ; 4,57 (slarge, NH2) ; 3,95 (t, 2H) ; 3,70-3,40 (m, 2H) ; 3,18 (dd, 1H) ; 2,96 (dd, 1 H) ; 2,14 (s, 3H).

### Exemple 236

### 2,3-dihydro-N-[2-(5-fluoro-2-nitrophénoxy)éthyl]-1-[[3'-(trifluorométhyl)[1,1'-biphényl]-4-yl]sulfonyl]-(2S)-1H-indole-2-carboxamide

En opérant de façon analogue à l'exemple 234, au départ du composé obtenu selon la préparation IC, on obtient le composé attendu sous forme d'une mousse jaune vif (rendement = 54 %).
RMN ¹H (DMSO, 250MHz) δ : 8,37 (t, NH) ; 8,10-7,60 (m, 9H) ; 7,49 (d, 1H) ;7,37 (dd, 1H) ; 7,24 (td, 1H) ; 7,20-6,90 (m, 3H) ; 4,85 (dd, 1 H) ; 4,30-4,10 (m, 2H) ; 3,70-3,40 (m, 2H) ; 3,15 (dd, 1 H) ; 2,94 (dd, 1 H).

### Exemple 237

### 2,3-dihydro-N-[2-(2-amino-5-fluorophénoxy)éthyl]-1-[[3'-(trifluorométhyl)[1,1'-biphényl]-4-yl]sulfonyl]-(2S)-1H-indole-2-carboxamide

En opérant de façon analogue à l'exemple 235, au départ du dérivé nitré obtenu selon l'exemple 236, on obtient le composé attendu sous forme d'une mousse rose pâle (rendement = 90 %).
RMN ¹H (DMSO, 250MHz) δ : 8,49 (t, NH) ; 8,10-7,65 (m, 8H) ; 7,49 (d, 1 H) ; 7,24 (td, 1H) ; 7,12 (d, 1 H) ; 7,02 (td, 1 H) ; 6,73 (dd, 1 H) ; 6,65-6,40 (m, 2H) ; 4,85 (dd, 1 H) ; 4,72 (slarge, NH2) ; 3,98 (t, 2H) ; 3,70-3,40 (m, 2H) ; 3,18 (dd, 1 H) ; 2,96 (dd, 1 H).

### Exemple 238

### 2,3-dihydro-N-[2-(2-nitrophénoxy)éthyl]-1-[[3-(trifluorométhyl)[1,1'-biphényl]-4-yl]sulfonyl]-(2S)-1H-indole-2-carboxamide

En opérant de façon analogue à l'exemple 234, au départ de 2-(2-nitrophénoxy)éthanamine, on obtient le composé attendu sous forme d'une mousse jaune (rendement = 51 %).
RMN ¹H (DMSO, 300MHz) δ : 8,39 (t, NH) ; 8,10-7,65 (m, 9H) ; 7,63 (td, 1 H) ; 7,49 (d, 1 H) ; 7,39 (d, 1 H) ; 7,25 (td, 1 H) ; 7,20-7,10 (m, 2H) ; 7,02 (td, 1 H) ; 4,86 (dd, 1 H) ; 4,30-4,15 (m, 2H) ; 3,65-3,40 (m, 2H) ; 3,15 (dd, 1 H) ; 2,94 (dd, 1 H).

### Exemple 239

### 2,3-dihydro-N-[2-(2-aminophénoxy)éthyl]-1-[[3'-(trifluorométhyl)[1,1'-biphényl]-4-yl]sulfonyl]-(2S)-1H-indole-2-carboxamide

En opérant de façon analogue à l'exemple 235, au départ du dérivé nitré obtenu selon l'exemple 238, on obtient le composé attendu sous forme d'une mousse blanche (rendement = 59 %).
RMN ¹H (DMSO, 250MHz) δ : 8,48 (t, NH) ; 8,10-7,65 (m, 8H) ; 7,49 (d, 1 H) ; 7,23 (td, 1 H) ; 7,12 (d, 1 H) ; 7,02 (td, 1 H) ; 6,78 (dd, 1 H) ; 6,75-6,60 (m, 2H) ; 6,55-6,40 (m, 1 H) ; 4,86 (dd, 1 H) ; 4,76 (slarge, NH2) ; 3,96 (t, 2H) ; 3,65-3,40 (m, 2H) ; 3,18 (dd, 1 H) ; 2,96 (dd, 1 H).

### Exemple 240

### Acide 3-[[3-[[[[(2S)-2,3-dihydro-1-[[3'-(trifluorométhyl)[1,1'-biphényl]-4-yl]sulfonyl]-1H-indol-2-yl]carbonyl]amino]méthyl]phényl]thio] propanoïque, éthyl ester

On prépare une solution de 129 mg (0,29 mmol) d'acide obtenu selon la préparation IV dans 3 ml de dichlorométhane puis on ajoute 61 mg (0,32 mmol) de EDCI et 44 mg (0,32 mmol) de HOAT. Après 10 minutes sous agitation à température ambiante, on ajoute 69 mg (0,29 mmol) du chlorhydrate d'ester éthylique de l'acide 3-[[3-(aminométhyl)phényl]thio]-propanoïque (préparation CIII). Le milieu réactionnel est ensuite agité à température ambiante pendant 6 heures. Le mélange est alors traité par addition de dichlorométhane et la phase organique est lavée par de l'eau. La phase organique est ensuite séchée sur sulfate de magnésium, puis concentrée sous pression réduite. Le produit brut obtenu est ensuite purifié par chromatographie sur gel de silice en éluant à l'aide d'un mélange dichlorométhane/acétate d'éthyle (98/2 ; v/v). On obtient ainsi le produit attendu sous forme d'une huile translucide (rendement = 49 %).
RMN ¹H (DMSO, 300MHz) δ : 8,79 (t, NH) ; 8,10-7,65 (m, 8H) ; 7,51 (d, 1 H) ; 7,35-7,00 (m, 7H) ; 4,91 (dd, 1 H) ; 4,34 (d, 2H) ; 4,05 (q, 2H) ; 3,30-3,10 (m, 3H) ; 2,97 (dd, 1 H) ; 2,60 (t, 2H) ; 1,16 (t, 3H).

### Exemple 241

### Acide 3-[[3-[[[[(2S)-2,3-dihydro-1-[[3'-(trifluorométhyl)[1,1'-biphényl]-4-yl]sulfonyl]-1H-indol-2-yl]carbonyl]amino]méthyl]phényl]thio] propanoïque

En opérant de façon analogue à l'exemple 22, au départ de l'ester obtenu selon l'exemple 240, on obtient le composé attendu sous forme d'une huile translucide (rendement = 22 %).
RMN ¹H (DMSO, 250MHz) δ : 12,30 (slarge, COOH) ; 8,79 (t, NH) ; 8,10-7,65 (m, 8H) ; 7,51 (d, 1 H) ; 7,35-7,00 (m, 7H) ; 4,90 (dd, 1 H) ; 4,34 (dd, 2H) ; 3,30-3,05 (m, 3H) ; 2,97 (dd, 1 H) ; 2,60-2,40 (m, 2H).

### Exemple 242

### Acide 3-[[[(2S)-1-[(2'-chloro-4'-fluoro-[1,1'-biphényl]-4-yl)sulfonyl]-2,3-dihydro-1H-indol-2-yl]carbonyl]amino]-4,4,4-trifluorobutanoique, éthyl ester

En opérant de façon analogue à l'exemple 21, au départ d'acide obtenu selon la préparation CVII et de l'ester éthylique de l'acide 3-amino-4,4,4-trifluorobutanoique, on obtient le composé attendu sous forme d'une poudre blanche (rendement = 55 %).
F = 57-60°C.

### Exemple 243

### N-[(3-amino-4-pyridinyl)méthyl]-2,3-dihydro-1-[[3'-(trifluorométhyl)-[1,1'-biphényl]-4-yl]sulfonyl]-(2S)-1H-indole-2-carboxamide

En opérant de façon analogue à l'exemple 234 (avec 3 équivalents de triéthylamine), au départ du dichlorhydrate de 3-amino-4-pyridine-méthanamine, on obtient le composé attendu sous forme d'une poudre blanche (rendement = 85 %).
F = 105-108°C.

### Exemple 244

### Acide 4-[3-[[[(2S)-2,3-dihydro-1-[[3'-(trifluorométhyl)[1,1'-biphényl]-4-yl]sulfonyl]-1H-indol-2-yl]carbonyl]amino]propyl]benzèneacétique, méthyl ester

En opérant de façon analogue à l'exemple 234, au départ du chlorhydrate de l'ester méthylique de l'acide 4-(3-aminopropyl)benzèneacétique, on obtient le composé attendu sous forme d'une huile blanche (rendement = 98%).
RMN ¹H (DMSO, 300MHz) δ : 8,25 (t, NH) ; 8,10-7,65 (m, 8H) ; 7,49 (d, 1 H) ; 7,24 (t, 1 H) ; 7,20-7,10 (m, 5H) ; 7,02 (td, 1 H) ; 4,81 (dd, 1H) ; 3,62 (s, 2H) ; 3,60 (s, 3H) ; 3,25-3,05 (m, 3H) ; 2,94 (dd, 1 H) ; 2,57 (t, 2H) ; 1,80-1,60 (m, 2H).

### Exemple 245

### Acide 4-[3-[[[(2S)-2,3-dihydro-1-[[3'-(trifluorométhyl)[1,1'-biphényl]-4-yl]sulfonyl]-1 H-indol-2-yl]carbonyl]amino]propyl]benzèneacétique

En opérant de façon analogue à l'exemple 5, au départ du composé obtenu selon l'exemple 244, on obtient le composé attendu sous forme d'une mousse blanche (rendement = 99 %).
RMN ¹H (DMSO, 300MHz) δ : 8,25 (t, NH) ; 8,10-7,65 (m, 8H) ; 7,49 (d, 1 H) ; 7,24 (t, 1 H) ; 7,20-7,10 (m, 5H) ; 7,02 (td, 1 H) ; 4,81 (dd, 1 H) ; 3,50 (s, 2H) ; 3,25-3,05 (m, 3H) ; 2,94 (dd, 1H) ; 2,57 (t, 2H) ; 1,80-1,60 (m, 2H).

### Exemple 246

### 1-[[2',4'-difluoro-[1,1'-biphényl]-4-yl]-sulfonyl]-2,3-dihydro-N-[(2-nitrophényl)méthyl]-(2S)-1H-indole-2-carboxamide

En opérant de façon analogue à la préparation XXXV, au départ de l'acide obtenu selon la préparation CV, on obtient le chlorure d'acide que l'on fait réagir, suivant le mode opératoire décrit pour l'exemple 98, avec la (2-nitrophényl)méthanamine. On obtient ainsi le produit attendu sous forme d'un solide blanc (rendement = 64 %).
F = 99-102°C.

### Exemple 247

### N-[(2-aminophényl)méthyl]-1-[[2',4-difluoro-[1,1'-biphényl]-4-yl]-sulfonyl]-2,3-dihydro-(2S)-1H-indole-2-carboxamide

En opérant de façon analogue à la préparation XCIII, mais sans ajouter de chlorure d'hydrogène, au départ du composé obtenu selon l'exemple 246, on obtient le composé attendu sous forme d'un solide blanc (rendement = 49 %).
F = 93-96°C.

### Exemple 248

### 2,3-dihydro-1-[[4'-fluoro-2'-méthyl-[1,1'-biphényl]-4-yl]-sulfonyl]-N-[(2-nitrophényl)méthyl]-(2S)-1H-indole-2-carboxamide

En opérant de façon analogue à l'exemple 246, au départ de l'acide obtenu selon la préparation CIV, on obtient le produit attendu sous forme d'un solide orange (rendement = 87 %).
F = 64-66°C.

### Exemple 249

### N-[(2-aminophényl)méthyl]-2,3-dihydro-1-[[4'-fluoro-2'-méthyl-[1,1'-biphényl]-4-yl]-sulfonyl]-(2S)-1H-indole-2-carboxamide

En opérant de façon analogue à l'exemple 247, au départ du composé obtenu selon l'exemple 248, on obtient le produit attendu sous forme d'un solide blanc (rendement = 58 %).
F = 92-96°C.

### Exemple 250

### Acide 4-[2-[[[(2S)-1-[(4'-fluoro-2'-méthoxy[1,1'-biphényl]-4-yl)-sulfonyl]-2,3-dihydro-1H-indol-2-yl]carbonyl]amino]éthoxy] benzèneacétique, méthyl ester

En opérant de façon analogue à l'exemple 192, au départ du chlorhydrate de l'ester méthylique de l'acide 4-(2-aminoéthoxy)benzèneacétique, on obtient le produit attendu sous forme d'un solide blanc (rendement = 64%).
F = 70-75°C.

### Exemple 251

### Acide 4-[2-[[[(2S)-1-[(4'-fluoro-2'-méthoxy[1,1'-biphényl]-4-yl)sulfonyl]-2,3-dihydro-1H-indol-2-yl]carbonyl]amino]éthoxy] benzèneacétique

En opérant de façon analogue à l'exemple 22, au départ du composé obtenu selon l'exemple 250, on obtient le produit attendu sous forme d'un solide blanc (rendement = 82 %).
F = 102-110°C.

### Exemple 252

### Acide 4-[[[[(2S)-1-[(4'-fluoro-2'-méthoxy[1,1'-biphényl]-4-yl)sulfonyl]-2,3-dihydro-1H-indol-2-yl]carbonyl]amino]méthyl]benzèneacétique, méthyl ester

En opérant de façon analogue à l'exemple 192, au départ du chlorhydrate de l'ester méthylique de l'acide 4-(aminométhyl)benzèneacétique, on obtient le produit attendu sous forme d'un solide blanc (rendement = 62 %).
F = 77-79°C.

### Exemple 253

### Acide 4-[[[[(2S)-1-[(4'-fluoro-2'-méthoxy[1,1'-biphényl]-4-yl)sulfonyl]-2,3-dihydro-1H-indol-2-yl]carbonyl]amino]méthyl]benzèneacétique

En opérant de façon analogue à l'exemple 22, au départ du composé obtenu selon l'exemple 252, on obtient le produit attendu sous forme d'un solide blanc (rendement = 95 %).
F=106-111°C

### Exemple 254

### N-[(2-aminophényl)méthyl]-1-[[4'-fluoro-2'-méthoxy[1,1'-biphényl]-4-yl]-sulfonyl]-2,3-dihydro-(2S)-1H-indole-2-carboxamide

En opérant de façon analogue à l'exemple 240, au départ de l'acide obtenu selon la préparation CXI et de (2-aminophényl)méthaneamine, on obtient le produit attendu sous forme d'une mousse blanche (rendement = 64%).
F = 92-97°C.

### Exemple 255

### Acide 4-[[[[(2S)-1-[(2',4'-difluoro[1,1'-biphényl]-4-yl)sulfonyl]-2,3-dihydro-1H-indol-2-yl]carbonyl]amino]méthyl]benzèneacétique, méthyl ester

En opérant de façon analogue à l'exemple 186, au départ de l'acide obtenu selon la préparation CV, on obtient le composé attendu sous forme d'un solide blanc (rendement = 59 %).
F = 68-72°C.

### Exemple 256

### Acide 4-[[[[(2S)-1-[(2',4'-difluoro[1,1'-biphényl]-4-yl)sulfonyl]-2,3-dihydro-1H-indol-2-yl]carbonyl]amino]méthyl]benzèneacétique

En opérant de façon analogue à l'exemple 22, au départ du composé obtenu selon l'exemple 255, on obtient le produit attendu sous forme d'un solide blanc (rendement = 81 %).
F = 117-121°C.

### Exemple 257

### Acide 4-[2-[[[(2S)-1-[(2',4'-difluoro[1,1'-biphényl]-4-yl)sulfonyl]-2,3-dihydro-1H-indol-2-yl]carbonyl]amino]éthoxy]benzèneacétique, méthyl ester

En opérant de façon analogue à l'exemple 250, au départ du chlorure de l'acide obtenu selon la préparation CV, on obtient le produit attendu sous forme d'un solide blanc (rendement = 49 %).
F = 60-64°C.

### Exemple 258

### Acide 4-[2-[[[(2S)-1-[(2',4'-difluoro[1,1'-biphényl]-4-yl)sulfonyl]-2,3-dihydro-1H-indol-2-yl]carbonyl]amino]éthoxy]benzèneacétique

En opérant de façon analogue à l'exemple 22, au départ du composé obtenu selon l'exemple 257, on obtient le produit attendu sous forme d'un solide blanc (rendement = 82 %).
F = 92-99°C.

### Exemple 259

### Acide 4-[[[[(2S)-1-[(3'-méthyl[1,1'-biphényl]-4-yl)sulfonyl]-2,3-dihydro-1H-indol-2-yl]carbonyl]amino]méthyl]benzèneacétique, méthyl ester

En opérant de façon analogue à l'exemple 186, au départ du chlorure de l'acide obtenu selon la préparation CVI, on obtient le composé attendu sous forme d'un solide blanc (rendement = 62 %)
F = 66-68°C.

### Exemple 260

### Acide 4-[[[[(2S)-1-[(3'-méthyl[1,1'-biphényl]-4-yl)sulfonyl]-2,3-dihydro-1H-indol-2-yl]carbonyl]amino]méthyl]benzèneacétique

En opérant de façon analogue à l'exemple 22, au départ du composé obtenu selon l'exemple 259, on obtient le produit attendu sous forme d'un solide blanc (rendement = 98 %).
F = 118-120°C.

### Exemple 261

### Acide 4-[2-[[[(2S)-1-[(2'-chloro-4'-fluoro[1,1'-biphényl]-4-yl)sulfonyl]-2,3-dihydro-1H-indol-2-yl]carbonyl]amino]éthoxy]benzèneacétique, méthyl ester

En opérant de façon analogue à l'exemple 184, au départ de l'acide obtenu selon la préparation CVII, on obtient le composé attendu sous forme d'un solide rose (rendement = 57 %).
F = 68°C.

### Exemple 262

### Acide 4-[2-[[[(2S)-1-[(2'-chloro-4'-fluoro[1,1'-biphényl]-4-yl)sulfonyl]-2,3-dihydro-1H-indol-2-yl]carbonyl]amino]éthoxy]benzèneacétique

En opérant de façon analogue à l'exemple 22, au départ du composé obtenu selon l'exemple 261, on obtient le produit attendu sous forme d'un solide blanc (rendement = 92 %).
F = 90°C.

### Exemple 263

### Acide 4-[[[[(2S)-1-[(2'-chloro-4-fluoro[1,1'-biphényl]-4-yl)sulfonyl]-2,3-dihydro-1H-indol-2-yl]carbonyl]amino]méthyl]benzèneacétique, méthyl ester

En opérant de façon analogue à l'exemple 186, au départ de l'acide obtenu selon la préparation CVII, on obtient le composé attendu sous forme d'un solide beige (rendement = 62 %).
F = 70°C.

### Exemple 264

### Acide 4-[[[[(2S)-1-[(2'-chloro-4'-fluoro[1,1'-biphényl]-4-yl)sulfonyl]-2,3-dihydro-1H-indol-2-yl]carbonyl]amino]méthyl]benzèneacétique

En opérant de façon analogue à l'exemple 22, au départ du composé obtenu selon l'exemple 263, on obtient le produit attendu sous forme d'un solide blanc (rendement = 85 %).
F = 119°C.

### Exemple 265

### N-[(2-aminophényl)méthyl]-1-[[2'-chloro-4'-fluoro[1,1'-biphényl]-4-yl]sulfonyl]-2,3-dihydro-(2S)-1H-indole-2-carboxamide

En opérant de façon analogue à l'exemple 254, au départ de l'acide obtenu selon la préparation CVII, on obtient le produit attendu sous forme d'un solide blanc (rendement = 53 %).
F = 85-90°C.

### Exemple 266

### Acide 4-[[[[(2S)-1-[(3'-chloro[1,1'-biphényl]-4-yl)sulfonyl]-2,3-dihydro-1H-indol-2-yl]carbonyl]amino]méthyl]benzèneacétique, méthyl ester

En opérant de façon analogue à l'exemple 186, au départ de l'acide obtenu selon la préparation CVIII, on obtient le composé attendu sous forme d'un solide blanc écru (rendement = 64 %).
F = 70°C.

### Exemple 267

### Acide 4-[[[[(2S)-1-[(3'-chloro[1,1'-biphényl]-4-yl)sulfonyl]-2,3-dihydro-1H-indol-2-yl]carbonyl]amino]méthyl]benzèneacétique

En opérant de façon analogue à l'exemple 22, au départ du composé obtenu selon l'exemple 266, on obtient le produit attendu sous forme d'un solide blanc (rendement = 85 %).
F = 120°C.

### Exemple 268

### Acide 4-[[[[(2S)-1-[(3'-éthyl[1,1'-biphényl]-4-yl)sulfonyl]-2,3-dihydro-1H-indol-2-yl]carbonyl]amino]méthyl]benzèneacétique, méthyl ester

En opérant de façon analogue à l'exemple 186, au départ de l'acide obtenu selon la préparation CIX, on obtient le composé attendu sous forme d'une poudre beige (rendement = 60 %).
F = 70-75°C.

### Exemple 269

### Acide 4-[[[[(2S)-1-[(3'-éthyl[1,1'-biphényl]-4-yl)sulfonyl]-2,3-dihydro-1H-indol-2-yl]carbonyl]amino]méthyl]benzèneacétique

En opérant de façon analogue à l'exemple 22, au départ du composé obtenu selon l'exemple 268, on obtient le produit attendu sous forme d'un solide blanc (rendement = 74 %).
F = 98°C.

### Exemple 270

### Acide 4-[2-[[[(2S)-1-[[4'-fluoro-2'-(trifluorométhyl)[1,1'-biphényl]-4-yl]sulfonyl]-2,3-dihydro-1H-indol-2-yl]carbonyl]amino]éthoxy] benzèneacétique, méthyl ester

En opérant de façon analogue à l'exemple 1, au départ du composé obtenu selon la préparation CX et de 1-bromo-4-fluoro-2-(trifluorométhyl)-, benzène on obtient le produit attendu sous forme d'une huile incolore (rendement = 44 %).
RMN ¹H (DMSO, 300MHz) δ : 8,41 (t, NH) ; 7,90-7,70 (m, 3H) ; 7,60 (td, 1H) ; 7,55-7,40 (m, 4H) ; 7,30-7,10 (m, 4H) ; 7,03 (t, 1 H) ; 6,88 (d, 2H) ; 4,88 (dd, 1 H) ; 4,10-3,95 (m, 2H) ; 3,60 (s, 2H) ; 3,59 (s, 3H) ; 3,60-3,30 (m, 2H) ; 3,03 (dd, 1 H) ; 2,93 (dd, 1 H).

### Exemple 271

**Acide 4-[2-[[[(2*S*)-1-[[4'-fluoro-2'-(trifluorométhyl)[1,1'-biphényl]-4-yl]sulfonyl]-2,3-dihydro-1*H*-indol-2-yl]carbonyl]amino]éthoxy] benzèneacétique**

En opérant de façon analogue à l'exemple 5, au départ du composé obtenu selon l'exemple 270, on obtient le produit attendu sous forme d'une mousse blanche (rendement = 99 %).
RMN ¹H (DMSO, 300MHz) δ : 8,41 (t, NH) ; 7,90-7,70 (m, 3H) ; 7,60 (td, 1 H) ; 7,55-7,40 (m, 4H) ; 7,23 (td, 1 H) ; 7,20-7,10 (m, 3H) ; 7,03 (td, 1H) ; 6,86 (dd, 2H) ; 4,88 (dd, 1 H) ; 4,10-3,95 (m, 2H) ; 3,45 (s, 2H) ; 3,60-3,30 (m, 2H) ; 3,03 (dd, 1 H) ; 2,93 (dd, 1 H).

### Exemple 272

### Acide 4-[2-[([(2S)-1-[(2'-cyano-4'-fluoro[1,1'-biphényl)-4-yl)sulfonyl]-2,3-dihydro-1H-indol-2-yl]carbonyl]amino]éthoxy]benzèneacétique, méthyl ester

En opérant de façon analogue à l'exemple 270, au départ du composé obtenu selon la préparation CX et de 2-bromo-5-fluorobenzonitrile on obtient le produit attendu sous forme d'une huile incolore (rendement = 73%).
RMN ¹H (DMSO, 300MHz) δ : 8,44 (t, NH) ; 8,10-7,85 (m, 3H) ; 7,80-7,65 (m, 4H) ; 7,48 (d, 1H); 7,24 (t, 1H) ; 7,20-7,10 (m, 3H) ; 7,03 (t, 1 H) ; 6,89 (d, 2H) ; 4,90 (dd, 1H) ; 4,02 (t, 2H) ; 3,60 (s, 2H) ; 3,59 (s, 3H) ; 3,60-3,30 (m, 2H) ; 3,14 (dd, 1H) ; 2,94 (dd, 1 H).

### Exemple 273

### Acide 4-[2-[[[(2S)-1-[(2'-cyano-4'-fluoro[1,1'-biphényl]-4-yl)sulfonyl]-2,3-dihydro-1H-indol-2-yl]carbonyl]amino]éthoxy]benzèneacétique

En opérant de façon analogue à l'exemple 5, au départ du composé obtenu selon l'exemple 272, on obtient le produit attendu sous forme d'une mousse blanche (rendement = 99 %).
RMN ¹H (DMSO, 300MHz) δ: 12,24 (slarge, COOH) ; 8,44 (t, NH) ; 8,05-7,9 (m, 3H) ; 7,80-7,65 (m, 4H) ; 7,48 (d, 1 H) ; 7,24 (td, 1 H) ; 7,20-7,10 (m, 3H) ; 7,02 (t, 1 H) ; 6,89 (d, 2H) ; 4,91 (dd, 1 H) ; 4,10-3,90 (m, 2H) ; 3,48 (s, 2H) ; 3,60-3,30 (m, 2H) ; 3,14 (dd, 1 H) ; 2,94 (dd, 1 H).

### Exemple 274

### Acide 4-[2-[[[(2S)-1-[(4-chloro[1,1'-biphényl]-4-yl)sulfonyl]-2,3-dihydro-1H-indol-2-yl]carbonyl]amino]éthoxy]benzèneacétique, méthyl ester

En opérant de façon analogue à l'exemple 1, au départ du composé obtenu selon la préparation VIIa et d'acide 4-chlorophénylboronique, on obtient le produit attendu sous forme d'une huile marron (rendement = 96 %).
RMN ¹H (DMSO, 300MHz) δ : 8,41 (t, NH) ; 7,87 (d, 2H) ; 7, 85 (d, 2H) ; 7,74 (dd, 2H) ; 7,60-7,40 (m, 3H) ; 7,30-7,10 (m, 4H) ; 7,02 (td, 1 H) ; 6,89 (d, 2H) ; 4,87 (dd, 1 H) ; 4,10-3,95 (m, 2H) ; 3,60 (s, 2H) ; 3,59 (s, 3H) ; 3,60-3,40 (m, 2H) ; 3,15 (dd, 1 H) ; 2,94 (dd, 1 H).

### Exemple 275

### Acide 4-[2-[[[(2S)-1-[[2'-chloro-5'-(trifluorométhyl)[1,1'-biphényl]-4-yl]sulfonyl]-2,3-dihydro-1H-indol-2-yl]carbonyl]amino]éthoxy] benzèneacétique, méthyl ester

En opérant de façon analogue à l'exemple 274, au départ d'acide 2-chloro-5-(trifluorométhyl)phénylboronique, on obtient le produit attendu sous forme d'une mousse beige (rendement = 51 %).
RMN ¹H (DMSO, 300MHz) δ : 8,44 (t, NH) ; 7,90 (d, 2H) ; 7,90-7,80 (m, 3H) ; 7, 67 (d, 2H) ; 7,47 (d, 1 H) ; 7,23 (td, 1 H) ; 7,20-7,10 (m, 3H) ; 7,03 (td, 1 H) ; 6,89 (dd, 2H) ; 4,89 (dd, 1H) ; 4,02 (t, 2H) ; 3,60 (s, 2H) ; 3,59 (s, 3H) ; 3,60-3,40 (m, 2H) ; 3,16 (dd, 1H) ; 2,94 (dd, 1 H).

### Exemple 276

### Acide 4-[2-[[[(2S)-1-[[2'-fluoro-5'-(trifluorométhyl)[1,1'-biphényl]-4-yl]sulfonyl]-2,3-dihydro-1H-indol-2-yl]carbonyl]amino]éthoxy] benzèneacétique, méthyl ester

En opérant de façon analogue à l'exemple 274, au départ d'acide 2-fluoro-5-(trifluorométhyl)phénylboronique, on obtient le produit attendu sous forme d'une poudre rose (rendement = 89 %).
F = 52-55°C.

### Exemple 277

### Acide 4-[2-[[[(2S)-1-[[2'-fluoro-5'-(trifluorométhyl)[1,1'-biphényl]-4-yl]sulfonyl]-2,3-dihydro-1H-indol-2-yl]carbonyl]amino]éthoxy] benzèneacétique

En opérant de façon analogue à l'exemple 5, au départ du composé obtenu selon l'exemple 276, on obtient le produit attendu sous forme d'une poudre blanche (rendement = 94 %).
F=81-84°C.

### Exemple 278

### Acide 4-[2-[[[(2S)-1-[(2'-fluoro-5'-méthyl[1,1'-biphényl]-4-yl)sulfonyl]2,3-dihydro-1H-indol-2-yl]carbonyl]amino]éthoxy]benzèneacétique, méthyl ester

En opérant de façon analogue à l'exemple 274, au départ d'acide 2-fluoro-5-méthylphénylboronique, on obtient le produit attendu sous forme d'une poudre rose (rendement = 90 %).
F = 55-58°C.

### Exemple 279

### Acide 4-[2-[[[(2S)-1-[(2'-fluoro-5'-méthyl[1,1'-biphényl]-4-yl)sulfonyl]-2,3-dihydro-1 H-indol-2-yl]carbonyl]amino]éthoxy]benzèneacétique

En opérant de façon analogue à l'exemple 22, au départ du composé obtenu selon l'exemple 278 on obtient le produit attendu sous forme d'une poudre beige (rendement = 95 %).
F = 82-85°C.

### Exemple 280

### Acide 4-[2-[[[(2S)-1-[(2'-méthoxy-5'-méthyl[1,1'-biphényl]-4-yl)-sulfonyl]-2,3-dihydro-1H-indol-2-yl]carbonyl]amino]éthoxy]benzèneacétique, méthyl ester

En opérant de façon analogue à l'exemple 274, au départ d'acide 2-méthoxy-5-méthylphénylboronique, on obtient le produit attendu sous forme d'une poudre rose (rendement = 91 %).
F = 57-60°C.

### Exemple 281

### Acide 4-[2-[[[(2S)-1-[(2-méthoxy-5'-méthyl[1,1'-biphényl]-4-yl)-sulfonyl]-2,3-dihydro-1H-indol-2-yl]carbonyl]amino]éthoxy]benzèneacétique

En opérant de façon analogue à l'exemple 22, au départ du composé obtenu selon l'exemple 280, on obtient le produit attendu sous forme d'une poudre jaune (rendement = 96 %).
F = 85-88°C.

### Exemple 282

### Acide 4-[2-[[[(2S)-1-[(2',5'-diméthyl[1,1'-biphényl]-4-yl)sulfonyl]-2,3-dihydro-1H-indol-2-yl]carbonyl]amino]éthoxy]benzèneacétique, méthyl ester

En opérant de façon analogue à l'exemple 274, au départ d'acide 2,5-diméthylphénylboronique, on obtient le produit attendu sous forme d'une poudre rose (rendement = 91 %).
F = 56-59°C.

### Exemple 283

### Acide 4-[2-[[[(2S)-1-[(2',5'-diméthyl[1,1'-biphényl]-4-yl)sulfonyl]-2,3-dihydro-1H-indol-2-yl]carbonyl]amino]éthoxy]benzèneacétique

En opérant de façon analogue à l'exemple 22, au départ du composé obtenu selon l'exemple 282, on obtient le produit attendu sous forme d'une poudre beige (rendement = 96 %).
F = 81-84°C.

### Exemple 284

### Acide 4-[2-[[[(2S)-1-[[2'-méthoxy-5'-(trifluorométhyl)[1,1'-biphényl]-4-yl]sulfonyl]-2,3-dihydro-1H-indol-2-yl]carbonyl]amino]éthoxy] benzèneacétique, méthyl ester

En opérant de façon analogue à l'exemple 274, au départ d'acide 2-méthoxy-5-(trifluorométhyl)phénylboronique, on obtient le produit attendu sous forme d'une poudre rose (rendement = 85 %).
F=61-64°C.

### Exemple 285

### Acide 4-[2-[[[(2S)-1-[[2'-méthoxy-5'-(trifluorométhyl)[1,1'-biphényl]-4-yl]sulfonyl]-2,3-dihydro-1H-indol-2-yl]carbonyl]amino]éthoxy] benzèneacétique

En opérant de façon analogue à l'exemple 22, au départ du composé obtenu selon l'exemple 284, on obtient le produit attendu sous forme d'une poudre blanche (rendement = 99 %).
F = 85-88°C.

### Exemple 286

### Acide 4-[2-[[[(2S)-1-[[2'-méthyl-5'-(trifluorométhyl)[1,1'-biphényl]-4-yl]sulfonyl]-2,3-dihydro-1 H-indol-2-yl]carbonyl]amino]éthoxy] benzèneacétique, méthyl ester

En opérant de façon analogue à l'exemple 274, au départ d'acide 2-méthyl-5-(trifluorométhyl)phénylboronique, on obtient le produit attendu sous forme d'une poudre rose (rendement = 80 %).
F = 46-49°C.

### Exemple 287

### Acide 4-[2-[[[(2S)-1-[[2'-méthyl-5'-(trifluorométhyl)[1,1'-biphényl]-4-yl]sulfonyl]-2,3-dihydro-1H-indol-2-yl]carbonyl]amino]éthoxy] benzèneacétique

En opérant de façon analogue à l'exemple 22, au départ du composé obtenu selon l'exemple 286, on obtient le produit attendu sous forme d'une poudre blanche (rendement = 95 %).
F = 80-84°C.

### Exemple 288

### Acide 4-[2-[[[(2S)-1-[(2'-chloro-5'-méthyl[1,1'-biphényl]-4-yl)sulfonyl]-2,3-dihydro-1H-indol-2-yl]carbonyl]amino]éthoxy]benzèneacétique, méthyl ester

En opérant de façon analogue à l'exemple 274, au départ d'acide 2-chloro-5-méthylphénylboronique, on obtient le produit attendu sous forme d'une poudre rose (rendement = 69 %).
F = 50-53°C.

### Exemple 289

### Acide 4-[2-([[(2S)-1-((2'-chloro-5'-méthyl[1,1'-biphényl]-4-yl)sulfonyl]-2,3-dihydro-1H-indol-2-yl]carbonyl]amino]éthoxy]benzèneacétique

En opérant de façon analogue à l'exemple 22, au départ du composé obtenu selon l'exemple 288, on obtient le produit attendu sous forme d'une poudre blanche (rendement = 96 %).
F = 77-81 °C.

### Exemple 290

### Acide 4-[2-[[[(2S)-1-[(2',5'-dichloro[1,1'-biphényl]-4-yl)sulfonyl]-2,3-dihydro-1H-indol-2-yl]carbonyl]amino]éthoxy]benzèneacétique, méthyl ester

En opérant de façon analogue à l'exemple 274, au départ d'acide 2,5-dichlorophénylboronique, on obtient le produit attendu sous forme d'une poudre jaune (rendement = 82 %).
F = 61-65°C.

### Exemple 291

### Acide 4-[2-[[[(2S)-1-[(2',5'-dichloro[1,1'-biphényl]-4-yl)sulfonyl]-2,3-dihydro-1H-indol-2-yl]carbonyl]amino]éthoxy]benzèneacétique

En opérant de façon analogue à l'exemple 22, au départ du composé obtenu selon l'exemple 290, on obtient le produit attendu sous forme d'une poudre jaune (rendement = 82 %).
F = 80-86°C.

### Exemple 292

### Acide 4-[2-[[[(2S)-1-[(2'-chloro-5'-fluoro[1,1'-biphényl]-4-yl)sulfonyl]-2,3-dihydro-1H-indol-2-yl]carbonyl]amino]éthoxy]benzèneacétique, méthyl ester

En opérant de façon analogue à l'exemple 272, au départ 1-bromo-2-chloro-5-fluorobenzène, on obtient le produit attendu sous forme d'une poudre blanche (rendement = 60 %).
F = 52-56°C.

### Exemple 293

### Acide 4-[2-[[[(2S)-1-[(2'-chloro-5'-fluoro[1,1'-biphényl]-4-yl)sulfonyl]-2,3-dihydro-1H-indol-2-yl]carbonyl]amino]éthoxy]benzèneacétique

En opérant de façon analogue à l'exemple 22, au départ du composé obtenu selon l'exemple 292, on obtient le produit attendu sous forme d'une poudre blanche (rendement = 97 %).
F = 78-84°C.

### Exemple 294

### Acide 4-[2-[[[(25)-1-[[2'-chloro-5'-(trifluorométhyl)[1,1'-biphényl]-4-yl]sulfonyl]-2,3-dihydro-1H-indol-2-yl]carbonyl]amino]éthoxy] benzoïque, 1,1-diméthyléthyl ester

En opérant de façon analogue à l'exemple 21, au départ de l'acide 1-[[2'-chloro-5'-(trifluorométhyl)[1,1'-biphényl]-4-yl]sulfonyl]-2,3-dihydro-1 *H*-(2S)-indole-2-carboxylique (préparation CXII) et de l'ester t-butylique de l'acide 4-(2-aminoéthoxy)benzoïque, on obtient le produit attendu sous forme d'une poudre blanche (rendement = 87 %).
F = 75-80°C.

### Exemple 295

### Acide 4-[2-[[[(2S)-1-[[2'-chloro-5'-(trifluorométhyl)[1,1'-biphényl]-4-yl]sulfonyl]-2,3-dihydro-1H-indol-2-yl]carbonyl]amino]éthoxy] benzoique

En opérant de façon analogue à l'exemple 25, au départ du composé obtenu selon l'exemple 294, on obtient le produit attendu sous forme d'une poudre blanche (rendement = 87 %).
F = 117-121 °C.

### Exemple 296

### Acide 4-[[2-[[[(2S)-1-[[2'-chloro-5'-(trifluorométhyl)[1,1'-biphényl]-4-yl]sulfonyl]-2,3-dihydro-1H-indol-2-yl]carbonyl]amino]éthyl]amino] benzoique, 1,1-diméthyléthyl ester

En opérant de façon analogue à l'exemple 21, en remplaçant l'HOAT par HOBT, au départ de l'acide obtenu selon la préparation CXII et de l'ester t-butylique de l'acide 4-[(2-aminoéthyl)amino]benzoïque, on obtient le produit attendu sous forme d'une huile jaune (rendement = 59 %).
RMN 1H (DMSO, 300 MHz) d : 8,40 (t, NH) ; 7,90 (d, 2H) ; 7,85-7,75 (m, 2H) ; 7,75-7,60 (m, 4H) ; 7,51 (d, 1 H) ; 7,25 (t, 1 H) ; 7,15-7,05 (m, 2H) ; 7,02 (t, 1 H) ; 6,60 (d, 2H) ; 4,93 (dd, 1 H) ; 3,50-3,00 (m, 5H) ; 2,96 (dd, 1H); 1,49 (s, 9H).

### Exemple 297

### Acide 4-[[2-[[[(2S)-1-[[2'-chloro-5'-(trifluorométhyl)[1,1'-biphényl]-4-yl]sulfonyl]-2,3-dihydro-1H-indol-2-yl]carbonyl]amino]éthyl]amino] benzoïque, méthyl ester

En opérant de façon analogue à l'exemple 296, au départ de l'acide obtenu selon la préparation CXII et de l'ester méthylique de l'acide 4-[(2-aminoéthyl)amino]benzoïque (obtenu par réaction à 120°C de l'ester méthylique de l'acide 4-aminobenzoique et de la 2-bromoéthylamine), on obtient le produit attendu sous forme d'un solide blanc (rendement = 53 %).
F = 96-98°C.

### Exemple 298

### Acide 4-[[2-[[[(2S)-1-[[2'-chloro-5'-(trifluorométhyl)[1,1'-biphényl]-4-yl]sulfonyl]-2,3-dihydro-1H-indol-2-yl]carbonyl]amino]éthyl]amino] benzoïque

On prépare une solution de 220 mg (0,31 mmol) de l'ester obtenu selon l'exemple 296 dans 5 ml d'acétate d'éthyle. On additionne, à 0°C, 1,5 ml d'une solution de chlorure d'hydrogène 4M dans le dioxane. Le milieu réactionnel est agité 16 heures à température ambiante. Le mélange est ensuite concentré sous pression réduite et le produit brut obtenu est purifié par chromatographie sur gel de silice en éluant à l'aide d'un mélange dichlorométhane/méthanol (98/2 ; v/v). On obtient ainsi le produit attendu sous forme d'une mousse blanche (rendement = 23 %).
F = 130°C.

### Exemple 299

### Acide 4-[[2-[[[(2S)-1-[[2'-chloro-5'-(trifluorométhyl)[1,1'-biphényl]-4-yl]sulfonyl]-2,3-dihydro-1H-indoi-2-yl]carbonyl]amino]éthyl]amino] benzèneacétique, éthyl ester

En opérant de façon analogue à l'exemple 296, au départ de l'ester éthylique de l'acide 4-[(2-aminoéthyl)amino]benzèneacétique, on obtient le produit attendu sous forme d'un solide blanc (rendement = 72 %).
F = 55-63°C.

### Exemple 300

### Acide 4-[[2-[[[(2S)-1-[[2'-chloro-5'-(trifluorométhyl)[1,1'-biphényl]-4-yl]sulfonyl]-2,3-dihydro-1H-indoi-2-yl]carbonyl]amino]éthyl]amino] benzèneacétique

En opérant de façon analogue à l'exemple 22, au départ de l'ester obtenu selon l'exemple 299, on obtient le produit attendu sous forme d'un solide blanc (rendement = 41 %).
F = 110-118°C.

### Exemple 301

### Acide 4-[2-[[[(2S)-1-[[2'-chloro-5'-(trifluorométhyl)[1,1'-biphényl]-4-yl]sulfonyl]-2,3-dihydro-1H-indol-2-yl]carbonyl]amino]éthoxy]-3-méthoxybenzèneacétique, méthyl ester

En opérant de façon analogue à l'exemple 296, au départ de l'ester obtenu selon la préparation LXXIX, on obtient le produit attendu sous forme d'un solide fin blanc (rendement = 62 %).
F = 51-58°C.

### Exemple 302

### Acide 4-[2-[[[(2S)-1-[[2'-chloro-5'-(trifluorométhyl)[1,1'-biphényl]-4-yl]sulfonyl]-2,3-dihydro-1H-indol-2-yl]carbonyl]amino]éthoxy]-3-méthoxybenzèneacétique

En opérant de façon analogue à l'exemple 22, au départ de l'ester obtenu selon l'exemple 301, on obtient le produit attendu sous forme d'un solide blanc (rendement = 96 %).
F = 74-76°C.

### PREPARATION CXIII

### Acide 1-[4-[2-[[(phénylméthoxy)carbonyl]amino]éthoxy]phényl] cyclopropanecarboxylique, méthyl ester

En opérant de façon analogue à la préparation LXVIII, au départ d'ester méthylique de l'acide 1-(4-hydroxyphényl)cyclopropanecarboxylique, on obtient le produit attendu sous forme d'une huile jaune (rendement = 58 %).
RMN ¹H (CDCl₃), 300MHz) δ : 7,40-7,30 (m, 7H); 6,82 (d, 2H); 5,22 (s large, 1 H); 5,22 (s, 2H); 4,03 (t, 2H); 3,65-3,55 (m, 5H); 1,65-1,55 (m, 2H); 1,20-1,10 (m, 2H)

### PREPARATION CXIV

### Acide 1-[4-(2-aminoéthoxy)phényl] cyclopropanecarboxylique, méthyl ester (chlorhydrate)

En opérant de façon analogue à la préparation LXXI, au départ du composé obtenu selon la préparation CXIII, on obtient le produit attendu sous forme d'un solide blanc (rendement = 96 %).
F = 119-122°C.

### Exemple 303

### Acide 1-[4-[2-[[[(2S)-1-[[2'-chloro-5'-(trifluorométhyl)[1,1'-biphényl]-4-yl]sulfonyl]-2,3-dihydro-1H-indoi-2-yl]carbonyl]amino]éthoxy] phényl]cyclopropanecarboxylique, méthyl ester

En opérant de façon analogue à l'exemple 296, au départ du composé obtenu selon la préparation CXIV, on obtient le produit attendu sous forme d'un solide fin blanc (rendement = 78 %).
F = 64-72°C.

### Exemple 304

### Acide 1-[4-[2-[[[(2S)-1-[[2'-chloro-5'-(trifluorométhyl)[1,1'-biphényl]-4-yl]sulfonyl]-2,3-dihydro-1 H-indoi-2-yl]carbonyl]amino]éthoxy] phényl]cyclopropanecarboxylique

En opérant de façon analogue à l'exemple 22, au départ de l'ester obtenu selon l'exemple 303, on obtient le produit attendu sous forme d'un solide blanc (rendement = 22 %).
F = 111°C.

### Exemple 305

### Acide 4-[2-[[[(2S)-1-[[2'-chloro-5'-(trifluorométhyl)[1,1'-biphényl]-4-yl]sulfonyl]-2,3-dihydro-1H-indol-2-yl]carbonyl]amino]éthoxy]-2-fluorobenzoïque, méthyl ester

En opérant de façon analogue à l'exemple 296, au départ du composé obtenu selon la préparation LXIX, on obtient le produit attendu sous forme d'un solide fin blanc (rendement = 38 %).
F = 84-85°C.

### Exemple 306

### Acide 4-[2-[[[(2S)-1-[[2'-chloro-5'-(trifluorométhyl)[1,1'-biphényl]-4-yl]sulfonyl]-2,3-dihydro-1H-indoi-2-yl]carbonyl]amino]éthoxy]-2-méthoxybenzoïque

En opérant de façon analogue à l'exemple 22, au départ de l'ester obtenu selon l'exemple 305, on obtient le produit attendu sous forme d'un solide blanc (rendement = 72 %).
F = 98-104°C.

### Exemple 307

### Acide 4-[2-[[[(2S)-1-[[2'-chloro-5'-(trifluorométhyl)[1,1'-biphényl]-4-yl]sulfonyl]-2,3-dihydro-1H-indol-2-yl]carbonyl]amino]éthoxy]-2-(trifluorométhyl)benzoïque, méthyl ester

En opérant de façon analogue à l'exemple 296, au départ du composé obtenu selon la préparation LXXXV, on obtient le produit attendu sous forme d'un solide fin blanc (rendement = 71 %).
F = 79°C.

### Exemple 308

### Acide 4-[2-[[[(2S)-1-[[2'-chloro-5'-(trifluorométhyl)[1,11-biphényl]-4-yl]sulfonyl]-2,3-dihydro-1H-indol-2-yl]carbonyl]amino]éthoxy]-2-(trifluorométhyl)benzoïque

En opérant de façon analogue à l'exemple 22, au départ de l'ester obtenu selon l'exemple 307, on obtient le produit attendu sous forme d'un solide blanc (rendement = 20 %).
F = 102-109°C.

### Exemple 309

### Acide 4-[2-[[[(2S)-1-[[2'-chloro-5'-(trifluorométhyl)[1,1'-biphényl]-4-yl]sulfonyl]-2,3-dihydro-1H-indol-2-yl]carbonyl]amino]éthoxy]-2,6-difluorobenzoïque, méthyl ester

En opérant de façon analogue à l'exemple 296, au départ du composé obtenu selon la préparation LXXXI, on obtient le produit attendu sous forme d'un solide fin blanc (rendement = 61 %).
F = 78°C.

### Exemple 310

### Acide 4-[2-[[[(2S)-1-[[21-chloro-5'-(trifluorométhyl)[1,1'-biphényl]-4-yl]sulfonyl]-2,3-dihydro-1H-indol-2-yl]carbonyl]amino]éthoxy]-2,6-difluorobenzoïque

En opérant de façon analogue à l'exemple 22, au départ de l'ester obtenu selon l'exemple 309, on obtient le produit attendu sous forme d'un solide blanc (rendement = 56 %).
F = 103-114°C.

### Exemple 311

### Acide 4-[[[[1-[(2',4'-difluoro[1,1'-biphényl]-4-yl)sulfonyl]-2,3-dihydro-1H-indol-2-yl]carbonyl]amino]méthyl]benzoïque

On prépare le chlorure de l'acide obtenu selon la préparation CV, selon le procédé décrit pour la préparation VI, et on le fait réagir selon le mode opératoire décrit à l'exemple 17, avec l'acide 4-(aminométhyl)benzoïque. On obtient ainsi le produit attendu sous forme d'un solide blanc (rendement = 69 %).
F = 115°C.

### Exemple 312

### Acide 4-[[[[1-[(2'-chloro-4'-fluoro[1,1'-biphényl]-4-yl)sutfonyl]-2,3-dihydro-1H-indol-2-yl]carbonyl]amino]méthyl]benzdique

En opérant de façon analogue à l'exemple 311, au départ de l'acide obtenu selon la préparation CVII, on obtient le produit attendu sous forme d'un solide blanc (rendement = 61 %).
F = 111-124°C.

### Exemple 313

### Acide 4-[2-[[[(2S)-1-[[2'-chloro-5'-(trifluorométhyl)[1,1'-biphényl]-4-yl]sulfonyl]-2,3-dihydro-1H-indol-2-yl]carbonyl]amino]éthoxy]-α-méthylbenzèneacétique, méthyl ester

En opérant de façon analogue à l'exemple 296, au départ de l'ester obtenu selon la préparation LXXVII, on obtient le produit attendu sous forme d'un solide fin blanc (rendement = 30 %).
F = 55-63°C.

### Exemple 314

### Acide 4-[2-[[[(2S)-1-[[2'-chloro-5'-(trifluorométhyl)[1,1'-biphényl]-4-yl]sulfonyl]-2,3-dihydro-1H-indol-2-yl]carbonyl]amino]éthoxy]-α-méthylbenzèneacétique

En opérant de façon analogue à l'exemple 22, au départ de l'ester obtenu selon l'exemple 313, on obtient le produit attendu sous forme d'un solide blanc (rendement = 98 %).
F = 91-100°C.

### PREPARATION CXV

### Acide 3-fluoro-4-[2-[[(phénylméthoxy)carbonyl]amino]éthoxy]benzèneacétique, méthyl ester

En opérant de façon analogue à la préparation LXVIII, au départ de l'ester méthylique de l'acide 3-fluoro-4-hydroxybenzèneacétique, on obtient le composé attendu sous forme d'un solide blanc avec un rendement de 69%.
F = 44-45°C.

### PREPARATION CXVI

### Acide, 4-(2-aminoéthoxy)-3-fluorobenzèneacétique, méthyl ester (chlorhydrate)

En opérant de façon analogue à la préparation LXXI au départ du composé obtenu selon la préparation CXV, on obtient le composé attendu sous forme d'un solide blanc avec un rendement de 94 %.
F = 143-144°C.

### Exemple 315

### Acide 4-[2-[[[(2S)-1-[[2'-chloro-5'-(trifluorométhyl)[1,1'-biphényl]-4-yl]sulfonyl]-2,3-dihydro-1H-indol-2-yl]carbonyl]amino]éthoxy]-3-fluorobenzèneacétique, méthyl ester

En opérant de façon analogue à l'exemple 296, au départ de l'ester obtenu selon la préparation CXVI, on obtient le produit attendu sous forme d'un solide fin blanc (rendement = 86 %).
F = 56-62°C.

### Exemple 316

### Acide 4-[2-[[[(2S)-1-[[2'-chloro-5-(trifluorométhyl)[1,1'-biphényl]-4-yl]sulfonyl]-2,3-dihydro-1H-indol-2-yl]carbonyl]amino]éthoxy]-3-fluorobenzèneacétique

En opérant de façon analogue à l'exemple 22, au départ de l'ester obtenu selon l'exemple 315, on obtient le produit attendu sous forme d'un solide blanc (rendement = 86 %).
F = 100°C.

### PREPARATION CXVII

### Acide 2-méthyl-4-[2-[[(phénylméthoxy)carbonyl]amino]éthoxy]benzèneacétique, méthyl ester

En opérant de façon analogue à la préparation LXVIII, au départ de l'ester méthylique de l'acide 2-méthyl-4-hydroxybenzèneacétique, on obtient le composé attendu sous forme d'une huile incolore avec un rendement de 22%.
RMN'H (DMSO, 300MHz) δ : 7,47 (t, 1 H); 7,45-7,25 (m, 5H); 7,07 (d, 1 H); 6,80-6,60 (m, 2H); 5,03 (s, 2H); 3,95 (t, 2H); 3,70-3,50 (m, 5H); 3,45-3,25 (m, 2H); 2,18 (s, 3H).

### PREPARATION CXVIII

### Acide, 4-(2-aminoéthoxy)- 2-méthylbenzèneacétique, méthyl ester (chlorhydrate)

En opérant de façon analogue à la préparation LXXI au départ du composé obtenu selon la préparation CXVII, on obtient le composé attendu sous forme d'un solide beige avec un rendement de 75 %.
F = 168-171 °C.

### Exemple 317

### Acide 4-[2-[[[(2S)-1-[[2'-chloro-5'-(trifluorométhyl)[1,1'-biphényl]-4-yl]sulfonyl]-2,3-dihydro-1H-indol-2-yl]carbonyl]amino]éthoxy]-2-méthylbenzèneacétique, méthyl ester

En opérant de façon analogue à l'exemple 296, au départ de l'ester obtenu selon la préparation CXVIII, on obtient le produit attendu sous forme d'une huile incolore (rendement = 53 %).
RMN'H (DMSO, 300MHz) δ : 7,80-7,20 (m, 9H); 7,15-7,05 (m, 3H); 6,75-6,65 (m, 2H); 4,68 (dd, 1 H); 4,10-3,95 (m, 2H); 3,80-3,50 (m, 5H); 3,57 (s, 2H); 3,28 (dd, 1 H); 2,82 (dd, 1 H); 2,27 (s, 3H).

### Exemple 318

### Acide 4-[2-[[[(2S)-1-[[2'-chloro-5'-(trifluorométhyl)[1,1'-biphényl]-4-yl]sulfonyl]-2,3-dihydro-1H-indol-2-yl]carbonyl]amino]éthoxy]-2-méthylbenzèneacétique

En opérant de façon analogue à l'exemple 22, au départ de l'ester obtenu selon l'exemple 317, on obtient le produit attendu sous forme d'un solide blanc (rendement = 46 %).
F = 110-115°C.

### Exemple 319

### Acide 4-[2-[[[(2S)-1-[[2'-chloro-5-(trifluorométhyl)[1,1'-biphényl]-4-yl]sulfonyl]-2,3-dihydro-1H-indol-2-yl]carbonyl]amino]éthoxy]-3-chlorobenzèneacétique, méthyl ester

En opérant de façon analogue à l'exemple 296, au départ de l'ester obtenu selon la préparation LXI, on obtient le produit attendu sous forme d'une poudre blanche (rendement = 63 %).
F = 68-76°C.

### Exemple 320

### Acide 4-[2-[[[(2S)-1-[[2'-chloro-5'-(trifluorométhyl)[1,1'-biphényl]-4-yl]sulfonyl]-2,3-dihydro-1H-indol-2-yl]carbonyl]amino]éthoxy]-3-chlorobenzèneacétique

En opérant de façon analogue à l'exemple 22, au départ de l'ester obtenu selon l'exemple 319, on obtient le produit attendu sous forme d'un solide blanc (rendement = 89 %).
F = 108-112°C.

### Exemple 321

### Acide 3-chloro-4-[2-[[[(2S)-1-[(4'-fluoro[1,1'-biphényl]-4-yl)sulfonyl]-2,3-dihydro-1H-indol-2-yl]carbonyl]amino]éthoxy]benzèneacétique, méthyl ester

En opérant de façon analogue à l'exemple 319, au départ de l'acide obtenu selon la préparation LXIII, on obtient le produit attendu sous forme d'une poudre blanche (rendement = 63 %).
F = 68-76°C.

### Exemple 322

### Acide 3-chloro-4-[2-[[[(2S)-1-[(4'-fluoro[1,1'-biphényl]-4-yl)sulfonyl]-2,3-dihydro-1H-indol-2-yl]carbonyl]amino]éthoxy]benzèneacétique

En opérant de façon analogue à l'exemple 22, au départ de l'ester obtenu selon l'exemple 321, on obtient le produit attendu sous forme d'un solide blanc (rendement = 85 %).
F = 102-110°C.

### PREPARATION CXIX

### Acide α,α-diméthyl-4-[2-[[(phénylméthoxy)carbonyl]amino]éthoxy]benzèneacétique, méthyl ester

En opérant de façon analogue à la préparation LXVIII, au départ de l'ester méthylique de l'acide α,α-diméthyl-4-hydroxybenzèneacétique, on obtient le composé attendu sous forme d'une huile jaune avec un rendement de 62%.
RMN ¹H (DMSO, 300 MHz) δ : 7,48 (t, 1 H); 7,40-7,25 (m, 5H); 7,20 (d, 2H); 6,87 (d, 2H); 5,02 (s, 2H); 3,97 (t, 2H); 3,56 (s, 3H); 3,35 (q, 2H); 1,47 (s, 6H)

### PREPARATION CXX

### Acide α,α-diméthyl-4-(2-aminoéthoxy)benzèneacétique, méthyl ester (chlorhydrate)

En opérant de façon analogue à la préparation LXXI au départ du composé obtenu selon la préparation CXIX, on obtient le composé attendu sous forme d'un solide jaune avec un rendement de 88 %.
F = 108-112°C.

### Exemple 323

### Acide α,α-diméthyl-4-[2-[[[(2S)-1-[[2'-chloro-5'-(trif)uorométhyl)[1,1'-biphényl]-4-yl]sulfonyl]-2,3-dihydro-1H-indol-2-yl]carbonyl]amino]éthoxy]benzèneacétique, méthyl ester

En opérant de façon analogue à l'exemple 296, au départ de l'ester obtenu selon la préparation CXX, on obtient le produit attendu sous forme d'une poudre blanche (rendement = 61 %).
F = 66-72°C.

### Exemple 324

### Acide α,α-diméthyl-4-[2-[([(2S)-1-[[2'-chloro-5'-(trifluorométhyl)[1,1'-biphényl]-4-yl]sulfonyl]-2,3-dihydro-1H-indol-2-yl]carbonyl]amino]éthoxy]benzèneacétique

En opérant de façon analogue à l'exemple 22, au départ de l'ester obtenu selon l'exemple 323, on obtient le produit attendu sous forme d'un solide blanc (rendement = 23 %).
F = 92-100°C.

### PREPARATION CXXI

### Acide 2-chloro-4-[2-[[(phénylméthoxy)carbonyl]amino]éthoxy]benzoïque, méthyl ester

En opérant de façon analogue à la préparation LXVIII, au départ de l'ester méthylique de l'acide 2-chloro-4-hydroxybenzoïque, on obtient le composé attendu sous forme d'un solide blanc avec un rendement de 84 %.
F = 88-90°C.

### PREPARATION CXXII

### Acide, 4-(2-aminoéthoxy)-2-chlorobenzoïque, méthyl ester (bromhydrate)

On prépare une solution de 500 mg (1,37 mmol) du composé obtenu selon la préparation CXXI dans 5 ml de dichlorométhane et on ajoute, à température ambiante, 3,7 ml (20 mmol) d'une solution de bromure d'hydrogène à 45% dans l'acide acétique. Le mélange réactionnel est agité pendant 1 heure à température ambiante, puis dilué par de l'éther éthylique. Le précipité obtenu est filtré et rincé à l'éther éthylique sur le filtre, puis séché à 40°C sous vide. On obtient ainsi le produit attendu sous forme d'une poudre blanche avec un rendement de 91 %.
F = 195-197°C.

### Exemple 325

### Acide 2-chloro-4-[2-[[[(2S)-1-[[2'-chloro-5'-(triftuorométhyl)[1,1'-biphényl]-4-yl]sulfonyl]-2,3-dihydro-1H-indol-2-yl]carbonyl]amino]éthoxy]benzoïque, méthyl ester

En opérant de façon analogue à l'exemple 296, au départ de l'ester obtenu selon la préparation CXXII, on obtient le produit attendu sous forme d'une poudre blanche (rendement = 73 %).
F = 70-76°C.

### Exemple 326

### Acide 2-chloro-4-[2-[[[(2S)-1-[[2'-chloro-5'-(trifluorométhyl)[1,1'-biphényl]-4-yl]sulfonyl]-2,3-dihydro-1H-indol-2-yl]carbonyl]amino]éthoxy]benzoïque

En opérant de façon analogue à l'exemple 22, au départ de l'ester obtenu selon l'exemple 325, on obtient le produit attendu sous forme d'un solide blanc (rendement = 46 %).
F = 106-112°C.

### Exemple 327

### Acide 2-chloro-4-[2-[[[(2S)-1-[(4'-fluoro[1,1'-biphényl]-4-yl)sulfonyl]-2,3-dihydro-1H-indol-2-yl]carbonyl]amino]éthoxy]benzoïque, méthyl ester

En opérant de façon analogue à l'exemple 325, au départ de l'acide obtenu selon la préparation LXIII, on obtient le produit attendu sous forme d'une poudre rose (rendement = 70 %).
F = 70-79°C.

### Exemple 328

### Acide 2-chloro-4-[2-[[[(2S)-1-[[3'-(trifluorométhyl)[1,1'-biphényl]-4-yl]sulfonyl]-2,3-dihydro-1H-indol-2-yl]carbonyl]amino]éthoxy] benzoïque, méthyl ester

En opérant de façon analogue à l'exemple 325, au départ de l'acide obtenu selon la préparation IV, on obtient le produit attendu sous forme d'une poudre blanche (rendement = 83 %).
F = 63-67°C.

### Exemple 329

### Acide 2-chloro-4-[2-[[[(2S)-1-[[3'-(trifluorométhyl)[1,1'-biphényl]-4-yl]sulfonyl]-2,3-dihydro-1H-indol-2-yl]carbonyl]amino]éthoxy] benzoïque

En opérant de façon analogue à l'exemple 22, au départ de l'ester obtenu selon l'exemple 328, on obtient le produit attendu sous forme d'un solide blanc (rendement = 39 %).
F = 104-108°C.

### PREPARATION CXXIII

### Acide α-(1-méthyléthyl)-4-[2-[[(phénylméthoxy)carbonyl]amino]éthoxy]benzèneacétique, méthyl ester

En opérant de façon analogue à la préparation LXVIII, au départ de l'ester méthylique de l'acide α-(1-méthyléthyl)-4-hydroxybenzèneacétique, on obtient le composé attendu sous forme d'une huile incolore avec un rendement de 79 %.
RMN ¹H (DMSO, 300MHz) δ : 7,48 (t, 1 H); 7,45-7,00 (m, 7H); 6,88 (d, 2H); 5,03 (s, 2H); 3,97 (t, 2H); 3,57 (s, 3H); 3,45-3.25 (m, 2H); 3,18 (d, 1 H); 2,30-2,10 (m, 1 H); 0,95 (d, 3H); 0,64 (d, 3H).

### PREPARATION CXXIV

### Acide α-(1-méthyléthyl)-4-(2-aminoéthoxy)benzèneacétique, méthyl ester (bromhydrate)

En opérant de façon analogue à la préparation CXXII au départ du composé obtenu selon la préparation CXXIII, on obtient le composé attendu sous forme d'une huile avec un rendement de 37 %.
RMN ¹H (DMSO, 300MHz) δ : 7,94 (s large , NH2); 7,25 (d, 2H); 6,94 (d, 2H); 4,14 (t, 2H); 3,56 (s, 3H); 3,20 (q, 2H); 2,30-2,10 (m, 1H); 0,94 (d, 3H); 0,63 (d, 3H).

### Exemple 330

### Acide α-(1-méthyléthyl)-4-[2-[[[(2S)-1-[[2'-chloro-5'-(trifluorométhyl)-[1,1'-biphényl]-4-yl]sulfonyl]-2,3-dihydro-1H-indol-2-yl]carbonyl]amino]éthoxy]benzèneacétique, méthyl ester

En opérant de façon analogue à l'exemple 296, au départ de l'ester obtenu selon la préparation CXXIV, on obtient le produit attendu sous forme d'une poudre blanche (rendement = 56 %).
F = 68-75°C.

### Exemple 331

### Acide α-(1-méthyléthyl)-4-[2-[[[(2S)-1-[[2'-chloro-5'-(trifluorométhyl)[1,1'-biphényl]-4-yl]sulfonyl]-2,3-dihydro-1H-indol-2-yl]carbonyl]amino]éthoxy]benzèneacétique

En opérant de façon analogue à l'exemple 22, au départ de l'ester obtenu selon l'exemple 330, on obtient le produit attendu sous forme d'un solide jaune (rendement = 18 %).
F = 134-143°C.

### Exemple 332

### Acide α-(1-méthyléthyl)-4-[2-[[[(2S)-1-[[-3'-(trifluorométhyl)[1,1'-biphényl]-4-yl]sulfonyl]-2,3-dihydro-1H-indol-2-yl]carbonyl]amino]éthoxy]benzèneacétique, méthyl ester

En opérant de façon analogue à l'exemple 330, au départ de l'acide obtenu selon la préparation IV, on obtient le produit attendu sous forme d'une poudre blanche (rendement = 63 %).
F = 68-76°C

### Exemple 333

### Acide α-(1-méthyléthyl)-4-[2-[[[(2S)-1-[[2'-chloro-5'-(trifluorométhyl)-[1,1'-biphényl]-4-yl]sulfonyl]-2,3-dihydro-1H-indol-2-yl]carbonyl]amino]éthoxy]benzèneacétique

En opérant de façon analogue à l'exemple 22, au départ de l'ester obtenu selon l'exemple 332, on obtient le produit attendu sous forme d'un solide blanc (rendement = 14 %).
F = 84-88°C.

### Exemple 334

### Acide 2-[[[[(2S)-1-[[-3'-(triftuorométhyl)[1,1'-biphényl]-4-yl]sulfonyl]-2,3-dihydro-1H-indol-2-yl]carbonyl]amino]méthyl]benzène pentanoique, méthyl ester

En opérant de façon analogue à l'exemple 21, au départ de l'acide obtenu selon la préparation IV et du chlorhydrate de l'ester méthylique de l'acide 2-(aminométhyl)benzènepentanoique, on obtient le produit attendu sous forme d'une huile incolore (rendement = 80 %).
RMN ¹H (DMSO, 300MHz) δ : 8,61 (t, NH); 8,10-8,00 (m, 2H); 8,00-7,85 (m, 4H); 7,85-7,70 (m, 2H); 7,50 (d, 1 H); 7,30-7,10 (m, 6H); 7,03 (td, 1 H); 4,93 (dd, 1 H); 4,45-4,25 (m, 2H); 3,57 (s, 3H); 3,18 (dd, 1 H); 2,96 (dd, 1 H); 2,62 (t, 2H); 2,34 (t, 2H); 1,65-1,45 (m, 4H).

### Exemple 335

### Acide 2-[[[[(2S)-1-[[-3'-(trifluorométhyl)[1,1'-biphényl]-4-yl]sulfonyl]-2,3-dihydro-1H-indol-2-yl]carbonyl]amino]méthyl]benzène pentanoique

En opérant de façon analogue à l'exemple 22, au départ de l'ester obtenu selon l'exemple 334, on obtient le produit attendu sous forme d'un solide blanc (rendement = 98 %).
F = 98-99°C.

### PREPARATION CXXV

### Acide [[2-[3-(diméthylamino)propoxy]phényl]méthyl]carbamique phénylméthyl ester

En opérant de façon analogue à la préparation XCVI, au départ de l'ester benzylique de l'acide [(2-hydroxyphényl)méthyl]carbamique et de 3-(diméthylamino)propanol, on obtient le produit attendu sous forme d'une huile incolore (rendement = 81 %).
RMN ¹H (DMSO, 300MHz) δ : 7.63 (t, 1 H); 7,40-7,15 (m, 7H); 6,95-6,80 (m, 2H); 5,04 (s, 2H); 4,19 (d, 2H); 4,00 (t, 2H); 2,39 (t, 2H); 2,14 (s, 6H); 1,85 (t, 2H).

### PREPARATION CXXVI

### 2-[3-(diméthylamino)propoxy]benzèneméthanamine

En opérant de façon analogue à l'exemple 61 mais en travaillant dans l'éthanol, au départ du composé obtenu selon la préparation CXXV, on obtient le produit attendu sous forme d'une huile incolore (rendement = 97%).
RMN ¹H (DMSO, 300MHz) δ : 7.29 (d, 1H); 7,18 (td, 1H); 6,95-6,85 (m, 2H); 4,00 (t, 2H); 3,69 (s, 1 H); 2,39 (t, 2H); 2,14 (s, 6H); 1,90-1,80 (m, 2H).

### Exemple 336

### 2,3-dihydro-N-[[2-[3-(diméthylamino)propoxy]phényl]méthyl]-1-[[3'-(trifluorométhyl)-[1,1'-biphényl]-4-yl]sulfonyl]-(2S)-1H-indole-2-carboxamide (chlorhydrate)

En opérant de manière analogue à la préparation 1, au départ de l'acide obtenu selon la préparation IV et de l'amine obtenue selon la préparation CXXVI, on obtient une huile que l'on acidifie à l'aide d'une solution de chlorure d'hydrogène dans l'acétate d'éthyle pour obtenir le chlorhydrate sous forme d'un solide blanc (rendement = 86 %).
F = 129-132°C.

### PREPARATION CXXVII

### Acide 4-[[2-[[[(phénylméthoxy)carbonyl]amino]méthyl]phényl]amino] butanoique, méthyl ester

Dans un réacteur adapté pour réaction sous micro-ondes, on prépare une solution de 1g (3,9 mmol) d'ester benzylique de l'acide [(2-aminophényl)-méthyl]carbamique dans 10 ml d'acétonitrile et on ajoute 1,06g (5,85 mmol) d'ester méthylique de l'acide 4-bromobutanoique et 0,81 g (5,85 mmol) de carbonate de potassium. Le mélange réactionnel est chauffé 1 heure à 150°C sous micro-ondes, puis refroidi et repris par de l'acétate d'éthyle et de l'eau. La phase organique est séparée, lavée à l'eau, séchée sur sulfate de magnésium et concentrée sous pression réduite. L'huile jaune obtenue est purifiée par chromatographie sur gel de silice en éluant à l'aide d'un mélange méthylcyclohexane/acétate d'éthyle (7/3 ; v/v). On obtient ainsi le produit attendu sous forme d'une huile jaune avec un rendement de 35 %.
RMN ¹H (DMSO, 300MHz) δ : 7,68 (t, NH); 7,40-7,25 (m, 5H); 7,06 (td, 1 H); 6,99 (dd, 1 H); 6,60-6,50 (m, 2H); 5,05 (s large, NH + 2H); 4,07 (d, 2H); 3,59 (s, 3H); 3,06 (q, 2H); 2,42 (t, 2H); 1,78 (qu, 2H).

### PREPARATION CXXVIII

### Acide 4-[[2-[[[(phénylméthoxy)carbonyl]amino]méthyl]phényl]amino] butanoique

En opérant de façon analogue à l'exemple 22, au départ de l'ester obtenu selon la préparation CXXVII, on obtient le produit attendu sous forme d'une huile (rendement = 93 %).
RMN ¹H (DMSO, 300MHz) δ : 12,06 (s, COOH); 7,69 (t, NH); 7,40-7,25 (m, 5H); 7,04 (td, 1 H); 6,97 (dd, 1 H); 6,60-6,50 (m, 2H); 5,05 (s large, NH + 2H); 4,07 (d, 2H); 3,05 (t, 2H); 2,33 (t, 2H); 1,78 (qu, 2H).

### PREPARATION CXXIX

### Acide 4-[[2-(aminométhyl)phényl]amino]butanoique

En opérant de façon analogue à la préparation XXXVII au départ du composé obtenu selon la préparation CXXVIII, on obtient le produit attendu sous forme d'un solide blanc (rendement = 80 %).
RMN ¹H (DMSO, 300 MHz) δ : 7,10-7,00 (m, 2H); 6,50-6,54 (m, 2H); 3,72 (s, 2H); 3,08 (t, 2H); 2,24 (t, 2H); 1,81 (q, 2H).

### PREPARATION CXXX

### Chlorure de l'acide 2,3-dihydro-1-[[3'-(trifluorométhyl)[1,1'-biphényl]-4-yl]sulfonyl]-(2S)-1H-indole-2-carboxylique

Ce composé est obtenu de façon analogue à la préparation VI au départ de l'acide obtenu selon la préparation IV et engagé directement dans la réaction suivante.

### Exemple 337

### Acide 4-[[2-[[[[(2S)-2,3-dihydro-1-[[3'-(trifluorométhyl)[1,1'-biphényl]-4-yl]sulfonyl]-1H-indol-2-yl]carbonyl]amino]méthyl]phényl]amino] butanoique

En opérant de façon analogue à l'exemple 17, au départ des composés obtenus selon les préparations CXXIX et CXXX, on obtient le composé attendu sous forme d'un solide blanc (rendement=17 %).
F = 77-81 °C

### Exemple 338

### N-[(2,3-dihydro-2-oxo-1H-indol-7-yl)méthyl]-2,3-dihydro-1-[[3'-(trifluorométhyl)[1,1'-biphényl]-4-yl]suifonyl]-(2S)-1H-indole-2-carboxamide

En opérant de manière analogue à la préparation I, au départ de l'acide obtenu selon la préparation IV et de 7-(aminométhyl)-1;3-dihydro-2*H-*indol-2-one, on obtient le produit attendu sous forme d'un solide orange (rendement = 12 %).
F = 50°C.

### Exemple 339

### Acide 4-[2-[[[(2S)-1-[(2'-chloro-4'-fluoro[1,1'-biphényl]-4-yl)sulfonyl]-2,3-dihydro-1H-indol-2-yl]carbonyl]amino]éthoxy]-2-fluorobenzoïque, méthyl ester

En opérant de façon analogue à l'exemple 230, au départ de l'acide obtenu selon la préparation CVII, on obtient le composé attendu sous forme d'un solide blanc (rendement = 52 %).
F = 71-76°C

### Exemple 340

### Acide 4-[2-[[[(2S)-1-[(2'-chloro-4'-fluoro[1,1'-biphényl]-4-yl)sulfonyl]-2,3-dihydro-1H-indol-2-yl]carbonyl]amino]éthoxy]-2-fluorobenzoique

En opérant de façon analogue à l'exemple 22, au départ de l'ester obtenu selon l'exemple 339, on obtient le composé attendu sous forme d'un solide blanc (rendement = 71 %).
F = 98-102°C.

### Exemple 341

### Acide 4-[2-[[[(2S)-1-[(4'-fluoro-2'-méthoxy[1,1'-biphényl]-4-yl) sulfonyl]-2,3-dihydro-1H-indol-2-yl]carbonyl]amino]éthoxy]-2-fluorobenzoïque, méthyl ester

En opérant de façon analogue à l'exemple 230, au départ de l'acide obtenu selon la préparation CXI, on obtient le composé attendu sous forme d'un solide blanc (rendement = 66 %).
F = 73-80°C

### Exemple 342

### Acide 4-[2-[[[(2S)-1-[(4'-fluoro-2'-méthoxy[1,1'-biphényl]-4-yl) sulfonyl]-2,3-dihydro-1H-indol-2-yl]carbonyl]amino]éthoxy]-2-fluorobenzoïque

En opérant de façon analogue à l'exemple 22, au départ de l'ester obtenu selon l'exemple 341, on obtient le composé attendu sous forme d'un solide blanc (rendement= 62 %).
F = 88-95°C.

### Exemple 343

### Acide 4-[2-[[[(2S)-1-[(2',4-difluoro[1,1'-biphényl]-4-yl)sulfonyl]-2,3-dihydro-1H-indol-2-yl]carbonyl]amino]éthoxy]-2-fluorobenzdique, méthyl ester

En opérant de façon analogue à l'exemple 230, au départ de l'acide obtenu selon la préparation CV, on obtient le composé attendu sous forme d'un solide blanc (rendement = 53 %).
F = 65-68°C

### Exemple 344

### Acide 4-[2-[[[(2S)-1-[(2',4-difluoro[1,1'-biphényl]-4-yl)sulfonyl]-2,3-dihydro-1H-indol-2-yl]carbonyl]amino]éthoxy]-2-fluorobenzdique

En opérant de façon analogue à l'exemple 22, au départ de l'ester obtenu selon l'exemple 343, on obtient le composé attendu sous forme d'un solide blanc (rendement = 85 %).
F = 91-94°C

### Exemple 345

### Acide 4-[2-[[[(2S)-1-[(4'-fluoro-2'-méthyl[1,1'-biphényl]-4-yl) sulfonyl]-2,3-dihydro-1H-indol-2-yl]carbonyl]amino]éthoxy]-2-fluorobenzoïque, méthyl ester

En opérant de façon analogue à l'exemple 230, au départ de l'acide obtenu selon la préparation CIV, on obtient le composé attendu sous forme d'un solide blanc (rendement = 83 %).
F = 66-75°C.

### Exemple 346

### Acide 4-[2-[[[(2S)-1-[(4'-fluoro-2'-méthyl[1,1'-biphényl]-4-yl) sulfonyl]-2,3-dihydro-1H-indol-2-yllcarbonyl]amino]éthoxy]-2-fluorobenzoïque

En opérant de façon analogue à l'exemple 22, au départ de l'ester obtenu selon l'exemple 345, on obtient le composé attendu sous forme d'un solide blanc. (rendement = 80 %).
F = 109-118°C.

### Exemple 347

### Acide 6-[[[(2S)-2,3-dihydro-1-[[3'-(trifluorométhyl)[1,1'-biphényl]-4-yl]sulfonyl]-1H-indol-2-yl]carbonyl]amino]hexanoïque, méthyl ester

En opérant de manière analogue à l'exemple 98, au départ d'ester méthylique de l'acide 6-aminohexanoïque, on obtient le composé attendu sous forme d'une pâte incolore (rendement = 52 %).
RMN ¹H (DMSO, 250 MHz) δ : 8,16 (t, NH); 8,10-8,00 (m, 2H); 8,00-7,85 (m, 4H); 7,85-7,72 (m, 2H); 7,49 (d, 1 H); 7,24 (td, 1 H); 7,13 (d, 1 H); 7,02 (td, 1 H); 4,80 (dd, 1 H); 3,57 (s, 3H); 3,20-3,00 (m, 3H); 2,92 (dd, 1 H); 2,28 (t, 2H); 1,60-1,35 (m, 4H); 1,35-1,20 (m, 2H).

### Exemple 348

### Acide 6-[[[(2S)-2,3-dihydro-1-[[3'-(trifluororméthyl)[1,1'-biphényl]-4-yl]sulfonyl]-1H-indol-2-yl]carbonyl]amino]hexanoïque

En opérant de façon analogue à l'exemple 22, au départ de l'ester obtenu selon l'exemple 347, on obtient le composé attendu sous forme d'un solide jaune (rendement= 81 %).
F = 50°C.
RMN ¹H (DMSO, 300 MHz) δ : 11,97 (s, COOH), 8,17 (t, NH); 8,10-8,00 (m, 2H); 8,00-7,85 (m, 4H); 7,85-7,72 (m, 2H); 7,49 (d, 1 H); 7,24 (td, 1 H); 7,13 (d, 1 H); 7,02 (td, 1 H); 4,80 (dd, 1 H); 3,20-3,00 (m, 3H); 2,92 (dd, 1 H); 2,19 (t, 2H); 1,60-1,35 (m, 4H); 1,35-1,20 (m, 2H).

### Exemple 349

### Acide 2-méthyl-4-[2-[[[(2S)-1-[[3'-(trifluorométhyl)[1,1'-biphényl]-4-yl]sulfonyl]-2,3-dihydro-1H-indol-2-yl]carbonyl]amino]éthoxy] benzèneacétique, méthyl ester

En opérant de façon analogue à l'exemple 21, au départ de l'acide obtenu selon la préparation IV et de l'amine obtenue selon la préparation CXVIII, on obtient le produit attendu sous forme d'un solide blanc (rendement = 61 %).
F = 53-57°C.

### Exemple 350

### Acide 2-méthyl-4-[2-[[[(2S)-1-[[3'-(trifluorométhyl)[1,1'-biphényl]-4-yl]sulfonyl]-2,3-dihydro-1H-indol-2-yl]carbonyl]amino]éthoxy] benzèneacétique

En opérant de façon analogue à l'exemple 22, au départ du composé obtenu selon l'exemple 349, on obtient le produit attendu sous forme d'une poudre blanche (rendement = 51 %).
F = 87-90°C.

### Exemple 351

### Acide 2-méthyl-4-[2-[[[(2S)-1-[[2'-(trfluorométhyl)[1,1'-biphényl]-4-yl]sulfonyl]-2,3-dihydro-1H-indol-2-yl]carbonyl]amino]éthoxy] benzèneacétique, méthyl ester

En opérant de façon analogue à l'exemple 349, au départ de l'acide obtenu selon la préparation V, on obtient le produit attendu sous forme d'une pâte blanche (rendement = 90 %).
RMN ¹H (DMSO, 300 MHz) δ : 8,40 (t, NH); 7,90-7,80 (m, 3H); 7,80-7,70 (m, 2H); 7,50-7,45 (m, 3H); 7,39 (d, 1 H); 7,24 (t, 1 H); 7,13 (d, 1 H); 7,04 (t, 2H); 6,80-6,70 (m, 2H); 4,88 (dd, 1 H); 3,99 (t, 2H); 3,58 (s, 5H); 3,58-3,40 (m, 2H); 3,10-2,90 (m, 2H); 2,17 (s, 3H)

### Exemple 352

### Acide 2-méthyl-4-[2-[[[(2S)-1-[[2'-(trifluorométhyl)[1,1'-biphényl]-4-yl]sulfonyl]-2,3-dihydro-1H-indol-2-yl]carbonyl]amino]éthoxy] benzèneacétique

En opérant de façon analogue à l'exemple 22, au départ du composé obtenu selon l'exemple 351, on obtient le produit attendu sous forme d'une poudre blanche (rendement = 68 %).
F = 85-95°C.

### PREPARATION CXXXI

### Acide 2-méthyl-4-[[(trifluorométhyl)suifonyl]oxy]benzèneacétique, méthyl ester

On ajoute 0,85 ml (6,11 mmol) de triéthylamine à une solution de 0,55 g (3,06 mmol) d'ester méthylique de l'acide 2-méthyl-4-hydroxybenzèneacétique dans 22 ml de DCM. Le mélange est refroidi à 0°C et on ajoute 0,62 ml (3,67 mmol) d'anhydride trifluorométhanesulfonique. Le mélange réactionnel est agité à 0°C pendant 1 heure puis dilué avec 60 ml de DCM et lavé à l'eau. La phase organique est séchée sur sulfate de magnésium et concentrée sous pression réduite. Le produit brut obtenu est purifié par chromatographie sur gel de silice en éluant à l'aide d'un mélange cyclohexane/acétate d'éthyle (9/1 ; v/v). On obtient ainsi 670 mg du produit attendu sous forme d'une huile incolore (rendement = 70 %).
RMN ¹H (DMSO, 250 MHz) δ : 7.38 (d, 1 H); 7,33 (d, 1 H); 7,26 (dd, 1 H); 3.77 (s, 2H); 3,63 (s, 3H); 2.27 (s, 3H).

### PREPARATION CXXXII

### Acide 4-cyano-2-méthylbenzèneacétique, méthyl ester

Dans un réacteur pour travail sous micro-ondes, on introduit 0,564 mg (1,8 mmol) du composé obtenu selon la préparation CXXXI, 5 ml de DMF, 208 mg de tétrakis(triphénylphosphine)Palladium et 211 mg (1,8 mmol) de cyanure de zinc. Le mélange réactionnel est agité à 150°C pendant 5 mn puis dilué avec 25 ml d'acétate d'éthyle et lavé à l'eau. La phase organique est séchée sur sulfate de magnésium et concentrée sous pression réduite. Le produit brut obtenu est purifié par chromatographie sur gel de silice en éluant à l'aide d'un mélange cyclohexane/acétate d'éthyle (8/2 ; v/v). On obtient ainsi 150 mg du produit attendu sous forme d'un solide blanc (rendement = 44 %).
F = 52-54°C.

### PREPARATION CXXXIII

### Acide 4-(aminométhyl)-2-méthylbenzèneacétique, méthyl ester (chlorhydrate)

En opérant de façon analogue à la préparation LXIX, au départ de l'ester obtenu selon la préparation CXXXII, on obtient le produit attendu sous forme d'un solide blanc (rendement = 98 %).
F = 218-220°C.

### Exemple 353

### Acide 2-méthyl-4-[[[[(2S)-1-[[3'-(trifluorométhyl)[1,1'-biphényl]-4-yl]sulfonyl]-2,3-dihydro-1H-indol-2-yl]carbonyl]amino]méthyl] benzèneacétique, méthyl ester

En opérant de façon analogue à l'exemple 349, au départ de l'amine obtenue selon la préparation CXXXIII, on obtient le produit attendu sous forme d'un solide blanc (rendement = 80 %).
F = 60-67°C.

### PREPARATION CXXXIV

### Acide 4-[[[[(2S)-2,3-dihydro-1-[(4-iodophényl)sulfonyl]-1H-indol-2-yl]carbonyl]amino]méthyl]benzoïque, méthyl ester

En opérant de manière analogue à la préparation VIIa, de l'ester méthylique de l'acide 4-(aminométhyl)benzoïque, on obtient le composé attendu sous forme d'un solide blanc (rendement = 92 %).
F = 80-85°C.

### PREPARATION CXXXV

### Acide 4-[[[[(2S)-2,3-dihydro-1-[(4-iodophényl)sulfonyl]-1H-indol-2-yl]carbonyl]amino]méthyl]benzoïque

En opérant de manière analogue à l'exemple 22, au départ de l'ester obtenu selon la préparation CXXXIV, on obtient le composé attendu sous forme d'une poudre blanche (rendement = 98 %).
F = 110-120°C.

### Exemple 354

### Acide 4-[[[[(2S)-1-[(2'-méthoxy-5'-méthyl[1,1'-biphényi]-4-yl)sulfonyl]-2,3-dihydro-1H-indol-2-yl]carbonyl]amino]méthyl]benzoïque

En opérant de façon analogue à l'exemple 1, au départ de l'acide obtenu selon la préparation CXXXV et d'acide 2-méthoxy-5-méthylphénylboronique, on obtient le produit attendu sous forme d'un solide blanc (rendement = 23 %).
F=153-165°C.

En opérant de façon analogue à l'exemple 354, au départ de différents acides phénylboroniques, on obtient les produits suivants :

### Exemple 355

### Acide 4-[[[[(2S)-1-[(2'-fluoro-5'-méthyl[1,1'-biphényl]-4-yl)sulfonyl]-2,3-dihydro-1H-indol-2-yl]carbonyl]amino]méthyl]benzoïque

solide blanc (rendement = 62 %).
F = 105-120°C.

### Exemple 356

### Acide 4-[[[[(2S)-1-[[2'-fluoro-5'-(trifluorométhyl)[1,1'-biphényl]-4-yl]sulfonyl]-2,3-dihydro-1H-indol-2-yl]carbonyl]amino]méthyl] benzoïque

solide blanc (rendement = 84 %).
F = 115-120°C.

### Exemple 357

### Acide 4-[[[[(2S)-1-[(2',5'-diméthyl[1,1'-biphényl]-4-yl)sulfonyl]-2,3-dihydro-1H-indoli-2-yl]carbonyl]amino]méthyl]benzoïque

solide blanc (rendement = 81 %).
F = 126-136°C.

### Exemple 358

### Acide 4-[[[[(2S)-1-[[2'-chloro-5'-(trifluorométhyl)[1,1'-biphényl]-4-yl]sulfonyl]-2,3-dihydro-1H-indol-2-yl]carbonyl]amino]méthyl] benzoïque

solide beige (rendement = 71 %).
F = 120-130°C.

### Exemple 359

### Acide 4-[[[(2S)-1-[(3'-méthyl[1,1'-biphényl]-4-yl)sulfonyl]-2,3-dihydro-1H-indol-2-yl]carbonyl]amino]méthyl]benzoïque

solide beige (rendement = 51 %).
F=118-130°C.

### Exemple 360

### Acide 4-[[[[(2S)-1-[[2'-méthoxy-5'-(trifluorométhyl)[1,1'-biphényl]-4-yl]sulfonyl]-2,3-dihydro-1H-indol-2-yl]carbonyl]amino]méthyl] benzoïque

solide jaune (rendement = 74 %).
F = 122-130°C.

### Exemple 361

### Acide 4-[[[[(2S)-1-[[2'-méthyl-5'-(trifluorométhyl)[1,1'-biphényl]-4-yl]sulfonyl]-2,3-dihydro-1H-indol-2-yl]carbonyl]amino]méthyl] benzoïque

solide beige (rendement = 76 %).
F = 120-128°C.

### Exemple 362

### Acide 4-[[[[(2S)-1-[(2'-chloro-5'-méthyl[1,1'-biphényl]-4-yl)sulfonyl]-2,3-dihydro-1H-indol-2-yl]carbonyl]amino]méthyl]benzoïque

solide blanc (rendement = 37 %).
F = 104-110°C.

En opérant de façon analogue aux préparations CXXXI à CXXXIII, au départ de l'ester méthylique de l'acide 4-hydroxy-3-méthoxybenzèneacétique, on obtient les composés suivants :

### PREPARATION CXXXVI

### Acide 3-méthoxy-4-[[(trifluorométhyl)sulfonyl]oxy]benzèneacétique, méthyl ester

solide blanc (rendement = 94 %).
F = 57-59°C.

### PREPARATION CXXXVII

### Acide 4-cyano-3-méthoxybenzèneacétique, méthyl ester

solide blanc (rendement = 36 %).
F = 57-60°C.

### PREPARATION CXXXVIII

### Acide 4-(aminométhyl)-3-méthoxybenzèneacétique, méthyl ester (chlorhydrate)

solide jaune clair (rendement = 98 %).
F = 180-184°C.

### Exemple 363

### Acide 3-méthoxy-4-[[[[(2S)-1-[[3'-(trifluorométhyl)[1,1'-biphényl]-4-yl]sulfonyl]-2,3-dihydro-1H-indol-2-yl]carbonyl]amino]méthyl] benzèneacétique, méthyl ester

En opérant de façon analogue à l'exemple 353, au départ de l'amine obtenue selon la préparation CXXXVIII, on obtient le produit attendu sous forme d'une pâte blanche (rendement = 82 %).
RMN ¹H (DMSO, 250 MHz) δ: 8,48 (t, NH); 8,10-8,00 (m, 2H); 8,00-7,85 (m, 4H); 7,85-7,70 (m, 2H); 7,50 (d, 1 H); 7,15 (td, 1 H); 7,20-7,10 (m, 2H); 7,03 (td, 1 H); 6,90 (d, 1 H); 6,79 (dd, 1 H); 4,95 (dd, 1 H); 4,30-4,15 (m, 2H); 3,80 (s, 3H); 3,65 (s, 2H); 3,61 (s, 3H); 3,16 (dd, 1 H); 2,97 (dd, 1 H).

### Exemple 364

### Acide 3-méthoxy-4-[[[[(2S)-1-[[3'-(trifluorométhyl)[1,1'-biphényl]-4-yl]sulfonyl]-2,3-dihydro-1H-indol-2-yl]carbonyl]amino]méthyl] benzèneacétique

En opérant de manière analogue à l'exemple 22, au départ de l'ester obtenu selon l'exemple 363, on obtient le composé attendu sous forme d'une poudre blanche (rendement = 95 %).
F = 99-105°C.

### Exemple 365

### Acide 3-fluoro-4-[[[[(2S)-1-[[3'-(trifluorométhyl)[1,1'-biphényl]-4-yl]sulfonyl]-2,3-dihydro-1H-indol-2-yl]carbonyl]amino]méthyl] benzèneacétique, méthyl ester

En opérant de façon analogue à l'exemple 353, au départ du chlorhydrate de l'ester méthylique de l'acide 4-(aminométhyl)-3-fluorobenzèneacétique, on obtient le produit attendu sous forme d'un solide blanc (rendement = 85 %).
F = 50-57°C.

### Exemple 366

### Acide 3-fluoro-4-[[[[(2S)-1-[[3'-(trifluorométhyl)[1,1'-biphényl]-4-yl]sulfonyl]-2,3-dihydro-1H-indol-2-yl]carbonyl]amino]méthyl] benzèneacétique

En opérant de manière analogue à l'exemple 22, au départ de l'ester obtenu selon l'exemple 365, on obtient le composé attendu sous forme d'une poudre blanche (rendement = 95 %).
F = 99-105°C.

### Exemple 367

### Acide 3-chloro-4-[[[[(2S)-1-[[3'-(trifluorométhyl)[1,1'-biphényl]-4-yl]sulfonyl]-2,3-dihydro-1H-indol-2-yl]carbonyl]amino]méthyl] benzèneacétique, méthyl ester

En opérant de façon analogue à l'exemple 353, au départ du chlorhydrate de l'ester méthylique de l'acide 4-(aminométhyl)-3-chlorobenzèneacétique, on obtient le produit attendu sous forme d'un solide blanc (rendement = 59%).
F = 124-126°C.

### Exemple 368

### Acide 3-chloro-4-[[[[(2S)-1-[[3'-(trifluorométhyl)[1,1'-biphényl]-4-yl]sulfonyl]-2,3-dihydro-1H-indol-2-yl]carbonyl]amino]méthyl] benzèneacétique

En opérant de manière analogue à l'exemple 22, au départ de l'ester obtenu selon l'exemple 367, on obtient le composé attendu sous forme d'une poudre blanche (rendement = 85 %).
F = 95-106°C.

### Exemple 369

### Acide 3-chloro-4-[[[[(2S)-1-[[3'-(trifluorométhyl)[1,1'-biphényl]-4-yl]sulfonyl]-2,3-dihydro-1H-indol-2-yl]carbonyl]amino]méthyl] benzoïque, méthyl ester

En opérant de façon analogue à l'exemple 353, au départ de l'ester méthylique de l'acide 4-(aminométhyl)-3-chlorobenzoïque, on obtient le produit attendu sous forme d'un solide blanc (rendement = 89 %).
F = 78-87°C.

### Exemple 370

### Acide 3-chloro-4-[[[[(2S)-1-[[3'-(trifluorométhyl)[1,1'-biphényl]-4-yl]sulfonyl]-2,3-dihydro-1H-indol-2-yl]carbonyl]amino]méthyl] benzoïque

En opérant de manière analogue à l'exemple 22, au départ de l'ester obtenu selon l'exemple 369, on obtient le composé attendu sous forme d'une poudre blanche (rendement = 88 %).
F = 110-120°C.

### Exemple 371

### Acide 3-méthoxy-4-[[[[(2S)-1-[[3'-(trifluorométhyl)[1,1'-biphényl]-4-yl]sulfonyl]-2,3-dihydro-1H-indol-2-yl]carbonyl]amino]méthyl] benzoïque, méthyl ester

En opérant de façon analogue à l'exemple 353, au départ de l'ester méthylique de l'acide 4-(aminométhyl)-3-méthoxybenzoïque, on obtient le produit attendu sous forme d'un solide blanc (rendement = 76 %).
F = 78-85°C.

### Exemple 372

### Acide 3-méthoxy-4-[[[[(2S)-1-[[3'-(trifluorométhyl)[1,1 -biphényl]-4-yl]sulfonyl]-2,3-dihydro-1H-indol-2-yl]carbonyl]amino]méthyl] benzoïque

En opérant de manière analogue à l'exemple 22, au départ de l'ester obtenu selon l'exemple 371, on obtient le composé attendu sous forme d'une poudre blanche (rendement = 93 %).
F = 108-120°C.

### Exemple 373

### N-[2-(4-cyanophénoxy)éthyl]-2,3-dihydro-1-[[3'-(trifluorométhyl)[1,1'-biphényl]-4-yl]sulfonyl]-(2S)-1H-indole-2-carboxamide

En opérant de manière analogue à l'exemple 349, au départ du 4-(2-aminoéthoxy)benzonitrile, on obtient le composé attendu sous forme d'une poudre blanche (rendement = 71 %).
F = 70-80°C.

### Exemple 374

### Acide 2,6-difluoro-4-[2-[[[(2S)-1-[(2',4'-difluoro[1,1'-biphényl]-4-yl)sulfonyl]-2,3-dihydro-1H-indol-2-yl]carbonyl]amino]éthoxy] benzoïque, méthyl ester

En opérant de façon analogue à l'exemple 21, au départ de l'acide obtenu selon la préparation CV et de l'amine obtenue selon la préparation LXXXI, on obtient le produit attendu sous forme d'un solide blanc (rendement = 59%).
F = 69-77°C

### Exemple 375

### Acide 2,6-difluoro-4-[2-[[[(2S)-1-[(4'-fluoro-2'-méthoxy[1,1'-biphényl]-4-yl)sulfonyl]-2,3-dihydro-1H-indol-2-yl]carbonyl]amino]éthoxy] benzoïque, méthyl ester

En opérant de façon analogue à l'exemple 374, au départ de l'acide obtenu selon la préparation CXI, on obtient le produit attendu sous forme d'un solide blanc (rendement = 58 %).
F = 65-76°C

### Exemple 376

### Acide 2,6-difluoro-4-[2-[[[(2S)-1-[(2'-chloro-4'-fluoro[1,1'-biphényl]-4-yl)sulfonyl]-2,3-dihydro-1H-indol-2-yl]carbonyl]amino]éthoxy] benzoïque, méthyl ester

En opérant de façon analogue à l'exemple 374, au départ de l'acide obtenu selon la préparation CVII, on obtient le produit attendu sous forme d'un solide blanc (rendement = 60 %).
F = 68-74°C.

### Exemple 377

### Acide 2,6-difluoro-4-[2-[[[(2S)-1-[(2'-chloro-4'-fluoro[1,1'-biphényl]-4-yl)sulfonyl]-2,3-dihydro-1H-indol-2-yl]carbonyl]amino]éthoxy] benzoïque

En opérant de manière analogue à l'exemple 22, au départ de l'ester obtenu selon l'exemple 376, on obtient le composé attendu sous forme d'une poudre blanche (rendement = 55 %).
F = 75-80°C.

### Exemple 378

### Acide 4-[2-[[[(2S)-1-[[4'-méthyl-2-(trifluorométhyl)[1,1'-biphényl]-4-yl]sulfonyl]-2,3-dihydro-1H-indol-2-yl]carbonyl]amino]éthoxy] benzèneacétique, méthyl ester

En opérant de façon analogue à l'exemple 1 au départ du composé obtenu selon la préparation XI et d'acide 4-méthylphénylboronique, on obtient le produit attendu sous forme d'un solide fin blanc (rendement = 81 %).
F = 60°C.

### Exemple 379

### Acide 4-[2-[[[(2S)-1-[[4'-méthyl-2-(trifluorométhyl)[1,1'-biphényl]-4-yl]sulfonyl]-2,3-dihydro-1H-Nindol-2-yl]carbonyl]amino]éthoxy] benzèneacétique

En opérant de manière analogue à l'exemple 22, au départ de l'ester obtenu selon l'exemple 378, on obtient le composé attendu sous forme d'une poudre blanche (rendement = 95 %).
F = 92°C.

### Exemple 380

### Acide 4-[2-[[[(2S)-1-[[4'-chloro-2-(trifluorométhyl)[1,1'-biphényl]-4-yl]sulfonyl]-2,3-dihydro-1H-indol-2-yl]carbonyl]amino]éthoxy] benzèneacétique, méthyl ester

En opérant de façon analogue à l'exemple 378 au départ d'acide 4-chlorophénylboronique, on obtient le produit attendu sous forme d'un solide fin blanc (rendement = 80 %).
F = 60°C.

### Exemple 381

### Acide 4-[2-[[[(2S)-1-[[4'-chloro-2-(trifluorométhyl)[1,1'-biphényl]-4-yl]sulfonyl]-2,3-dihydro-1H-indol-2-yl]carbonyl]amino]éthoxy] benzèneacétique

En opérant de manière analogue à l'exemple 22, au départ de l'ester obtenu selon l'exemple 380, on obtient le composé attendu sous forme d'une poudre blanche (rendement = 92 %).
F = 92°C.

### Exemple 382

### Acide 4-[2-[[[(2S)-1-[[4'-fluoro-2-(trifluorométhyl)[1,1'-biphényl]-4-yl]sulfonyl]-2,3-dihydro-1H-indol-2-yl]carbonyl]amino]éthoxy] benzèneacétique, méthyl ester

En opérant de façon analogue à l'exemple 378 au départ d'acide 4-fluorophénylboronique, on obtient le produit attendu sous forme d'un solide fin blanc (rendement = 80 %).
F = 60°C.

### Exemple 383

### Acide 4-[2-[[[(2S)-1-[[4'-fluoro-2-(trifluorométhyl)[1,1'-biphényl]-4-yl]sulfonyl]-2,3-dihydro-1H-indol-2-yl]carbonyl]amino]éthoxy] benzèneacétique

En opérant de manière analogue à l'exemple 22, au départ de l'ester obtenu selon l'exemple 382, on obtient le composé attendu sous forme d'une poudre blanche (rendement = 97 %).
F = 92°C.

### PREPARATION CXXXIX

### Acide 4-[2-[[[(2S)-1-[(4-bromo-3-fluorophényl)sulfonyl]-2,3-dihydro-1H-indol-2-yl]carbonyl]amino]éthoxy]benzèneacétique, méthyl ester

En opérant de façon analogue à la préparation XI, au départ du chlorure de 4-bromo-3-fluorobenzènesulfonyle, on obtient le composé attendu sous forme d'une pâte blanche (rendement = 67 %).
RMN ¹H (DMSO, 250MHz) δ : 8,42 (t, NH); 7,95-7,81 (m, 2H); 7,55 (dd, 1 H); 7,43 (d, 1 H); 7,35-7,10 (m, 4H); 7,03 (td, 1 H); 6,95-6,80 (m, 2H); 4,89 (dd, 1 H); 4,10-3,90 (m, 2H); 3,59 (s, 5H); 3,59-3,40 (m, 2H); 3,20 (dd, 1 H); 2,93 (dd, 1 H)

### Exemple 384

### Acide 4-[2-[[[(2S)-1-[[2-fluoro-3'-(trifluorométhyl)[1,1'-biphényl]-4-yl]sulfonyl]-2,3-dihydro-1H-indol-2-yl]carbonyl]amino]éthoxy] benzèneacétique, méthyl ester

En opérant de façon analogue à l'exemple 378 au départ d'acide 3-(trifluorométhyl)phénylboronique et du composé obtenu selon la préparation CXXXIX, on obtient le produit attendu sous forme d'une mousse rose (rendement = 69 %).
RMN ¹H (DMSO, 250MHz) δ : 8,46 (t, NH); 7,92-7,70 (m, 7H); 7,48 (d, 1 H); 7,30-7,10 (m, 4H); 7,03 (td, 1 H); 6,95-6,85 (m, 2H); 4,95 (dd, 1 H); 4,02 (t, 2H); 3.59 (s, 5H); 3,59-3,40 (m, 2H); 3,25 (dd, 1 H); 2,96 (dd, 1 H)

### Exemple 385

### Acide 4-[2-[[[(2S)-1-[[2-fluoro-3'-(trifluorométhyl)[1,1'-biphényl]-4-yl]sulfonyl]-2,3-dihydro-1H-indol-2-yl]carbonyl]amino]éthoxy] benzèneacétique

En opérant de manière analogue à l'exemple 22, au départ de l'ester obtenu selon l'exemple 384, on obtient le composé attendu sous forme d'une poudre jaune (rendement = 93 %).
F = 82°C.

### PREPARATION CXL

### Acide 4-[2-[[[(2S)-1-[(4-bromo-3-chlorophényl)sulfonyl]-2,3-dihydro-1H-indol-2-yl]carbonyl]amino]éthoxy]benzèneacétique, méthyl ester

En opérant de façon analogue à la préparation XI, au départ du chlorure de 4-bromo-3-chlorobenzènesulfonyle, on obtient le composé attendu sous forme d'une mousse blanche (rendement = 87 %).
RMN ¹H (DMSO, 250MHz) δ: 8,44 (t, NH); 8,01 (d, 1 H); 7,97 (d, 1 H); 7,63 (dd, 1 H); 7,42 (d, 1 H); 7,30-7,10 (m, 4H); 7,04 (td, 1 H); 6,95-6,85 (m, 2H); 4,91 (dd, 1 H); 4,00 (t, 2H); 3,59 (s, 5H); 3,59-3,40 (m, 2H); 3,20 (dd, 1 H); 2,92 (dd, 1H).

### Exemple 386

### Acide 4-[2-[[[(2S)-1-[[2-chloro-3'-(trifluorométhyl)[1,1'-biphényl]-4-yl]sulfonyl]-2,3-dihydro-1H-indol-2-yl]carbonyl]amino]éthoxy] benzèneacétique, méthyl ester

En opérant de façon analogue à l'exemple 384 au départ du composé obtenu selon la préparation CXL, on obtient le produit attendu sous forme d'une mousse jaune (rendement = 84 %).
F = 60°C.

### Exemple 387

### Acide 4-[2-[[[(2S)-1-[[2-fluoro-3'-(trifluorométhyl)[1,1'-biphényl]-4-yl]sulfonyl]-2,3-dihydro-1H-indol-2-yl]carbonyl]amino]éthoxy] benzèneacétique

En opérant de manière analogue à l'exemple 22, au départ de l'ester obtenu selon l'exemple 386, on obtient le composé attendu sous forme d'une mousse blanche (rendement = 98 %).
F = 92°C.

### PREPARATION CXLI

### Acide 4-[2-[[[(2S)-1-[(4-bromo-3-méthylphényl)sulfonyl]-2,3-dihydro-1H-indol-2-yl]carbonyl]amino]éthoxy]benzèneacétique, méthyl ester

En opérant de façon analogue à la préparation XI, au départ du chlorure de 4-bromo-3-méthylbenzènesulfonyle, on obtient le composé attendu sous forme d'une mousse blanche (rendement = 87 %).
RMN ¹H (DMSO, 250MHz) δ: 8,36 (t, NH); 7,80 (d, 1 H); 7,74 (d, 1 H); 7,50-7,40 (m, 2H); 7,35-7,10 (m, 4H); 7,02 (td, 1H); 6,95-6,85 (m, 2H); 4,86 (dd, 1 H); 4,00 (t, 2H); 3,60 (s, 5H); 3,60-3,35 (m, 2H); 3,13 (dd, 1 H); 2,91 (dd, 1 H).

### Exemple 388

### Acide 4-[2-[[[(2S)-1-[[2-méthyl-3'-(trifluorométhyl)[1,1'-biphényl]-4-yl]sulfonyl]-2,3-dihydro-1H-indol-2-yl]carbonyl]amino]éthoxy] benzèneacétique, méthyl ester

En opérant de façon analogue à l'exemple 384 au départ du composé obtenu selon la préparation CXLI, on obtient le produit attendu sous forme d'une poudre blanche (rendement = 84 %).
F = 60°C.

### Exemple 389

### Acide 4-[2-[[[(2S)-1-[[2-méthyl-3'-(trifluorométhyl)[1,1'-biphényl]-4-yl]sulfonyl]-2,3-dihydro-1H-indol-2-yl]carbonyl]amino]éthoxy] benzèneacétique

En opérant de manière analogue à l'exemple 22, au départ de l'ester obtenu selon l'exemple 388, on obtient le composé attendu sous forme d'une mousse blanche (rendement = 94 %).
F = 92°C.

### PREPARATION CXLII

### Acide 5-cyano-2-pyridineacétique, 1,1-diméthyléthyl ester

Dans un réacteur pour travail sous micro-ondes, on introduit 0,715 g (10 mmol) de zinc, quelques paillettes d'iode, et 0,845 ml (5.4 mmol) de bromoacétate de t-butyle dans 10 ml de THF. Le mélange est chauffé 5 min à 110°C sous micro-ondes, puis filtré et ajouté à une solution de 0,5 g (2,7 mmol) de 6-bromo-3-pyridinecarbonitrile dans 15 ml de THF.

On ajoute 316 mg de tétrakis(triphénylphosphine)Palladium et le mélange réactionnel est agité à 120°C pendant 5 mn sous micro-ondes puis refroidi et dilué avec une solution de chlorure d'ammonium et extrait par l'acétate d'éthyle. La phase organique est lavée à l'eau puis séchée sur sulfate de magnésium et concentrée sous pression réduite. Le produit brut obtenu est purifié par chromatographie sur gel de silice en éluant à l'aide d'un mélange méthylcyclohexane/acétate d'éthyle (9/1 ; v/v). On obtient ainsi 254 mg du produit attendu sous forme d'un solide jaune (rendement = 43%).
RMN¹ H (DMSO, 250MHz) δ: 8,95 (d, 1 H); 8,27 (dd, 1 H); 7,57 (d, 1 H); 3,87 (s, 2H); 1,39 (s, 9H).

### PREPARATION CXLIII

### Acide 5-(aminométhyl)-2-pyridineacétique, 1,1-diméthyléthyl ester

On prépare une solution de 90 mg (0,41 mmol) du composé obtenu selon la préparation CXLII dans 5 ml de méthanol et on ajoute 1520 mg de Nickel préparé selon Raney. Le mélange est agité sous atmosphère d'hydrogène, à pression atmosphérique et à température ambiante pendant 3 heures, puis filtré et concentré sous pression réduite. On obtient ainsi le produit attendu sous forme d'une huile jaune (rendement = 90%).
RMN¹ H (DMSO, 250MHz) δ: 8,41 (d, 1 H); 7,68 (dd, 1 H); 7,24 (d, 1 H); 3,70 (s, 2H); 3,68 (s, 2H); 1,39 (s, 9H)

### Exemple 390

### Acide 5-[[[[(2S)-2,3-dihydro-1-[[3'-(trifluorométhyl)[1,1'-biphényl]-4-yl]sulfonyl]-1H-indol-2-yl]carbonyl]amino]méthyl]-2-pyridineacétique, 1,1-diméthyléthyl ester

En opérant de façon analogue à l'exemple 349, au départ du composé obtenu selon la préparation CXLIII, on obtient le produit attendu sous forme d'une mousse jaune (rendement = 75 %).
F = 70°C.

### Exemple 391

### N-[(3-cyanophényl)méthyl]-2,3-dihydro-1-[[3'-(trifluorométhyl)[1,1'-biphényl]-4-yl]sulfonyl]-(2S)-1H-indole-2-carboxamide

En opérant de manière analogue à l'exemple 349, au départ du 3-(aminométhyl)benzonitrile, on obtient le composé attendu sous forme d'une mousse blanche (rendement = 96 %).
RMN¹ H (DMSO,300MHz) δ: 8,88 (t, NH); 8,10-8,00 (m, 2H); 8,00-7,90 (m, 4H); 7,80 (d, 1 H); 7,75-7,70 (m, 3H); 7,70-7,60 (m, 1 H); 7,60-7,50 (m, 2H); 7,26 (td, 1 H); 7,15 (d, 1 H); 7,04 (td, 1 H); 4,91 (dd, 1 H); 4.41 (d, 2H); 3,20 (dd, 1 H); 2,99 (dd, 1 H)

### Exemple 392

### N-[[3-(aminométhyl)phényl]méthyl]-2,3-dihydro-1-[[3'-(trifluorométhyl)[1,1'-biphényl]-4-yl]sulfonyl]-(2S)-1H-indole-2-carboxamide (chlorhydrate)

En opérant de manière analogue à la préparation XV, au départ du composé obtenu selon l'exemple 391, on obtient le composé attendu sous forme d'une mousse blanche (rendement = 96 %).
F = 116°C.

### Exemple 393

### N-[(2-amino-3-pyridinyl)méthyl]-2,3-dihydro-1-[[3'-(trifluorométhyl)-[1,1'-biphényl]-4-yl]sulfonyl]-(2S)-1H-indole-2-carboxamide

En opérant de manière analogue à l'exemple 349, au départ de 2-amino-3-pyridineméthanamine, on obtient le composé attendu sous forme d'un solide blanc (rendement = 32 %).
F = 94°C

### PREPARATION CXLIV

### Acide 4-[2-[[(phénylméthoxy)carbonyl]amino]éthoxy]benzoïque, 1,1-diméthyléthyl ester

On prépare une solution de 33 g (0,17 mole) d'ester t-butylique de l'acide 4-hydroxybenzoïque dans 550 ml d'acétonitrile et on ajoute 43,8 g (0,17 mole) d'ester benzylique de l'acide 2-bromoéthylcarbamique et 55,3 g (0,17mole) de carbonate de césium. Le mélange est agité pendant 1 heure à 50°C, puis 16 heures à température ambiante. Ce milieu réactionnel est ensuite filtré et concentré sous pression réduite. Le résidu d'évaporation est repris dans l'acétate d'éthyle, lavé par une solution de soude 2N, puis à l'eau, et séché sur sulfate de magnésium. Après concentration de cette phase organique sous pression réduite, le produit brut est purifié par chromatographie sur gel de silice en éluant à l'aide d'un mélange dichlorométhane/acétate d'éthyle (98/2 ; v/v). On obtient ainsi 37 g du composé attendu sous forme d'une huile incolore (rendement = 58%).
RMN ¹H (DMSO, 300 MHz) δ: 7,83 (d, 2H); 7,50 (t, NH); 7,40-7,35 (m, 5H); 7,01 (d, 2H); 5,03 (s, 2H); 4.06 (t, 2H); 2,39 (q, 2H); 1,52 (s, 9H).

### PREPARATION CXLV

### Acide 4-[2-(amino)éthoxy]benzoïque, 1,1-diméthyléthyl ester

En opérant de manière analogue à l'exemple 61, au départ du composé obtenu selon la préparation CXLIV, on obtient le composé attendu sous forme d'un solide rose (rendement = 96 %).
RMN ¹H (DMSO, 300 MHz) δ: 7,84 (d, 2H); 7,01 (d, 2H); 4.00 (t, 2H); 2,91 (t, 2H); 2,80 (s large, NH2); 1,52 (s, 9H).

### PREPARATION CXLVI

### Acide 4-[2-[[[(2S)-2,3-dihydro-1-[(4-iodophényl)sulfonyl]-1H-indol-2-yl]carbonyl]amino]éthoxy]benzoique, 1,1-diméthyléthyl ester

En opérant de manière analogue à la préparation I, au départ des composés obtenus selon les préparations IVa et CXLV, on obtient le composé attendu sous forme d'une mousse blanche (rendement = 96 %). RMN ¹H (DMSO, 300 MHz) δ : 8,42 (t, NH); 7,93 (d, 2H); 7,84 (d, 2H); 7,53 (d, 2H); 7,42 (d, 1 H); 7,22 (td, 1 H); 7,13 (d 1 H); 7,05-6,95 (m, 3H); 4,81 (dd, 1 H); 4,10 (t, 2H); 3,60-3,40 (m, 2H); 3,13 (dd, 1 H); 2,90 (dd, 1 H); 1,53 (s, 9H).

### Exemple 394

### Acide 4-[2-[[[(2S)-1-[(4'-fluoro-2'-méthyl[1,1'-biphényl]-4-yl)sulfonyl]-2,3-dihydro-1H-indol-2-yl]carbonyl]amino]éthoxy]benzoïque, 1,1-diméthyléthyl ester

En opérant de façon analogue à l'exemple 1, au départ de l'ester obtenu selon la préparation CXLVI et d'acide 4-fluoro-2-méthylphénylboronique, on obtient le produit attendu sous forme d'une poudre blanche (rendement = 91 %).
F = 84°C.

### Exemple 395

### Acide 4-[2-[[[(2S)-1-[(4'-fluoro-2'-méthyl[1,1'-biphényl]-4-yl)sulfonyl]-2,3-dihydro-1H-indol-2-yl]carbonyl]amino]éthoxy]benzoïque

En opérant de façon analogue à l'exemple 298, au départ de l'ester obtenu selon l'exemple 394, on obtient le produit attendu sous forme d'une poudre blanche (rendement = 98 %).
F = 96°C.

### Exemple 396

### Acide 4-[2-[[[(2S)-1-[(2',4'-difluoro[1,1'-biphényl]-4-yl)sulfonyl]-2,3-dihydro-1H-indol-2-yl]carbonyl]amino]éthoxy]benzoïque, 1,1-diméthyléthyl ester

En opérant de façon analogue à l'exemple 394, au départ de l'acide 2,4-difluorophénylboronique, on obtient le produit attendu sous forme d'une poudre beige (rendement = 88 %).
F = 80°C.

### Exemple 397

### Acide 4-[2-[[[(2S)-1-[(2',4'-difluoro[1,1'-biphényl]-4-yl)sulfonyl]-2,3-dihydro-1H-indol-2-yl]carbonyl]amino]éthoxy]benzoïque

En opérant de façon analogue à l'exemple 298, au départ de l'ester obtenu selon l'exemple 396, on obtient le produit attendu sous forme d'une poudre jaune (rendement = 99 %).
F = 92°C.

### Exemple 398

### Acide 4-[2-[[[(2S)-1-[(2'-chloro- 4'-fluoro[1,1'-biphényl]-4-yl)sulfonyl]-2,3-dihydro-1H-indol-2-yl]carbonyl]amino]éthoxy]benzdique, 1,1-diméthyléthyl ester

En opérant de façon analogue à l'exemple 394, au départ de l'acide 2-chloro-4-fluorophénylboronique, on obtient le produit attendu sous forme d'une poudre blanche (rendement = 95 %).
F = 78°C.

### Exemple 399

### Acide 4-[2-[[[(2S)-1-[(2'-chloro- 4'-fluoro[1,1'-biphényl]-4-yl)sulfonyl]-2,3-dihydro-1H-indol-2-yl]carbonyl]amino]éthoxy]benzoïque

En opérant de façon analogue à l'exemple 298, au départ de l'ester obtenu selon l'exemple 398, on obtient le produit attendu sous forme d'une poudre jaune (rendement = 98 %).
F = 90°C.

### Exemple 400

### Acide 4-[2-[[[(2S)-1-[(4'-fluoro-2'-méthoxy[1,1'-biphényl]-4-yl)sulfonyl]-2,3-dihydro-1H-indol-2-yllcarbonyl]amino]éthoxy] benzoïque, 1,1-diméthyléthyl ester

En opérant de façon analogue à l'exemple 394, au départ de l'acide 4-fluoro-2-méthoxyphénylboronique, on obtient le produit attendu sous forme d'une poudre blanche (rendement = 94 %).
F = 86°C.

### Exemple 401

### Acide 4-[2-[[[(2S)-1-[(4'-fluoro-2'-méthoxy[1,1'-biphényl]-4-yl)sulfonyl]-2,3-dihydro-1H-indol-2-yl]carbonyl]amino]éthoxy] benzoïque

En opérant de façon analogue à l'exemple 298, au départ de l'ester obtenu selon l'exemple 400, on obtient le produit attendu sous forme d'une poudre blanche (rendement = 94%).
F = 102°C.

### PREPARATION CXLVII

### Acide 2,3-dihydro-1-[[3'-(trifluorométhyl)[1,1'-biphényl]-4-yl]sulfonyl]-1H-indole-2-carboxylique

En opérant de façon analogue à la préparation IV, au départ de l'acide 1,2-dihydro-indole-2-carboxylique, on obtient le produit attendu sous forme d'une poudre rose (rendement = 73 % pour le dérivé intermédiaire iodé et 60% pour le composé attendu).
F = 166-170°C.

### Exemple 402

### Acide 4-[[[[-2,3-dihydro-1-[[3'-(trifluorométhyl)[1,1'-biphényl]-4-yl]sulfonyl]-1H-indol-2-yl]carbonyl]amino]méthyl]benzèneacétique, méthyl ester

En opérant de façon analogue à l'exemple 84, au départ de l'acide racémique obtenu selon la préparation CXLVII, on obtient le produit attendu sous forme d'une poudre blanche (rendement = 90 %).
F = 74°C.

### Exemple 403

### Acide 4-[[[[-2,3-dihydro-1-[[3'-(trifluorométhyl)[1,1'-biphényl]-4-yl]sulfonyl]-1H-indol-2-yl]carbonyl]amino]méthyl]benzèneacétique

En opérant de façon analogue à l'exemple 22, au départ du composé obtenu selon l'exemple 402, on obtient le produit attendu sous forme d'une poudre blanche (rendement = 84 %).
F = 96°C.

### Exemple 404

### Acide 4-[2-[[[-2,3-dihydro-1-[[3'-(trifluorométhyl)[1,1'-biphényl]-4-yl]sulfonyl]-1H-indol-2-yl]carbonyl]amino]éthoxy]benzèneacétique, méthyl ester

En opérant de façon analogue à l'exemple 21, au départ de l'acide racémique obtenu selon la préparation CXLVII et du chlorhydrate de l'ester méthylique de l'acide 4-(2-aminoéthoxy)benzèneacétique, on obtient le produit attendu sous forme d'une poudre jaune clair (rendement = 62 %).
F = 85°C.

### Exemple 405

### Acide 4-[2-[[[-2,3-dihydro-1-[[3'-(trifluorométhyl)[1,1'-biphényl]-4-yl]sulfonyl]-1H-indol-2-yl]carbonyl]amino]éthoxy]benzèneacétique

En opérant de façon analogue à l'exemple 22, au départ du composé obtenu selon l'exemple 404, on obtient le produit attendu sous forme d'une poudre blanche (rendement = 75 %).
F = 92°C.

### Exemple 406

### Acide 4-[2-[[[-2,3-dihydro-4-méthoxy-1-[[3'-(trifluorométhyl)[1,1'-biphényl]-4-yl]sulfonyl]-1H-indol-2-yl]carbonyl]amino]éthoxy] benzèneacétique, méthyl ester

En opérant de façon analogue à l'exemple 170, au départ du chlorhydrate de l'ester méthylique de l'acide 4-(2-aminoéthoxy)benzèneacétique, on obtient le produit attendu sous forme d'une poudre jaune clair (rendement = 81 %).
F = 64°C.

### Exemple 407

### Acide 4-[2-[[[-2,3-dihydro-4-méthoxy-1-[[3'-(trifluorométhyl)[1,1'-biphényl]-4-yl]sulfonyl]-1H-indol-2-yl]carbonyl]amino]éthoxy] benzèneacétique

En opérant de façon analogue à l'exemple 22, au départ du composé obtenu selon l'exemple 406, on obtient le produit attendu sous forme d'une poudre blanche (rendement = 78 %).
F = 96°C

### PREPARATION CXLVIII

### Acide 2,3-dihydro-1-[[2'-chloro-5'-(trifluorométhyl)[1,1'-biphényl]-4-yl]sulfonyl]-1H-indole-2-carboxylique

En opérant de façon analogue à la préparation CXLVII, au départ de l'acide 2-chloro-5-(trifluorométhyl)phénylboronique, on obtient le produit attendu sous forme d'une poudre blanche (rendement = 95 % pour la seconde partie du procédé).
F = 90°C.

### Exemple 408

### Acide 4-[2-[[[-2,3-dihydro-1-[[2'-chloro-5'-(trifluorométhyl)[1,1'-biphényl]-4-yl]sulfonyl]-1H-indol-2-yl]carbonyl]amino]éthoxy] benzèneacétique, méthyl ester

En opérant de façon analogue à l'exemple 404, au départ de l'acide racémique obtenu selon la préparation CXLVIII, on obtient le produit attendu sous forme d'une poudre blanche (rendement = 76 %).
F = 60°C.

### Exemple 409

### Acide 4-[2-[[[-2,3-dihydro-1-[[2'-chloro-5'-(trifluorométhyl)[1,1'-biphényl]-4-yl]sulfonyl]-1H-indol-2-yl]carbonyl]amino]éthoxy] benzèneacétique

En opérant de façon analogue à l'exemple 22, au départ du composé obtenu selon l'exemple 408, on obtient le produit attendu sous forme d'une poudre blanche (rendement = 75 %).
F = 84°C.

### PREPARATION CIL

### 2,3-dihydro-N-[2-[4-[(hydroxyamino)iminométhyl]phénoxy]éthyl]-1-[[3'-(trifluorométhyl)[1,1'-biphenyl]-4-yl]sulfonyl]-(2S)-1H-indole-2-carboxamide

On prépare un mélange de 208 mg (0,35 mmol) du composé obtenu selon l'exemple 373 dans 4 ml d'éthanol et on ajoute 100 mg (1,54 mmol) d'hydroxylamine et 0,23 ml (1,6 mmol) de triéthylamine. Le mélange est agité pendant 7 heures à reflux du solvant. Ce milieu réactionnel est ensuite filtré et concentré sous pression réduite. Le produit brut est purifié par chromatographie sur gel de silice en éluant à l'aide d'un mélange dichlorométhane/éthanol (97/3 ; v/v). On obtient ainsi le composé attendu sous forme d'un solide gris (rendement = 84 %).

### Exemple 410

### N-[2-[4-(4,5-dihydro-5-oxo-1,2,4-oxadiazol-3-yl)phénoxy]éthyl]-2,3-dihydro-1-[[3'-(trifluorométhyl)[1,1'-biphényl]-4-yl]sulfonyl]-(25)-1H-indole-2-carboxamide

On prépare une solution de 182 mg (0,29 mmol) du composé obtenu selon la préparation CIL dans 8 ml de pyridine et on ajoute 0,07 ml (0,73 mmol) de chloroformate d'éthyle. Le mélange est agité pendant 24 heures à 100°C, puis refroidi et dilué dans 50 ml d'acétate d'éthyle, lavé par une solution d'acide chlorhydrique N, puis à l'eau, et séché sur sulfate de magnésium. Après concentration de cette phase organique sous pression réduite, le produit brut est purifié par chromatographie HPLC en phase inverse sur colonne de silice greffée C18, en éluant à l'aide d'un mélange acétonitrile/eau/0,1 % de TFA en gradient. On obtient le composé attendu sous forme d'un solide blanc (rendement = 30 %).
F =123-130°C.

### Exemple 411

### N-[(4-cyanophényl)méthyl]-2,3-dihydro-1-[[3'-(trifluorométhyl)[1,1'-biphényl]-4-yl]sulfonyl]-(2S)-1H-indole-2-carboxamide

En opérant de manière analogue à l'exemple 373, au départ du 4-(aminométhyl)benzonitrile, on obtient le composé attendu sous forme d'une poudre blanche (rendement = 39 %).
F = 80-89°C.

### PREPARATION CL

### 2,3-dihydro-N-[[4-[(hydroxyamino)iminométhyl]phényl]méthyl]-1-[[3'-(trifluorométhyl)[1,1'-biphenyl]-4-yl]sulfonyl]-(2S)-1H-indole-2-carboxamide

En opérant de manière analogue la préparation CIL, au départ du composé obtenu selon l'exemple 411, on obtient le composé attendu sous forme d'une poudre blanche (rendement = 88 %).
F = 110-115°C.

### Exemple 412

### N-[[4-(4,5-dihydro-5-oxo-1,2,4-oxadiazol-3-yl)phényl]méthyl]-2,3-dihydro-1-[[3'-(trifluorométhyl)[1,1'-biphényl]-4-yl]sulfonyl]-(2S)-1H-indole-2-carboxamide

En opérant de manière analogue à l'exemple 410, au départ du composé obtenu selon la préparation CL, on obtient le composé attendu sous forme d'une poudre blanche (rendement = 58 %).
F = 171-173°C.

### PREPARATION CLI

### 4-(aminométhyl)benzèneacétonitrile

### a) 4-(azidométhyl)benzeneacétonitrile

On prépare une solution de 3,10 g (14,8 mmol) de 4-(bromométhyl)-benzèneacétonitrile dans 15 ml d'éthanol et on ajoute 20 mg d'iodure de lithium, 25 mg de sulfate de tétrabutylammonium et 1,06 g (16,3 mmol) d'azoture de sodium. Le mélange est agité pendant 1 heure à reflux, puis concentré sous pression réduite et repris dans 50 ml de MTBE. Le précipité blanc formé est éliminé par filtration sur un lit de silice et le filtrat est concentré sous pression réduite. On obtient le composé attendu sous forme d'une huile orange (rendement = 96 %).

### b) 4-(aminométhyl)benzèneacétonitrile.

On mélange 2,45 g (14,2 mmol) du composé obtenu ci-dessus dans 33 ml de THF et on ajoute 5,58 g (21,3 mmol) de triphénylphosphine. Le milieu réactionnel est agité pendant 15 mn à température ambiante, puis on ajoute 1,3 ml d'eau et on laisse sous agitation pendant 12 heures. Le mélange est concentré à température ambiante sous pression réduite et repris dans 150 ml d'acide chlorhydrique N. Cette phase aqueuse est lavée 2 fois par 50 ml de DCM, puis amenée à pH basique par addition d'une solution diluée de soude et extraite par 100 ml de DCM. La phase organique est lavée une fois à l'eau, séchée sur sulfate de magnésium et concentrée sous pression réduite. On obtient le composé attendu sous forme d'une huile jaune (rendement = 80 %).
RMN ¹H (DMSO, 300 MHz) δ : 7,35 (d, 2H) ; 7.27 (d, 2H) ; 3,98 (s, 2H) ; 3,47 (s, 2H) ; 2,70 (s large, 2H).

### Exemple 413

### N-[[4-(cyanométhyl)phényl]méthyl]-2,3-dihydro-1 -[[3'-(trifluorométhyl)[1,1'-biphényl]-4-yl]sulfonyl]-(2S)-1H-indole-2-carboxamide

En opérant de manière analogue à l'exemple 198, au départ du composé obtenu selon la préparation CLI, on obtient le composé attendu sous forme d'une poudre blanche (rendement = 76 %).
F = 75-80°C.

### Exemple 414

### 2,3-dihydro-N-[2-(3-pyridazinyloxy)éthyl]-1-[[3'-(trifluorométhyl)[1,1'-biphényl]-4-yl]sulfonyl]-(2S)-1H-indole-2-carboxamide

En opérant de manière analogue à l'exemple 198, au départ de 2-(3-pyridazinyloxy)éthanamine, on obtient le composé attendu sous forme d'une poudre blanche (rendement = 37 %).
F = 65-71 °C.

### Exemple 415

### Acide 2,6-difluoro-4-[2-[[[(2S)-1-[(4'-fluoro-2'-méthyl[1,1'-biphényl]-4-yl)sulfonyl]-2,3-dihydro-1H-indol-2-yl]carbonyl]amino]éthoxy] benzoïque, méthyl ester

En opérant de manière analogue à l'exemple 21, au départ des composés obtenus selon les préparations CIV et LXXXI, on obtient le composé attendu sous forme d'une poudre blanche (rendement = 42 %).
F = 65-72°C.

### Exemple 416

### Acide 2,6-difluoro-4-[2-[[[(2S)-1-[(4'-fluoro-2'-méthyl[1,1'-biphényl]-4-yl)sulfonyl]-2,3-dihydro-1H-indol-2-yl]carbonyl]amino]éthoxy] benzoïque

En opérant de manière analogue à l'exemple 22, au départ de l'ester obtenu selon l'exemple 415, on obtient le composé attendu sous forme d'une poudre blanche (rendement = 58 %).
F = 97-111 °C.

### PREPARATION CLII

### Acide 2-fluoro-4-[2-[[(phénylméthoxy)carbonyl]amino]éthoxy] benzèneacétique, méthyl ester

En opérant de façon analogue à la préparation CXLIV, au départ de l'ester méthylique de l'acide 2-fluoro-4-hydroxybenzèneacétique, on obtient le composé attendu sous forme d'une huile incolore (rendement = 51 %).
RMN ¹H (DMSO, 300 MHz) δ : 7,50 (t, NH) ; 7,45-7,30 (m, 5H) ; 7,23 (t, 1H) ; 6,85-6,70 (m, 2H); 5,03 (s, 2H); 3,99 (t, 2H); 3,64 (s, 2H) ; 3,61 (s, 3H) ; 3,45-3,30 (m, 2H).

### PREPARATION CLIII

### Acide 4-(2-aminoéthoxy)-2-fluorobenzèneacétique, méthyl ester (chlorhydrate)

En opérant de façon analogue à l'exemple 61, en présence d'acide chlorhydrique N dans le milieu d'hydrogénation, au départ du composé obtenu selon la préparation CLII, on obtient le composé attendu sous forme d'un solide jaune clair (rendement = 98 %).
F = 199°C.

### Exemple 417

### Acide 2-fluoro-4-[2-[[[(2S)-1-[[2'-chloro-5'-(trifluorométhyl)[1,1'-biphényl]-4-yl]sulfonyl]-2,3-dihydro-1H-indol-2-yl]carbonyl]amino] éthoxy]benzèneacétique, méthyl ester

En opérant de manière analogue à l'exemple 21, au départ des composés obtenus selon les préparations CXII et CLIII, on obtient le composé attendu sous forme d'une huile incolore (rendement = 22 %).
RMN ¹H (CDCl₃, 300 MHz) δ : 7,85-7,35 (m, 6H); 7,35-20 (m, 3H); 7,20-7,05 (m, 3H); 6,70-6,50 (m, 2H); 4,66 (dd, 1 H); 4,10-3,95 (m, 2H); 3,80-3,55 (m, 7H); 3,32 (dd, 1 H); 2,83 (dd, 1 H).

### Exemple 418

### Acide 2-fluoro-4-[2-[[[(2S)-1-[[2'-chloro-5'-(trifluorométhyl)[1,1'-biphényl]-4-yl]sulfonyl]-2,3-dihydro-1H-indol-2-yl]carbonyl]amino] éthoxy]benzèneacétique,

En opérant de manière analogue à l'exemple 22, au départ de l'ester obtenu selon l'exemple 417, on obtient le composé attendu sous forme d'une poudre blanche (rendement = 80 %).
F = 105°C.

En opérant de façon analogue à l'exemple 21, mais en remplaçant HOAT par HOBT, pour le couplage, et à l'exemple 22 pour obtenir les exemples sous forme acide, au départ d'acides et d'amines précédemment décrites, on obtient les composés suivants :

### Exemple 419

### Acide 4-[2-[[[(2S)-1-[(4'-fluoro-2'-méthoxy[1,1'-biphényl]-4-yl)sulfonyl]-2,3-dihydro-1H-indol-2-yl]carbonyl]amino]éthoxy]-3-méthylbenzoïque, méthyl ester

Poudre blanche (rendement = 60 %).
F = 82-84°C.

### Exemple 420

### Acide 4-[2-[[[(2S)-1-[(4'-fluoro-2'-méthoxy[1,1'-biphényl]-4-yl)sulfonyl]-2,3-dihydro-1H-indol-2-yl]carbonyl]amino]éthoxy]-3-méthylbenzoïque

Poudre blanche (rendement = 50 %).
F= 114°C.

### Exemple 421

### Acide 4-[2-[[[(2S)-1-[(4'-fluoro-2'-méthoxy[1,1'-biphényl]-4-yl)sulfonyl]-2,3-dihydro-1H-indol-2-yl]carbonyl]amino]éthoxy]-3-(trifluorométhyl)benzoïque, méthyl ester

Poudre blanche (rendement = 47 %).
F=81°C.

### Exemple 422

### Acide 4-[2-[[[(2S)-1-[(4'-fluoro-2'-méthoxy[1,1'-biphényl]-4-yl)sulfonyl]-2,3-dihydro-1H-indol-2-yl]carbonyl]amino]éthoxy]-3-(trifluorométhyl)benzdique

Poudre blanche (rendement = 67 %).
F = 123°C.

### Exemple 423

### Acide 4-[2-[[[(2S)-1-[(4'-fluoro-2'-méthoxy[1,1'-biphényi]-4-yl)sulfonyl]-2,3-dihydro-1H-indol-2-yl]carbonyl]amino]éthoxy]-3,5-diméthylbenzoïque, méthyl ester

Poudre beige (rendement = 70 %).
F = 90-93°C.

### Exemple 424

### Acide 4-[2-[[[(2S)-1-[(4'-fluoro-2'-méthoxy[1,1'-biphényl]-4-yl)sulfonyl]-2,3-dihydro-1 H-indol-2-yl]carbonyl]amino]éthoxy]-3,5-diméthylbenzoïque

Poudre blanche (rendement = 63 %).
F = 129°C.

### Exemple 425

### Acide 4-[2-[[[(2S)-1-[(4'-fluoro-2'-méthyl[1,1'-biphényl]-4-yl)sulfonyl]-2,3-dihydro-1H-indol-2-yl]carbonyl]amino]éthoxy]-3-(trifluorométhyl)-benzoïque, méthyl ester

Poudre blanche (rendement = 53 %).
F = 65°C.

### Exemple 426

### Acide 4-[2-[[[(2S)-1-[(4'-fluoro-2'-méthyl[1,1'-biphényl]-4-yl)sulfonyl]-2,3-dihydro-1H-indol-2-yl]carbonyl]amino]éthoxy]-3-(trifluorométhyl)-benzoïque

Poudre blanche (rendement = 88 %).
F = 113-118°C.

### Exemple 427

### Acide 4-[2-[[[(2S)-1-[(2',4'-difluoro[1,1'-biphényl]-4-yl)sulfonyl]-2,3dihydro-1H-indol-2-yl]carbonyl]amino]éthoxy]-3,5-diméthylbenzoïque, méthyl ester

Poudre blanche (rendement = 68 %).
F = 85-89°C.

### Exemple 428

### Acide 4-[2-[[[(2S)-1-[(2',4'-difluoro[1,1'-biphényl]-4-yl)sulfonyl]-2,3-dihydro-1H-indol-2-yl]carbonyl]amino]éthoxy]-3,5-diméthylbenzoïque

Poudre blanche (rendement = 67 %).
F = 122°C.

### Exemple 429

### Acide 3-fluoro-4-[2-[[[(2S)-1-[(4'-fluoro-2'-méthoxy[1,1'-biphényl]-4-yl)sulfonyl]-2,3-dihydro-1H-indol-2-yl]carbonyl]amino]éthoxy] benzoïque, méthyl ester

Poudre blanche (rendement = 65 %).
F = 87°C.

### Exemple 430

### Acide 3-fluoro-4-[2-[[[(2S)-1-[(4'-fluoro-2'-méthoxy[1,1'-biphényl]-4-yl)sulfonyl]-2,3-dihydro-1H-indol-2-yl]carbonyl]amino]éthoxy] benzoïque

Poudre blanche (rendement = 73 %).
F = 106-108°C.

### Exemple 431

### Acide 4-[2-[[[(2S)-1-[(2'-chloro-4'-fluoro[1,1'-biphényl]-4-yl)sulfonyl]-2,3-dihydro-1H-indol-2-yl]carbonyl]amino]éthoxy]-3-fluorobenzoïque, méthyl ester

Poudre blanche (rendement = 69 %).
F = 84°C.

### Exemple 432

### Acide 4-[2-[[[(2S)-1-[(2'-chloro-4'-fluoro[1,1'-biphényl]-4-yl)sulfonyl]-2,3-dihydro-1 H-indol-2-yl]carbonyl]amino]éthoxy]-3-fluorobenzdoïque

Poudre blanche (rendement = 64 %).
F= 118°C.

### Exemple 433

### Acide 4-[2-[[[(2S)-1-[(2',4'-difluoro[1,1'-biphényl]-4-yl)sulfonyl]-2,3-dihydro-1H-indol-2-yl]carbonyl]amino]éthoxy]-3-fluorobenzoïque, méthyl ester

Poudre blanche (rendement = 51 %).
F = 58-61 °C.

### Exemple 434

### Acide 4-[2-[[[(2S)-1-[(2',4'-difluoro[1,1'-biphényl]-4-yl)sulfonyl]-2,3-dihydro-1H-indol-2-yl]carbonyl]amino]éthoxy]-3-fluorobenzoïque

Poudre blanche (rendement = 76 %).
F = 105-110°C (décomposition).

### Exemple 435

### Acide 3-fluoro-4-[2-[[[(2S)-1-[(4'-fluoro-2'-méthyl[1,1'-biphényl]-4-yl)sulfonyl]-2,3-dihydro-1H-indol-2-yl]carbonyl]amino]éthoxyl benzoïque, méthyl ester

Poudre blanche (rendement = 67 %).
F = 74-79°C.

### Exemple 436

### Acide 3-fluoro-4-[2-[[[(2S)-1-[(4'-fluoro-2'-méthyl[1,1'-biphényl]-4-yl)sulfonyl]-2,3-dihydro-1H-indol-2-yl]carbonyl]amino]éthoxy] benzoïque

Poudre blanche (rendement = 76 %).
F = 118°C.

### Exemple 437

### Acide 4-[2-[[[(2S)-1-[(2',4'-difluoro[1,1'-biphényl]-4-yl)sulfonyl]-2,3-dihydro-1H-indol-2-yl]carbonyl]amino]éthoxy]-3-(trifluorométhyl) benzoïque, méthyl ester

Poudre blanche (rendement = 51 %).
F = 97°C.

### Exemple 438

### Acide 4-[2-[[[(2S)-1-[(2',4'-difluoro[1,1'-biphényl]-4-yl)sulfonyl]-2,3-dihydro-1H-indol-2-yl]carbonyl]amino]éthoxy]-3-(trifluorométhyl) benzoïque

Poudre blanche (rendement = 72 %).
F = 202-205°C.

### Exemple 439

### Acide 4-[2-[[[(2S)-1-[(2'-chloro-4'-fluoro[1,1'-biphényl]-4-yl)sulfonyl]-2,3-dihydro-1H-indol-2-yl]carbonyl]amino]éthoxy]-3-(trifluorométhyl) benzoïque, méthyl ester

Poudre blanche (rendement = 56 %).
F = 65-70°C.

### Exemple 440

### Acide 4-[2-[[[(2S)-1-[(2'-chloro-4'-fluoro[1,1'-biphényl]-4-yl)sulfonyl]-2,3-dihydro-1 H-indol-2-yl]carbonyl]amino]éthoxy]-3-(trifluorométhyl) benzoïque

Poudre blanche (rendement = 69 %).
F = 218-222°C.

### Exemple 441

### Acide 4-[2-[[[(2S)-1-[(2'-chloro-4'-fluoro[1,1'-biphényl]-4-yl)sulfonyl]-2,3-dihydro-1H-indol-2-yl]carbonyl]amino]éthoxy]-3,5-diméthyl benzoïque, méthyl ester

Poudre blanche (rendement = 50 %).
F = 87°C.

### Exemple 442

### Acide 4-[2-[[[(2S)-1-[(2'-chloro-4'-fluoro[1,1'-biphényl]-4-yl)sulfonyl]-2,3-dihydro-1H-indol-2-yl]carbonyl]amino]éthoxy]-3,5-diméthyl benzoïque

Poudre blanche (rendement = 46 %).
F = 124°C.

### Exemple 443

### Acide 4-[2-[[[(2S)-1-[(2'-chloro-4'-fluoro[1,1'-biphényl]-4-yl)sulfonyl]-2,3-dihydro-1H-indol-2-yl]carbonyl]amino]éthoxy]-3-méthyl benzoïque, méthyl ester

Poudre blanche (rendement = 51 %).
F = 88°C.

### Exemple 444

### Acide 4-[2-[[[(2S)-1-[(2'-chloro-4'-fluoro[1,1'-biphényl]-4-yl)sulfonyl]-2,3-dihydro-1H-indol-2-yl]carbonyl]amino]éthoxy]-3-méthyl benzoïque

Poudre blanche (rendement = 47 %).
F = 116°C.

### Exemple 445

### Acide 3,5-diméthyl-4-[2-[[[(2S)-1-[(4'-fluoro-2'-méthyl[1,1'-biphényl]-4-yl)sulfonyl]-2,3-dihydro-1H-indol-2-yl]carbonyl]amino]éthoxy] benzoïque, méthyl ester

Poudre blanche (rendement = 67 %).
F = 74°C.

### Exemple 446

### Acide 3,5-diméthyl-4-[2-[[[(2S)-1-[(4'-fluoro-2'-méthy)[1,1'-biphényl]-4-yl)sulfonyl]-2,3-dihydro-1H-indol-2-yl]carbonyl]amino]éthoxy] benzoïque

Poudre blanche (rendement = 49 %).
F = 119°C.

### Exemple 447

### Acide 4-[2-[[[(2S)-1-[(4'-fluoro-2'-méthyl[1,1'-biphényl]-4-yl)sulfonyl]-2,3-dihydro-1H-indol-2-yl]carbonyl]amino]éthoxy]-3-méthyl benzoïque, méthyl ester

Huile incolore (rendement = 34%).

### Exemple 448

### Acide 4-[2-[[[(2S)-1-[(4'-fluoro-2'-méthyl[1,1'-biphényl]-4-yl)sulfonyl]-2,3-dihydro-1H-indol-2-yl]carbonyl]amino]éthoxy]-3-méthyl benzoïque

Cet acide est obtenu par saponification par la soude de l'ester ci-dessus.
Poudre blanche (rendement = 40 %).
F = 108-112°C.

### Exemple 449

### 1-[[2'-chloro-4'-fluoro[1,1'-biphényl]-4-yl]-sulfonyl]-2,3-dihydro-N-[3-(4-méthyl-1-pipérazinyl)propyl]-(2S)-1H-indole-2-carboxamide

Solide blanc (rendement = 45 %).
F = 67°C.

### Exemple 450

### 1-[[2'chloro-4-fluoro[1,1'-biphényl]-4-yl]-sulfonyl]-2,3-dihydro-N-[3-(1-pyrrolidinyl)propyl]-(2S)-1H-indole-2-carboxamide

Solide blanc amorphe (rendement = 32 %).
F = 70°C.

### Exemple 451

### 1-[[2'-chloro-4'-fluoro[1,1'-biphényl]-4-yl]-sulfonyl]-2,3-dihydro-N-[4-(1-pyrrolidinyl)butyl]-(2S)-1H-indole-2-carboxamide

Solide jaune (rendement = 59 %).
F = 60°C.

### Exemple 452

### 1-[[2'-chloro-4'-fluoro[1,1'-biphényl]-4-yl]-sulfonyl]-2,3-dihydro-N-[2-(1-pipéridinyl)éthyl]-(2S)-1H-indole-2-carboxamide

Solide blanc amorphe (rendement = 57 %).
F = 84°C.

### Exemple 453

### 1-[[2'-chloro-4'-fluoro[1,1'-biphényl]-4-yl]-sulfonyl]-2,3-dihydro-N-[4-(diméthylamino)butyl]-(2S)-1H-indole-2-carboxamide

Solide blanc amorphe (rendement = 52 %).
F = 59-63°C.

### Exemple 454

### Acide 4-[2-[[[(2S)-2,3-dihydro-1-[(4'-fluoro-2'-méthoxy[1,1'-biphényl]-4-yl)sulfonyl]-1H-indol-2-yl]carbonyl]amino]éthoxy]-3-méthyl benzoïque, méthyl ester

Poudre blanche (rendement = 60 %).
F = 82-84°C.

### Exemple 455

### Acide 4-[2-[[[(2S)-1-[(2',4-difluoro[1,1'-biphényl]-4-yl)sulfonyl]-2,3-dihydro-1H-indol-2-yl]carbonyl]amino]éthoxy]-3-méthylbenzoïque, 1,1-diméthyléthyl ester

Poudre blanche (rendement = 47 %).
F = 68-72°C.

### Exemple 456

### Acide 4-[2-[[[(2S)-1-[(2',4'-difluoro[1,1'-biphényl]-4-yl)sulfonyl]-2,3-dihydro-1H-indol-2-yl]carbonyl]aminoléthoxy]-3-méthylbenzoïque

Cet acide est obtenu selon le procédé décrit à l'exemple 298, au départ de l'ester ci-dessus.
Poudre blanche (rendement = 80 %).
F = 106-111°C.

### Exemple 457

### 1-[[2'-chloro-4'-fluoro[1,1'-biphényl]-4-yl]-sulfonyl]-2,3-dihydro-N-[(2-amino-3-pyridinyl)méthyl]-(2S)-1H-indole-2-carboxamide

Solide blanc (rendement = 48 %).
F = 88-100°C.

### Exemple 458

### 1-[[2'-chloro-4'-fluoro[1,1'-biphényl]-4-yl]-sulfonyl]-2,3-dihydro-N-[(2-amino-N-oxyde-3-pyridinyl)méthyl]-(2S)-1H-indole-2-carboxamide

On prépare une solution de 80 mg (0,14 mmol) du composé obtenu selon l'exemple 457 dans 2 ml de chloroforme et on ajoute 51 mg (0,28 mmol) d'acide 3-chloroperbenzoïque. Le mélange est agité à température ambiante pendant 16 heures, puis dilué par une solution de bicarbonate de sodium et extrait par l'acétate d'éthyle. La phase organique est lavée à l'eau, séchée sur sulfate de magnésium et concentrée sous pression réduite. Le produit brut est purifié par chromatographie sur gel de silice en éluant à l'aide d'un mélange DCM/méthanol (95/5 ; v/v). On obtient le produit attendu sous forme d'un solide blanc (rendement = 43 %).
F = 135-147°C.

### PREPARATION CLIV

### Acide 6-[2-[(1,1-diméthyléthoxy)carbonyl]amino]éthoxy]-3-pyridine carboxylique, méthyl ester

En opérant de façon analogue à la préparation LVII, en remplaçant DIAD par DBAD, au départ de l'ester méthylique de l'acide 6-hydroxy-3-pyridine carboxylique et de (2-hydroxyéthyl)carbamate de *t*-butyle, on obtient le composé attendu sous forme d'une huile qui cristallise (rendement = 45%).

### PREPARATION CLV

### Acide 6-(2-aminoéthoxy)-3-pyridinecarboxylique, méthyl ester (trifluoroacétate)

En opérant de façon analogue à la préparation II, au départ du composé obtenu selon la préparation CLIV, on obtient le composé attendu sous forme d'une huile jaune (rendement = 99 %).

### Exemple 459

### Acide 6-[2-[[[(2S)-1-[(2'chloro-4'-fluoro[1,1'-biphényl]-4-yl)sulfonyl]-2,3-dihydro-1H-indol-2-yl]carbonyl]amino]éthoxy]-3-pyridinecarboxylique, méthyl ester

En opérant de façon analogue à l'exemple 296, au départ des composés obtenus selon les préparations CVII et CLV, on obtient le composé attendu sous forme d'une poudre blanche (rendement = 89 %).
F = 78-85°C.

### Exemple 460

### Acide 6-[2-[[[(2S)-1-[(2'-chloro-4'-fluoro[1,1'-biphényl]-4-yl)sulfonyl]. 2,3-dihydro-1H-indol-2-yl]carbonyl]amino]éthoxy)-3-pyridinecarboxylique

En opérant de façon analogue à l'exemple 22, au départ de l'ester obtenu selon l'exemple 459, on obtient le composé attendu sous forme d'une poudre blanche (rendement = 81 %).
F = 88-98°C.

### PREPARATION CLVI

### N-[2-[[[[(2S)-2,3-dihydro-1-[(4-iodophényl)-sulfonyl]-1H-indol-2-yl]carbonyl]amino]méthyl]phényl]-β-alanine, méthyl ester

En opérant de façon analogue à l'exemple 66, au départ des composés obtenus selon les préparations IVa et XXIII, on obtient le produit attendu sous forme d'un solide blanc (rendement = 93 %).
F= 67-75°C.

### PREPARATION CLVII

### N-[2-[[[[(2S)-2,3-dihydro-1-[(4-iodophényl)-sulfonyl]-1H-indol-2-yl)carbonyl]amino]méthyl]phényl]-N-méthyl-β-alanine, méthyl ester

On prépare une solution de 300 mg (0,48 mmol) du composé obtenu selon la préparation CLVI dans 4 ml d'acétonitrile et on ajoute 0,18 ml (2,4 mmol) de paraformaldéhyde, puis, après 15 mn sous agitation à température ambiante, 60 mg (0,96 mmol) de borohydrure de sodium. Le mélange est agité à température ambiante pendant 15 mn puis on ajoute 0,15 ml d'acide acétique.Le milieu réactionnel est agité pendant 5 heures à température ambiante et concentré sous pression réduite. Le résidu est dilué dans du DCM et lavé par une solution de soude N puis à l'eau. On obtient le produit sous forme d'une huile incolore (rendement = 98 %).

### Exemple 461

### N-[2-[[[[(2S)-1-[(2'-chloro-4'-fluoro[1,1'-biphényl]-4-yl)-sulfonyl]-2,3-dihydro-1H-indol-2-yl]carbonyl]amino]méthyl]phényl]-N-méthyl-β-alanine, méthyl ester

En opérant de façon analogue à l'exemple 1, au départ du composé obtenu selon la préparation CLVII et d'acide 2-chloro-4-fluorophényl-boroniqué, on obtient le produit attendu sous forme d'un solide blanc (rendement = 96 %).
F= 88°C.

### Exemple 462

### N-[2-[[[[(2S)-1-[(2'-chloro-4'-fluoro[1,1'-biphényl]-4-yl)-sulfonyl]-2,3-dihydro-1H-indol-2-yl]carbonyl]amino]méthyl]phényl]-N-méthyl-β-alanine

En opérant de façon analogue à l'exemple 22, au départ du composé obtenu selon l'exemple 461 on obtient le produit attendu sous forme d'un solide blanc (rendement = 96 %).
F= 75-83°C.

### Exemple 463

### 1-[[2'-chloro-4'-fluoro[1,1'-biphényl]-4-yl]-sulfonyl]-2,3-dihydro-N-[(3-amino-4-pyridinyl)méthyl]-(2S)-1H-indole-2-carboxamide

En opérant de façon analogue à l'exemple 21, au départ du composé obtenu selon la préparation CVII et de (3-amino-4-pyridinyl)méthanamine dichlorhydrate, on obtient le composé attendu sous forme d'une pâte blanche (rendement = 80 %).
RMN ¹H (DMSO, 250 MHz) δ : 8,75 (t, NH) ; 7,94 (s, 1 H) ; 7,89 (d, 2H) ; 7,72 (d, 1H) ; 7,65-7,55 (m, 3H) : 7,55-7,40 (m, 2H) ; 7,40-7,10 (m, 3H) ; 7,05 (t, 1H) ; 6,96 (d, 1 H) ; 5,22 (s, NH₂) ; 4,93 (dd, 1 H) ; 4,17 (t, 2H) 3,19 (dd, 1 H) ; 2,99 (dd, 1 H).

### Exemple 464

### 1-[[2'chloro-4'fluoro[1,1'-biphényl]-4-yl]-sulfonyl]-2,3-dihydro-N-[(3-amino-N-oxyde-4-pyridinyl)méthyl]-(2S)-1H-indole-2-carboxamide

En opérant de façon analogue à l'exemple 458, au départ du composé obtenu selon l'exemple 463, on obtient le produit attendu sous forme d'une poudre jaune (rendement = 58 %).
F= 142-148°C.

### PREPARATION CLVIII

### Acide 1-[(4-iodophényl)sulfonyl]-2,3-dihydro-5-méthoxy-1H-indole-2-carboxylique, méthyl ester

En opérant de façon analogue à la préparation LXV, au départ de l'ester méthylique de l'acide 2,3-dihydro-5-méthoxy-1*H*-indole-2-carboxylique, on obtient le produit attendu sous forme d'un solide beige (rendement = 81 %).
F= 154°C.

### PREPARATION CLIX

### Acide 2,3-dihydro-1-[[2',4'-difluoro-[1,1'-biphényl]-4-yl]sulfonyl]-5-méthoxy-1H-indole-2-carboxylique, méthyl ester

En opérant de façon analogue à l'exemple 1, au départ de l'ester obtenu selon la préparation CLVIII et d'acide 2,4-difluorophénylboronique, on obtient le produit attendu sous forme d'une mousse (rendement = 77 %).
RMN ¹H (DMSO, 300 MHz) δ : 7,87 (d, 2H) ; 7,71 (d, 2H) ; 7,70-7,55 (m, 1 H) ; 7,45-7,30 (m, 2H) ; 7,22 (td, 1 H) ; 6,85-6,70 (m, 2H) ; 5,08 (dd, 1H) ; 3,72 (s, 3H) ; 3,68 (s, 3H) ; 3,23 (dd, 1 H) ; 3,04 (dd, 1 H).

### PREPARATION CLX

### Acide 2,3-dihydro-1-[[2',4'-difluoro-[1,1'-biphényl]-4-yl]sulfonyl]-5-méthoxy-1H-indole-2-carboxylique

En opérant de façon analogue à l'exemple 5, au départ de l'ester obtenu selon la préparation CLIX, on obtient le produit attendu sous forme d'une mousse jaune (rendement = 83 %).
RMN ¹H (DMSO, 250 MHz) δ : 7,87 (d, 2H) ; 7,70 (d, 2H) ; 7,70-7,55 (m, 1 H) ; 7,45-7,30 (m, 2H) ; 7,22 (td, 1 H) ; 6,85-6,70 (m, 2H) ; 4,92 (dd, 1H) ; 3,67 (s, 3H) ; 3,17 (dd, 1 H) ; 2,98 (dd, 1 H).

### PREPARATION CLXI

### Chlorure de l'acide 2,3-dihydro-1-[[2',4'-difluoro-[1,1'-biphényl]-4-yl]-sulfonyl]-5-méthoxy-1H-indole-2-carboxylique

En opérant de façon analogue à la préparation VI, au départ de l'acide obtenu selon la préparation CLX, on obtient le produit attendu sous forme d'une mousse jaune (utilisé sans autre purification pour le couplage suivant).

### Exemple 465

### Acide 4-[[[[1-[(2',4'-difluoro[1,1'-biphényl]-4-yl)sulfonyl]-2,3-dihydro-5-méthoxy-1H-indol-2-yl]carbonyl]amino]méthyl]benzdique

En opérant de façon analogue à l'exemple 17, au départ du composé obtenu selon la préparation CLXI et d'acide 4-(aminométhyl)benzoïque, on obtient le produit attendu sous forme d'un solide blanc (rendement = 36 %).
F = 143°C.
Ce composé racémique a été dédoublé par chromatographie HPLC préparative à l'aide d'une colonne remplie d'une phase chirale Chiralpack AD-H 5 µm (Daicel) 250*20mm, L'éluant utilisé est un mélange constitué de 20 % d'hexane et 80 % de 2-propanol avec 0,05 % d'acide formique, et un débit de 18,9 ml/mn. La détection est effectuée par UV à 205 nm et la température de travail est fixée à 40°C. Les composés séparés sont analysés par chromatographie sur colonne chirale Chiralpack AD-H 5 µm, 250*4,6mm. L'éluant est un mélange constitué de 50 % d'hexane et 50 % de 2-propanol avec 0,05 % d'acide formique, et un débit de 1 ml/mn. La détection est effectuée par UV à 205 nm et la température de travail est de 40°C. Dans ces conditions, les temps de rétention sont respectivement de 10,70 mn et 12,42 mn. L'excès énantiomérique est supérieur à 95,5 % pour chacun des énantiomères.

### Exemple 466

### Acide 4-[2-[[[-1-[(2',4'-difluoro[1,1'-biphényl]-4-yl)sulfonyl]-2,3-dihydro-5-méthoxy-1H-indol-2-yl]carbonyl]amino]éthoxy]benzoïque, 1,1-diméthyléthyl ester

En opérant de façon analogue à l'exemple 294, au départ de l'acide obtenu selon la préparation CLX, on obtient le produit attendu sous forme d'une mousse blanche (rendement = 73 %).
RMN ¹H (DMSO, 300 MHz) δ : 8,38 (t, NH) ; 7,90-7,75 (m, 4H) ; 7,69 (dd, 2H) ; 7,62 (td, 1H) ; 7,42 (d, 2H) ; 7,20 (td, 1H) ; 7,01 (d, 2H) ; 6,81 (dd, 1H) ; 6,72 (d, 1H) ; 4,83 (dd, 1H) ; 4,09 (t, 2H) ; 3,67 (s, 3H) ; 3,60-3,35 (m, 2H) ; 3,05-2,80 (m, 2H) ; 1,52 (s, 9H).

### Exemple 467

### Acide 4-[2-[[[-1-[(2',4'-difluoro[1,1'-biphényl]-4-yl)sulfonyl]-2,3-dihydro-5-méthoxy-1H-indol-2-yl]carbonyl]amino]éthoxy]benzoïque

En opérant de façon analogue à l'exemple 298, au départ de l'ester obtenu selon l'exemple 466, on obtient le produit attendu sous forme d'un solide beige (rendement = 76 %).
F=91°C.

### PREPARATION CLXII

### Acide 2,3-dihydro-1-[[4'-fluoro-[1,1'-biphényl]-4-yl]sulfonyl]-4-méthoxy-1H-indole-2-carboxylique, méthyl ester

En opérant de façon analogue à la préparation LXVI, au départ de l'ester obtenu selon la préparation LXV et d'acide 4-fluorophénylboronique, on obtient le composé attendu sous forme d'une mousse orange (rendement 64%).
RMN ¹H (DMSO, 250 MHz) δ : 7,91 (d, 2H) ; 7,84 (d, 2H) ; 7,80-7,65 (m, 2H) ; 7,32 (t, 2H) ; 7,22 (t, 1H) ; 7,04 (d, 1H) ; 6,68 (d, 1H) ; 5,11 (dd, 1H) ; 3,74 (s, 3H) ; 3,72 (s, 3H) ; 3,26 (dd, 1 H) ; 2,94 (dd, 1 H).

### PREPARATION CLXIII

### Acide 2,3-dihydro-1-[[4'-fluoro-[1,1'-biphényl]-4-yl]sulfonyl]-4-méthoxy-1H-indole-2-carboxylique

En opérant de façon analogue à l'exemple 5, au départ de l'ester obtenu selon la préparation CLXII on obtient le produit attendu sous forme d'une mousse blanche (rendement = 94 %).
RMN ¹H (DMSO, 250 MHz) δ : 7,92 (d, 2H) ; 7,89 (d, 2H) ; 7,80-7,65 (m, 2H) ; 7,32 (t, 2H) ; 7,20 (t, 1 H) ; 7,03 (d, 1 H) ; 6,67 (d, 1 H) ; 4,97 (dd, 1H) : 3,72 (s, 3H) ; 3,24 (dd, 1 H) ; 2,89 (dd, 1 H).

### Exemple 468

### Acide 4-[2-[[[-1-[(4'-fluoro[1,1'-biphényl]-4-yl)sulfonyl]-2,3-dihydro-4-méthoxy-1H-indol-2-yl]carbonyl]amino]éthoxy]benzoïque, 1,1-diméthyléthyl ester

En opérant de façon analogue à l'exemple 294, au départ de l'acide obtenu selon la préparation CLXIII, on obtient le produit attendu sous forme d'une mousse blanche (rendement = 70 %).
RMN ¹H (DMSO, 250 MHz) δ : 8,42 (t, NH) ; 7,90-7,70 (m, 8H) ; 7,40-7,15 (m, 3H) ; 7,10 (d, 1 H) ; 7,02 (d, 2H) ; 6,68 (d, 1 H) ; 4,88 (dd, 1 H) ; 4,11 (t, 2H) ; 3,70 (s, 3H) ; 3,65-3,40 (m, 2H) ; 3,02 (dd, 1 H) ; 2,78 (dd, 1 H) ; 1,53 (s, 9H).

### Exemple 469

### Acide 4-[2-[[[-1-[(4'-fluoro[1,1'-biphényl]-4-yl)sulfonyl]-2,3-dihydro-4-méthoxy-1H-indol-2-yl]carbonyl]amino]éthoxy]benzoïque

En opérant de façon analogue à l'exemple 298, au départ de l'ester obtenu selon l'exemple 468, on obtient le produit attendu sous forme d'un solide blanc (rendement = 80 %).
F = 113°C.

### PREPARATION CLXIV

### Acide 2,3-dihydro-1-[[2',4'-difluoro-[1,1'-biphényl]-4-yl]sulfonyl]-4-méthoxy-1H-indole-2-carboxylique, méthyl ester

En opérant de façon analogue à la préparation CLXII, au départ d'acide 2,4-difluorophénylboronique, on obtient le produit attendu sous forme d'un solide marron (rendement = 98 %).
F = 162°C.

### PREPARATION CLXV

### Acide 2,3-dihydro-1-[[2',4'-difluoro-[1,1'-biphényl]-4-yl]sulfonyl]-4-méthoxy-1H-indole-2-carboxylique

En opérant de façon analogue à l'exemple 5, au départ de l'ester obtenu selon la préparation CLXIV on obtient le produit attendu sous forme d'une huile (rendement = 95 %).
RMN ¹H (DMSO, 300 MHz) δ : 7,94 (d, 2H); 7,71 (d, 2H); 7,75-7,55 (m, 1 H) ; 7,50-7,35 (m, 1 H) ; 7,30-7,15 (m, 2H) ; 7,02 (d, 1 H) ; 6,67 (d, 1H) ; 4,97 (dd, 1 H) ; 3,73 (s, 3H) ; 3,25 (dd, 1 H) ; 2,90 (dd, 1 H).

### Exemple 470

### Acide 4-[2-[[[-1-[(2',4'-difluoro[1,1'-biphényl]-4-yl)sulfonyl]-2,3-dihydro-4-méthoxy-1H-indol-2-yl]carbonyl]amino]éthoxy]benzoïque, 1,1-diméthyléthyl ester

En opérant de façon analogue à l'exemple 294, au départ de l'acide obtenu selon la préparation CLXV, on obtient le produit attendu sous forme d'un solide blanc (rendement = 66 %).
F = 135°C.

### Exemple 471

### Acide 4-[2-[[[-1-[(2',4'-difluoro[1,1'-biphényl]-4-yl)sulfonyl]-2,3-dihydro-4-méthoxy-1H-indol-2-yl]carbonyl]amino]éthoxy]benzoïque

En opérant de façon analogue à l'exemple 298, au départ de l'ester obtenu selon l'exemple 470, on obtient le produit attendu sous forme d'un solide beige (rendement = 84 %).
F = 206°C.

### PREPARATION CLXVI

### Chlorure de l'acide 2,3-dihydro-1-[[2',4'-difluoro-[1,1'-biphényl]-4-yl]sulfonyl]-4-méthoxy-1H-indole-2-carboxylique

En opérant de façon analogue à la préparation VI, au départ de l'acide obtenu selon la préparation CLXV, on obtient le produit attendu sous forme d'une pâte beige (utilisé sans autre purification pour le couplage suivant).

### Exemple 472

### Acide 4-[[[[1-[(2',4'-difluoro[1,1'-biphényl]-4-yl)sulfonyl]-2,3-dihydro-4-méthoxy-1H-indol-2-yl]carbonyl]amino]méthyl]benzoïque

En opérant de façon analogue à l'exemple 17, au départ du composé obtenu selon la préparation CLXVI et d'acide 4-(aminométhyl)benzoïque, on obtient le produit attendu sous forme d'un solide blanc (rendement = 10 %).
F = 139°C.

### Exemple 473

### Acide 4-[[[[1-[(2',4'-difluoro[1,1'-biphényl]-4-yl)sulfonyl]-2,3-dihydro-4-méthoxy-1H-indol-2-yl]carbonyl]amino]méthyl]benzèneacétique, méthyl ester

En opérant de façon analogue à l'exemple 472, au départ du chlorhydrate de l'ester méthylique de l'acide 4-(aminométhyl)benzèneacétique, on obtient le produit attendu sous forme d'une huile (rendement = 77 %).
RMN ¹H (DMSO, 300 MHz) δ : 8,72 (t, NH) ; 7,91 (dd, 2H) ; 7,71 (dd, 2H) ; 7,70-7,55 (m, 1 H) ; 7,45-7,30 (m, 1 H) ; 7,30-7,15 (m, 6H) ; 7,12 (d, 1H) ; 6,70 (d, 1 H) ; 4,91 (dd, 1 H) ; 4,32 (t, 2H) ; 3,72 (s, 3H) ; 3,65 (s, 2H) ; 3,60 (s, 3H) ; 3,07 (dd, 1 H) ; 2,82 (dd, 1 H).

### Exemple 474

### Acide 4-[[[[1-[(2',4'-difluoro[1,1'-biphényl]-4-yl)sulfonyl]-2,3-dihydro-4-méthoxy-1H-indol-2-yl]carbonyl]amino]méthyl]benzèneacétique

En opérant de façon analogue à l'exemple 5, au départ de l'ester obtenu selon l'exemple 473, on obtient le produit attendu sous forme d'une mousse blanche (rendement = 98 %).
RMN ¹H (DMSO, 250 MHz) δ : 8,70 (t, NH) ; 7,90 (dd, 2H) ; 7,71 (dd, 2H) ; 7,70-7,55 (m, 1H) ; 7,50-7,35 (m, 1H) ; 7,30-7,15 (m, 6H) ; 7,12 (d, 1H) ; 6,70 (d, 1H) ; 4,91 (dd, 1 H) ; 4,32 (t, 2H) ; 3,72 (s, 3H) ; 3,54 (s, 2H) ; 3,07 (dd, 1H) ; 2,82 (dd, 1 H).

### PREPARATION CLXVII

### Acide 7-méthoxy-2,3-dihydro-1H-indole-2-carboxylique, méthyl ester

En opérant de façon analogue à la préparation LXIV, au départ de l'ester méthylique de l'acide 7-méthoxy-2-indolecarboxylique, on obtient le produit attendu sous forme d'une huile beige (rendement = 98 %).
RMN ¹H (DMSO, 250 MHz) δ : 6,75-6,50 (m, 3H); 5,33 (d, NH); 4,37 (ddd, 1 H) ; 3,74 (s, 3H) ; 3,65 (s, 3H) ; 3,32 (dd, 1H) ; 3,08 (dd, 1 H).

### PREPARATION CLXVIII

### Acide 1-[(4-iodophényl)sulfonyl]-2,3-dihydro-7-méthoxy-1H-indole-2-carboxylique, méthyl ester

En opérant de façon analogue à la préparation CLVIII, au départ de l'ester méthylique de l'acide 2,3-dihydro-7-méthoxy-1*H*-indole-2-carboxylique, on obtient le produit attendu sous forme d'un solide blanc (rendement = 62 %).
F= 161°C.

### PREPARATION CLXIX

### Acide 2,3-dihydro-1-[[2',4'difluoro-[1,1'-biphényl]-4-yl]sulfonyl]-7-méthoxy-1H-indole-2-carboxylique, méthyl ester

En opérant de façon analogue à la préparation CLXIV, au départ de l'ester obtenu selon la préparation CLXVIII, on obtient le produit attendu sous forme d'une huile beige (rendement = 57 %).
RMN ¹H (DMSO, 300 MHz) δ : 8,01 (d, 2H) ; 7,76 (d, 2H) ; 7,75-7,65 (m, 1 H) ; 7,50-7,35 (m, 1 H) ; 7,30-7,15 (m, 1H) ; 7,05 (t, 1H) ; 6,84 (d, 2H) ; 5,50 (dd, 1 H) ; 3,72 (s, 3H) ; 3,50 (s, 3H) ; 3,45 (dd, 1 H) ; 3,15 (dd, 1 H).

### PREPARATION CLXX

### Acide 2,3-dihydro-1-[[2',4'-difluoro-[1,1'-biphényl]-4-yl]sulfonyl]-7-méthoxy-1H-indole-2-carboxylique

En opérant de façon analogue à l'exemple 5, au départ de l'ester obtenu selon la préparation CLXIX, on obtient le produit attendu sous forme d'une mousse beige (rendement = 98 %).
RMN ¹H (DMSO, 300 MHz) δ : 8,05 (d, 2H) ; 7,80-7,60 (m, 3H) ; 7,50-7,35 (m, 1 H) ; 7,30-7,15 (m, 1 H) ; 7,04 (t, 1 H) ; 6,83 (d, 2H) ; 5,35 (dd, 1H) ; 3,49 (s, 3H) ; 3,33 (dd, 1 H) ; 3,11 (dd, 1 H).

### Exemple 475

### Acide 4-[2-[[[-1-[(2',4'-difluoro[1,1'-biphényl]-4-yl)sulfonyl]-2,3-dihydro-7-méthoxy-1H-indol-2-yl]carbonyl]amino]éthoxy]benzoïque, 1,1-diméthyléthyl ester

En opérant de façon analogue à l'exemple 470, au départ de l'acide obtenu selon la préparation CLXX, on obtient le produit attendu sous forme d'une huile incolore (rendement = 81 %).
RMN ¹H (DMSO, 250MHz) δ : 8,25 (t, NH) ; 7,91 (dd, 2H) ; 7,80 (dd, 2H) ; 7,70-7,50 (m, 3H) ; 7,45-7,30 (m, 1 H) ; 7,28-7,10 (m, 1 H) ; 7,10-6,90 (m, 3H) ; 6,90-6,75 (m, 2H) ; 5,26 (dd, 1 H) ; 4,08 (t, 2H) ; 3,53 (s, 3H) ; 3,60-3,40 (m, 2H) ; 3,19 (dd, 1 H) ; 2,92 (dd, 1 H) ; 1,52 (s, 9H).

### Exemple 476

### Acide 4-[2-[[[-1-[(2',4'-difluoro[1,1'-biphényl]-4-yl)sulfonyl]-2,3-dihydro-7-méthoxy-1H-indol-2-yl]carbonyl]amino]éthoxy]benzoïque

En opérant de façon analogue à l'exemple 298, au départ de l'ester obtenu selon l'exemple 475, on obtient le produit attendu sous forme d'un solide blanc (rendement = 83 %).
F = 234°C.

### Exemple 477

### Acide 4-[[[[1-[(2',4'-difluoro[1,1'-biphényl]-4-yl)sulfonyl]-2,3-dihydro-7-méthoxy-1H-indol-2-yl]carbonyl]amino]méthyl]benzoïque

En opérant de façon analogue à la préparation CLXVI et à l'exemple 472, au départ du composé obtenu selon la préparation CLXX, on obtient le produit attendu sous forme d'un solide blanc (rendement = 24 %).
F =218°C.

### PREPARATION CLXXI

### Acide 2,3-dihydro-1-[[2'-chloro-4'-fluoro-[1,1'-biphényl]-4-yl]sulfonyl]-4-méthoxy-1H-indole-2-carboxylique, méthyl ester

En opérant de façon analogue à l'exemple 2, au départ de l'ester obtenu selon la préparation LXV et d'acide 2-chloro-4-fluorophénylboronique, on obtient le produit attendu sous forme d'une poudre blanche (rendement = 78%).
F = 80°C.

### PREPARATION CLXXII

### Acide 2,3-dihydro-1-[[2'-chloro-4'-fluoro-[1,1'-biphényl]-4-yl]sulfonyl]-4-méthoxy-1H-indole-2-carboxylique

En opérant de façon analogue à l'exemple 5, au départ de l'ester obtenu selon la préparation CLXXI, on obtient le produit attendu sous forme d'une poudre blanche (rendement = 82 %).
F = 137-138°C.

### Exemple 478

### Acide 4-[[[[1-[(2'-chloro-4'-fluoro[1,1'-biphényl]-4-yl)sulfonyl]-2,3-dihydro-4-méthoxy-1H-indol-2-yl]carbonyl]amino]méthyl]benzoïque

En opérant de façon analogue à la préparation CLXVI et à l'exemple 472, au départ du composé obtenu selon la préparation CLXXII, on obtient le produit attendu sous forme d'un solide blanc (rendement = 36 %).
F =225-234°C.

### Exemple 479

### Acide 4-[2-[[[-1-[(2'-chloro-4'-fluoro[1,1'-biphényl]-4-yl)sulfonyl]-2,3-dihydro-4-méthoxy-1H-indol-2-yl]carbonyl]amino]éthoxy]benzoïque, 1,1-diméthyléthyl ester

En opérant de façon analogue à l'exemple 470, au départ de l'acide obtenu selon la préparation CLXXII, on obtient le produit attendu sous forme d'un solide blanc (rendement = 94 %).
F= 79-85°C.

### Exemple 480

### Acide 4-[2-[[[-1-[(2'-chloro-4'-fluoro[1,1'-biphényl]-4-yl)sulfonyl]-2,3-dihydro-4-méthoxy-1H-indol-2-yl]carbonyl]amino]éthoxy]benzoïque

En opérant de façon analogue à l'exemple 298, au départ de l'ester obtenu selon l'exemple 479, on obtient le produit attendu sous forme d'un solide orange pâle (rendement = 65 %).
F = 190-204°C.

### PREPARATION CLXXIII

### 2-(difluorométhoxy)-4-fluoro-bromobenzène

Ce composé est obtenu sous forme d'une huile (non purifié) en chauffant à reflux pendant une nuit un mélange de 2-bromo-5-fluorophénol et de bromodifluoroacétate d'éthyle dans l'acétone en présence de carbonate de potassium.

### PREPARATION CLXXIV

### Acide (2S)-2,3-dihydro-1-[[2'-(difluorométhoxy)-4'-fluoro-[1,1'-biphényl]-4-yl]sulfonyl]-1H-indole-2-carboxylique

En opérant de façon analogue à la préparation CX au départ du composé obtenu selon la préparation IVa, on obtient l'acide (2S)-2,3-dihydro-1-[[4-(4,4,5,5-tétraméthyl-1,3,2-dioxaborolan-2-yl)phényl]sulfonyl]-1*H*-indole-2-carboxylique, que l'on fait ensuite réagir selon l'exemple 1 avec le composé obtenu selon la préparation CLXXIII pour obtenir le produit attendu sous forme d'un solide beige (rendement = 52 %).

### Exemple 481

### Acide 4-[2-[[[(2S)-1-[[2'-(difluorométhoxy)-4'-fluoro[1,1'-biphényl]-4-yl]sulfonyl]-2,3-dihydro-1H-indol-2-yl]carbonyl]amino]éthoxy] benzoïque, 1,1-diméthyléthyl ester

En opérant de façon analogue à l'exemple 470, au départ de l'acide obtenu selon la préparation CLXXIV, on obtient le produit attendu sous forme d'un solide blanc (rendement = 61 %).
F= 60-70°C.

### Exemple 482

### Acide 4-[2-[[[(2S)-1-[[2'-(difluorométhoxy)-4'-fluoro[1,1'-biphényl]-4-yl]sulfonyl]-2,3-dihydro-1H-indol-2-yl]carbonyl]amino]éthoxy] benzoïque

En opérant de façon analogue à l'exemple 298, au départ de l'ester obtenu selon l'exemple 481, on obtient le produit attendu sous forme d'un solide blanc (rendement = 78 %).
F = 70°C.

### Exemple 483

### Acide 4-[[[[(2S)-1-[[2'-(difluorométhoxy)-4'-fluoro[1,1'-biphényl]-4-yl]sulfonyl]-2,3-dihydro-1H-indol-2-yl]carbonyl]amino]méthyl]benzoïque

En opérant de façon analogue à la préparation CLXVI et à l'exemple 472, au départ du composé obtenu selon la préparation CLXXIV, on obtient le produit attendu sous forme d'un solide blanc (rendement = 26 %).
F =92-97°C.

En opérant de façon analogue aux préparations ou exemples CLXXIV, 481 à 483, on obtient les composés suivants :

### Exemple 484

### Acide 4-[2-[[[1-[[2'-(difluorométhoxy)-4'-fluoro[1,1'-biphényl]-4-yl]sulfonyl]-2,3-dihydro-4-méthoxy-1H-indol-2-yl]carbonyl]amino]éthoxy]benzoïque, 1,1-diméthyléthyl ester

Solide blanc (rendement = 71 %).
F = 60-76°C.

### Exemple 485

### Acide 4-[2-[[[1-[[2'-(difluorométhoxy)-4'-fluoro[1,1'-biphényl]-4-yl]sulfonyl]-2,3-dihydro-4-méthoxy-1H-indol-2-yl]carbonyl]amino]éthoxy]benzoïque

Solide blanc (rendement = 98 %).
F = 69-73°C.

### Exemple 486

### Acide 4-[[[[1-[[2'-(difluorométhoxy)-4'-fluoro[1,1'-biphényl]-4-yl]sulfonyl]-2,3-dihydro-4-méthoxy-1H-indol-2-yl]carbonyl]amino]méthyl]benzoïque

Solide blanc (rendement = 60 %).
F = 230-240°C.

Les structures chimiques des composés décrits précédemment sont répertoriées dans le tableau suivant :

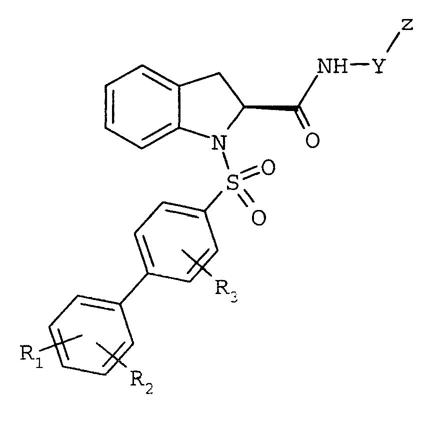

| Ex | R1 | R2 | R3 | Y | Z |
|---|---|---|---|---|---|
| 1 | 4'-CF₃ | H | H | (CH₂)₂ | 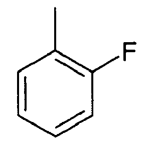 |
| 2 | 3'-CF₃ | H | H | (CH₂)₂ | 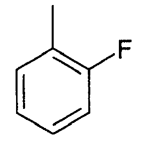 |
| 3 | 2'- CF₃ | H | H | (CH₂)₂ | 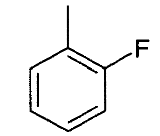 |
| 4 | 3'-CF₃ | H | H | (CH₂)₂ | 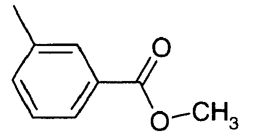 |
| 5 | 3'-CF₃ | H | H | (CH₂)₂ | 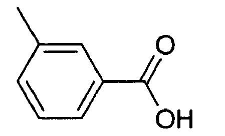 |
| 6 | 3'-CF₃ | H | H | 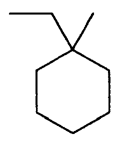 | 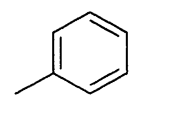 |
| 7 | 2'-CF₃ | H | H | (CH₂)₂ | 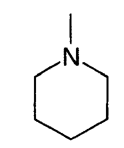 |
| 8 | 2'-CF₃ | H | H | (CH₂)₃ | 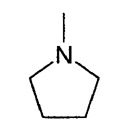 |
| 9 | 2'-CF₃ | H | H | (CH₂)₃ | 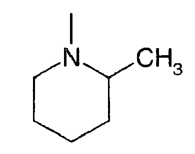 |
| 10 | 2'-CF₃ | H | H | (CH₂)₄ | 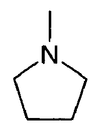 |
| 11 | 2'-CF₃ | H | H | (CH₂)₂ | 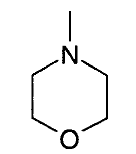 |
| 12 | 2'-CF₃ | H | H | (CH₂)₃ | 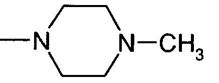 |
| 13 | 2'-CF₃ | H | H | (CH₂)₃-O- | -CH₃ |
| 14 | 2'-CF₃ | H | H | (CH₂)₃ | 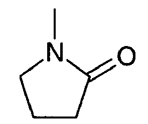 |
| 15 | 2'-CF₃ | H | H | CH₂ | CF₃ |
| 16 | 2'-CF₃ | H | H | (CH₂)₃ | 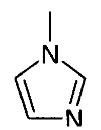 |
| 17 | 2'-CF₃ | H | H | CH₂ | 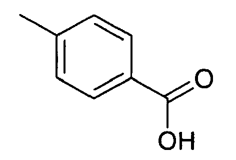 |
| 18 | 2'-CF₃ | H | H | (CH₂)₂-O- | 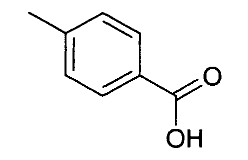 |
| 19 | 2'-CF₃ | H | H | (CH₂)₄ | 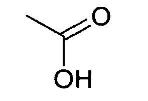 |
| 20 | 2'-CF₃ | H | H | (CH₂)₃ | CF₃ |
| 21 | 2'-CF₃ | H | H | CH₂ | 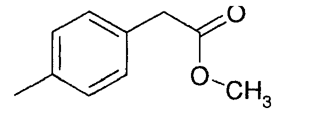 |
| 22 | 2'-CF₃ | H | H | CH₂ | 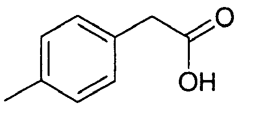 |
| 23 | 2'-CF₃ | H | H | (CH₂)₂ | CF₃ |
| 24 | 2'-CF₃ | H | H | (CH₂)₂ | 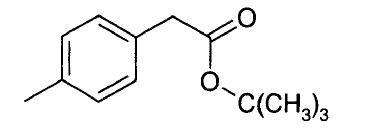 |
| 25 | 2'-CF₃ | H | H | (CH₂)₂ | 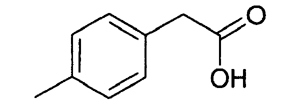 |
| 26 | 2'-CF₃ | H | H | (CH₂)₂ | 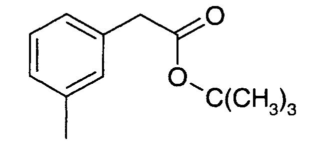 |
| 27 | 2'-CF₃ | H | H | (CH₂)₂ | 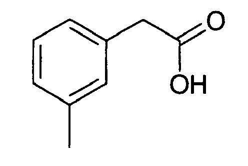 |
| 28 | 2'-CF₃ | H | H | (CH₂)₅ | 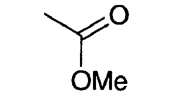 |
| 29 | 2'-CF₃ | H | H | (CH₂)₅ | 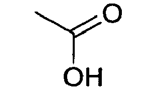 |
| 30 | 2'-CF₃ | H | H | (CH₂)₄ | 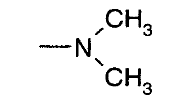 |
| 31 | 2'-CF₃ | H | H | (CH₂)₃ | 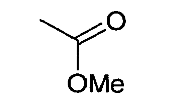 |
| 32 | 2'-CF₃ | H | H | (CH₂)₃ | 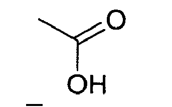 |
| 33 | 2'-CF₃ | H | H | (CH₂)₂-O- | 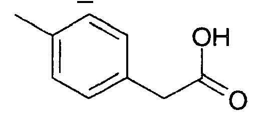 |
| 34 | 3'-CF₃ | 4'-Cl | H | (CH₂)₂-O- | 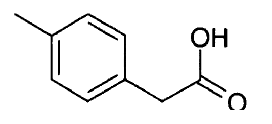 |
| 35 | 5'-CF₃ | 2'-Cl | H | (CH₂)₂-O- | 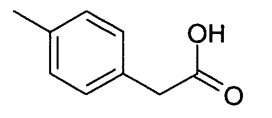 |
| 36 | 4'-CF₃ | 3'-Cl | H | (CH₂)₂-O- | 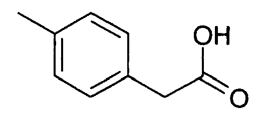 |
| 37 | 2'-O-CF₃ | H | H | (CH₃)₂-O- | 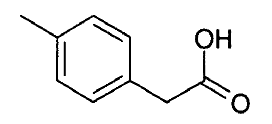 |
| 38 | 3'-O-CF₃ | H | H | (CH₂)₂-O- | 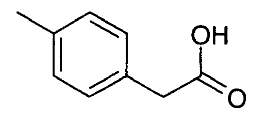 |
| 39 | 3'-O-CH₃ | H | H | (CH₂)₂-O- | 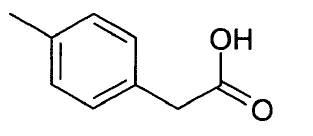 |
| 40 | 2'-F | H | H | (CH₂)₂-O- | 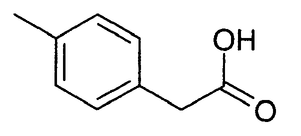 |
| 41 | 2'-CH₃ | H | H | (CH₂)₂-O- | 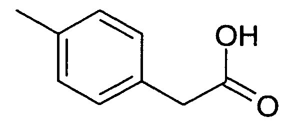 |
| 42 | 3'-iPr | H | H | (CH₂)₂-O- | 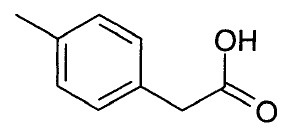 |
| 43 | 2'-O-CH₃ | H | H | (CH₂)₂-O- | 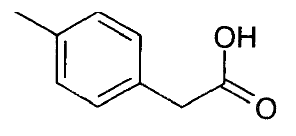 |
| 44 | 3'-Cl | H | H | (CH₂)₂-O- | 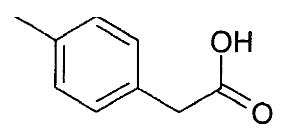 |
| 45 | 4'-Cl | H | H | (CH₂)₂-O- | 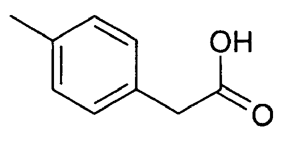 |
| 46 | 2'-Cl | H | H | (CH₂)₂-O- | 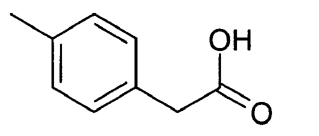 |
| 47 | 3'-F | H | H | (CH₂)₂-O- | 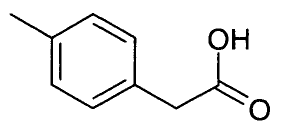 |
| 48 | 2'-CF₃ | H | H | (CH₂)₂-O- | 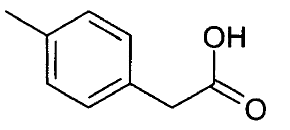 |
| 49 | 3'-CH₃ | H | H | (CH₂)₂-O- | 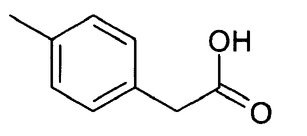 |
| 50 | 3'-CF₃ | H | H | (CH₂)₂ | 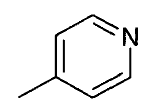 |
| 51 | 3'-CF₃ | H | H | (CH₂)₂ | 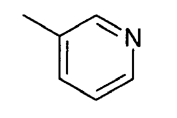 |
| 52 | 3'-CF₃ | H | H | CH₂ | 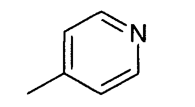 |
| 53 | 3'-CF₃ | H | H | CH₂ | 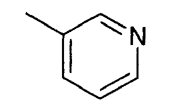 |
| 54 | 3'-CF₃ | H | H | (CH₂)₂-O- | 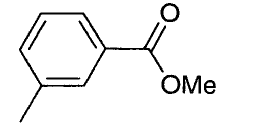 |
| 55 | 3'-CF₃ | H | H | (CH₂)₂-O- | 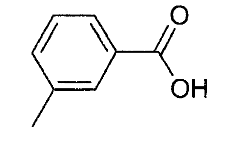 |
| 56 | H | H | 2-CF₃ | (CH₂)₂-O- | 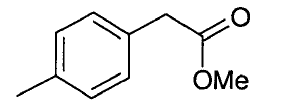 |
| 57 | H | H | 2-CF₃ | (CH₂)₂-O- | 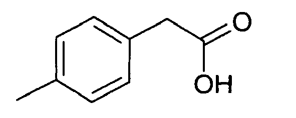 |
| 58 | 3'- CF₃ | H | H | (CH₂)₂-O- | 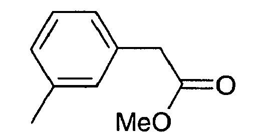 |
| 59 | 3'-CF₃ | H | H | (CH₂)₂-O- | 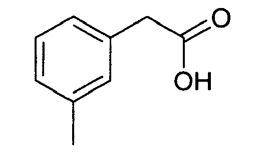 |
| 60 | 3'-CF₃ | H | H | CH₂ | 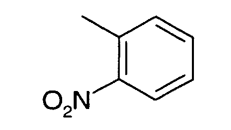 |
| 61 | 3'- CF₃ | H | H | CH₂ | 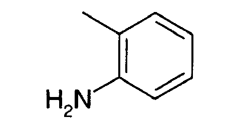 |
| 62 | 3'-CF₃ | H | H | CH₂ | 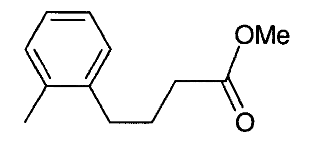 |
| 63 | 3'- CF₃ | H | H | CH₂ | 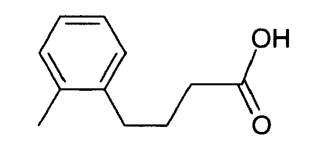 |
| 64 | 3'- CF₃ | H | H | CH₂ | 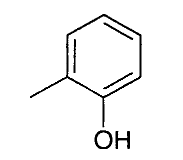 |
| 65 | 3'- CF₃ | H | H | CH₂ | 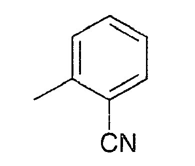 |
| 66 | 3'- CF₃ | H | H | CH₂ | 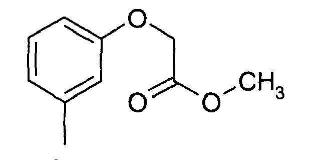 |
| 67 | 3'- CF₃ | H | H | CH₂ | 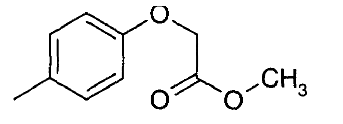 |
| 68 | 3'- CF₃ | H | H | CH₂ | 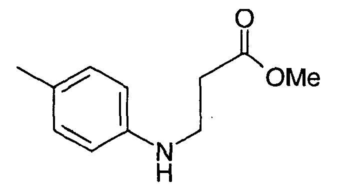 |
| 69 | 3'- CF₃ | H | H | CH₂ | 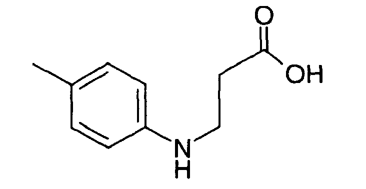 |
| 70 | 3'- CF₃ | H | H | CH₂ | 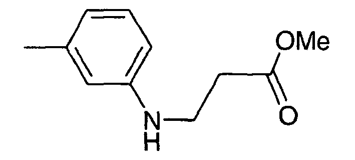 |
| 71 | 3'- CF₃ | H | H | CH₂ | 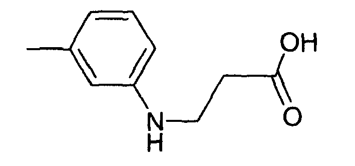 |
| 72 | 3'- CF₃ | H | H | CH₂ | 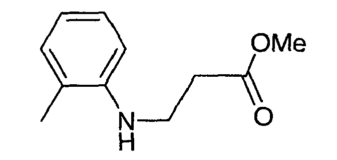 |
| 73 | 3'-CF₃ | H | H | CH₂ | 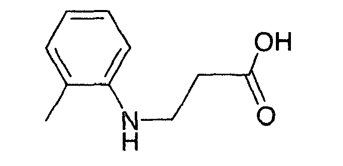 |
| 74 | 3'-CF₃ | H | H | CH₂ | 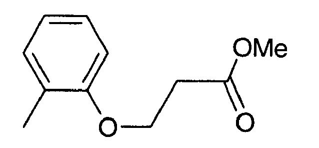 |
| 75 | 3'- CF₃ | H | H | CH₂ | 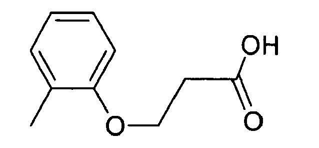 |
| 76 | 3'- CF₃ | H | H | CH₂ | 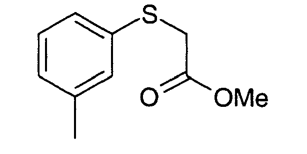 |
| 77 | 3'-CF₃ | H | H | CH₂ | 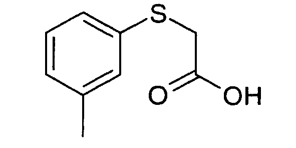 |
| 78 | 3'- CF₃ | H | H | CH₂ | 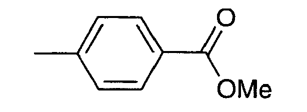 |
| 79 | 3'- CF₃ | H | H | CH₂ | 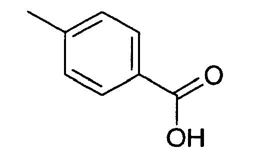 |
| 80 | 3'- CF₃ | H | H | CH₂ | 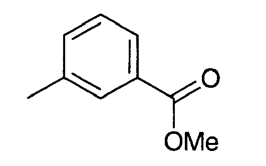 |
| 81 | 3'- CF₃ | H | H | CH₂ | 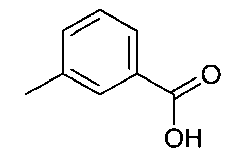 |
| 82 | 3'-CF₃ | H | H | CH₂ | 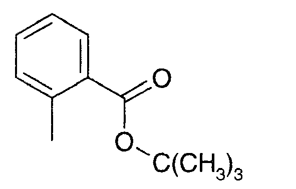 |
| 83 | 3'-CF₃ | H | H | CH₂ | 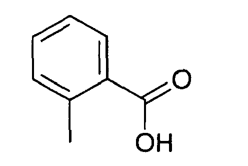 |
| 84 | 3'- CF₃ | H | H | CH₂ | 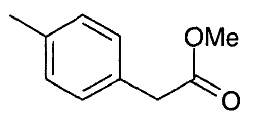 |
| 85 | 3'- CF₃ | H | H | CH₂ | 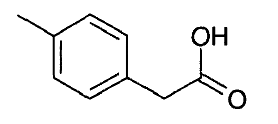 |
| 86 | 3'- CF₃ | H | H | CH₂ | 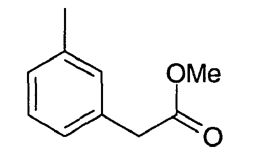 |
| 87 | 3'- CF₃ | H | H | CH₂ | 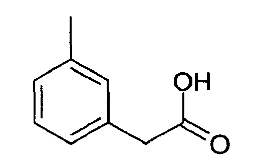 |
| 88 | 3'- CF₃ | H | H | CH₂ | 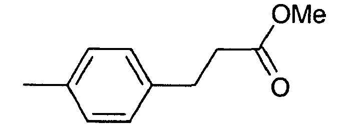 |
| 89 | 3'- CF₃ | H | H | CH₂ | 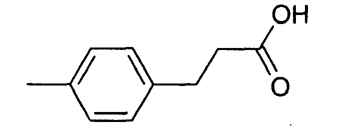 |
| 90 | 3'- CF₃ | H | H | CH₂ | 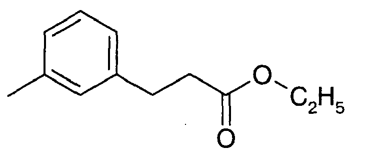 |
| 91 | 3'- CF₃ | H | H | CH₂ | 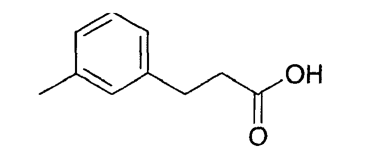 |
| 92 | 3'- CF₃ | H | H | CH₂ | 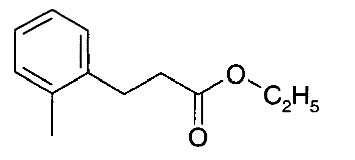 |
| 93 | 3'- CF₃ | H | H | CH₂ | 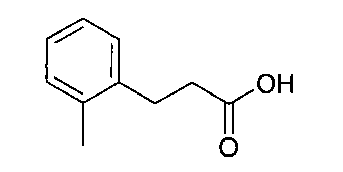 |
| 94 | 3'- CF₃ | H | H | CH₂ | 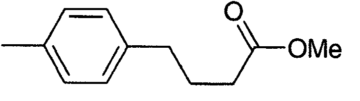 |
| 95 | 3'-CF₃ | H | H | CH₂ | 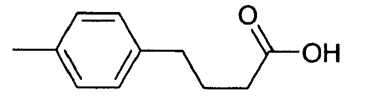 |
| 96 | 3'- CF₃ | H | H | CH₂ | 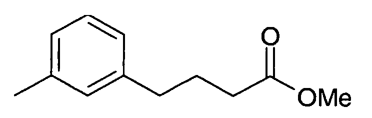 |
| 97 | 3'- CF₃ | H | H | CH₂ | 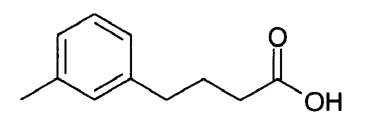 |
| 98 | 3'- CF₃ | H | H | (CH₂)₂-O- | 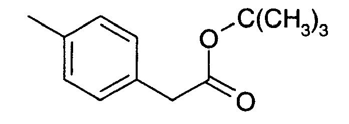 |
| 99 | 3'-CF₃ | H | H | (CH₂)₂-O- | 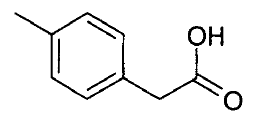 |
| 100 | 3'- CF₃ | H | H | (CH₂)₂-O- | 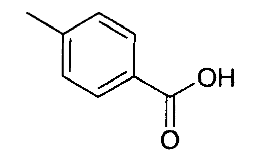 |
| 101 | 3'- CF₃ | H | H | (CH₂)₂ | 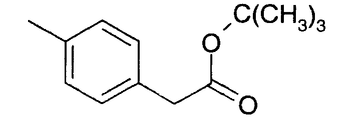 |
| 102 | 3'- CF₃ | H | H | (CH₂)₂ | 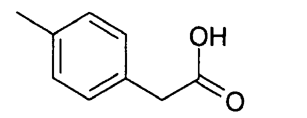 |
| 103 | 3'- CF₃ | H | H | (CH₂)₂ | 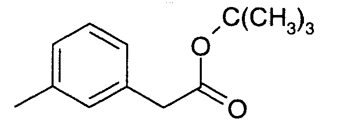 |
| 104 | 3'- CF₃ | H | H | (CH₂)₂ | 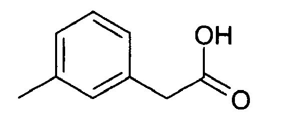 |
| 105 | 3'- CF₃ | H | H | (CH₂)₂-O- | 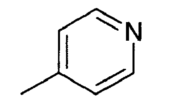 |
| 106 | 3'- CF₃ | H | H | (CH₂)₂-O- | 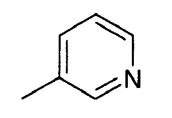 |
| 107 | 3'- CF₃ | H | H | 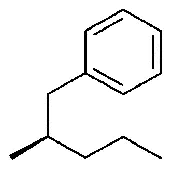 | -COOH |
| 108 | 3'- CF₃ | H | H | 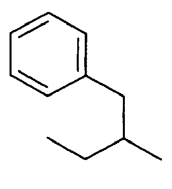 | -COOH |
| 109 | 3'-CF₃ | H | H | (CH₂)₆ | -COOH |
| 110 | 3'-CF₃ | H | H | (CH₂)₈ | -COOH |
| 111 | 3'-CF₃ | H | H | 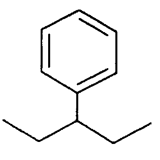 | -COOH |
| 112 | 3'-CF₃ | H | H | 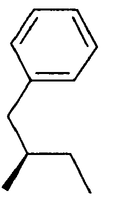 | -COOC(CH₃)₃ |
| 113 | 3'-CF₃ | H | H | 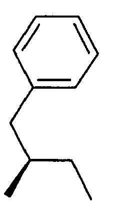 | -COOH |
| 114 | 3'- CF₃ | H | H | CH₂ | 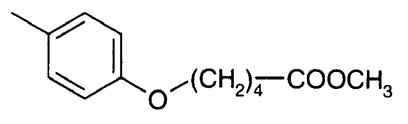 |
| 115 | 3'- CF₃ | H | H | CH₂ | 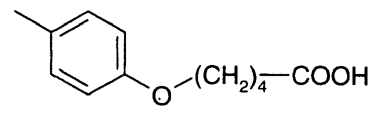 |
| 116 | 3'- CF₃ | H | H | CH₂ | 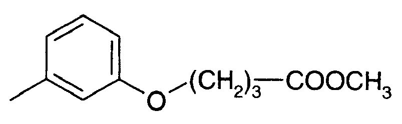 |
| 117 | 3'- CF₃ | H | H | CH₂ | 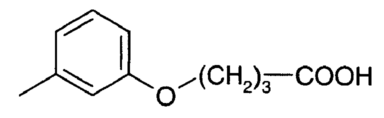 |
| 118 | 3'- CF₃ | H | H | (CH₂)₂-O- | 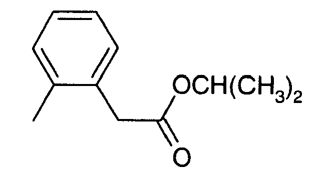 |
| 119 | 3'-CF₃ | H | H | (CH₂)₂-O- | 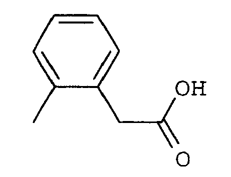 |
| 120 | 3'-CF₃ | H | H | CH₂ | 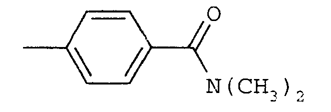 |
| 121 | 3'- CF₃ | H | H | (CH₂)₂ | 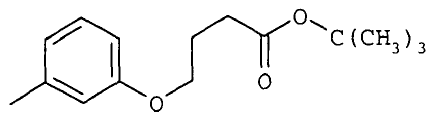 |
| 122 | 3'- CF₃ | H | H | (CH₂)₂ | 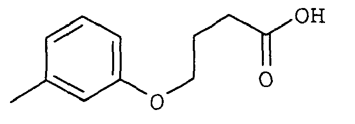 |
| 123 | 3'- CF₃ | H | H | (CH₂)₂ | 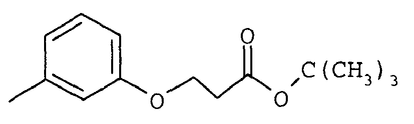 |
| 124 | 3'- CF₃ | H | H | (CH₂)₂ | 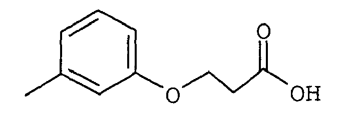 |
| 125 | 3'- CF₃ | H | H | (CH₂)₂ | 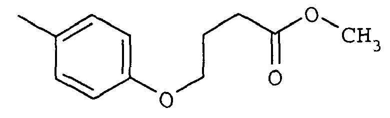 |
| 126 | 3'- CF₃ | H | H | (CH₂)₂ | 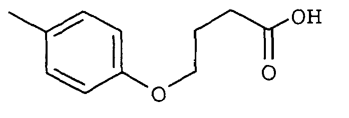 |
| 127 | 3'- CF₃ | H | H | (CH₂)₂ | 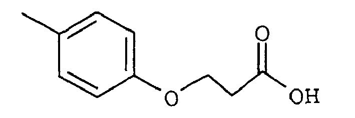 |
| 128 | 3'- CF₃ | H | H | CH₂ | 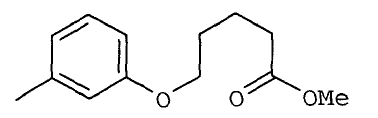 |
| 129 | 3'- CF₃ | H | H | CH₂ | 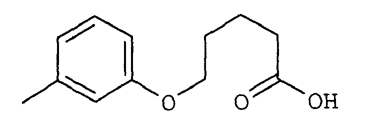 |
| 130 | 3'-CF₃ | H | H | CH₂ | 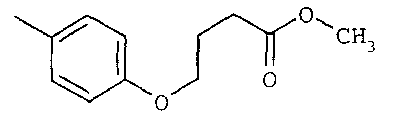 |
| 131 | 3'-CF₃ | H | H | CH₂ | 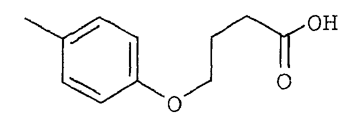 |
| 132 | 3'- CF₃ | H | H | CH₂ | 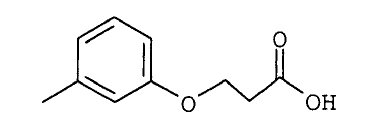 |
| 133 | 3'- CF₃ | H | H | CH₂ | 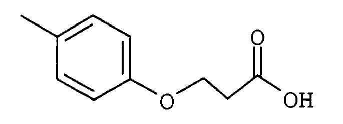 |
| 134 | 3'- CF₃ | H | H | (CH₂)₂ | 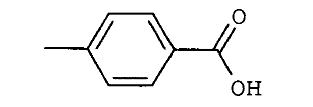 |
| 135 | 2'- CF₃ | H | H | CH₂ | 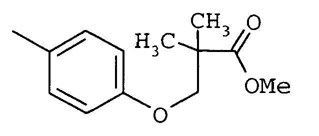 |
| 136 | 2'- CF₃ | H | H | CH₂ | 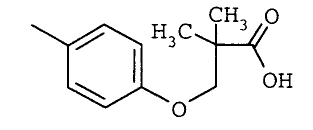 |
| 137 3' | -CF₃ | H | H | CH₂ | 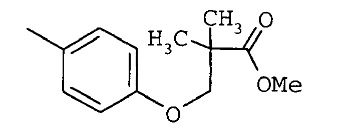 |
| 138 | 3'-CF₃ | H | H | CH₂ | 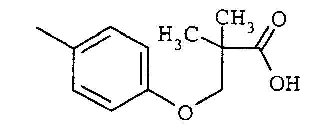 |
| 139 | 3'- CF₃ | H | H | CH₂ | 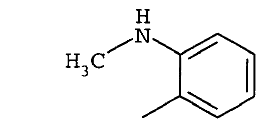 |
| 140 | 3'- CF₃ | H | H | CH₂ | 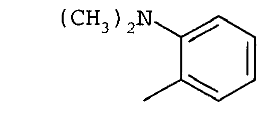 |
| 141 | 3'-CF₃ | H | H | (CH₂)₂ | 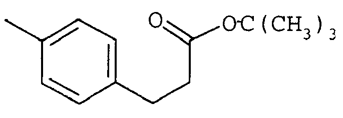 |
| 142 | 3'- CF₃ | H | H | (CH₂)₂ | 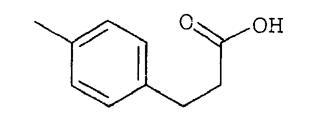 |
| 143 | 2'- CF₃ | H | H | -(CH₂)₂-S- | 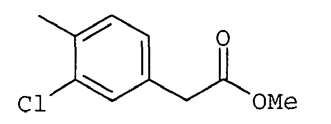 |
| 144 | 2'- CF₃ | H | H | -(CH₂)₂-S- | 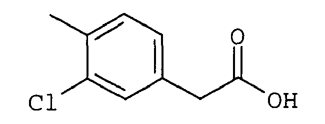 |
| 145 | 3'- CF₃ | H | H | (CH₂)₂-S- | 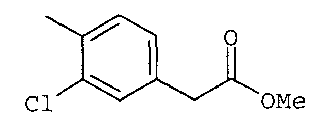 |
| 146 | 3'- CF₃ | H | H | (CH₂)₂-S- | 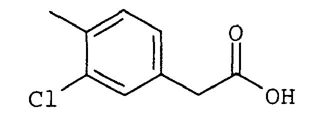 |
| 147 | 3'- CF₃ | H | H | (CH₂)₂-O- | 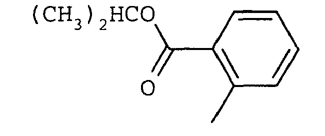 |
| 148 | 3'- CF₃ | H | H | (CH₂)₂-O- | 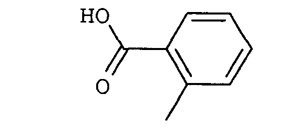 |
| 149 | 2'- CF₃ | H | H | CH₂ | 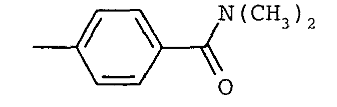 |
| 150 | 2'- CF₃ | H | H | CH₂ | 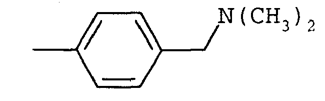 |
| 151 | 3'- CF₃ | H | H | CH₂ | 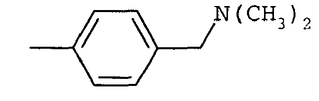 |
| 152 | 3'- CF₃ | H | H | (CH₂)₂-O- | 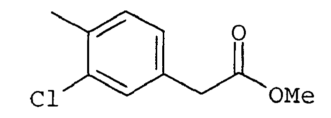 |
| 153 | 3'- CF₃ | H | H | (CH₂)₂-O- | 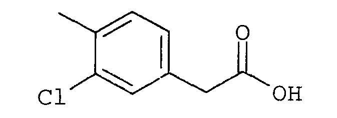 |
| 154 | 3'- CF₃ | H | H | (CH₂)₂-O- | 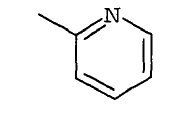 |
| 155 | 3'-CF₃ | H | H | CH₂ | 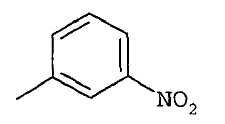 |
| 156 | 3'- CF₃ | H | H | CH₂ | 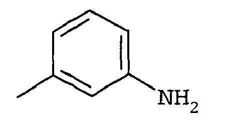 |
| 157 | 3'- CF₃ | H | H | CH₂ | 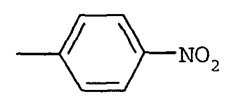 |
| 158 | 3'-CF₃ | H | H | CH₂ | 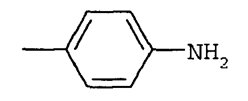 |
| 159 | 4'-F | H | H | CH₂ | 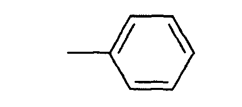 |
| 160 | 4'-F | H | H | (CH₂)₂ | 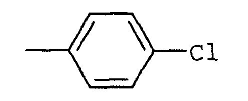 |
| 161 | 4'-F | H | H | (CH₂)₂-O- | 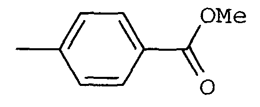 |
| 162 | 4'-F | H | H | (CH₂)₂-O- | 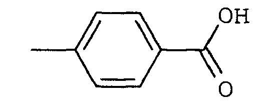 |
| 163 | 4'-F | H | H | CH₂ | 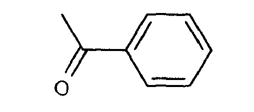 |
| 164 | 4'-F | H | H | 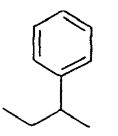 | 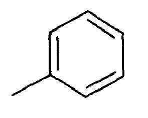 |
| 165 | 4'-F | H | H | 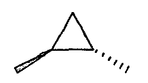 | 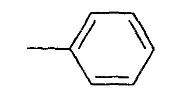 |
| 166 | 4'-F | H | H | (CH₂)₂-O- | 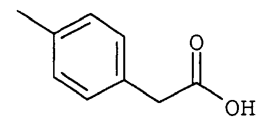 |
| 167 168 | 4'-F | H | H | 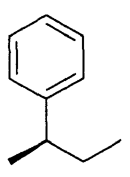 | -COOC(CH₃)₃ |
| 168 | 4'-F | H | H | 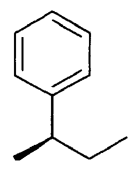 | -COOH |
| 169 | 3'- CF₃ | H | H | CH₂ | 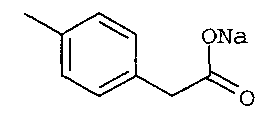 |
| 170* | 3'- CF₃ | H | H | CH₂ | 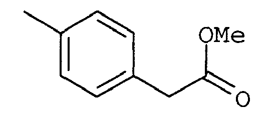 |
| 171 | 3'-CF₃ | H | H | CH₂ | 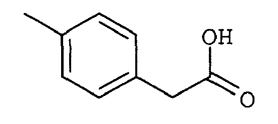 |
| 172 | 4'-F | H | H | CH₂ | 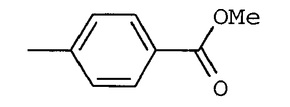 |
| 173 | 4'-F | H | H | 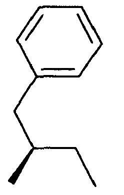 | -COOC(CH₃)₃ |
| 174 | 3'-CF₃ | H | H | CH₂ | 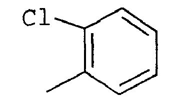 |
| 175 | 3'-CF₃ | H | H | CH₂ | 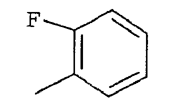 |
| 176 | 3'- CF₃ | H | H | CH₂ | 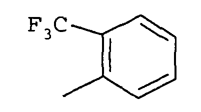 |
| 177 | 3'-CF₃ | H | H | CH₂ | 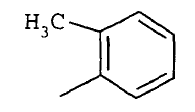 |
| 178 | 3'-CF₃ | H | H | CH₂ | 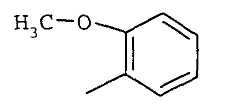 |
| 179 | 3'- CF₃ | H | H | CH₂ | 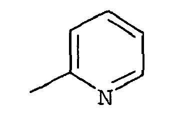 |
| 180 | 3'-CF₃ | H | H | 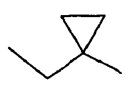 | 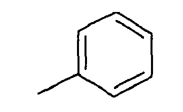 |
| 181 | 3'-CF₃ | H | H | CH₂ | 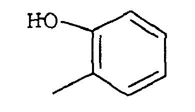 |
| 182 | 3'- CF₃ | H | H | CH₂ | 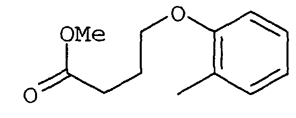 |
| 183 | 3'-CF₃ | H | H | CH₂ | 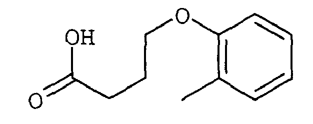 |
| 184 | 2'-CH₃ | 4'-F | H | (CH₂)₂-O- | 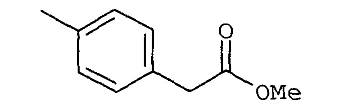 |
| 185 | 2'-CH₃ | 4'-F | H | (CH₂)₂-O- | 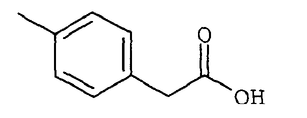 |
| 186 | 2'-CH₃ | 4'-F | H | CH₂ | 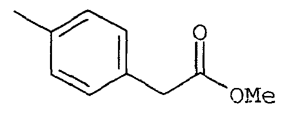 |
| 187 | 2'-CH₃ | 4'-F | H | CH₂ | 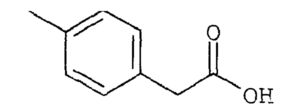 |
| 188 | 2'-CH₃ | 4'-F | H | CH₂ | 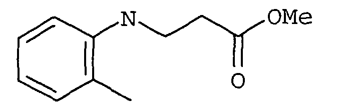 |
| 189 | 2'-CH₃ | 4'-F | H | CH₂ | 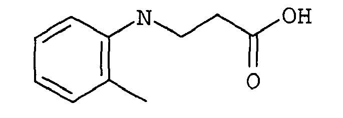 |
| 190 | 2'-Cl | 4'-F | H | CH₂ | 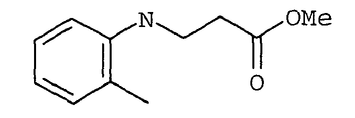 |
| 191 | 2'-Cl | 4'-F | H | CH₂ | 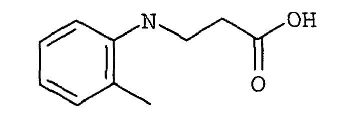 |
| 192 | 2'-OCH₃ | 4'-F | H | CH₂ | 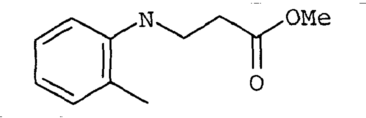 |
| 193 | 2'-OCH₃ | 4'-F | H | CH₂ | 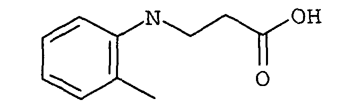 |
| 194 | 2'-F | 4'-F | H | CH₂ | 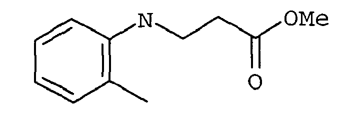 |
| 195 | 2'-F | 4'-F | H | CH₂ | 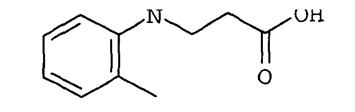 |
| 196 | 3'-CF₃ | H | H | CH₂ | 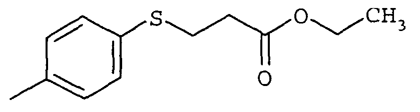 |
| 197 | 3'-CF₃ | H | H | CH₂ | 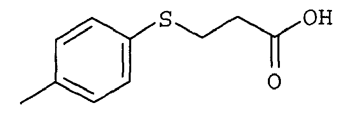 |
| 198 | 3'-CF₃ | H | H | CH₂ | 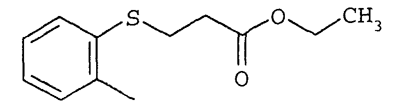 |
| 199 | 3'-CF₃ | H | H | CH₂ | 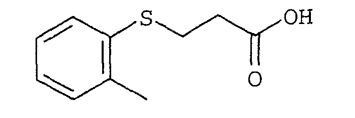 |
| 200 | 3'-CF₃ | H | H | (CH₂)₂-O- | 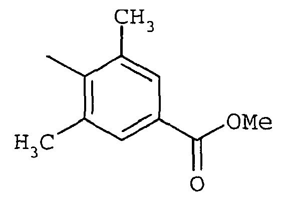 |
| 201 | 3'-CF₃ | H | H | (CH₂)₂-O- | 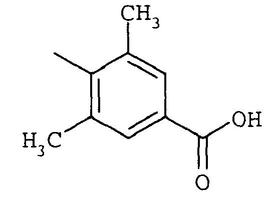 |
| 202 | 4'-F | H | H | (CH₂)₂-O- | 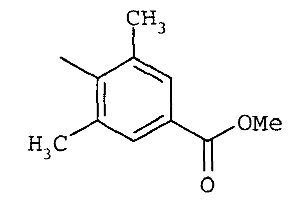 |
| 203 | 4'-F | H | H | (CH₂)₂-O- | 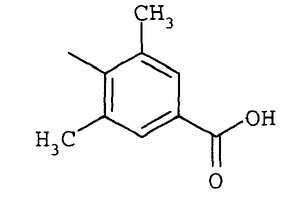 |
| 204 | 3'-CF₃ | H | H | (CH₂)₂-O- | 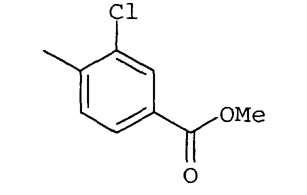 |
| 205 | 3'-CF₃ | H | H | (CH₂)₂-O- | 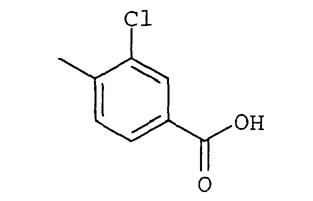 |
| 206 | 4'-F | H | H | (CH₂)₂-O- | 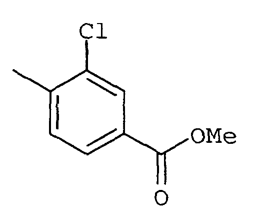 |
| 207 | 4'-F | H | H | (CH₂)₂-O- | 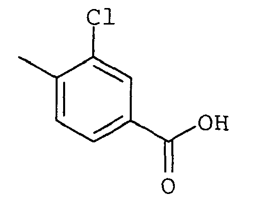 |
| 208 | 3'-CF₃ | H | H | (CH₂)₂-O- | 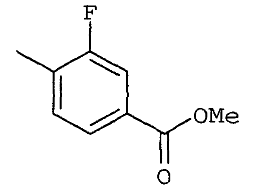 |
| 209 | 3'-CF₃ | H | H | (CH₂)₂-O- | 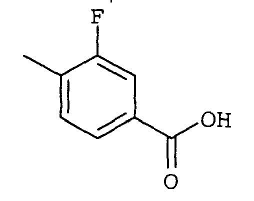 |
| 210 | 4'-F | H | H | (CH₂)₂-O- | 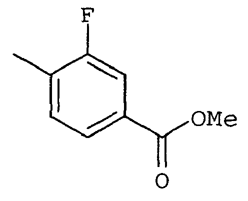 |
| 211 | 4'-F | H | H | (CH₂)₂-O- | 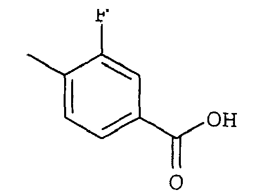 |
| 212 | 3'-CF₃ | H | H | (CH₂)₂-O- | 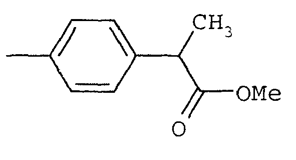 |
| 213 | 3'-CF₃ | H | H | (CH₂)₂-O- | 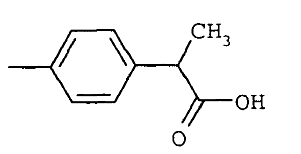 |
| 214 | 3'-CF₃ | H | H | (CH₂)₂-O- | 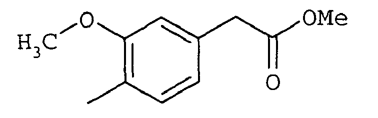 |
| 215 | 3'-CF₃ | H | H | (CH₂)₂-O- | 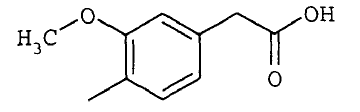 |
| 216 | 3'-CF₃ | H | H | CH₂ | 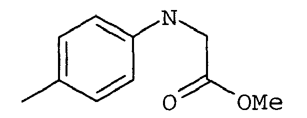 |
| 217 | 3'-CF₃ | H | H | CH₂ | 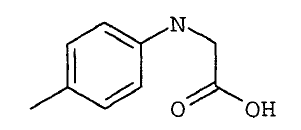 |
| 218 | 3'-CF₃ | H | H | (CH₂)₂-O- | 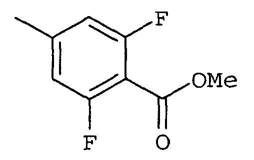 |
| 219 | 4'-F | H | H | (CH₂)₂-O- | 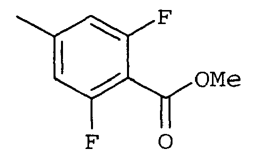 |
| 220 | 3'-CF₃ | H | H | (CH₂)₂-O- | 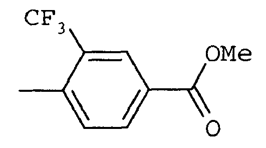 |
| 221 | 3'-CF₃ | H | H | (CH₂)₂-O- | 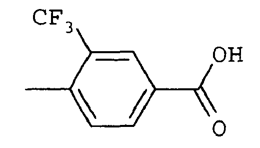 |
| 222 | 4' -F | H | H | (CH₂)₂-O- | 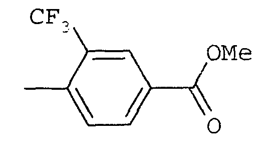 |
| 223 | 4'-F | H | H | (CH₂)₂-O- | 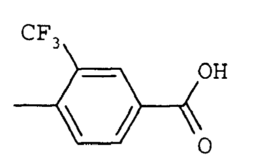 |
| 224 | 3'-CF₃ | H | H | (CH₂)₂-O- | 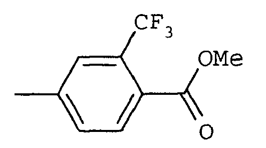 |
| 225 | 4'-F | H | H | (CH₂)₂-O- | 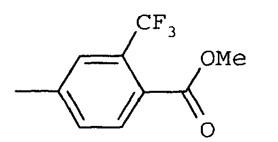 |
| 226 | 3'-CF₃ | H | H | (CH₂)₂-O- | 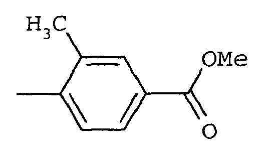 |
| 227 | 3-CF₃ | H | H | (CH₂)₂-O- | 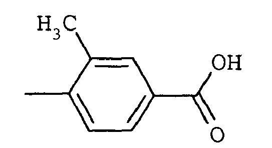 |
| 228 | 4'-F | H | H | (CH₂)₂-O- | 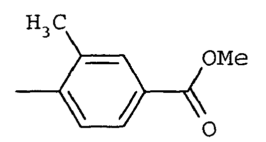 |
| 229 | 4'-F | H | H | (CH₂)₂-O- | 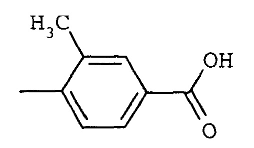 |
| 230 | 4'-F | H | H | (CH₂)₂-O- | 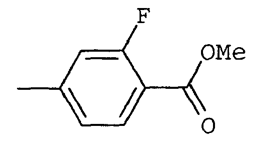 |
| 231 | 4'-F | H | H | (CH₂)₂-O- | 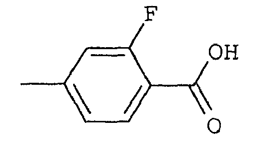 |
| 232 | 3'-CF₃ | H | H | (CH₂)₂-O- | 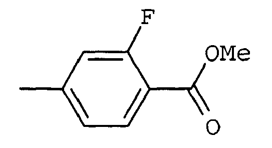 |
| 233 | 3'-CF₃ | H | H | (CH₂)₂-O- | 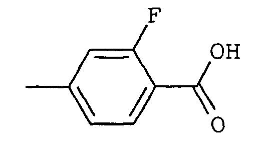 |
| 234 | 3'-CF₃ | H | H | (CH₂)₂-O- | 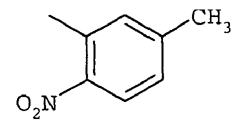 |
| 235 | 3'-CF₃ | H | H | (CH₂)₂-O- | 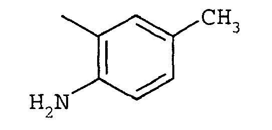 |
| 236 | 3'-CF₃ | H | H | (CH₂)₂-O- | 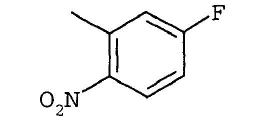 |
| 237 | 3'-CF₃ | H | H | (CH₂)₂-O- | 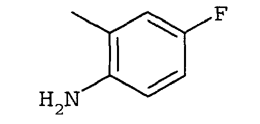 |
| 238 | 3'-CF₃ | H | H | (CH₂)₂-O- | 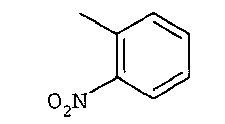 |
| 239 | 3'-CF₃ | H | H | (CH₂)₂-O- | 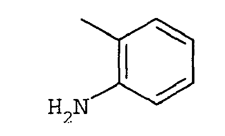 |
| 240 | 3'-CF₃ | H | H | CH₂ | 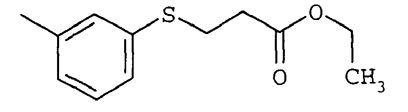 |
| 241 | 3'-CF₃ | H | H | CH₂ | 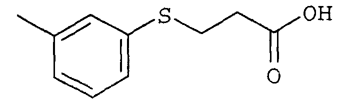 |
| 242 | 2'-Cl | 4'-F | H | 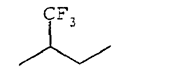 | -COOH |
| 243 | 3'-CF₃ | H | H | CH₂ | 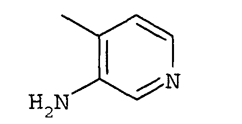 |
| 244 | 3'-CF₃ | H | H | (CH₂)₃ | 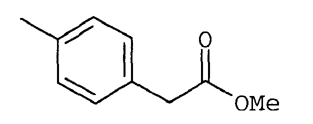 |
| 245 | 3'-CF₃ | H | H | (CH₂)₃ | 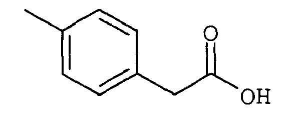 |
| 246 | 2'-F | 4'-F | H | CH₂ | 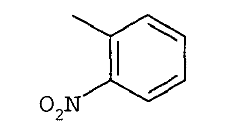 |
| 247 | 2'-F | 4'-F | H | CH₂ | 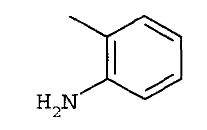 |
| 248 | 2'- CH₃ | 4'-F | H | CH₂ | 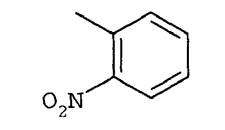 |
| 249 | 2'- CH₃ | 4'-F | H | CH₂ | 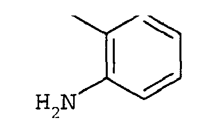 |
| 250 | 2'- OCH₃ | 4'-F | H | (CH₂)₂-O | 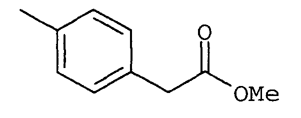 |
| 251 | 2'- OCH₃ | 4'-F | H | (CH₂)₂-O- | 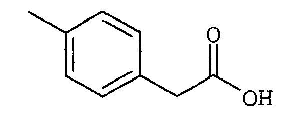 |
| 252 | 2'- OCH₃ | 4'-F | H | CH₂ | 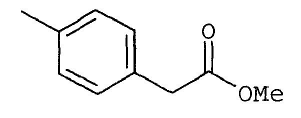 |
| 253 | 2'-OCH₃ | 4'-F | H | CH₂ | 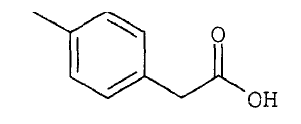 |
| 254 | 2'-OCH₃ | 4'-F | H | CH₂ | 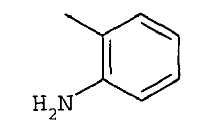 |
| 255 | 2'-F | 4'-F | H | CH₂ | 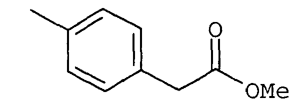 |
| 256 | 2'-F | 4'-F | H | CH₂ | 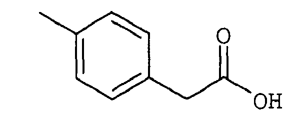 |
| 257 | 2'-F | 4'-F | H | (CH₂)₂-O- | 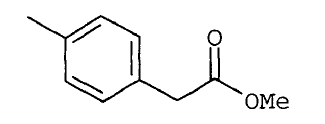 |
| 258 | 2'-F | 4'-F | H | (CH₂)₂-O- | 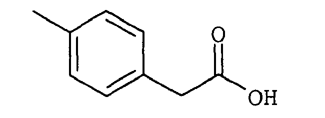 |
| 259 | 3'- CH₃ | H | H | CH₂ | 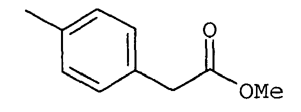 |
| 260 | 3'- CH₃ | H | H | CH₂ | 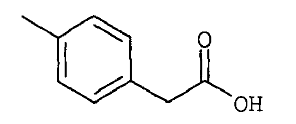 |
| 261 | 2'-Cl | 4'-F | H | (CH₂)₂-O- | 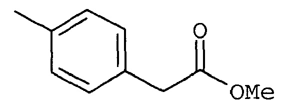 |
| 262 | 2'-Cl | 4'-F | H | (CH₂)₂-O- | 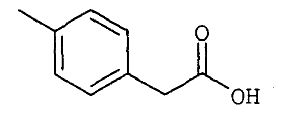 |
| 263 | 2'-Cl | 4'-F | H | CH₂ | 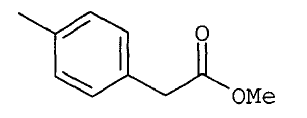 |
| 264 | 2'-Cl | 4'-F | H | CH₂ | 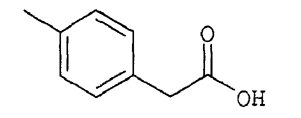 |
| 265 | 2'-Cl | 4'-F | H | CH₂ | 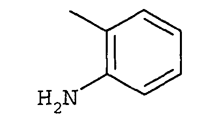 |
| 266 | 3'-Cl | H | H | CH₂ | 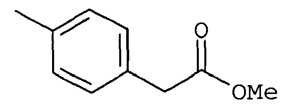 |
| 267 | 3'-Cl | H | H | CH₂ | 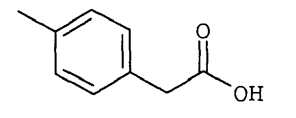 |
| 268 | 3'-C₂H₅ | H | H | CH₂ | 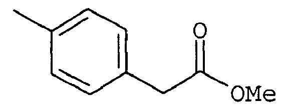 |
| 269 | 3'-C₂H₅ | H | H | CH₂ | 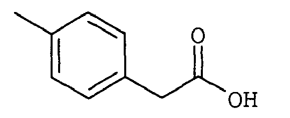 |
| 270 | 2'-CF₃ | 4'-F | H | (CH₂)₂-O- | 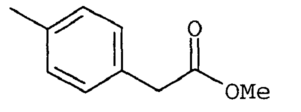 |
| 271 | 2'-CF₃ | 4'-F | H | (CH₂)₂-O- | 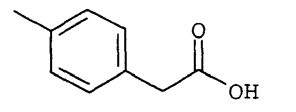 |
| 272 | 2'-CN | 4'-F | H | (CH₂)₂-O- | 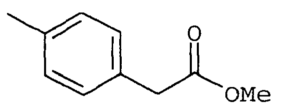 |
| 273 | 2'-CN | 4'-F | H | (CH₂)₂-O- | 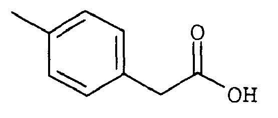 |
| 274 | 4'-Cl | H | H | (CH₂)₂-O- | 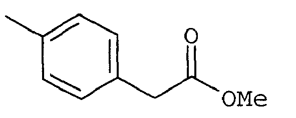 |
| 275 | 2'-Cl | 5'-CF₃ | H | (CH₂)₂-O- | 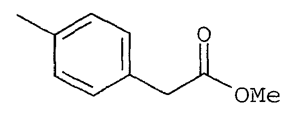 |
| 276 | 2'-F | 5'-CF₃ | H | (CH₂)₂-O- | 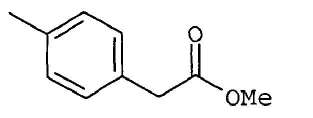 |
| 277 | 2'-F | 5'-CF₃ | H | (CH₂)₂-O- | 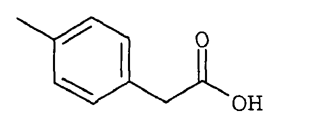 |
| 278 | 2'-F | 5'-CH₃ | H | (CH₂)₂-O- | 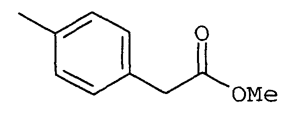 |
| 279 | 2'-F | 5'-CH₃ | H | (CH₂)₂-O- | 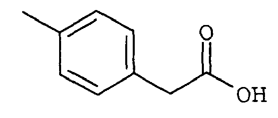 |
| 280 | 2'-OCH₃ | 5'-CH₃ | H | (CH₂)₂-O- | 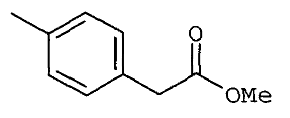 |
| 281 | 2'-OCH₃ | 5'-CH₃ | H | (CH₂)₂-O- | 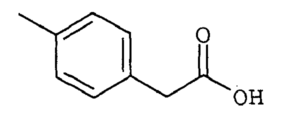 |
| 282 | 2'-CH₃ | 5'-CH₃ | H | (CH₂)₂-O- | 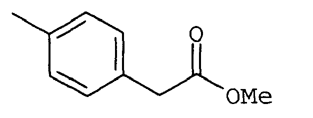 |
| 283 | 2'-CH₃ | 5'-CH₃ | H | (CH₂)₂-O- | 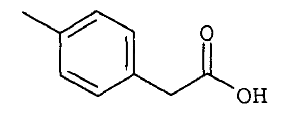 |
| 284 | 2'-OCH₃ | 5'-CF₃ | H | (CH₂)₂-O- | 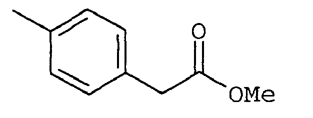 |
| 285 | 2'-OCH₃ | 5'-CF₃ | H | (CH₂)₂-O- | 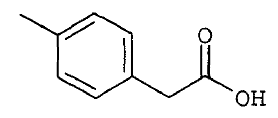 |
| 286 | 2'-CH₃ | 5'-CF₃ | H | (CH₂)₂-O- | 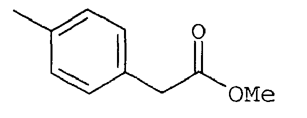 |
| 287 | 2'-CH₃ | 5'-CF₃ | H | (CH₂)₂-O- | 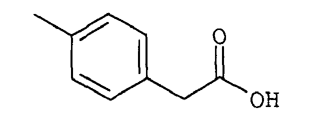 |
| 288 | 2'-Cl | 5'-CH₃ | H | (CH₂)₂-O- | 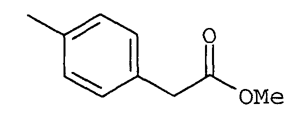 |
| 289 | 2'-Cl | 5'-CH₃ | H | (CH₂)₂-O- | 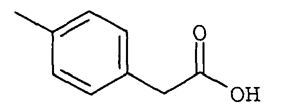 |
| 290 | 2'-Cl | 5'-Cl | H | (CH₂)₂-O- | 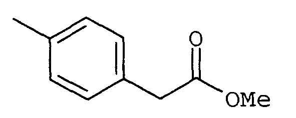 |
| 291 | 2'-Cl | 5'-Cl | H | (CH₂)₂-O- | 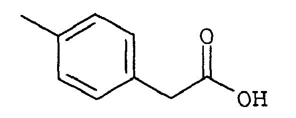 |
| 292 | 2'-Cl | 5'-F | H | (CH₂)₂-O- | 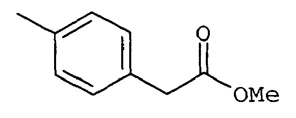 |
| 293 | 2'-Cl | 5'-F | H | (CH₂)₂-O- | 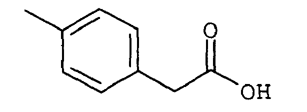 |
| 294 | 2'-Cl | 5'- CF₃ | H | (CH₂)₂-O- | 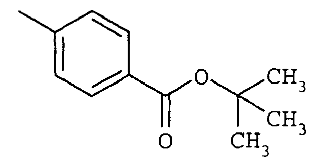 |
| 295 | 2'-Cl | 5'-CF₃ | H | (CH₂)₂-O- | 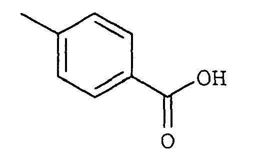 |
| 296 | 2'-Cl | 5'- CF₃ | H | (CH₂)₂-NH- | 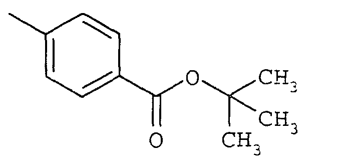 |
| 297 | 2'-Cl | 5'-CF₃ | H | (CH₂)₂-NH- | 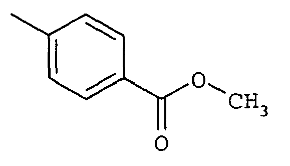 |
| 298 | 2'-Cl | 5'-CF₃ | H | (CH₂)₂-NH- | 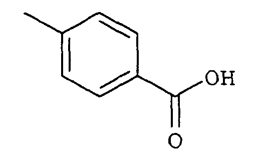 |
| 299 | 2'-Cl | 5'-CF₃ | H | (CH₂)₂-NH- | 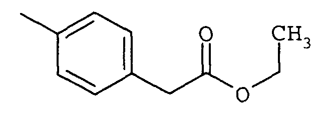 |
| 300 | 2'-Cl | 5'-CF₃ | H | (CH₂)₂-NH- | 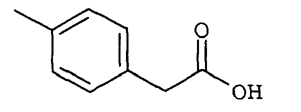 |
| 301 | 2'-Cl | 5'-CF₃ | H | (CH₂)₂-O- | 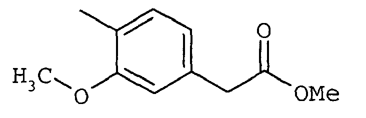 |
| 302 | 2'-Cl | 5'-CF₃ | H | (CH₂)₂-O- | 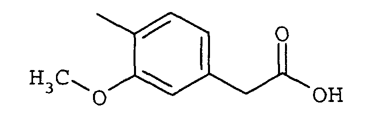 |
| 303 | 2'-Cl | 5'-CF₃ | H | (CH₂)₂-O- | 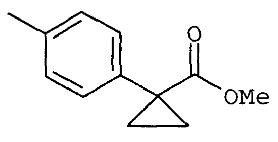 |
| 304 | 2'-Cl | 5'-CF₃ | H | (CH₂)₂-O- | 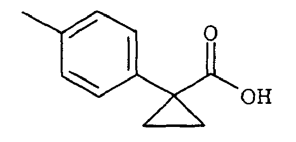 |
| 305 | 2'-Cl | 5'-CF₃ | H | (CH₂₎₂-O- | 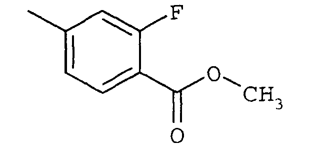 |
| 306 | 2'-Cl | 5'-CF₃ | H | (CH₂)₂-O- | 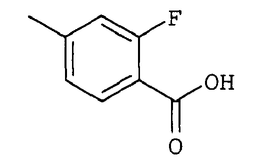 |
| 307 | 2'-Cl | 5'-CF₃ | H | (CH₂)₂-O- | 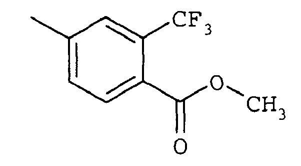 |
| 308 | 2'-Cl | 5'-CF₃ | H | (CH₂)₂-O- | 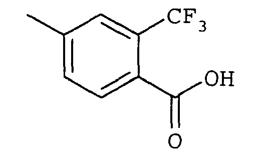 |
| 309 | 2'-Cl | 5'-CF₃ | H | (CH₂)₂-O- | 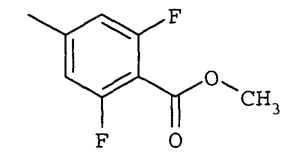 |
| 310 | 2'-Cl | 5'-CF₃ | H | (CH₂)₂-O- | 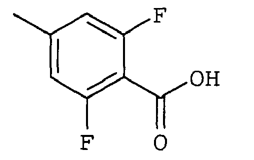 |
| 311 | 4'-F | 2'-F | H | -CH- | 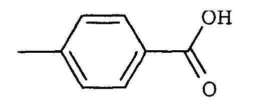 |
| 312 | 4'-F | 2'-Cl | H | -CH₂- | 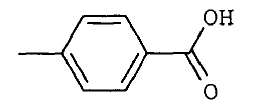 |
| 313 | 2'-Cl | 5'-CF₃ | H | (CH₂)₂-O- | 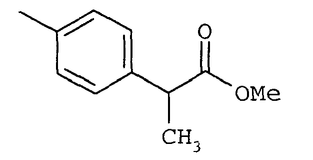 |
| 314 | 2'-Cl | 5'-CF₃ | H | (CH₂)₂O- | 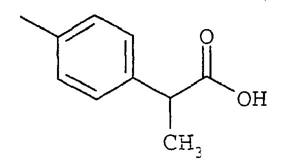 |
| 315 | 2'-Cl | 5'-CF₃ | H | (CH₂)₂-O- | 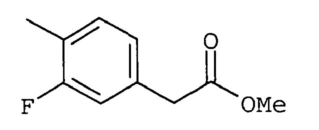 |
| 316 | 2'-Cl | 5'-CF₃ | H | (CH₂)₂-O- | 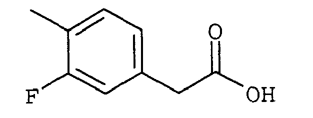 |
| 317 | 2'-Cl | 5'-CF₃ | H | (CH₂)₂-O- | 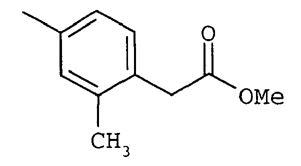 |
| 318 | 2'-Cl | 5'-CF₃ | H | (CH₂)₂-O- | 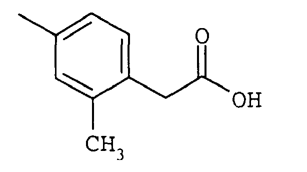 |
| 319 | 2'-Cl | 5'-CF₃ | H | (CH₂)₂-O- | 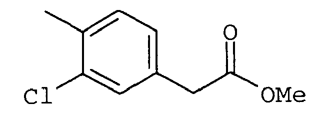 |
| 320 | 2'-Cl | 5'-CF₃ | H | (CH₂)₂-O- | 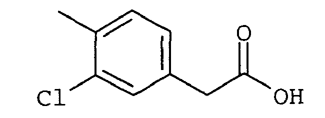 |
| 321 | 4'-F | H | H | (CH₂)₂-O- | 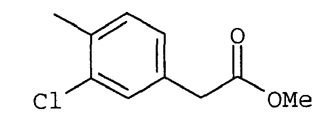 |
| 322 | 4'-F | H | H | (CH₂)₂-O- | 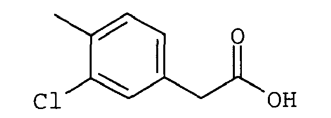 |
| 323 | 2'-Cl | 5'-CF₃ | H | (CH₂)₂-O- | 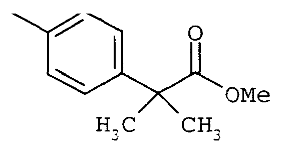 |
| 324 | 2'-Cl | 5'-CF₃ | H | (CH₂)₂-O- | 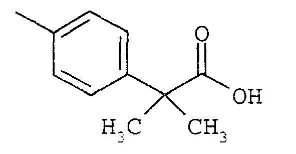 |
| 325 | 2'-Cl | 5'-CF₃ | H | (CH₂)₂-O- | 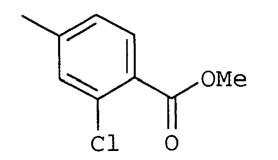 |
| 326 | 2'-Cl | 5'-CF₃ | H | (CH₂)₂-O- | 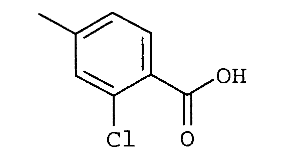 |
| 327 | 4'-F | H | H | (CH₂)₂-O- | 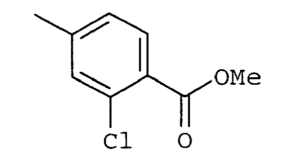 |
| 328 | 3'-CF₃ | H | H | (CH₂)₂-O- | 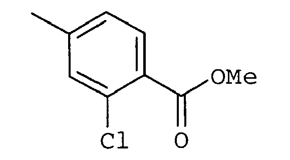 |
| 329 | 3'-CF₃ | H | H | (CH₂)₂-O- | 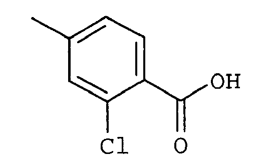 |
| 330 | 2'-Cl | 5'-CF₃ | H | (CH₂)₂-O- | 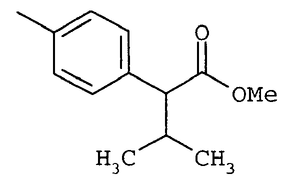 |
| 331 | 2'-Cl | 5'-CF₃ | H | (CH₂)₂-O- | 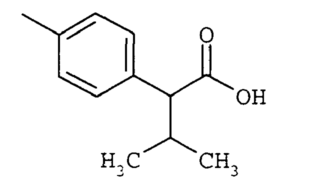 |
| 332 | 3'-CF₃ | H | H | (CH₂)₂-O- | 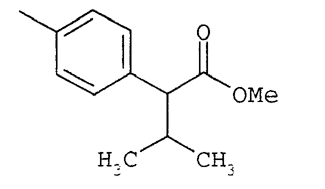 |
| 333 | 3'-CF₃ | H | H | (CH₂)₂-O- | 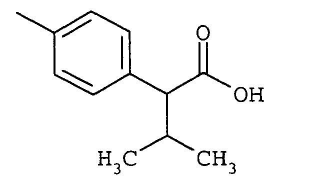 |
| 334 | 3'-CF₃ | H | H | -CH₂- | 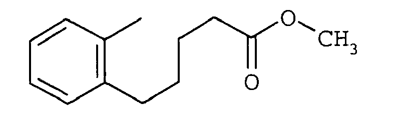 |
| 335 | 3'-CF₃ | H | H | -CH₂- | 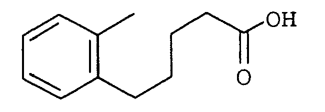 |
| 336 | 3'-CF₃ | H | H | -CH₂- | 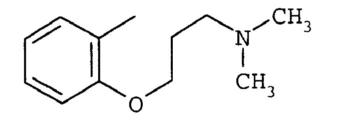 |
| 337 | 3'-CF₃ | H | H | -CH₂- | 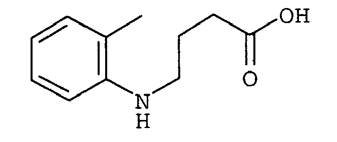 |
| 338 | 3'-CF₃ | H | H | -CH₂- | 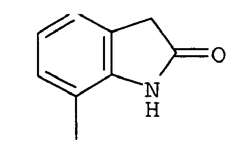 |
| 339 | 2'-Cl | 4'-F | H | (CH₂)₂-O- | 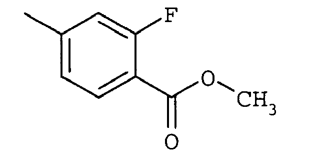 |
| 340 | 2'-Cl | 4'-F | H | (CH₂)₂-O- | 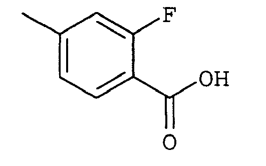 |
| 341 | 2'-OCH₃ | 4'-F | H | (CH₂)₂-O- | 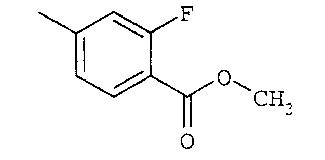 |
| 342 | 2'-OCH₃ | 4'-F | H | (CH₂)₂-O- | 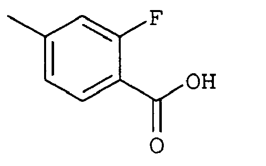 |
| 343 | 2'-F | 4'-F | H | (CH₂)₂-O- | 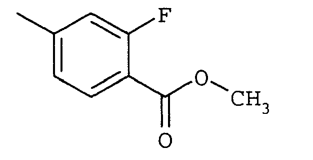 |
| 344 | 2'-F | 4'-F | H | (CH₂)₂-O- | 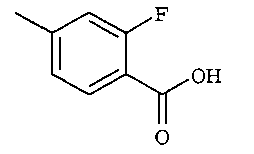 |
| 345 | 2'-CH₃ | 4'-F | H | (CH₂)₂-O- | 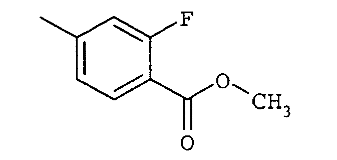 |
| 346 | 2'-CH₃ | 4'-F | H | (CH₂)₂-O- | 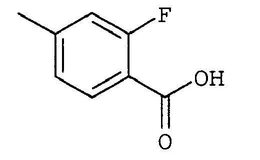 |
| 347 | 3'-CF₃ | H | H | -(CH₂)₅- | 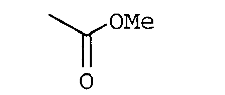 |
| 348 | 3'-CF₃ | H | H | -(CH₂)₅- | 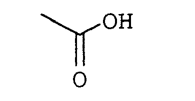 |
| 349 | 3'-CF₃ | H | H | (CH₂)₂-O- | 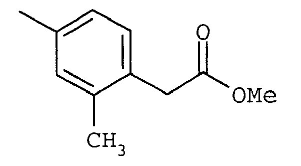 |
| 350 | 3'-CF₃ | H | H | (CH₂)₂-O- | 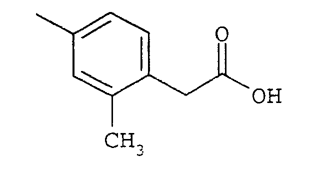 |
| 351 | 2'-CF₃ | H | H | (CH₂)₂-O- | 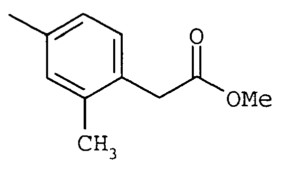 |
| 352 | 2'-CF₃ | H | H | (CH₂)₂-O- | 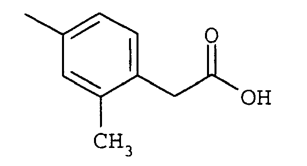 |
| 353 | 3'-CF₃ | H | H | -CH₂- | 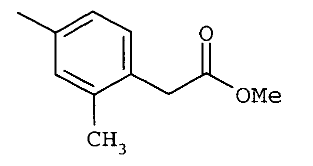 |
| 354 | 2'-OCH₃ | 5'-CH₃ | H | -CH₂- | 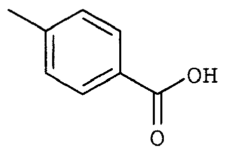 |
| 355 | 2'-F | 5'-CH₃ | H | -CH₂- | 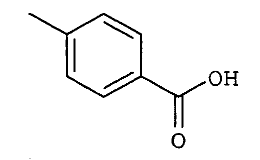 |
| 356 | 2'-F | 5'-CF₃ | H | -CH₂- | 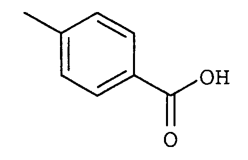 |
| 357 | 2'-CH₃ | 5'-CH₃ | H | -CH₂- | 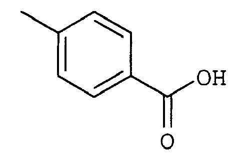 |
| 358 | 2'-Cl | 5'-CF₃ | H | -CH₂- | 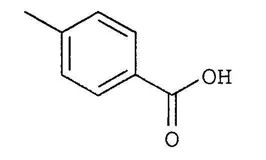 |
| 359 | 3'-CH₃ | H | H | -CH₂- | 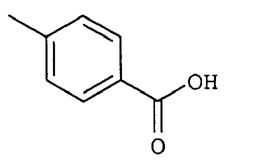 |
| 360 | 2'-OCH₃ | 5'-CF₃ | H | -CH₂- | 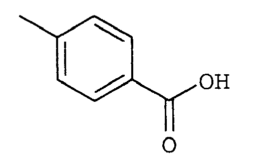 |
| 361 | 2'-CH₃ | 5'-CF₃ | H | -CH₂- | 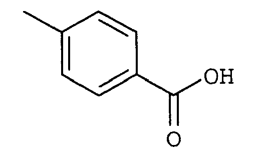 |
| 362 | 2'-Cl | 5'-CH₃ | H | -CH₂- | 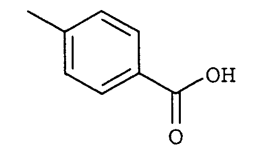 |
| 363 | 3'-CF₃ | H | H | -CH₂- | 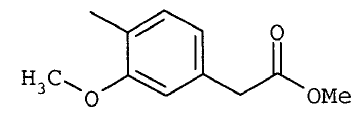 |
| 364 | 3'-CF₃ | H | H | -CH₂- | 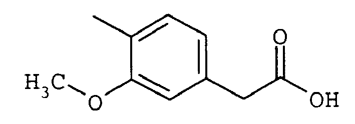 |
| 365 | 3'-CF₃ | H | H | -CH₂- | 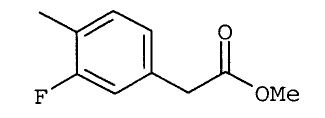 |
| 366 | 3'-CF₃ | H | H | -CH₂- | 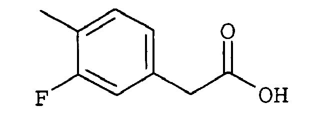 |
| 367 | 3'-CF₃ | H | H | -CH₂- | 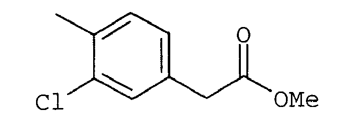 |
| 368 | 3'-CF₃ | H | H | -CH₂- | 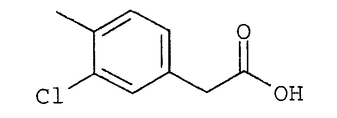 |
| 369 | 3'-CF₃ | H | H | -CH₂- | 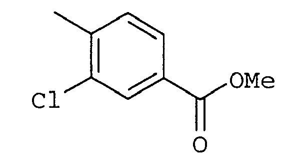 |
| 370 | 3'-CF₃ | H | H | -CH₂- | 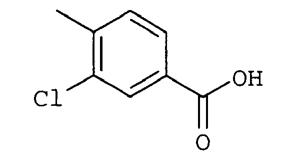 |
| 371 | 3'-CF₃ | H | H | -CH₂- | 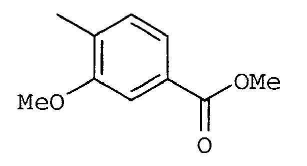 |
| 372 | 3'-CF₃ | H | H | -CH₂- | 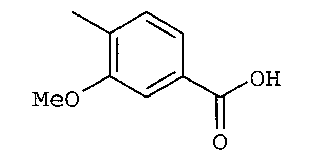 |
| 373 | 3'-CF₃ | H | H | -(CH₂)₂-O- | 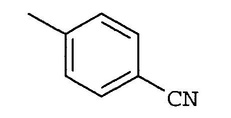 |
| 374 | 2'-F | 4'-F | H | -(CH₂)₂-O- | 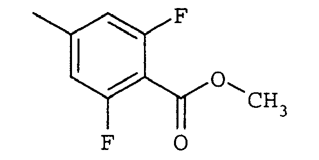 |
| 375 | 2'-OCH₃ | 4'-F | H | -(CH₂)₂-O- | 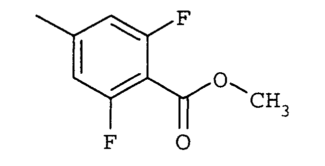 |
| 376 | 2'-Cl | 4'-F | H | -(CH₂)₂-O- | 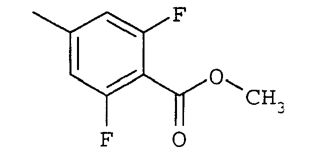 |
| 377 | 2'-Cl | 4'-F | H | -(CH₂)₂-O- | 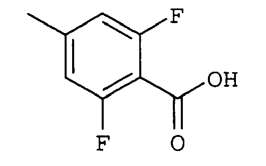 |
| 378 | 4'-CH₃ | H | 2-CF₃ | -(CH₂)₂-O- | 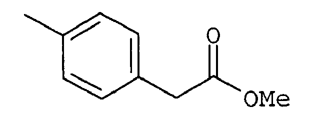 |
| 379 | 4'-CH₃ | H | 2-CF₃ | -(CH₂)₂-O- | 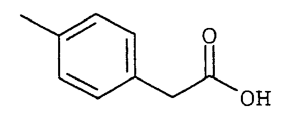 |
| 380 | 4'-Cl | H | 2-CF₃ | -(CH₂)₂-O- | 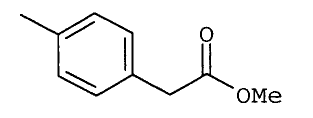 |
| 381 | 4'-Cl | H | 2-CF₃ | -(CH₂)₂-O- | 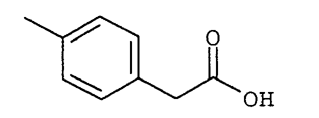 |
| 382 | 4'F | H | 2-CF₃ | -(CH₂)₂-O- | 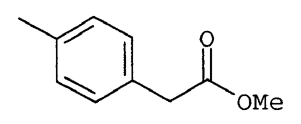 |
| 383 | 4'-F | H | 2-CF₃ | -(CH₂)₂-O- | 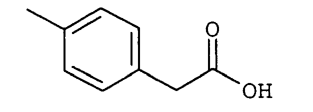 |
| 384 | 3'-CF₃ | H | 2-F | -(CH₂)₂-O- | 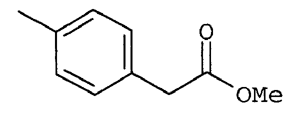 |
| 385 | 3'-CF₃ | H | 2-F | -(CH₂)₂-O- | 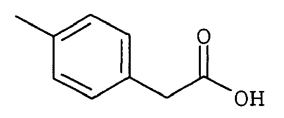 |
| 386 | 3'-CF₃ | H | 2-C1 | -(CH₂)₂-O- | 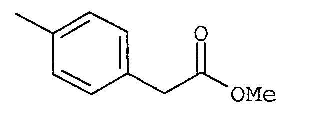 |
| 387 | 3'-CF₃ | H | 2-Cl | -(CH₂)₂-O- | 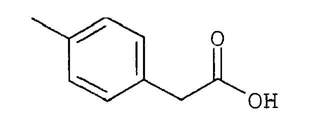 |
| 388 | 3'-CF₃ | H | 2-CH₃ | -(CH₂)₂-O- | 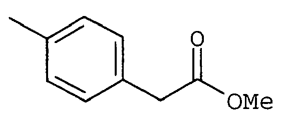 |
| 389 | 3'-CF₃ | H | 2-CH₃ | -(CH₂)₂-O- | 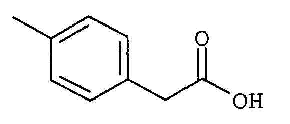 |
| 390 | 3'-CF₃ | H | H | -(CH₂)- | 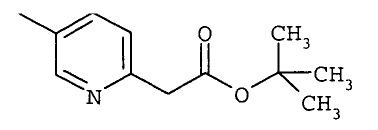 |
| 391 | 3'-CF₃ | H | H | -(-CH2)- | 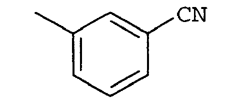 |
| 392 | 3'-CF₃ | H | H | -(CH₂)- | 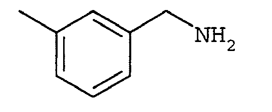 |
| 393 | 3'-CF₃ | H | H | -(CH₂)- | 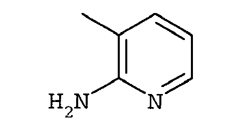 |
| 394 | 4'-F | 2'-CH₃ | H | -(CH₂)₂-O- | 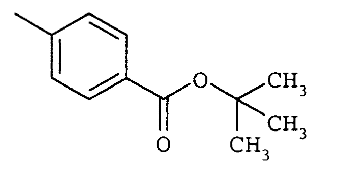 |
| 395 | 4'-F | 2'-CH₃ | H | -(CH₂)₂-O- | 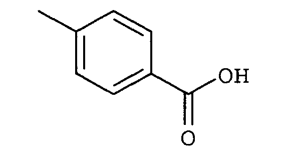 |
| 396 | 2'-F | 4'-F | H | -(CH₂)₂-O- | 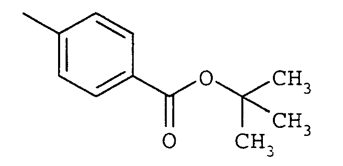 |
| 397 | 2'-F | 4'-F | H | -(CH₂)₂-O- | 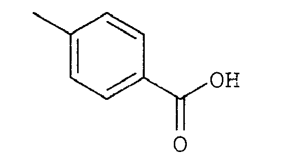 |
| 398 | 2'-Cl | 4'-F | H | -(CH₂)₂-O- | 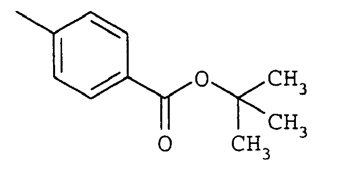 |
| 399 | 2'-Cl | 4'-F | H | -(CH₂)₂-O- | 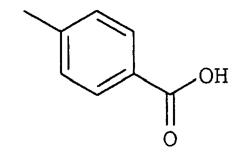 |
| 400 | 2'-OCH₃ | 4'-F | H | -(CH₂)₂-O- | 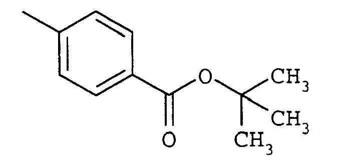 |
| 401 | 2'-OCH₃ | 4'-F | H | -(CH₂)₂-O- | 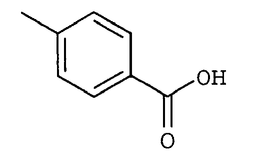 |
| 402 | 3'-CF₃ | H | H | -CH₂- | 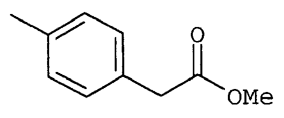 |
| 403 | 3'-CF₃ | H | H | -CH₂- | 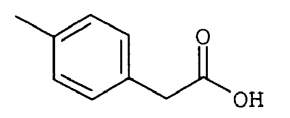 |
| 404 | 3'-CF₃ | H | H | -(CH₂)₂-O- | 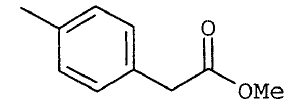 |
| 405 | 3'-CF₃ | H | H | -(CH₂)₂-O- | 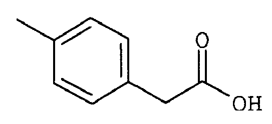 |
| 406* | 3'-CF₃ | H | H | -(CH₂)₂-O- | 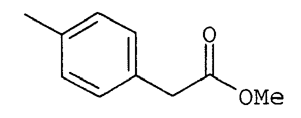 |
| 407* | 3'-CF₃ | H | H | -(CH₂)₂-O- | 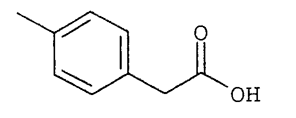 |
| 408 | 2'-Cl | 5'-CF₃ | H | -(CH₂)₂-O- | 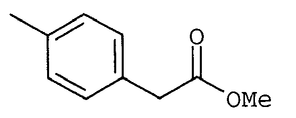 |
| 409 | 2'-Cl | 5'-CF₃ | H | -(CH₂)₂-O- | 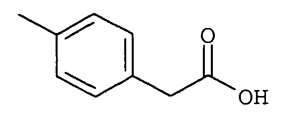 |
| 410 | 3'-CF₃ | H | H | -(CH₂)₂-O- | 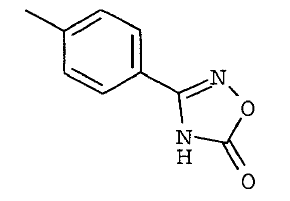 |
| 411 | 3'-CF₃ | H | H | -CH₂- | 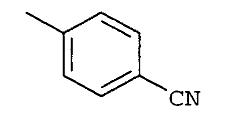 |
| 412 | 3'-CF₃ | H | H | -CH₂- | 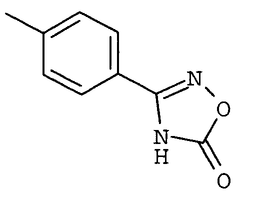 |
| 413 | 3'-CF₃ | H | H | -CH₂- | 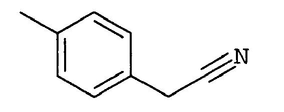 |
| 414 | 3'-CF₃ | H | H | -(CH₂)₂-O- | 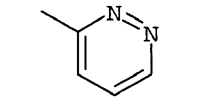 |
| 415 | 2'-CH₃ | 4'-F | H | -(CH₂)₂-O- | 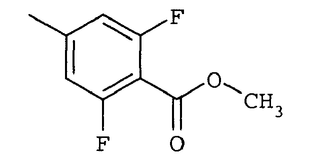 |
| 416 | 2'-CH₃ | 4'-F | H | -(CH₂)₂-O- | 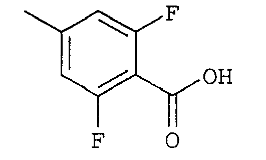 |
| 417 | 2'-Cl | 5'-CF₃ | H | -(CH₂)₂-O- | 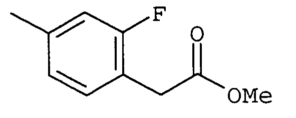 |
| 418 | 2'-Cl | 5'-CF₃ | H | -(CH₂)₂-O- | 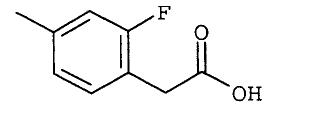 |
| 419 | 2'-OCH₃ | 4'-F | H | -(CH₂)₂-O- | 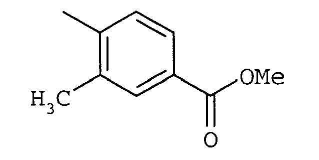 |
| 420 | 2'-OCH₃ | 4'-F | H | -(CH₂)₂-O- | 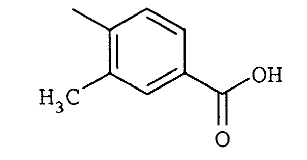 |
| 421 | 2'-OCH₃ | 4'-F | H | -(CH₂)₂-O- | 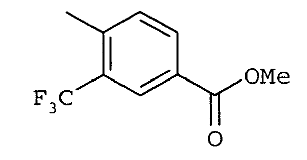 |
| 422 | 2'-OCH₃ | 4'-F | H | -(CH₂)₂-O- | 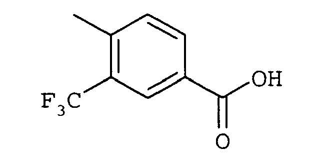 |
| 423 | 2'-OCH₃ | 4'-F | H | -(CH₂)₂-O- | 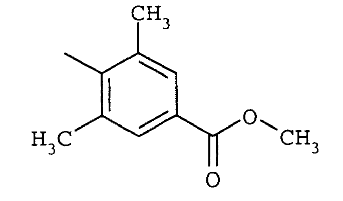 |
| 424 | 2'-OCH₃ | 4'-F | H | -(CH₂)₂-O- | 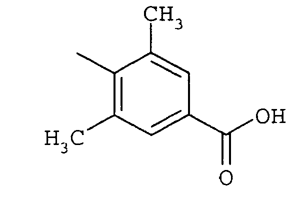 |
| 425 | 2'-CH₃ | 4'-F | H | -(CH₂)₂-O- | 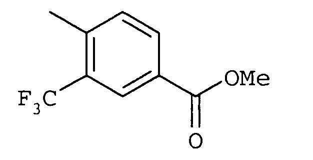 |
| 426 | 2'-CH₃ | 4'-F | H | -(CH₂)₂-O- | 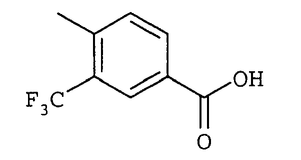 |
| 427 | 2'-F | 4'-F | H | -(CH₂)₂-O- | 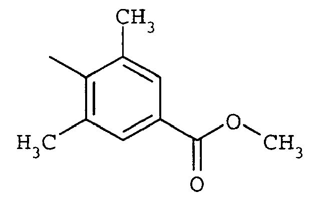 |
| 428 | 2'-F | 4'-F | H | -(CH₂)₂-O- | 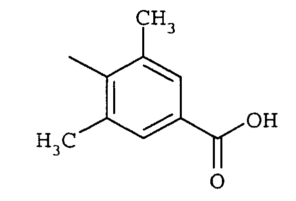 |
| 429 | 2'-OCH₃ | 4'-F | H | -(CH₂)₂-O- | 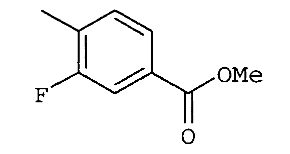 |
| 430 | 2'-OCH₃ | 4'-F | H | -(CH₂)₂-O- | 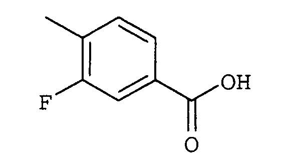 |
| 431 | 2'-Cl | 4'-F | H | -(C₂)₂-O- | 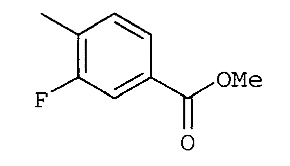 |
| 432 | 2'-Cl | 4'-F | H | -(CH₂)₂-O- | 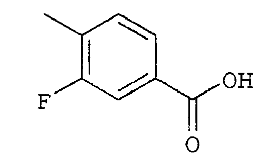 |
| 433 | 2'-F | 4'-F | H | -(CH₂)₂-O- | 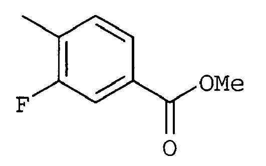 |
| 434 | 2'-F | 4'-F | H | -(CH₂)₂-O- | 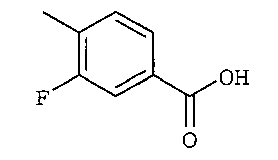 |
| 435 | 2'-CH₃ | 4'-F | H | -(CH₂)₂-O- | 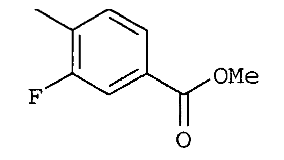 |
| 436 | 2'-CH₃ | 4'-F | H | -(CH₂)₂-O- | 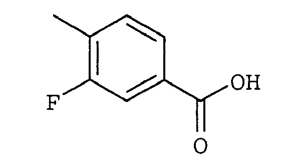 |
| 437 | 2'-F | 4'-F | H | -(CH₂)₂-O- | 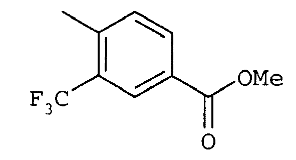 |
| 438 | 2'-F | 4'-F | H | -(CH₂)₂-O- | 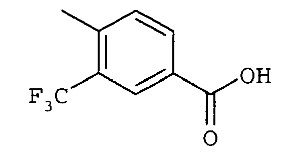 |
| 439 | 2'-Cl | 4'-F | H | -(CH₂)₂-O- | 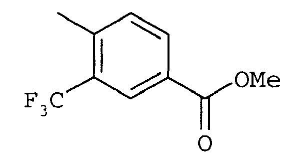 |
| 440 | 2'-Cl | 4'-F | H | -(CH₂)₂-O- | 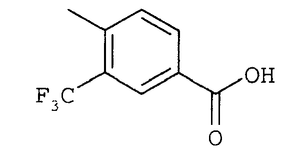 |
| 441 | 2'-Cl | 4'-F | H | -(CH₂)₂-O- | 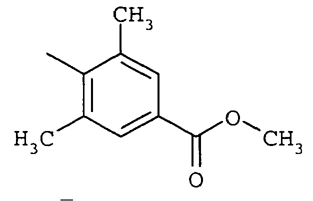 |
| 442 | 2'-Cl | 4'-F | H | -(CH₂)₂-O- | 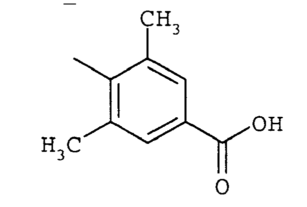 |
| 443 | 2'-Cl | 4'-F | H | -(CH₂)₂-O- | 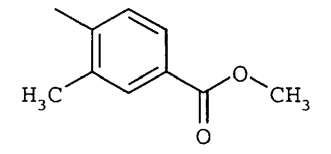 |
| 444 | 2'-Cl | 4'-F | H | -(CH₂)₂-O- | 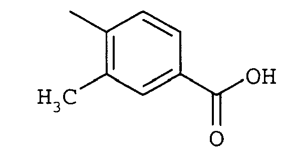 |
| 445 | 2'-CH₃ | 4'-F | H | -(CH₂)₂-O- | 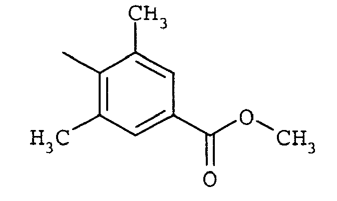 |
| 446 | 2'-CH₃ | 4'-F | H | -(CH₂)₂-O- | 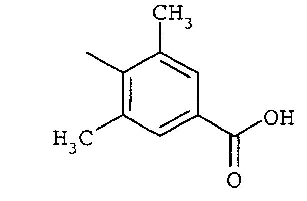 |
| 447 | 2'-CH₃ | 4'-F | H | -(CH₂)₂-O- | 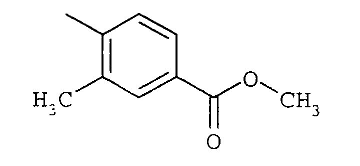 |
| 448 | 2'-CH₃ | 4'-F | H | -(CH₂)₂-O- | 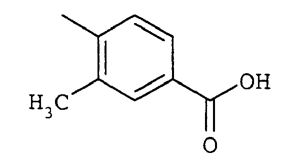 |
| 449 | 2'-Cl | 4'-F | H | -(CH₂)₃- | 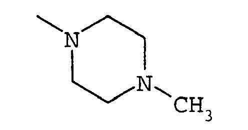 |
| 450 | 2'-Cl | 4'-F | H | -(CH₂)₃- | 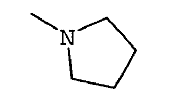 |
| 451 | 2'-Cl | 4'-F | H | -(CH₂)₄- | 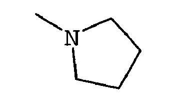 |
| 452 | 2'-Cl | 4'-F | H | -(CH₂)₂- | 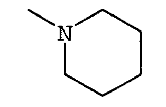 |
| 453 | 2'-Cl | 4'-F | H | -(CH₂)₄- | 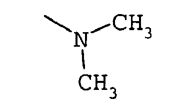 |
| 454 | 2'-OCH₃ | 4'-F | H | -(CH₂)₂-O- | 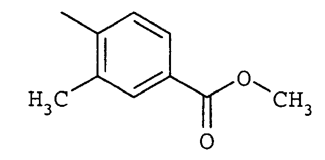 |
| 455 | 2'-F | 4'-F | H | -(CH₂)₂-O- | 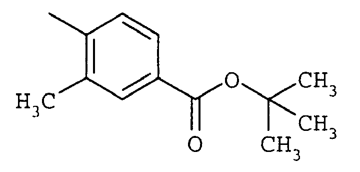 |
| 456 | 2'-F | 4'-F | H | -(CH₂)₂-O- | 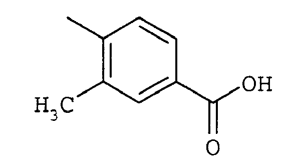 |
| 457 | 2'-Cl | 4'-F | H | -CH₂- | 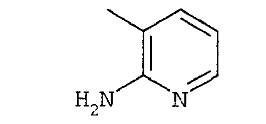 |
| 458 | 2'-Cl | 4'-F | H | -CH₂- | 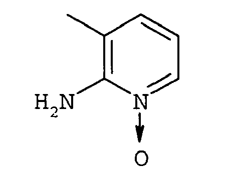 |
| 459 | 2'-Cl | 4'-F | H | -(CH₂)₂-O- | 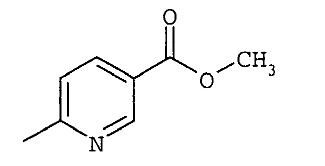 |
| 460 | 2'-Cl | 4'-F | H | -(CH₂)₂-O- | 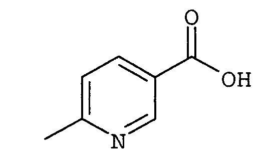 |
| 461 | 2'-Cl | 4'-F | H | -CH₂- | 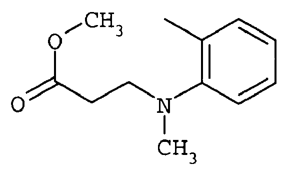 |
| 462 | 2'-Cl | 4'-F | H | -CH₂- | 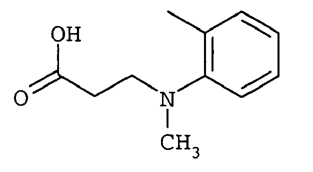 |
| 463 | 2'-Cl | 4'-F | H | -CH₂- | 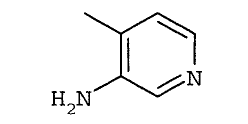 |
| 464 | 2'-Cl | 4'-F | H | -CH₂- | 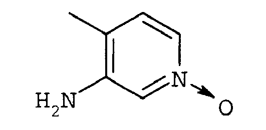 |
| 465 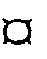 | 2'-F | 4'-F | H | -CH₂- | 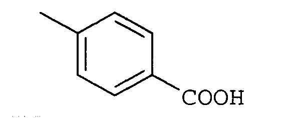 |
| 466 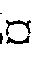 | 2'-F | 4'-F | H | -(CH₂)₂-O- | 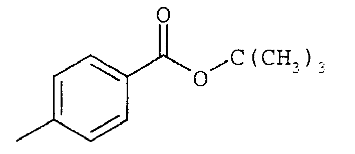 |
| 467 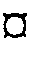 | 2'-F | 4'-F | H | -(CH₂)₂-O- | 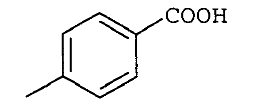 |
| 468* | 4'-F | H | H | -(CH₂)₂-O- | 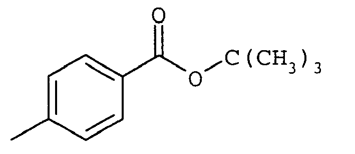 |
| 469* | 4'-F | H | H | -(CH₂)₂-O- | 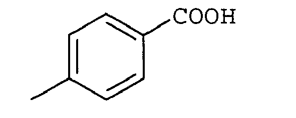 |
| 470* | 2'-F | 4'-F | H | -(CH₂)₂-O- | 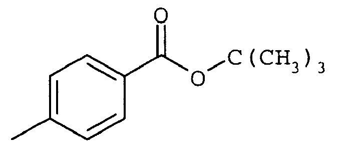 |
| 471* | 2'-F | 4'-F | H | -(CH₂)₂-O- | 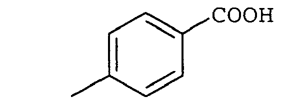 |
| 472* | 2'-F | 4'-F | H | -CH₂- | 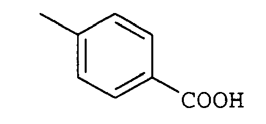 |
| 473* | 2'-F | 4'-F | H | -CH₂- | 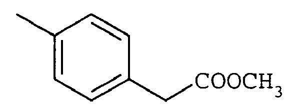 |
| 474* | 2'-F | 4'-F | H | -CH₂- | 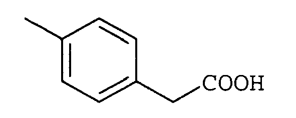 |
| 475# | 2'-F | 4'-F | H | -(CH₂)₂-O- | 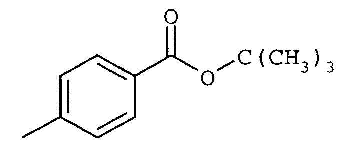 |
| 476# | 2'-F | 4'-F | H | -(CH₂)₂-O- | 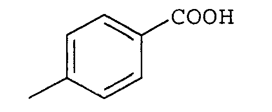 |
| 477# | 2'-F | 4'-F | H | -CH₂- | 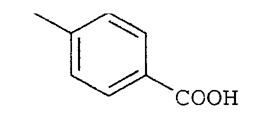 |
| 478* | 2'-Cl | 4'-F | H | -CH₂- | 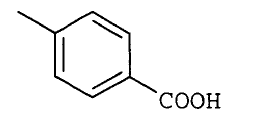 |
| 479* | 2'-Cl | 4'-F | H | -(CH₂)₂-O- | 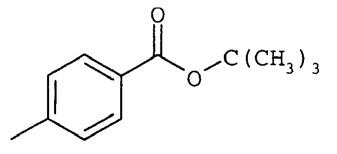 |
| 480* | 2'-Cl | 4'-F | H | -(CH₂)₂-O- | 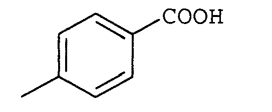 |
| 481 | 2'-O-CHF₂ | 4'-F | H | -(CH₂)₂-O- | 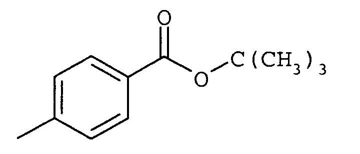 |
| 482 | 2'-O-CHF₂ | 4'-F | H | -(CH₂)₂-O- | 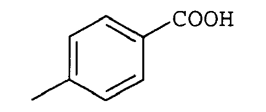 |
| 483 | 2'-O-CHF₂ | 4'-F | H | -CH₂- | 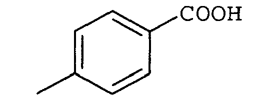 |
| 484* | 2'-O-CHF₂ | 4'-F | H | -(CH₂)₂-O- | 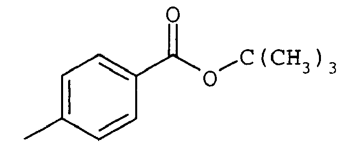 |
| 485* | 2'-O-CHF₂ | 4'-F | H | -(CH₂)₂-O- | 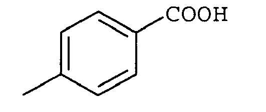 |
| 486* | 2'-O-CHF₂ | 4'-F | H | -CH₂- | 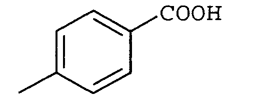 |

| | | | | | |
|---|---|---|---|---|---|
| IPr = 1-méthyléthyl (ou : isopropyl) * Les composés 170, 171, 406, 407, 468, 469, 470, 471, 472, 473, 474, 478, 479, 480, 484, 485 et 486 sont des dérivés de l'acide 4-méthoxy-indoline-2-carboxylique racémique Les composés 465, 466, 467, sont des dérivés de l'acide 5-méthoxy-indoline-2-carboxylique racémique # Les composés 475, 476, 477, sont des dérivés de l'acide 7-méthoxy-indoline-2-carboxylique racémique | | | | | |

### Activité biologique

### Test de transactivation des chimères GAL4-LXR après transfection transitoire en cellules COS7.

Les tests de transactivation reposent sur la capacité des récepteurs nucléaires :
(1) à se lier à une séquence ADN spécifique (RE = Response Element) située devant un promoteur, via leur domaine de liaison à l'ADN (ou DBD = DNA Binding Domain).
(2) et à augmenter la transcription d'un gène sous contrôle de ce promoteur en présence d'un ligand agoniste, via leur domaine de liaison au ligand (ou LBD = Ligand Binding Domain).

Le test de transactivation en cellules COS7 développé ici vise à évaluer l'effet de composés sur l'activité des LXRs humains : il permet de valider l'interaction des composés avec les LXRs et de déterminer l'EC50 de l'interaction. Ce test est basé sur l'utilisation de protéines chimères « Gal4-LXR » contenant le LBD du LXR (LXRa humain ou LXRβ humain) fusionné avec le DBD de Gal4. Les cellules COS7 sont ainsi co-transfectées de façon transitoire avec :
- un vecteur d'expression codant pour la protéine chimère « Gal4(DBD)-LXRα(LBD) ou un vecteur d'expression codant pour la chimère « Gal4(DBD)-LXRβ(LBD) »
- un vecteur rapporteur comportant l'élément de réponse Gal4-RE (Gal4-Response Element) reconnaissant le DBD du Gal4, et situé devant le promoteur minimal P_{TK} qui contrôle le gène de la luciférase.

L'activité de la luciférase ainsi produite génère de la luminescence en présence d'un excès de substrat, donnée quantifiable qui reflète l'interaction du composé avec le LBD du LXR.

Les composés selon l'invention sont évalués par rapport à un composé de référence (T-0901317, CAS RN : 293754-55-9).

Selon ce test les composés selon l'invention présentent une EC 50 inférieure à 1 µM, avec pour certains composés une valeur de 3 à 4 nM.

Les propriétés biologiques des composés selon l'invention démontrent leur intérêt potentiel et leur utilité pour leur application en tant que substances actives de médicaments destinés au traitement ou à la prévention des maladies dépendantes d'une dérégulation des fonctions des récepteurs LXR α et LXR β, notamment les hypercholestérolémies, les dyslipidémies, ainsi que l'obésité, le diabète, les maladies cardiovasculaires, certaines neurodégénérescences et les maladies inflammatoires.

L'invention concerne également les compositions pharmaceutiques destinées à la prévention ou au traitement des maladies précédemment citées lorsqu'elles contiennent en tant que principe actif au moins l'un des composés de formule I selon l'invention.

Ces compositions pharmaceutiques peuvent être formulées de façon classique et comprennent au moins un excipient pharmaceutiquement acceptable choisi selon la forme pharmaceutique et le mode d'administration souhaités.

Les composés selon l'invention sont susceptibles d'être administrés par voie orale, parentérale, ou toute autre voie désirable, sous forme de doses unitaires contenant de 5 à 250 mg de principe actif, ces doses pouvant être administrées une à trois fois par jour.

Les composés selon l'invention peuvent être notamment administrés par voie orale sous forme de comprimés ou de gélules comprenant des excipients classiques tels que stabilisants, surfactants, liants, lubrifiants, désintégrants, comme par exemple: lactose, amidon, mannitol, dérivés cellulosiques, dérivés de pyrrolidone, lauryl sulfate de sodium, stéarate de magnésium.

## Revendications

1. Composé de sulfonylindoline, **caractérisé en ce qu'**il est choisi parmi :
i) les composés de formule : 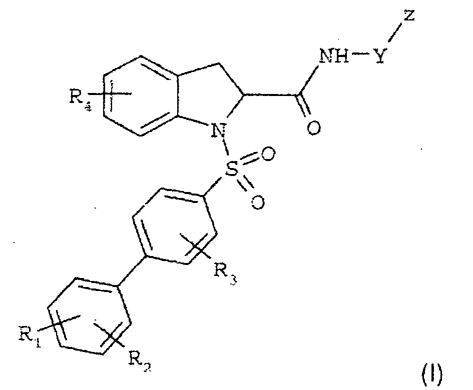 dans laquelle :
- R₁ représente un atome d'hydrogène, un halogène, un groupe alkyle en C₁-C₄, un groupe alcoxy en C₁-C₄, un groupe trifluorométhyle ou un groupe méthoxy totalement ou partiellement halogéné,
- R₂ représente un atome d'hydrogène, un halogène, un groupe alkyle en C₁-C₄ ou un groupe trifluorométhyle,
- R₃ représente un atome d'hydrogène, un halogène, un groupe alkyle en C₁-C₄ ou un groupe trifluorométhyle, avec la condition que R₁, R₂ et R₃ ne représentent pas simultanément un atome d'hydrogène,
- R₄ représente un atome d'hydrogène ou un groupe alcoxy en C₁-C₄,
- Y représente un groupe alkylène en C₁-C₈ linéaire, ramifié, éventuellement substitué par un groupe trifluorométhyle ou par un noyau phényle, ou comportant un groupe cyclopropyle ou cyclohexyle, ou représente un groupe -(CH₂)ₙ-W-,
- W représente un atome d'oxygène, un groupe -NH- ou un atome de soufre,
- n représente 2, 3 ou 4,
- Z représente un groupe alkyle en C₁-C₄, éventuellement partiellement halogéné, trifluorométhyle, -CORₐ, -CH₂-N(R)₂, un noyau aromatique, hétéroaromatique ou hétérocyclique choisi parmi les groupes phényle, pyrrolidinyle, pyrrolidinyi-one, imidazolyle, pyridinyle, pyridinyle-oxyde, pipéridinyle, pipérazinyle, pyridazinyle, morpholinyle, indolinylone, éventuellement substitué par un, deux ou trois substituants, identiques ou différents, choisis parmi un halogène, un groupe alkyle en C₁-C₄, alcoxy en C₁-C₄, trifluorométhyle, nitro, N(R)₂, -CH₂-N(R)₂, -O-(CH₂)ₙ-N(R)₂, hydroxy, cyano, cyanoalkyle en C₂-C₃, 5-oxo-1,2,4-oxadiazolidinyle ou un groupe de formule -X-[C(R)₂]ₚ-CORₐ,
- X représente une liaison simple, un atome d'oxygène, -O-CH₂-, un atome de soufre, un groupe -NR-, ou un groupe 1,1-cyclopropylène,
- Rₐ représente OR ou N(R)₂,
- R représente l'atome d'hydrogène ou un groupe alkyle en C₁-C₄,
- p est égal à 0, 1, 2, 3 ou 4;
ii) les sels pharmaceutiquement acceptables desdits composés de formule (I).

2. Composé de formule I selon la revendication 1, **caractérisé en ce que** le carbone asymétrique du groupe indoline est de configuration S.

3. Composé de formule I selon la revendication 1 ou 2, **caractérisé en ce que** R₁ représente un atome de fluor ou un groupe trifluorométhyle.

4. Composé de formule I selon la revendication 1, 2 ou 3, **caractérisé en ce que** Y représente un groupe -CH₂- ou un groupe -(CH₂)₂-O- et Z représente un noyau aromatique substitué par un groupe contenant une fonction acide carboxylique, ledit noyau aromatique comportant éventuellement un ou deux autres substituants choisis parmi un halogène, un groupe alkyle en C₁-C₄, de préférence méthyle, un groupe alcoxy en C₁-C₄, de préférence méthoxy, un groupe trifluorométhyle.

5. Composé selon l'une des revendications 1 à 4, pour son utilisation en tant que substance pharmacologiquement active.

6. Utilisation d'un composé selon l'une des revendications 1 à 4, pour la fabrication d'un médicament destiné à traiter les neurodégénérescences, les maladies cardiovasculaires, inflammatoires, les hypercholestérolémies, les dyslipidémies, l'obésité et le diabète.

7. Composition pharmaceutique **caractérisée en ce qu'**elle contient au moins un composé selon l'une des revendications 1 à 4 en tant que substance active.

8. Composition selon la revendication 7, destinée au traitement des maladies cardiovasculaires et des hypercholestérolémies, les dyslipidémies et l'obésité.

9. Composition selon la revendication 7, destinée au traitement du diabète.

10. Composition selon la revendication 7, destinée au traitement des neurodégénérescences.

11. Composition selon la revendication 7, destinée au traitement des maladies inflammatoires.

12. Procédé de préparation d'un composé selon l'une des revendications 1 à 4, **caractérisé en ce qu'**il comprend les étapes consistant à:
faire réagir un chlorure de benzènesulfonyle de formule 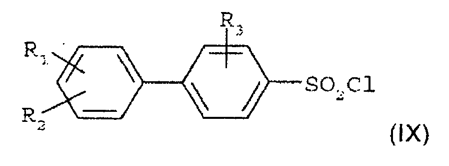
avec un dérivé d'indoline de formule 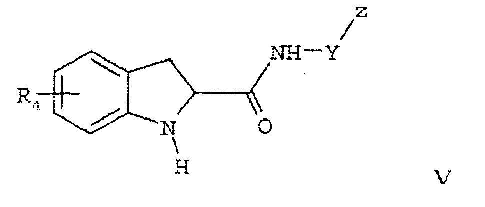
dans un solvant anhydre tel que par exemple le dichlorométhane, à température ambiante et pendant 2 à 10 heures, pour obtenir le composé de formule 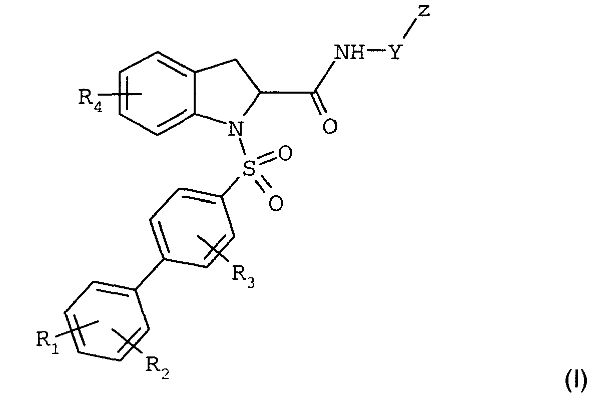
dans laquelle R₁, R₂, R₃, R₄, Y, W, n, X, Ra, R, p conservent la même signification que dans les composés de départ et Z représente un groupe alkyle en C₁-C_{4,} éventuellement partiellement halogéné, trifluorométhyle, CORₐ, CH₂-N(R)₂, un noyau aromatique, hétéroaromatique ou hétérocyclique choisi parmi les groupes phényle, 1- pyrrolidinyle, pyrrolidinyl-one, 1-imidazolyle, pyridinyle, 1-pipéridinyle, 4- alkyl-1-pipérazinyle, pyridazinyle, 4-morpholinyle, indolinylone, éventuellement substitué par un, deux ou trois substituants identiques ou différents choisis parmi un halogène, un groupe alkyle en C₁-C₄, alcoxy en C₁-C₄, trifluorométhyle, nitro, N(R)₂, -CH₂-N(R)₂, -O-(CH₂)ₙ-N(R)₂, hydroxy, cyano, cyanoalkyl en C₂-C₃ ou un groupe de formule -X-[C(R)₂]ₚ- CORₐ.

## Claims

1. Sulfonylindoline compound, **characterized in that** it is selected from:
i) the compounds of the formula 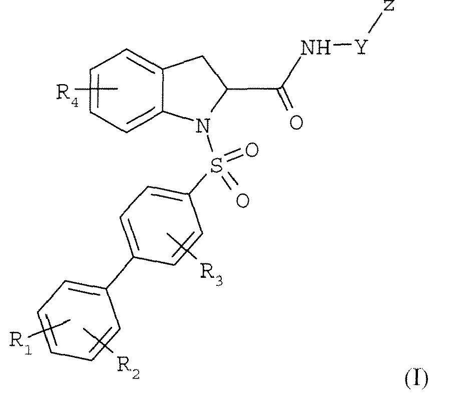 in which:
- R₁ is a hydrogen atom, a halogen, a C₁-C₄ alkyl group, a C₁-C₄ alkoxy group, a trifluoromethyl group or a totally or partially halogenated methoxy group,
- R₂ is a hydrogen atom, a halogen, a C₁-C₄ alkyl group or a trifluoromethyl group,
- R₃ is a hydrogen atom, a halogen, a C₁-C₄ alkyl group or a trifluoromethyl group, with the proviso that R₁, R₂ and R₃ are not simultaneously a hydrogen atom,
- R₄ is a hydrogen atom or a C₁-C₄ alkoxy group,
- Y is a linear or branched C₁-C₈ alkylene group optionally substituted by a trifluoromethyl group or by a phenyl ring, or containing a cyclopropyl or cyclohexyl group, or is a group -(CH₂)ₙ-W-,
- W is an oxygen atom, a group -NH- or a sulfur atom,
- n is 2, 3 or 4,
- Z is an optionally partially halogenated C₁-C₄ alkyl group, trifluoromethyl, -CORₐ, -CH₂-N(R)₂, or an aromatic, heteroaromatic or heterocyclic ring selected from phenyl, pyrrolidinyl, pyrrolidinylone, imidazolyl, pyridinyl, pyridinyl oxide, piperidinyl, piperazinyl, pyridazinyl, morpholinyl and indolinylone groups, and optionally substituted by one, two or three identical or different substituents selected from a halogen, a C₁-C₄ alkyl group, C₁-C₄ alkoxy, trifluoromethyl, nitro, N(R)₂, -CH₂-N(R)₂, -O-(CH₂)ₙ-N(R)₂, hydroxyl, cyano, C₂-C₃ cyanoalkyl, 5-oxo-1,2,4-oxadiazolidinyl and a group of the formula
- X-[C(R₂)]ₚ-CORₐ,
- X is a single bond, an oxygen atom, -O-CH₂-, a sulfur atom, a group -NR- or a 1,1-cyclopropylene group,
- Rₐ is OR or N(R)₂,
- R is a hydrogen atom or a C₁-C₄ alkyl group, and
- p is equal to 0, 1, 2, 3 or 4; and
ii) the pharmaceutically acceptable salts of said compounds of formula (I).

2. Compound of formula I according to claim 1, **characterized in that** the asymmetric carbon of the indoline group is of S configuration.

3. Compound of formula I according to claim 1 or 2, **characterized in that** R₁ is a fluorine atom or a trifluoromethyl group.

4. Compound of formula I according to claim 1, 2 or 3, **characterized in that** Y is a group -CH₂- or a group -(CH₂)₂-O- and Z is an aromatic ring substituted by a group containing a carboxylic acid group, said aromatic ring optionally containing one or two other substituents selected from a halogen, a C₁-C₄ alkyl group, preferably methyl, a C₁-C₄ alkoxy group, preferably methoxy, and a trifluoromethyl group.

5. Compound according to one of claims 1 to 4 for its use as a pharmacologically active substance.

6. Use of a compound according to one of claims 1 to 4 for the manufacture of a drug for the treatment of neurodegeneration, cardiovascular disease, inflammatory disease, hypercholesterolemia, dyslipidemia, obesity and diabetes.

7. Pharmaceutical composition, **characterized in that** it contains at least one compound according to one of claims 1 to 4 as the active substance.

8. Composition according to claim 7 for the treatment of cardiovascular disease, hypercholesterolemia, dyslipidemia and obesity.

9. Composition according to claim 7 for the treatment of diabetes.

10. Composition according to claim 7 for the treatment of neurodegeneration.

11. Composition according to claim 7 for the treatment of inflammatory disease.

12. Process for the preparation of a compound according to one of claims 1 to 4, **characterized in that** it involves the steps consisting in:
reacting a benzenesulfonyl chloride of the formula 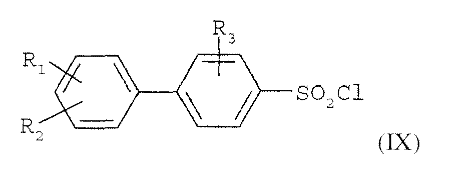
with an indoline derivative of the formula 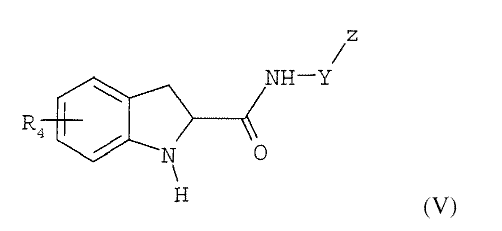
in an anhydrous solvent such as dichloromethane, at room temperature, for 2 to 10 hours, to give the compound of the formula 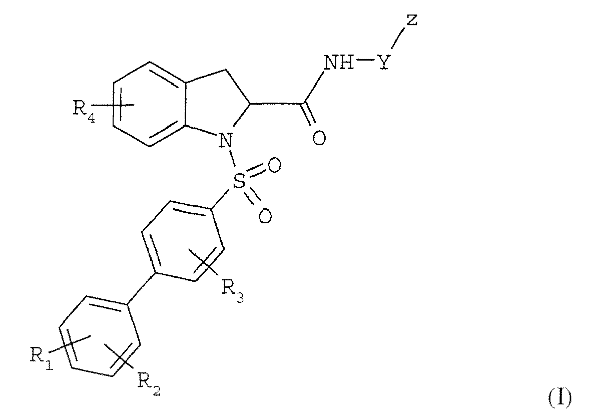
in which R₁, R₂, R₃, R₄, Y, W, n, X, Rₐ, R and p are as defined in the starting compounds and Z is an optionally partially halogenated C₁-C₄ alkyl group, trifluoromethyl, CORₐ, CH₂-N(R)₂, or an aromatic, heteroaromatic or heterocyclic ring selected from phenyl, 1-pyrrolidinyl, pyrrolidinylone, 1-imidazolyl, pyridinyl, 1-piperidinyl, 4-alkyl-1-piperazinyl, pyridazinyl, 4-morpholinyl and indolinylone groups, and optionally substituted by one, two or three identical or different substituents selected from a halogen, a C₁-C₄ alkyl group, C₁-C₄ alkoxy, trifluoromethyl, nitro, N(R)₂, -CH₂-N(R)₂, -O-(CH₂)ₙ-N(R)₂, hydroxyl, cyano, C₂-C₃ cyanoalkyl and a group of the formula -X-[C(R)₂]ₚ-CORₐ.

## Patentansprüche

1. Sulfonylindolinverbindung, **dadurch gekennzeichnet, daß** sie ausgewählt ist aus:
i) den Verbindungen folgender Formel: 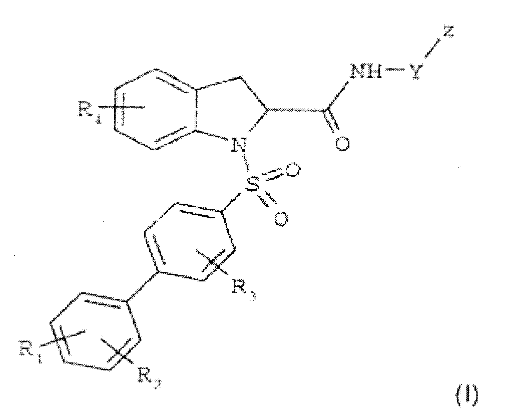 worin:
- R₁ ein Wasserstoffatom, ein Halogen, eine C₁-C₄-Alkylgruppe, eine C₁-C₄-Alkoxygruppe, eine Trifluormethylgruppe oder eine vollständig oder partiell halogenierte Methoxygruppe darstellt,
- R₂ ein Wasserstoffatom, ein Halogen, eine C₁-C₄-Alkylgruppe oder eine Trifluormethylgruppe darstellt,
- R₃ ein Wasserstoffatom, ein Halogen, eine C₁-C₄-Alkylgruppe oder eine Trifluormethylgruppe darstellt, mit der Bedingung, daß R₁, R₂ und R₃ nicht gleichzeitig ein Wasserstoffatom darstellen,
- R₄ ein Wasserstoffatom oder eine C₁-C₄-Alkoxygruppe darstellt,
- Y eine lineare, verzweigte C₁-C₈-Alkylengruppe darstellt, die eventuell durch eine Trifluormethylgruppe oder durch einen Phenylkern substituiert ist oder eine Cyclopropyl- oder Cyclohexylgruppe umfaßt, oder eine -(CH₂)ₙ-W-Gruppe darstellt,
- W ein Sauerstoffatom, eine -NH-Gruppe oder ein Schwefelatom darstellt,
- n 2, 3 oder 4 darstellt,
- Z folgendes darstellt, nämlich eine eventuell partiell halogenierte C₁-C₄-Alkylgruppe, Trifluormethyl, -CORₐ, -CH₂-N(R)₂, einen aromatischen, heteroaromatischen oder heterozyklischen Kern ausgewählt aus den Phenyl-, Pyrrolidinyl-, Pyrrolidinyl-on-, Imidazolyl-, Pyridinyl-, Pyridinyl-Oxid-, Piperidinyl-, Piperazinyl-, Pyridazinyl-, Morpholinyl-, Indolinylon-Gruppen, eventuell substituiert durch einen, zwei oder drei identische oder unterschiedliche Substituenten, die ausgewählt sind aus einem Halogen, einer C₁-C₄-Alkylgruppe, C₁-C₄-Alkoxy, Trifluormethyl, Nitro, N(R)₂, -CH₂-N(R)₂, -4-(CH₂)ₙ-N(R)₂, Hydroxy, Cyano, C₂-C₃-Cyanoalkyl, 5-oxo-1,2,4-Oxadiazolidinyl oder einer Gruppe der Formel -X-[C(R)₂]ₚ-CORₐ,
- X eine einfache Verbindung, ein Sauerstoffatom -O-CH₂-, ein Schwefelatom, eine -NR-Gruppe oder eine 1,1-Cyclopropylen-Gruppe darstellt,
- Rₐ OR oder N(R)₂ darstellt,
- R das Wasserstoffatom oder eine C₁-C₄-Alkylgruppe darstellt,
- p gleich 0, 1, 2, 3 oder 4 ist;
ii) den pharmazeutisch akzeptablen Salzen der Verbindungen der Formel (I).

2. Verbindung der Formel 1 nach Anspruch 1, **dadurch gekennzeichnet, daß** der asymmetrische Kohlenstoff der Indolingruppe eine S-Konfiguration aufweist.

3. Verbindung der Formel I nach Anspruch 1 oder 2, **dadurch gekennzeichnet, daß** R₁ ein Fluoratom oder eine Trifluormethylgruppe darstellt.

4. Verbindung der Formel I nach Anspruch 1, 2 oder 3, **dadurch gekennzeichnet, daß** Y eine -CH₂-Gruppe oder eine -(CH₂)₂-O-Gruppe und Z einen aromatischen Kern substituiert durch eine Gruppe enthaltend eine Carboxylsäurefunktion darstellt, wobei der aromatische Kern eventuell einen oder zwei weitere Substituenten ausgewählt aus einem Halogen, einer C₁-C₄-Alkylgruppe, vorzugsweise Methyl, einer C₁-C₄-Alkoxygruppe, vorzugsweise Methoxy, einer Trifluormethylgruppe enthält.

5. Verbindung nach einem der Ansprüche 1 bis 4 für ihre Verwendung als pharmakologisch wirksame Substanz.

6. Verwendung einer Verbindung nach einem der Ansprüche 1 bis 4 für die Herstellung eines Medikaments zur Behandlung von neurodegenerativen Erkrankungen, kardiovaskulären Erkrankungen, entzündlichen Krankheiten, Hypercholesterinämien, Dyslipidämien, Fettleibigkeit und Diabetes.

7. Pharmazeutische Zusammensetzung, **dadurch gekennzeichnet, daß** sie wenigstens eine Verbindung nach einem der Ansprüche 1 bis 4 als Wirkstoff enthält.

8. Zusammensetzung nach Anspruch 7 zur Behandlung von kardiovaskulären Erkrankungen und Hypercholesterinämien, Dyslipidämien und Fettleibigkeit.

9. Zusammensetzung nach Anspruch 7 zur Behandlung von Diabetes.

10. Zusammensetzung nach Anspruch 7 zur Behandlung von neurodegenerativen Erkrankungen.

11. Zusammensetzung nach Anspruch 7 zur Behandlung von entzündlichen Krankheiten.

12. Verfahren zur Herstellung einer Verbindung nach einem der Ansprüche 1 bis 4, **dadurch gekennzeichnet, daß** es die Schritte umfaßt, die darin bestehen:
ein Benzolsulfonylchlorid der Formel 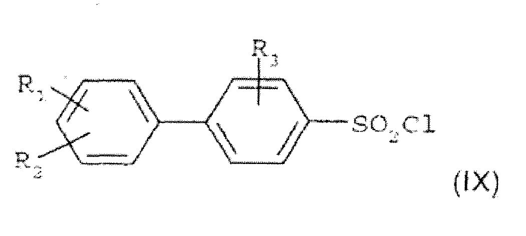
mit einem Indolinderivat der Formel 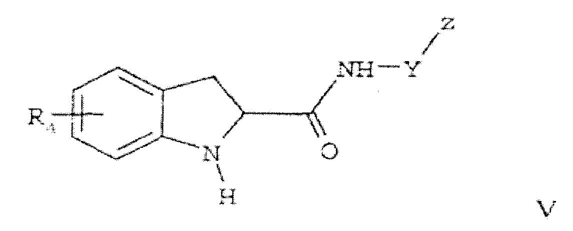
in einem wasserfreien Lösungsmittel, wie zum Beispiel Dichlormethan, bei Umgebungstemperatur für 2 bis 10 Stunden reagieren zu lassen, um die Verbindung folgender Formel zu erhalten 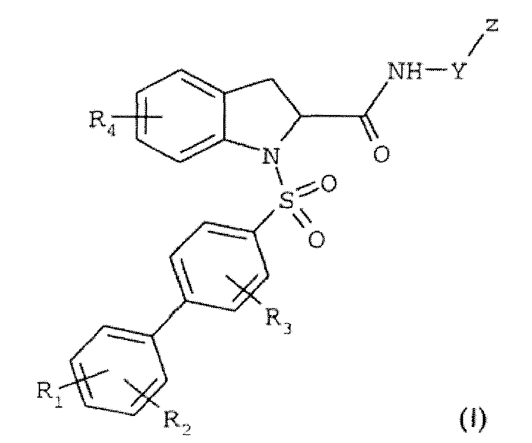
worin R₁, R₂, R₃, R₄, Y, W, n, X, Ra, R, p die gleiche Bedeutung wie bei den Ausgangsverbindungen behalten und Z folgendes darstellt, nämlich eine eventuell partiell halogenierte C₁-C₄-Alkylgruppe, Trifluormethyl, CORₐ, CH₂-N(R)₂, einen aromatischen, heteroaromatischen oder heterozyklischen Kern ausgewählt aus den Phenyl-, 1-Pyrrolidinyl-, Pyrrolidinyl-on-, 1-Imidazolyl-, Pyridinyl-, 1-Piperidinyl-, 4-Alkyl-1-Piperazinyl-, Pyridazinyl-, 4-Morpholinyl-, Indolinylon-Gruppen, eventuell substituiert durch einen, zwei oder drei identische oder unterschiedliche Substituenten, die ausgewählt sind aus einem Halogen, einer C₁-C₄-Alkylgruppe, C₁-C₄-Alkoxy, Trifluormethyl, Nitro, N(R)₂, -CH₂-N(R)₂, -O-(CH₂)ₙ-N(R)₂, Hydroxy, Cyano, C₂-C₃-Cyanoalkyl oder einer Gruppe der Formel -X-[C(R)₂]ₚ-CORₐ.
